# EUROPEAN PATENT APPLICATION

(11) **EP 4 818 187 A1**
(43) Date of publication of application: **07.10.2026**
(21) Application number: 24896775.4
(22) Date of filing: 29.11.2024
(51) Int. Cl.: C07K 5/027, C07K 5/02, A61K 31/4192, A61P 35/00

(54) **COMPOUND AND USE THEREOF**

(30) Priority: 30.11.2023 CN 202311630342
(71) Applicant: Ganzhou Hemay Pharmaceutical, Co., Ltd, Jiangxi Province (CN)
(72) Inventor: ZHANG, Hesheng, Ganzhou, Jiangxi 341600 (CN); CUI, Jianxin, Ganzhou, Jiangxi 341600 (CN); CHEN, Yingwei, Ganzhou, Jiangxi 341600 (CN); ZENG, Guanghuai, Ganzhou, Jiangxi 341600 (CN); LV, Jingjing, Ganzhou, Jiangxi 341600 (CN)
(74) Representative: ABG Intellectual Property Law, S.L.
(86) International application number: PCT/CN2024/135962
(87) International publication number: WO 2025/113696

(57) **Abstract**

Provided is a compound represented by general formula (A), or a stereoisomer, a cis-trans isomer, or a pharmaceutically acceptable salt thereof.

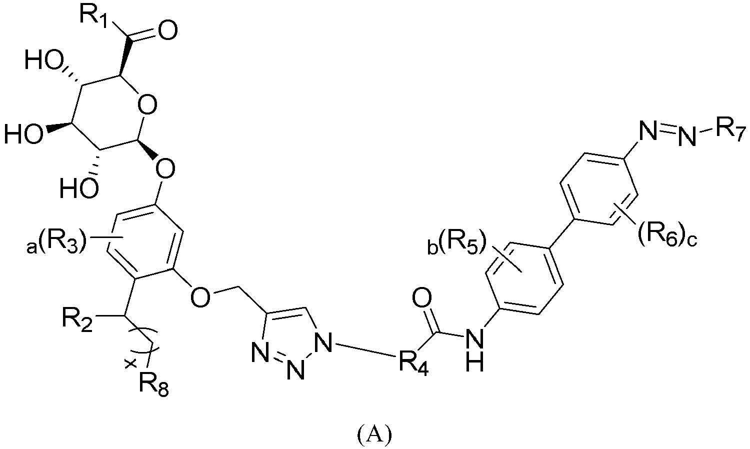

## Description

### CROSS-REFERENCE TO RELATED APPLICATION

The present disclosure claims the priority and rights to Chinese Patent Application No. 202311630342.7, the entire content of which is incorporated herein by reference.

### TECHNICAL FIELD

The present disclosure relates to the fields of organic chemistry and pharmaceutical chemistry.

### BACKGROUND

Cancers are major killers seriously threatening human health. At present, chemotherapy remains one of the main therapies for cancers, but conventional chemotherapeutic drugs lack tumor cell specificity, not only kill tumor cells, but also cause high toxicity to normal cells, thus easily resulting in severe systemic toxic side effects. In addition, many chemotherapeutic drugs have problems such as poor water solubility and low bioavailability, thereby restricting clinical applications thereof.

### SUMMARY

In the first aspect, the present disclosure provides a compound represented by general formula (A), or a stereoisomer, a cis-trans isomer, or a pharmaceutically acceptable salt thereof: 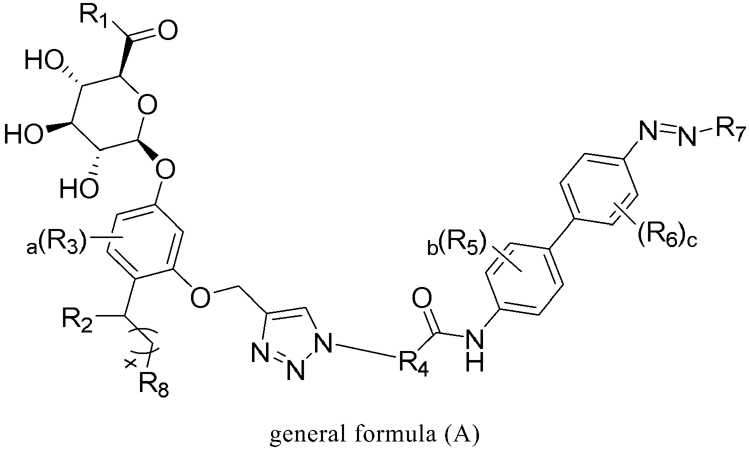 wherein
R₁ is selected from a hydroxy group, a C₁-C₁₀ hydrocarbyloxy group, an -O⁻ alkali metal ion, an -O⁻ammonium ion, and -O⁻;
R₂ is selected from hydrogen and a C₁-C₁₀ hydrocarbyl group;
R₃ is, at each occurrence, independently selected from hydrogen, a halogen, a C₁-C₁₀ hydrocarbyl group, a C₁-C₁₀ halohydrocarbyl group, a C₁-C₁₀ hydrocarbyloxy group, a C₁-C₁₀ halohydrocarbyloxy group, a C₆-C₁₈ aryl group, a nitro group, and - NR₂₁R₂₂;
R₄ is selected from a C₁-C₁₀ hydrocarbylene group, a C₁-C₁₀ hydrocarbylenoxy group, a C₆-C₁₂ arylene group, a C₁-C₁₀ hydrocarbylene-C₆-C₁₂ arylene group, a C₁-C₁₀ hydrocarbylene-C₆-C₁₂ arylene-C₁-C₁₀ hydrocarbylene group, a (C₁-C₁₀ hydrocarbylenoxy)ₐ-C₁-C₁₀ hydrocarbylene group, a C₁-C₁₀ hydrocarbylenoxy-C₆-C₁₂ arylene group, -(CH₂)ₘ-CO-NR₂₃-(CHR₂₄)ⱼ-(CO-NR₂₅-(CHR₂₆)ₖ)ₚ-(CH₂)ₙ-, and - ((CH₂)ₘ-NR₂₃-(CHR₂₄)ⱼ)_{q}-(CH₂)ₙ-;
R₅ and R₆ are, at each occurrence, each independently selected from hydrogen, a halogen, a C₁-C₁₀ hydrocarbyl group, a C₁-C₁₀ halohydrocarbyl group, a C₁-C₁₀ hydrocarbyloxy group, a C₁-C₁₀ halohydrocarbyloxy group, and a C₆-C₁₈ aryl group;
R₇ is selected from: 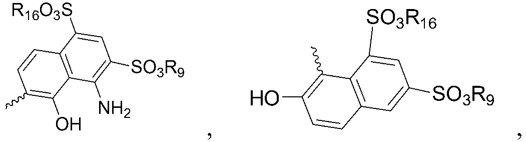 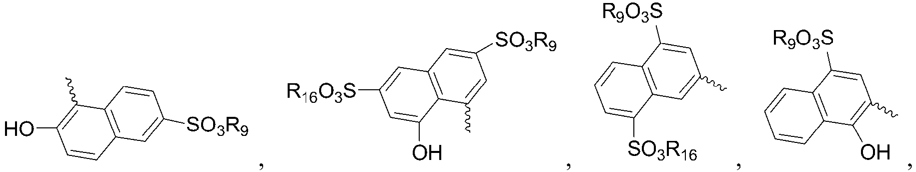 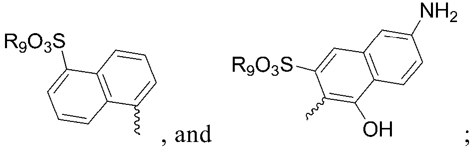
R₈ is selected from R₁₀, R₁₁, R₁₂, R₁₇, -OCO-R₁₀, -OCO-R₁₂, and -OCO-R₁₇: 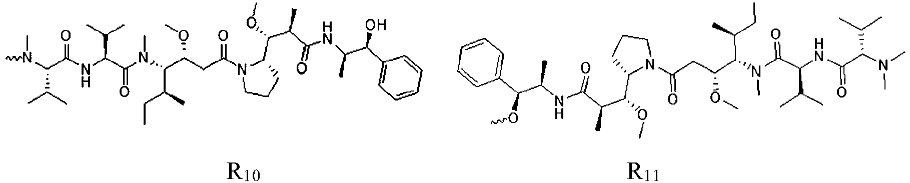 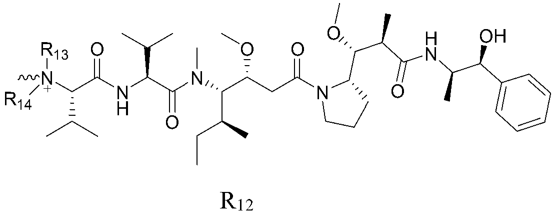 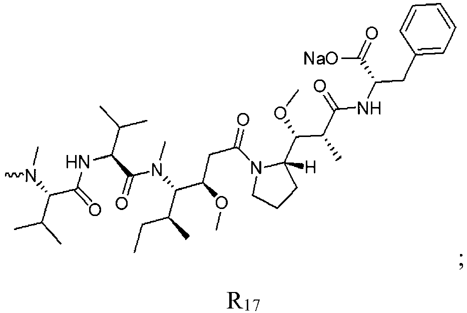
R₁₃ and R₁₄ are each independently selected from hydrogen, a C₁-C₈ hydrocarbyl group, a C₆-C₁₂ aryl group, and a C₁-C₈ hydrocarbylene-C₆-C₁₂ aryl group, and the R₁₃ and the R₁₄ optionally form, together with a nitrogen atom to which they are attached, a 3-17-membered nitrogen-containing heterocycle group;
R₉ and R₁₆ are each independently selected from hydrogen, an alkali metal ion, and an ammonium ion;
R₂₁ and R₂₂ are each independently selected from hydrogen and a C₁-C₆ hydrocarbyl group;
R₂₃ and R₂₅ are each independently selected from hydrogen, a C₁-C₆ hydrocarbyl group, and a C₁-C₆ hydrocarbyloxy group;
R₂₄ and R₂₆ are each independently selected from hydrogen, a C₁-C₆ hydrocarbyl group, a C₁-C₆ hydrocarbyloxy group, a C₆-C₁₂ aryl group, and a C₇-C₁₃ arylhydrocarbyl group; and
x is 0 or 1; a is an integer from 0 to 3; b and c are each independently an integer from 0 to 4; d is an integer from 0 to 4; j, k, m, n, and p are each independently an integer from 0 to 5, and q is an integer from 1 to 5;
wherein 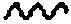 represents a linkage site.

In some embodiments, a compound represented by general formula (I), or a stereoisomer, a cis-trans isomer, or a pharmaceutically acceptable salt thereof is provided: 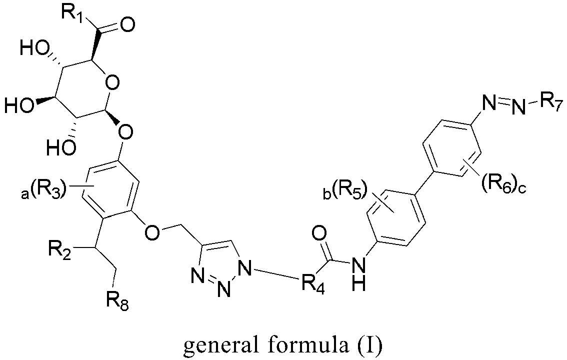 wherein
R₁ is selected from a hydroxy group, a C₁-C₁₀ hydrocarbyloxy group, an -O⁻ alkali metal ion, an -O⁻ammonium ion, and -O⁻;
R₂ is selected from hydrogen and a C₁-C₁₀ hydrocarbyl group;
R₃ is, at each occurrence, independently selected from hydrogen, a halogen, a C₁-C₁₀ hydrocarbyl group, a C₁-C₁₀ halohydrocarbyl group, a C₁-C₁₀ hydrocarbyloxy group, a C₁-C₁₀ halohydrocarbyloxy group, a C₆-C₁₈ aryl group, nitro group, and - NR₂₁R₂₂;
R₄ is selected from a C₁-C₁₀ hydrocarbylene group, a C₁-C₁₀ hydrocarbylenoxy group, a C₆-C₁₂ arylene group, a C₁-C₁₀ hydrocarbylene-C₆-C₁₂ arylene group, a C₁-C₁₀ hydrocarbylene-C₆-C₁₂ arylene-C₁-C₁₀ hydrocarbylene group, a C₁-C₁₀ hydrocarbylenoxy-C₆-C₁₂ arylene group, -(CH₂)ₘ-CO-NR₂₃-(CHR₂₄)ⱼ-(CO-NR₂₅-(CHR₂₆)ₖ)ₚ-(CH₂)ₙ-, and -((CH₂)ₘ-NR₂₃-(CHR₂₄)ⱼ)_{q}-(CH₂)ₙ-; or alternatively, R₄ is selected from a C₁-C₁₀ hydrocarbylene group, a C₁-C₁₀ hydrocarbylenoxy group, a C₆-C₁₂ arylene group, a C₁-C₁₀ hydrocarbylene-C₆-C₁₂ arylene group, a C₁-C₁₀ hydrocarbylene-C₆-C₁₂ arylene-C₁-C₁₀ hydrocarbylene group, a (C₁-C₁₀ hydrocarbylenoxy)ₐ-C₁-C₁₀ hydrocarbylene group, a C₁-C₁₀ hydrocarbylenoxy-C₆-C₁₂ arylene group, -(CH₂)ₘ-CO-NR₂₃-(CHR₂₄)ⱼ-(CO-NR₂₅-(CHR₂₆)ₖ)ₚ-(CH₂)ₙ-, and - ((CH₂)ₘ-NR₂₃-(CHR₂₄)ⱼ)_{q}-(CH₂)ₙ-;
R₅ and R₆ are, at each occurrence, each independently selected from hydrogen, a halogen, a C₁-C₁₀ hydrocarbyl group, a C₁-C₁₀ halohydrocarbyl group, a C₁-C₁₀ hydrocarbyloxy group, a C₁-C₁₀ halohydrocarbyloxy group, and a C₆-C₁₈ aryl group;
R₇ is selected from: 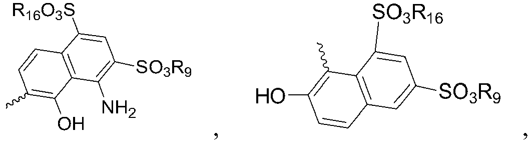 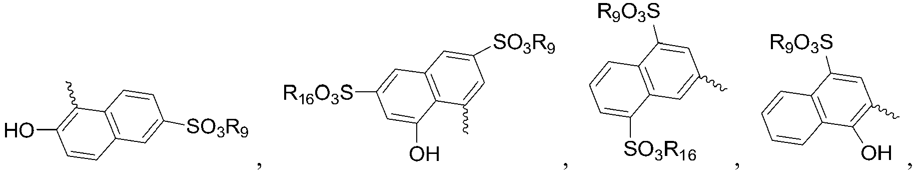 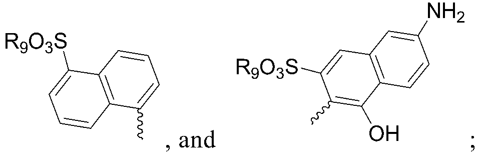
R₈ is selected from R₁₀, R₁₁, R₁₂, R₁₇, -OCO-R₁₀, -OCO-R₁₂, and -OCO-R₁₇: 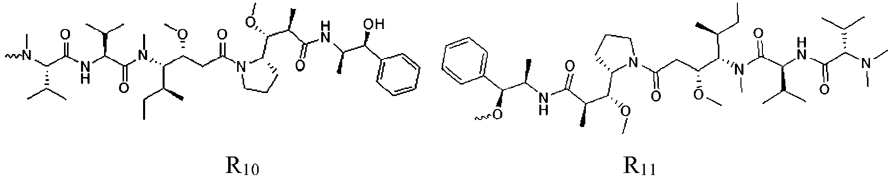 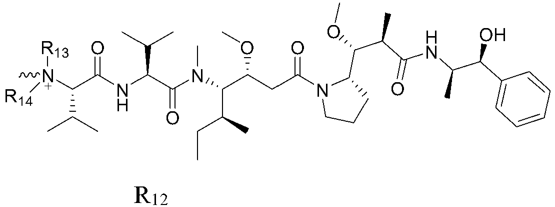 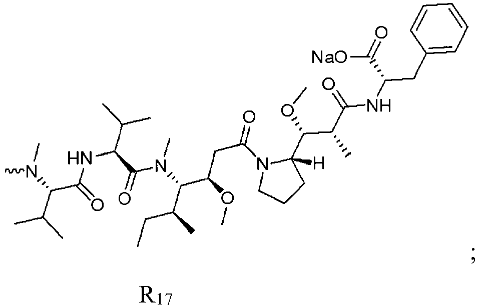
R₁₃ and R₁₄ are each independently selected from hydrogen, a C₁-C₈ hydrocarbyl group, a C₆-C₁₂ aryl group, and a C₁-C₈ hydrocarbylene-C₆-C₁₂ aryl group, and R₁₃ and R₁₄ optionally form, together with a nitrogen atom to which they are attached, a 3-17-membered nitrogen-containing heterocycle group;
R₉ and R₁₆ are each independently selected from hydrogen, an alkali metal ion, and an ammonium ion;
R₂₁ and R₂₂ are each independently selected from hydrogen and a C₁-C₆ hydrocarbyl group;
R₂₃ and R₂₅ are each independently selected from hydrogen, a C₁-C₆ hydrocarbyl group, and a C₁-C₆ hydrocarbyloxy group;
R₂₄ and R₂₆ are each independently selected from hydrogen, a C₁-C₆ hydrocarbyl group, a C₁-C₆ hydrocarbyloxy group, a C₆-C₁₂ aryl group, and a C₇-C₁₃ arylhydrocarbyl group; and
a is an integer from 0 to 3; b and c are each independently an integer from 0 to 4; j, k, m, n, and p are each independently an integer from 0 to 5, and q is an integer from 1 to 5; or alternatively, a is an integer from 0 to 3; b and c are each independently an integer from 0 to 4; d is an integer from 0 to 4; j, k, m, n, and p are each independently an integer from 0 to 5, and q is an integer from 1 to 5;
wherein 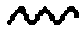 represents a linkage site.

In some embodiments, a compound represented by general formula (II), or a stereoisomer, a cis-trans isomer, or a pharmaceutically acceptable salt thereof is provided: 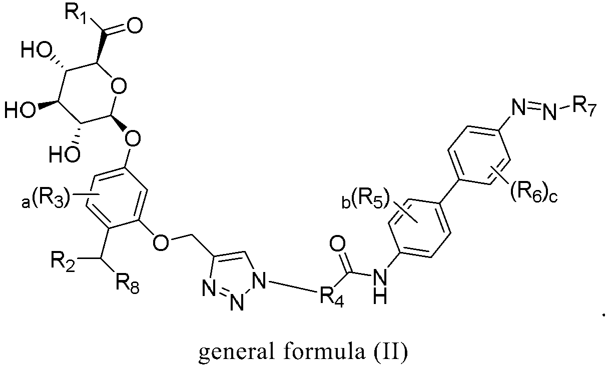 wherein
R₁ is selected from a hydroxy group, a C₁-C₁₀ hydrocarbyloxy group, an -O⁻ alkali metal ion, an -O⁻ammonium ion, and -O⁻;
R₂ is selected from hydrogen and a C₁-C₁₀ hydrocarbyl group;
R₃ is, at each occurrence, independently selected from hydrogen, a halogen, a C₁-C₁₀ hydrocarbyl group, a C₁-C₁₀ halohydrocarbyl group, a C₁-C₁₀ hydrocarbyloxy group, a C₁-C₁₀ halohydrocarbyloxy group, a C₆-C₁₈ aryl group, nitro group, and - NR₂₁R₂₂;
R₄ is selected from a C₁-C₁₀ hydrocarbylene group, a C₁-C₁₀ hydrocarbylenoxy group, a C₆-C₁₂ arylene group, a C₁-C₁₀ hydrocarbylene-C₆-C₁₂ arylene group, a C₁-C₁₀ hydrocarbylene-C₆-C₁₂ arylene-C₁-C₁₀ hydrocarbylene group, a (C₁-C₁₀ hydrocarbylenoxy)ₐ-C₁-C₁₀ hydrocarbylene group, a C₁-C₁₀ hydrocarbylenoxy-C₆-C₁₂ arylene group, -(CH₂)ₘ-CO-NR₂₃-(CHR₂₄)ⱼ-(CO-NR₂₅-(CHR₂₆)ₖ)ₚ-(CH₂)ₙ-, and - ((CH₂)ₘ-NR₂₃-(CHR₂₄)ⱼ)_{q}-(CH₂)ₙ-;
R₅ and R₆ are, at each occurrence, each independently selected from hydrogen, a halogen, a C₁-C₁₀ hydrocarbyl group, a C₁-C₁₀ halohydrocarbyl group, a C₁-C₁₀ hydrocarbyloxy group, a C₁-C₁₀ halohydrocarbyloxy group, and a C₆-C₁₈ aryl group;
R₇ is selected from: 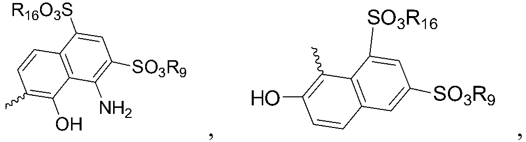 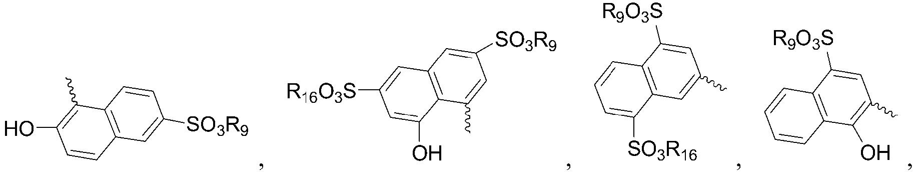 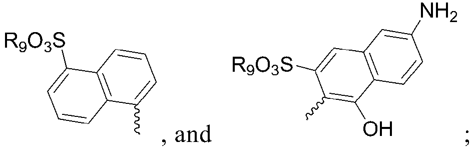
R₈ is selected from R₁₀, R₁₁, R₁₂, R₁₇, -OCO-R₁₀, -OCO-R₁₂, and -OCO-R₁₇: 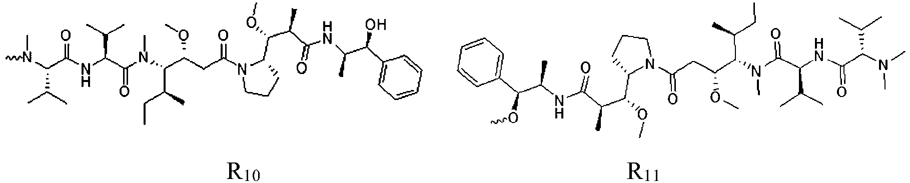 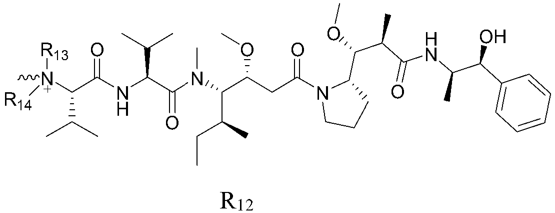 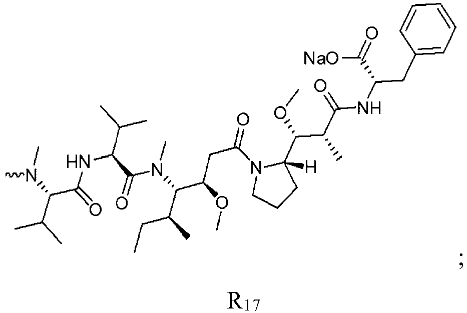
R₁₃ and R₁₄ are each independently selected from hydrogen, a C₁-C₈ hydrocarbyl group, a C₆-C₁₂ aryl group, and a C₁-C₈ hydrocarbylene-C₆-C₁₂ aryl group, and R₁₃ and R₁₄ optionally form, together with a nitrogen atom to which they are attached, a 3-17-membered nitrogen-containing heterocycle group;
R₉ and R₁₆ are each independently selected from hydrogen, an alkali metal ion, and an ammonium ion;
R₂₁ and R₂₂ are each independently selected from hydrogen and a C₁-C₆ hydrocarbyl group;
R₂₃ and R₂₅ are each independently selected from hydrogen, a C₁-C₆ hydrocarbyl group, and a C₁-C₆ hydrocarbyloxy group;
R₂₄ and R₂₆ are each independently selected from hydrogen, a C₁-C₆ hydrocarbyl group, a C₁-C₆ hydrocarbyloxy group, a C₆-C₁₂ aryl group, and a C₇-C₁₃ arylhydrocarbyl group; and
a is an integer from 0 to 3; b and c are each independently an integer from 0 to 4; d is an integer from 0 to 4; j, k, m, n, and p are each independently an integer from 0 to 5, and q is an integer from 1 to 5;
wherein 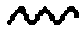 represents a linkage site.

In some embodiments, in the general formulas described herein,
R₁ is selected from a hydroxy group, a C₁-C₁₀ alkoxy group, -O⁻Li⁺, -O⁻Na⁺, - O⁻K⁺, -O⁻Cs⁺, -O⁻NH₄⁺, and -O⁻;
R₂ is selected from hydrogen and a C₁-C₁₀ alkyl group;
R₃ is, at each occurrence, independently selected from hydrogen, F, Cl, Br, I, a C₁-C₁₀ alkyl group, a C₁-C₁₀ haloalkyl group, a C₁-C₁₀ alkoxy group, a C₁-C₁₀ haloalkoxy group, a C₆-C₁₈ aryl group, nitro group, and -NR₂₁R₂₂;
R₄ is selected from a C₁-C₁₀ alkylene group, a C₁-C₁₀ alkylenoxy group, a C₆-C₁₂ arylene group, a C₁-C₁₀ alkylene-C₆-C₁₂ arylene group, a C₁-C₁₀ alkylene-C₆-C₁₂ arylene-C₁-C₁₀ alkylene group, a C₁-C₁₀ alkylenoxy-C₆-C₁₂ arylene group, -(CH₂)ₘ-CO-NR₂₃-(CHR₂₄)ⱼ-(CO-NR₂₅-(CHR₂₆)ₖ)ₚ-(CH₂)ₙ-, and -((CH₂)ₘ-NR₂₃-(CHR₂₄)ⱼ)_{q}-(CH₂)ₙ-;
R₅ and R₆ are, at each occurrence, each independently selected from hydrogen, F, Cl, Br, I, a C₁-C₁₀ alkyl group, a C₁-C₁₀ haloalkyl group, a C₁-C₁₀ alkoxy group, a C₁-C₁₀ haloalkoxy group, and a C₆-C₁₈ aryl group;
R₇ is selected from: 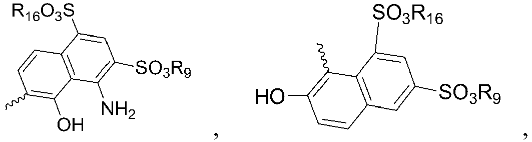 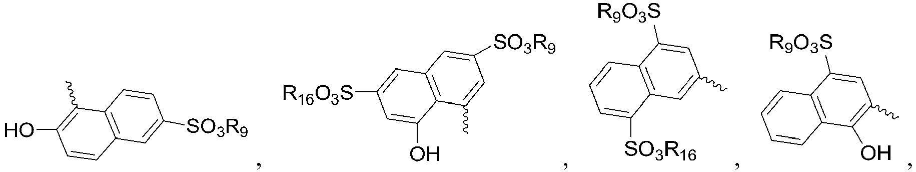 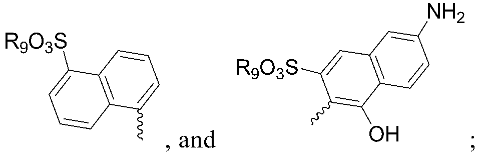
R₈ is selected from R₁₀, R₁₁, R₁₂, R₁₇, -OCO-R₁₀, -OCO-R₁₂, and -OCO-R₁₇: 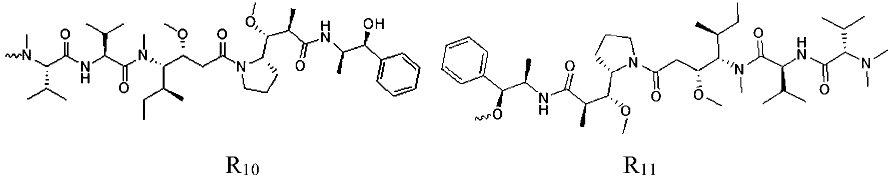 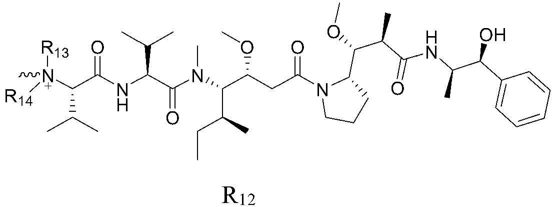 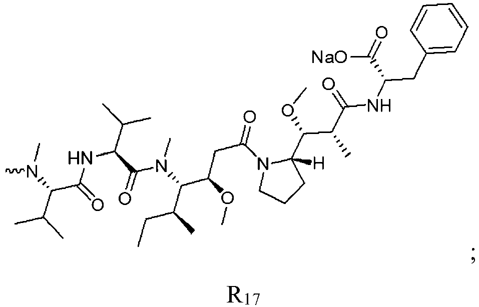
R₁₃ and R₁₄ are each independently selected from hydrogen, a C₁-C₈ alkyl group, a C₆-C₁₂ aryl group, and a C₁-C₈ alkylene-C₆-C₁₂ aryl group, and R₁₃ and R₁₄ optionally form, together with the nitrogen atom to which they are attached, a 3-6-membered nitrogen-containing heterocycle group;
R₉ and R₁₆ are each independently selected from hydrogen, Li⁺, Na⁺, K⁺, Cs⁺, and NH₄⁺;
R₂₁ and R₂₂ are each independently selected from hydrogen and a C₁-C₆ alkyl group;
R₂₃ and R₂₅ are each independently selected from hydrogen, a C₁-C₆ alkyl group, and a C₁-C₆ alkoxy group;
R₂₄ and R₂₆ are each independently selected from hydrogen, a C₁-C₆ alkyl group, a C₁-C₆ alkoxy group, a C₆-C₁₂ aryl group, and a C₇-C₁₃ arylalkyl group; and
a is an integer from 0 to 3; b and c are each independently an integer from 0 to 4; j, k, m, n, and p are each independently an integer from 0 to 5, and q is an integer from 1 to 5;
wherein 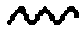 represents a linkage site.

In some other embodiments, in the general formulas described herein,
R₁ is selected from a hydroxy group, -OCH₃, -OCH₂CH₃, -O⁻Li⁺, -O⁻Na⁺, -O⁻ K⁺, -O⁻Cs⁺, -O⁻NH₄⁺, and -O⁻;
R₂ is selected from hydrogen, -CH₃, and -CH₂CH₃;
R₃ is, at each occurrence, independently selected from hydrogen, F, Cl, Br, - CH₃, -CH₂CH₃, -CH₂CH₂CH₃, -OCH₃, -OCH₂CH₃, a phenyl group, -NO₂, -NH₂, - NH(CH₃), and -N(CH₃)₂;
R₄ is selected from -CH₂CH₂CH₂CH₂CH₂-, -CH₂CH₂CH₂CH₂CH₂CH₂-, - CH₂CH₂CH₂CH₂CH₂CH₂CH₂-, -(CH₂CH₂O)₃CH₂-, -(CH₂CH₂CH₂O)₂CH₂CH₂-, - (CH₂CH₂NH)₃CH₂-, -CH₂CH₂C₆H₄-, -CH₂CH₂C₁₀H₆-, -CH₂CH₂C₆H₄C₆H₄-, - CH₂CH₂C₆H₄CH₂CH₂CH₂-, -CH₂CH₂OCH₂CH₂-, -CH₂CH₂OCH₂CH₂CH₂-, - CH₂CH₂CH₂OCH₂CH₂CH₂-, -(CON(CH₃)CH₂)₂-, -(CH₂CONH)₂CH₂-, - CH₂CONHCH(CH₃)CONHCH(CH₃)-, and -CH₂CONHCH(CH₃)CONHCH(CH₂C₆H₅)-;
R₅ and R₆ are, at each occurrence, each independently selected from hydrogen, F, Cl, Br, -CH₃, -CH₂CH₃, -OCH₃, -OCH₂CH₃, and a phenyl group;
R₇ is selected from: 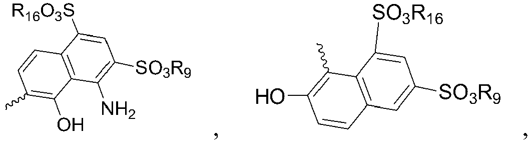 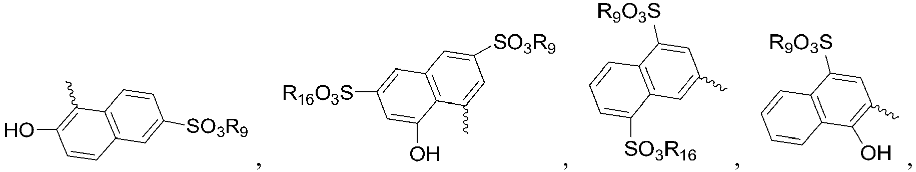 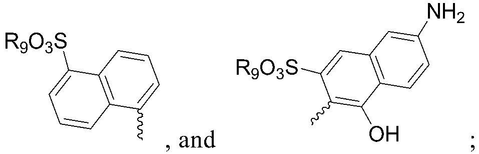
R₈ is selected from R₁₀, R₁₁, R₁₂, R₁₃, R₁₄, R₁₅, R₁₇, -OCO-R₁₀, -OCO-R₁₂, and -OCO-R₁₇; 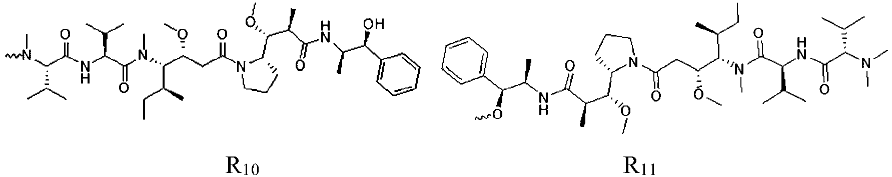 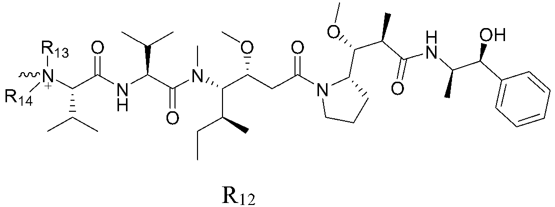 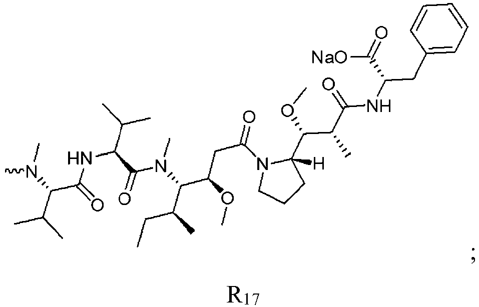
R₁₃ and R₁₄ are each independently selected from hydrogen, a methyl group, an ethyl group, an isopropyl group, a butyl group, a phenyl group, a benzyl group, and a phenethyl group,
when R₁₃ and R₁₄ together with the nitrogen atom to which they are attached form a ring, R₁₂ is: 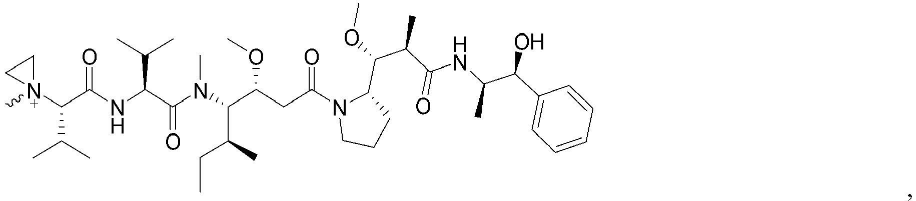 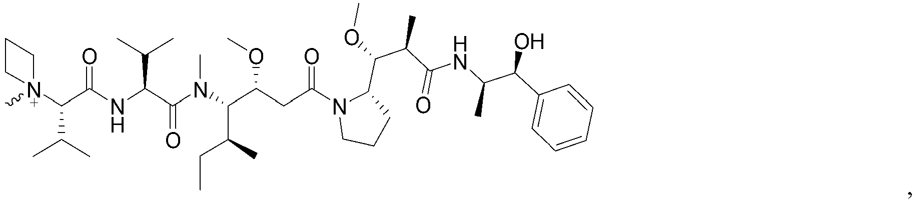 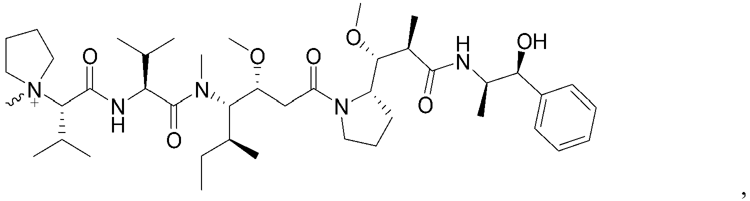 or 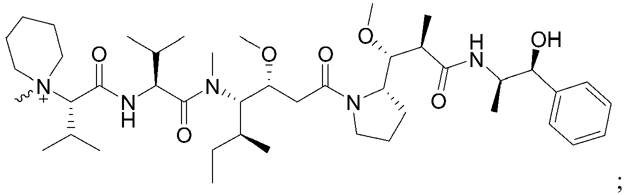
R₉ and R₁₆ are each independently selected from hydrogen, Li⁺, Na⁺, K⁺, Cs⁺, and NH₄⁺; and
a is an integer from 0 to 3; and b and c are each independently an integer from 0 to 4;
wherein 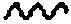 represents a linkage site.

In still some other embodiments, the compound described herein may be any one of the compounds listed in Table 1 below.

In the second aspect, the present disclosure provides a pharmaceutical composition, comprising the compound represented by the general formulas described herein, or the stereoisomer, the cis-trans isomer, or the pharmaceutically acceptable salt thereof, and a pharmaceutically acceptable excipient.

In some embodiments, the pharmaceutical composition comprises an effective amount (e.g., a therapeutically effective amount) of the compound represented by the general formulas described herein, or the stereoisomer, the cis-trans isomer, or the pharmaceutically acceptable salt thereof, and a pharmaceutically acceptable excipient. In some other embodiments, the excipient may be a sodium chloride solution vehicle. In still some other embodiments, the pharmaceutical composition described herein is prepared into a formulation for injection. In yet some other embodiments, the pharmaceutical composition described herein is prepared into an injection, a sterile powder for injection, and a concentrated solution for injection.

In the third aspect, the present disclosure provides use of the compound represented by the general formulas described herein, or the stereoisomer, the cis-trans isomer, or the pharmaceutically acceptable salt thereof, or the pharmaceutical composition described herein for the manufacture of a medicament for treating a tumor or a cancer.

In some embodiments, the tumor or the cancer is selected from colorectal cancer, liver cancer, cervical cancer, ovarian cancer, gastric cancer, esophagus cancer, breast cancer, pancreatic cancer, bladder cancer, liver cancer, intestinal cancer, head and neck cancer, uterine cancer, urothelial carcinoma, osteosarcoma, sarcoma, renal cancer, melanoma, prostate cancer, glioma, neuroglioma, and leukemia.

In the fourth aspect, the present disclosure provides the compound represented by the general formulas described herein, or the stereoisomer, the cis-trans isomer, or the pharmaceutically acceptable salt thereof, or the pharmaceutical composition described herein for use in the treatment of a tumor or a cancer.

In some embodiments, the tumor or the cancer is selected from colorectal cancer, liver cancer, cervical cancer, ovarian cancer, gastric cancer, esophagus cancer, breast cancer, pancreatic cancer, bladder cancer, liver cancer, intestinal cancer, head and neck cancer, uterine cancer, urothelial carcinoma, osteosarcoma, sarcoma, renal cancer, melanoma, prostate cancer, glioma, neuroglioma, and leukemia.

In the fifth aspect, the present disclosure provides a method for treating a tumor or a cancer, comprising administering to an individual in need thereof an effective amount (e.g., a therapeutically effective amount) of the compounds represented by the general formulas (A), (I), and (II), or the stereoisomers, the cis-trans isomers, or the pharmaceutically acceptable salts thereof, or the pharmaceutical composition described herein.

In some embodiments, the tumor or the cancer is selected from colorectal cancer, liver cancer, cervical cancer, ovarian cancer, gastric cancer, esophagus cancer, breast cancer, pancreatic cancer, bladder cancer, liver cancer, intestinal cancer, head and neck cancer, uterine cancer, urothelial carcinoma, osteosarcoma, sarcoma, renal cancer, melanoma, prostate cancer, glioma, neuroglioma, and leukemia.

### DETAILED DESCRIPTION

Specific embodiments below are used to provide detailed description of the present disclosure, but do not imply any unfavorable limitation on the present disclosure. The present disclosure has been described in detail based on the specific embodiments. For those skilled in the art, it will be apparent to make various alterations to the specific embodiments without departing from the spirit and scope of the present disclosure.

Unless otherwise required in the present disclosure, throughout the specification and the claims, the words "comprise (comprising)" and "include (including)" should be construed as an open-ended and inclusive meaning, i.e., "including but not limited to."

Reference herein to "an embodiment" or "another embodiment" or "embodiment" or "some embodiments" means that in at least one embodiment, an element, a structure, or a feature associated with the embodiment is included. Therefore, the phrase "an embodiment" or "embodiment" or "another embodiment" at different occurrences herein does not necessarily refer to a given embodiment. In addition, elements, structures, or features may be combined in any suitable manner in one or more embodiments.

It should be understood that the wording "a/an" in a singular form used throughout the specification and the claims comprises plural objects, unless the context explicitly dictates otherwise.

### Definitions

Unless otherwise stated, the following terms used in the specification and the claims have the following meanings:
Abbreviated symbols preceding some chemical groups named in the present disclosure indicate the total number of carbon atoms present in the indicated chemical group. For example, a C₁-C₁₀ alkyl group depicts an alkyl group totally having from 1 to 10 carbon atoms as defined below, while a C₃-C₁₀ cycloalkyl group depicts a cycloalkyl group totally having from 3 to 10 carbon atoms as defined below. The total number of carbon atoms in an abbreviated symbol does not include carbon atoms that may be present in a substituent of the group.

In the present disclosure, the term "halogen" refers to fluorine, chlorine, bromine, or iodine.

In the present disclosure, the term "hydroxy" refers to a group -OH.

In the present disclosure, the term "amino" refers to a group -NH₂.

In the present disclosure, the term "carboxy" refers to a group -COOH.

In the present disclosure, the term "cyano" refers to a group -CN.

In the present disclosure, the term "nitro" refers to a group -NO₂.

In the present disclosure, the term "alkali metal" refers to six metallic elements in Group IA of the periodic table of elements except for hydrogen (H), namely lithium (Li), sodium (Na), potassium (K), rubidium (Rb), cesium (Cs), and francium (Fr).

In the present disclosure, the term "ammonium ion" refers to an ammoniaderived ammoniated proton, and is expressed as NH₄⁺.

In the present disclosure, the term "hydrocarbyl" refers to an aliphatic hydrocarbyl group. The hydrocarbyl moiety may be a "saturated hydrocarbyl" group, meaning that it does not contain any alkene or alkyne moiety. The hydrocarbyl moiety may also be an "unsaturated hydrocarbyl" moiety, meaning that it contains at least one alkene or alkyne moiety. The "alkene" moiety refers to a linear or branched hydrocarbon chain group consisting of 2 to 10 carbon atoms and at least one carbon-carbon double bond, which is attached to the remainder of a molecule through a single bond, such as vinyl, prop-1-enyl, but-1-enyl, pent-1-enyl, or pent-1,4-dienyl. The "alkyne" moiety refers to a linear or branched hydrocarbon chain group consisting of 2 to 10 carbon atoms and at least one carbon-carbon triple bond which is attached to the remainder of a molecule through a single bond. The hydrocarbyl moiety, whether saturated or unsaturated, may be branched or linear.

The hydrocarbyl group may have from 1 to 10 carbon atoms (at each occurrence in the present disclosure, a numerical range such as "from 1 to 8" refers to every integer within the given range; and "from 1 to 10" means that the hydrocarbyl group may have, e.g., 1, 2, 3, or 4 carbon atoms, up to and including 10 carbon atoms, although this definition further covers occurrence of the term "hydrocarbyl" where no numerical range is specified).

The hydrocarbyl group may be optionally substituted, i.e., substituted or unsubstituted. When the hydrocarbyl group is substituted, the substituent is selected individually and independently from: cyclohydrocarbyl, aryl, heteroaryl, heteroalicyclyl, hydroxy, hydrocarbyloxy, aryloxy, mercapto, hydrocarbylthio, arylthio, cyano, halo, carbonyl, thiocarbonyl, O-carbamoyl, N-carbamoyl, O-thiocarbamoyl, N-thiocarbamoyl, C-amido, N-amido, S-sulfinylamido, N-sulfinylamido, C-carboxyl, O-carboxyl, isocyanato, thiocyanato, isothiocyanato, nitro group, silyl, a trihalomethanesulfonyl, -NR'R" (R' and R" are each hydrocarbyl as defined in the present disclosure) or amino including mono- and di-substituted amino, and a protected derivative thereof. Generally, the hydrocarbyl group includes, but is not limited to, methyl, ethyl, propyl, isopropyl, butyl, isobutyl, tert-butyl, pentyl, hexyl, vinyl, propenyl, butenyl, ethynyl, propynyl, and butynyl. Whenever a group is described as "optionally substituted," the group may be substituted with one of the above substituents.

In some embodiments, "C₁-C₄ hydrocarbyl" refers to a hydrocarbyl group containing from one to four carbon atoms as defined above. The C₁-C₄ hydrocarbyl group may be optionally substituted as defined for the hydrocarbyl group.

In some embodiments, "C₁-C₆ hydrocarbyl" refers to a hydrocarbyl group containing from one to six carbon atoms as defined above. The C₁-C₆ hydrocarbyl group may be optionally substituted as defined for the hydrocarbyl group.

In some embodiments, "C₁-C₁₀ hydrocarbyl" refers to a hydrocarbyl group containing from one to ten carbon atoms as defined above. The C₁-C₁₀ hydrocarbyl group may be optionally substituted as defined for the hydrocarbyl group.

In some embodiments, "C₂-C₆ hydrocarbyl" refers to a hydrocarbyl group containing from two to six carbon atoms as defined above. The C₂-C₆ hydrocarbyl group may be optionally substituted as defined for the hydrocarbyl group.

In some embodiments, "C₃-C₆ hydrocarbyl" refers to a hydrocarbyl group containing from three to six carbon atoms as defined above. The C₃-C₆ hydrocarbyl group may be optionally substituted as defined for the hydrocarbyl group.

In some embodiments, "C₃-C₁₀ hydrocarbyl" refers to a hydrocarbyl group containing from three to ten carbon atoms as defined above. The C₃-C₁₂ hydrocarbyl group may be optionally substituted as defined for the hydrocarbyl group.

In some embodiments, "C₆-C₁₀ hydrocarbyl" refers to a hydrocarbyl group containing from six to ten carbon atoms as defined above. The C₆-C₁₂ hydrocarbyl group may be optionally substituted as defined for the hydrocarbyl group.

In some embodiments, "C₇-C₁₂ hydrocarbyl" refers to a hydrocarbyl group containing from seven to twelve carbon atoms as defined above. The C₇-C₁₂ hydrocarbyl group may be optionally substituted as defined for the hydrocarbyl group.

In some embodiments, the term "halohydrocarbyl" refers to a hydrocarbyl group substituted with one or more substituents individually and independently selected from halogen.

In some embodiments, the term "hydrocarbylene" refers to a hydrocarbyl group formed by removing two hydrogen atoms from a hydrocarbon molecule. Representative examples of the hydrocarbylene group include, but are not limited to, - CH₂-, -CH₂CH₂-, and -CH₂CH₂CH₂-.

In the present disclosure, the term "hydrocarbyloxy" refers to a general formula of -O-hydrocarbyl group, wherein the hydrocarbyl group is as defined in the present disclosure. Representative examples of the hydrocarbyloxy group include, but are not limited to, methoxy, ethoxy, n-propoxy, 1-methylethoxy (isopropoxy), n-butoxy, isobutoxy, sec-butoxy, tert-butoxy, pentyloxy, and tert-pentyloxy.

In the present disclosure, the term "aryl" refers to a carbocyclic ring (allcarbon) or a fused ring of two or more rings (rings sharing two adjacent carbon atoms) with a fully delocalized Pi electron system. The aryl group includes, but is not limited to, fluorenyl, phenyl, and naphthyl. The aryl group may have, for example, from six to twelve carbon atoms. The aryl group in the present disclosure may be substituted or unsubstituted. When the aryl group is substituted, the hydrogen atom is replaced with one or more groups independently selected from the following substituents: hydrocarbyl, cyclohydrocarbyl, aryl, heteroaryl, heteroalicyclyl, hydroxy, protected hydroxy, hydrocarbyloxy, aryloxy, mercapto, hydrocarbylthio, arylthio, cyano, halo, carbonyl, thiocarbonyl, O-carbamoyl, N-carbamoyl, O-thiocarbamoyl, N-thiocarbamoyl, C-amido, N-amido, S-sulfinylamido, N-sulfinylamido, C-carboxyl, protected C-carboxyl, O-carboxyl, isocyanato, thiocyanato, isothiocyanato, nitro, silyl, trihalomethanesulfonyl, -NR'R" (R' and R" are each hydrocarbyl as defined in the present disclosure), and protected amino. Whenever a group is described as "optionally substituted," the group may be substituted with one of the above substituents.

In the present disclosure, the term "arylene" refers to a carbocyclic ring (allcarbon) or a fused ring of two or more rings (rings sharing two adjacent carbon atoms) with a fully delocalized Pi electron system. The arylene group includes, but is not limited to, fluorenylidene, phenylene, and naphthylene. The arylene group may have, for example, from six to twelve carbon atoms. The arylene group in the present disclosure may be substituted or unsubstituted. When the arylene group is substituted, the hydrogen atom is replaced with one or more groups independently selected from the following substituents: hydrocarbyl, cyclohydrocarbyl, aryl, heteroaryl, heteroalicyclyl, hydroxy, protected hydroxy, hydrocarbyloxy, aryloxy, mercapto, hydrocarbylthio, arylthio, cyano, halo, carbonyl, thiocarbonyl, O-carbamoyl, N-carbamoyl, O-thiocarbamoyl, N-thiocarbamoyl, C-amido, N-amido, S-sulfinylamido, N-sulfinylamido, C-carboxyl, protected C-carboxyl, O-carboxyl, isocyanato, thiocyanato, isothiocyanato, nitro, silyl, trihalomethanesulfonyl, -NR'R" (R' and R" are each hydrocarbyl as defined in the present disclosure), or protected amino. Whenever a group is described as "optionally substituted," the group may be substituted with one of the above substituents. The arylene may be linked to the remainder of a molecule through one carbon atom in the group or through any two carbon atoms in the group.

In the present disclosure, the term "aryl" refers to a carbocyclic ring (allcarbon) or a fused ring of two or more rings (rings sharing two adjacent carbon atoms) with a fully delocalized Pi electron system. The aryl group includes, but is not limited to, fluorenyl, phenyl, and naphthyl. The aryl group may have, for example, from six to twelve carbon atoms. The aryl group in the present disclosure may be substituted or unsubstituted. When the aryl group is substituted, the hydrogen atom is replaced with one or more groups independently selected from the following substituents: hydrocarbyl, cyclohydrocarbyl, aryl, heteroaryl, heteroalicyclyl, hydroxy, protected hydroxy, hydrocarbyloxy, aryloxy, mercapto, hydrocarbylthio, arylthio, cyano, halo, carbonyl, thiocarbonyl, O-carbamoyl, N-carbamoyl, O-thiocarbamoyl, N-thiocarbamoyl, C-amido, N-amido, S-sulfinylamido, N-sulfinylamido, C-carboxyl, protected C-carboxyl, O-carboxyl, isocyanato, thiocyanato, isothiocyanato, nitro, silyl, trihalomethanesulfonyl, -NR'R" (R' and R" are each hydrocarbyl as defined in the present disclosure), or protected amino. Whenever a group is described as "optionally substituted," the group may be substituted with one of the above substituents.

In the present disclosure, the term "heteroaryl" refers to a 5-18-membered aromatic ring consisting of one to seventeen carbon atoms and one to ten heteroatoms selected from nitrogen, oxygen, and sulfur atoms. In some embodiments, the heteroaryl group may be a monocyclic, bicyclic, tricyclic, or tetracyclic ring system, which may comprise a fused or bridged ring system; the nitrogen, carbon, or sulfur atom in the heteroaryl group may optionally be oxidized; and the nitrogen atom may optionally be quaternized. Representative examples of the heteroaryl group include, but are not limited to, azepinyl, acridyl, benzimidazolyl, benzothiazolyl, benzoindolyl, benzodioxolyl, benzofuranyl, benzooxazolyl, benzothiazolyl, benzothiadiazolyl, benzo[b][1,4]dioxepinyl, 1,4-benzodioxanyl, benzonaphthofuranyl, benzoxazolyl, benzodioxolyl, benzodioxinyl, benzopyranyl, benzopyronyl, benzofuranyl, benzofuranonyl, benzothienyl, benzotriazolyl, benzo[4,6]imidazo[1,2-a]pyridinyl, carbazolyl, cinnolinyl, dibenzofuranyl, dibenzothienyl, furanyl, furanonyl, isothiazolyl, imidazolyl, indazolyl, indolyl, indazolyl, isoindolyl, indolinyl, isoindolinyl, isoquinolyl, indolizinyl, isoxazolyl, naphthyridinyl, oxadiazolyl, 2-oxoazepinyl, oxazolyl, oxiranyl, 1-phenyl-1H-pyrrolyl, phenazinyl, phenothiazinyl, phenoxazinyl, phthalazinyl, pteridinyl, purinyl, pyrrolyl, pyrazolyl, pyridinyl, pyrazinyl, pyrimidinyl, pyridazinyl, pyrrolyl, quinazolinyl, quinoxalyl, quinolyl, quinuclidinyl, isoquinolyl, tetrahydroquinolyl, thiazolyl, thiadiazolyl, triazolyl, tetrazolyl, triazinyl, and thienyl. The heteroaryl group in the present disclosure may be substituted or unsubstituted. When the heteroaryl group is substituted, the hydrogen atom is replaced with one or more groups independently selected from the following substituents: hydrocarbyl, cyclohydrocarbyl, aryl, heteroaryl, heteroalicyclyl, hydroxy, protected hydroxy, hydrocarbyloxy, aryloxy, mercapto, hydrocarbylthio, arylthio, cyano, halo, carbonyl, thiocarbonyl, O-carbamoyl, N-carbamoyl, O-thiocarbamoyl, N-thiocarbamoyl, C-amido, N-amido, S-sulfinylamido, N-sulfinylamido, C-carboxyl, protected C-carboxyl, O-carboxyl, isocyanato, thiocyanato, isothiocyanato, nitro, silyl, trihalomethanesulfonyl, -NR'R" (R' and R" are each hydrocarbyl as defined in the present disclosure), or protected amino. Whenever a group is described as "optionally substituted," the group may be substituted with one of the above substituents.

In the present disclosure, the term "heteroarylene" refers to a 5-18-membered divalent aromatic ring consisting of one to seventeen carbon atoms and one to ten heteroatoms selected from nitrogen, oxygen, and sulfur atoms. In some embodiments, the heteroarylene group may be a monocyclic, bicyclic, tricyclic, or tetracyclic ring system, which may comprise a fused or bridged ring system; the nitrogen, carbon, or sulfur atom in the heteroarylene group may optionally be oxidized; and the nitrogen atom may optionally be quaternized. Representative examples of the heteroarylene group include, but are not limited to, azepinylene, acridylene, benzimidazolylene, benzothiazolylene, benzoindolylene, benzodioxolylidene, benzofuranylene, benzooxazolylene, benzothiazolylene, benzothiadiazolylene, benzo[b][1,4]dioxepinylene, 1,4-benzodioxanylene, benzonaphthofuranylene, benzoxazolylene, benzodioxolylidene, benzodioxinylidene, benzopyranylene, benzopyronylene, benzofuranylene, benzofuranonylene, benzothienylene, benzotriazolylene, benzo[4,6]imidazo[1,2-a]pyridinylene, carbazolylene, cinnolinylene, dibenzofuranylene, dibenzothienylene, furanylene, furanonylene, isothiazolylene, imidazolylene, indazolylene, indolylene, indazolylene, isoindolylene, indolinylene, isoindolinylene, isoquinolylene, indolizinylene, isoxazolylene, naphthyridinylene, oxadiazolylene, 2-oxoazepinylene, oxazolylene, oxiranylene, 1-phenyl-1H-pyrrolylene, phenazinylene, phenothiazinylene, phenoxazinylene, phthalazinylene, pteridinylene, purinylene, pyrrolylene, pyrazolylene, pyridinylene, pyrazinylene, pyrimidinylene, pyridazinylene, pyrrolylene, quinazolinylene, quinoxalylene, quinolylene, quinuclidinylene, isoquinolylene, tetrahydroquinolylene, thiazolylene, thiadiazolylene, triazolylene, tetrazolylene, triazinylene, and thienylene. The heteroarylene group in the present disclosure may be substituted or unsubstituted. When the heteroarylene group is substituted, the hydrogen atom is replaced with one or more groups independently selected from the following substituents: hydrocarbyl, cyclohydrocarbyl, aryl, heteroaryl, heteroalicyclyl, hydroxy, protected hydroxy, hydrocarbyloxy, aryloxy, mercapto, hydrocarbylthio, arylthio, cyano, halo, carbonyl, thiocarbonyl, O-carbamoyl, N-carbamoyl, O-thiocarbamoyl, N-thiocarbamoyl, C-amido, N-amido, S-sulfinylamido, N-sulfinylamido, C-carboxyl, protected C-carboxyl, O-carboxyl, isocyanato, thiocyanato, isothiocyanato, nitro, silyl, trihalomethanesulfonyl, -NR'R" (R' and R" are each hydrocarbyl as defined in the present disclosure), or protected amino. Whenever a group is described as "optionally substituted," the group may be substituted with one of the above substituents. The heteroarylene may be linked to the remainder of a molecule through one carbon atom in the group or through any two carbon atoms in the group.

In the present disclosure, the term "hetero-aromatic ring group" refers to a 5-18-membered aromatic ring consisting of one to seventeen carbon atoms and one to ten heteroatoms selected from nitrogen, oxygen, and sulfur atoms. In some embodiments, the hetero-aromatic ring group may be a monocyclic, bicyclic, tricyclic, or tetracyclic ring system, which may comprise a fused or bridged ring system; the nitrogen, carbon, or sulfur atom in the hetero-aromatic ring group may optionally be oxidized; and the nitrogen atom may optionally be quaternized. Representative examples of the hetero-aromatic ring group include, but are not limited to, azepinyl, acridyl, benzimidazolyl, benzothiazolyl, benzoindolyl, benzodioxolyl, benzofuranyl, benzooxazolyl, benzothiazolyl, benzothiadiazolyl, benzo[b][1,4]dioxepinyl, 1,4-benzodioxanyl, benzonaphthofuranyl, benzoxazolyl, benzodioxolyl, benzodioxinyl, benzopyranyl, benzopyronyl, benzofuranyl, benzofuranonyl, benzothienyl, benzotriazolyl, benzo[4,6]imidazo[1,2-a]pyridinyl, carbazolyl, cinnolinyl, dibenzofuranyl, dibenzothienyl, furanyl, furanonyl, isothiazolyl, imidazolyl, indazolyl, indolyl, indazolyl, isoindolyl, indolinyl, isoindolinyl, isoquinolyl, indolizinyl, isoxazolyl, naphthyridinyl, oxadiazolyl, 2-oxoazepinyl, oxazolyl, oxiranyl, 1-phenyl-1H-pyrrolyl, phenazinyl, phenothiazinyl, phenoxazinyl, phthalazinyl, pteridinyl, purinyl, pyrrolyl, pyrazolyl, pyridinyl, pyrazinyl, pyrimidinyl, pyridazinyl, pyrrolyl, quinazolinyl, quinoxalyl, quinolyl, quinuclidinyl, isoquinolyl, tetrahydroquinolyl, thiazolyl, thiadiazolyl, triazolyl, tetrazolyl, triazinyl, and thienyl. The hetero-aromatic ring group in the present disclosure may be substituted or unsubstituted. When the hetero-aromatic ring group is substituted, the hydrogen atom is replaced with one or more groups independently selected from the following substituents: hydrocarbyl, cyclohydrocarbyl, aryl, heteroaryl, heteroalicyclyl, hydroxy, protected hydroxy, hydrocarbyloxy, aryloxy, mercapto, hydrocarbylthio, arylthio, cyano, halo, carbonyl, thiocarbonyl, O-carbamoyl, N-carbamoyl, O-thiocarbamoyl, N-thiocarbamoyl, C-amido, N-amido, S-sulfinylamido, N-sulfinylamido, C-carboxyl, protected C-carboxyl, O-carboxyl, isocyanato, thiocyanato, isothiocyanato, nitro, silyl, trihalomethanesulfonyl, -NR'R" (R' and R" are each hydrocarbyl as defined in the present disclosure), or protected amino. Whenever a group is described as "optionally substituted," the group may be substituted with one of the above substituents.

In the present disclosure, the term "cyclohydrocarbyl" refers to a stable nonaromatic monocyclic or bicyclic hydrocarbyl group that consists solely of carbon and hydrogen atoms, has from three to fifteen carbon atoms, in some embodiments from three to twelve carbon atoms, and is saturated or unsaturated, attached to the remainder of a molecule through a single bond, such as cyclopropyl, cyclobutyl, cyclopentyl, cyclohexyl, or cyclodecyl. Unless otherwise explicitly stated in the present disclosure, the term "cyclohydrocarbyl" is intended to include the above defined cyclohydrocarbyl group optionally substituted with one or more groups selected from substituents of: cyclohydrocarbyl, aryl, heteroaryl, cycloaryl, heterocycloaryl, heteroalicyclyl, hydroxy, hydrocarbyloxy, aryloxy, mercapto, hydrocarbylthio, arylthio, cyano, halo, carbonyl, thiocarbonyl, O-carbamoyl, N-carbamoyl, O-thiocarbamoyl, N-thiocarbamoyl, C-amido, N-amido, S-sulfinylamido, N-sulfinylamido, C-carboxyl, O-carboxyl, isocyanato, thiocyanato, isothiocyanato, nitro, silyl, trihalomethanesulfonyl, -NR'R" (R' and R" are each hydrocarbyl as defined in the present disclosure) or amino including mono- and di-substituted amino, and a protected derivative thereof.

In some embodiments, "C₃-C₆ cyclohydrocarbyl" refers to a cyclohydrocarbyl group containing from three to six carbon atoms as defined above. The C₃-C₆ cyclohydrocarbyl group may be optionally substituted as defined for the above cyclohydrocarbyl.

In some embodiments, "C₃-C₁₀ cyclohydrocarbyl" refers to a cyclohydrocarbyl group containing from three to ten carbon atoms as defined above. The C₃-C₁₀ cyclohydrocarbyl group may be optionally substituted as defined for the above cyclohydrocarbyl.

In the present disclosure, the heterocycle may contain at least one of B, O, N, P, Si, or S as a heteroatom used to form the ring. When the heterocycle contains two or more heteroatoms, the two or more heteroatoms may be identical to or different from each other. The heterocycle may be a monocyclic heterocycle or a polycyclic heterocycle, and may include a heterocyclic hydrocarbyl group, such as a heteroaryl group. The number of ring carbon atoms used to form a heterocycle may be from 2 to 30, from 2 to 20, or from 2 to 10. The number of ring heteroatoms used to form a heterocycle may be from 1 to 10, from 1 to 5, or from 1 to 3, such as 1, 2, 3, 4, 5, 6, 7, 8, or 9. Examples of the heterocycle may include, but are not limited, thiophene, furan, pyrrole, imidazole, thiazole, oxazole, oxadiazole, triazole, pyridine, bipyridine, pyrimidine, triazine, acridine, pyridazine, pyrazine, quinoline, quinazoline, quinoxaline, phenoxazine, phthalazine, pyridopyrimidine, pyridopyrazine, pyrazinopyrazine, isoquinoline, indole, carbazole, N-arylcarbazole, N-heteroarylcarbazole, N-alkylcarbazole, benzoxazole, benzimidazole, benzothiazole, benzocarbazole, benzothiophene, dibenzothiophene, thienothiophene, benzofuran, phenanthroline, isoxazole, thiadiazole, phenothiazine, dibenzosilole, dibenzofuran, etc.

In the present disclosure, the term "alkylene" or "hydrocarbylene" refers to a divalent hydrocarbon chain consisting solely of carbon and hydrogen, and may be a linear alkyl group, a branched alkyl group, or a cycloalkyl group, that is, a linear or branched divalent hydrocarbon chain linked to the remainder of a molecule, such as methylene, ethylene, propylene, n-butylene, vinylene, propenylene, or n-butenylene. The alkylene chain may be linked to the remainder of a molecule through one carbon atom in the chain or through any two carbon atoms in the chain.

In the present disclosure, the term "C₁-C₉ hydrocarbylene" refers to a bifunctional linear, branched, or cyclic hydrocarbyl having from one to nine carbon atoms. That is, "C₁-C₉ hydrocarbylene" refers to a linear or branched divalent hydrocarbon chain consisting solely of carbon and hydrogen, having from one to nine carbon atoms, and linked to the remainder of a molecule, such as methylene, ethylene, propylene, n-butylene, vinylene, propenylene, or n-butenylene. The hydrocarbylene chain may be linked to the remainder of a molecule through one carbon atom in the chain or through any two carbon atoms in the chain. In the present disclosure, the term "alkyleneoxy" or "hydrocarbyleneoxy" refers to a general formula of alkylene-O- or hydrocarbylene-O-, wherein the alkylene or hydrocarbylene is as defined in the present disclosure. Representative examples of the alkyleneoxy include, but are not limited to, methyleneoxy, ethyleneoxy, and n-propyleneoxy. In the present disclosure, the term "C₁-C₉ alkyleneoxy" or "C₁-C₉ hydrocarbyleneoxy" refers to a bifunctional linear, branched, or cyclic alkoxy or hydrocarbyl having from one to nine carbon atoms.

In the present disclosure, the term "cyclohydrocarbyloxy" refers to -O-cyclohydrocarbyl group, wherein the cyclohydrocarbyl group is as defined in the present disclosure. Representative examples of the cyclohydrocarbyloxy group include, but are not limited to, cyclopropyloxy, cyclobutyloxy, cyclopentyloxy, cyclohexyloxy, and cyclodecyloxy.

In the present disclosure, the term "hydrocarbylcarbonylamino" refers to - NHC(=O)-hydrocarbyl group, wherein the hydrocarbyl group is as defined in the present disclosure. Representative examples of the hydrocarbylcarbonylamino group include, but are not limited to, acetamido, propioamido, butanamido, and pentanamido.

In the present disclosure, the term "hydrocarbylcarbonyloxy" refers to - OC(=O)-hydrocarbyl group, wherein the hydrocarbyl group is as defined in the present disclosure. Representative examples of the hydrocarbylcarbonyloxy group include, but are not limited to, acetoxy, propionyloxy, butanoyloxy, and pentanoyloxy.

In the present disclosure, the term "hydrocarbyloxycarbonylamino" refers to - NHC(=O)O-hydrocarbyl group, wherein the hydrocarbyl group is as defined in the present disclosure. Representative examples of the hydrocarbyloxycarbonylamino group include, but are not limited to, methoxycarbonylamino, ethoxycarbonylamino, propoxycarbonylamino, and tert-butoxycarbonylamino.

In the present disclosure, the term "hydrocarbyloxycarbonyl" refers to - C(=O)O-hydrocarbyl group, wherein the hydrocarbyl group is as defined in the present disclosure. Representative examples of the hydrocarbyloxycarbonyl group include, but are not limited to, methoxycarbonyl, ethoxycarbonyl, propoxycarbonyl, isopropoxycarbonyl, and butoxycarbonyl.

In the present disclosure, the term "carbonylamino" refers to -C(=O)-NH-.

In addition, sequentially named prefixes mean that substituents are recited in sequence. For example, the arylalkoxy group refers to an aryl-substituted alkoxy group, the alkoxycarbonyl group refers to an alkoxy-substituted carbonyl group, and the arylcarbonylalkenyl group refers to an arylcarbonyl-substituted alkenyl group, wherein the arylcarbonyl group may be an aryl-substituted carbonyl group.

In the present disclosure, an expression similar to "-NH-," "-NR-," or the like means that two chemical bonds of the N atom are linked to the remainder, and should not be construed as "-NH-R-".

In the present disclosure, the variable defined in a general formula, when represented as 0 or absent, means that the general formula should skip the group represented by the variable being 0 or absent and continue to link to a group that is linked to the group represented as 0 or absent, rather than being construed as no further linkage to any group.

In the present disclosure, when general formula A, I, or II is a quaternary ammonium salt, the compound of the general formula can be linked to a negative ion. The negative ion includes, e.g., halogen negative ions (fluorine, chlorine, bromine, or iodine), nitrate, sulfate, phosphate, methane sulfonate, triflate, benzenesulfonate, p-methylbenzenesulfonate, camphorsulfonate, citrate, fumarate, lactate, malate, oxalate, formate, acetate, maleate, succinate, tartrate, ascorbate, benzoate, gentisate, oleate, pamoate, stearate, and sorbate.

In the present disclosure, the term "mammal" refers to an animal including, for example, a dog, a cat, a cattle, a sheep, a horse, a human, and the like. In some embodiments, the mammal includes a human.

In the present disclosure, the term "patient" refers to an animal (e.g., a human), a companion animal (e.g., a dog, a cat, or a horse), and a domestic animal (e.g., a cattle, a pig, and a sheep). In some embodiments, the patient is a mammal including both male and female. In some embodiments, the patient is a human.

In the present disclosure, the term "pharmaceutically acceptable" refers to a vehicle, a diluent, and/or a salt that must be compatible with other ingredients of the formulation and will not be harmful to a recipient thereof.

In the present disclosure, the term "optional" or "optionally" means that the subsequently described event or condition may or may not occur, and that the specification includes a case where the event or condition occurs and a case where the event or condition does not occur.

In the present disclosure, the term "pharmaceutically acceptable excipient" includes, but is not limited to, various forms of vehicles that have been approved by the U.S. Food and Drug Administration for use in humans or animals and have no adverse effects on the ingredients of the pharmaceutical composition, such as any adjuvant, vehicle, glidant, sweetener, diluent, preservative, dye/colorant, flavor enhancer, surfactant, wetting agent, dispersant, suspending aid, stabilizer, isosmotic agent, solvent or emulsifier.

In the present disclosure, the term "vehicle" is defined as a compound that facilitates introduction of a compound into cells or tissues. For example, dimethyl sulfoxide (DMSO) is often used as a vehicle because it readily introduces some organic compounds into cells or tissues of an organism.

In the present disclosure, the term "pharmaceutically acceptable salt" includes an "acceptable acid addition salt" and an "acceptable base addition salt."

In the present disclosure, the term "acceptable acid addition salt" refers to a salt retaining bioavailability and properties of a free base, being biologically or otherwise suitable, and being formed from an inorganic or organic acid, such as, but not limited to, hydrochloric acid, hydrobromic acid, sulfuric acid, nitric acid, phosphoric acid, etc., and the organic acid includes, but is not limited to, acetic acid, 2,2-dichloroacetic acid, adipic acid, alginic acid, ascorbic acid, aspartic acid, benzenesulfonic acid, phenylcarboxylic acid, 4-acetamidophenylcarboxylic acid, camphoric acid, camphor-10-sulfonic acid, decanoic acid, hexanoic acid, octanoic acid, carbonic acid, cinnamic acid, citric acid, cyclohexylaminosulfonic acid, dodecylsulfuric acid, ethane-1,2-disulfonic acid, ethanesulfonic acid, 2-hydroxyethanesulfonic acid, formic acid, fumaric acid, mucic acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecenoic acid, etc.

In the present disclosure, the term "acceptable base addition salt" refers to a salt retaining bioavailability and properties of a free acid, and being biologically or otherwise suitable. The salt is prepared by adding an inorganic or organic base to a free acid. Salts derived from inorganic bases include, but are not limited to, salts of sodium, potassium, lithium, ammonium, calcium, magnesium, iron, zinc, copper, manganese, aluminum and the like. In some embodiments, the inorganic salt is salt of ammonium, sodium, potassium, calcium, or magnesium. Salts derived from organic bases include, but are not limited to, salts of primary, secondary, and tertiary amines, salts of substituted amines including naturally occurring substituted amines, salts of cycloamine, and salts of basic ion exchange resins, such as ammonia, isopropylamine, trimethylamine, diethylamine, triethylamine, tripropylamine, diethanolamine, ethanolamine, 2-dimethylaminoethanol, 2-diethylaminoethanol, dicyclohexylamine, lysine, arginine, histidine, caffeine, procaine, hydrabamine, choline, betaine, benzylamine, phenylethylenediamine, ethylenediamine, glucosamine, methylglucamine, theobromine, triethanolamine, tromethamine, purine, piperazine, piperidine, N-ethylpiperidine, and polyamine resin. In some embodiments, the organic bases include isopropylamine, diethylamine, ethanolamine, trimethylamine, dicyclohexylamine, choline, and caffeine.

In the present disclosure, the term "solvent or solvent mixture" refers to any and all solvents. In some embodiments, the solvent or solvent mixture is an organic solvent and water, including but not limited to methanol, ethanol, 2-propanol, n-butanol, isobutanol, acetone, methyl ethyl ketone, ethyl acetate, 1,4-dioxane, diethyl ether, methyl tert-butyl ether, tetrahydrofuran, acetonitrile, dichloromethane, chloroform, N,N-dimethylformamide, cyclohexane, cyclopentane, n-hexane, n-heptane, n-pentane, toluene, o-xylene, p-xylene, dimethyl sulfoxide (DMSO), pyridine, acetic acid, anisole, butyl acetate, cumene, ethyl formate, formic acid, isobutyl acetate, isopropyl acetate, methyl acetate, 3-methyl-1-butanol, methyl isobutyl ketone, 2-methyl-1-propanol, 1-pentanol, propyl acetate, ethylene glycol, and 1-methyl-2-pyrrolidone, as well as any and all mixtures of two or more of such solvents. In some embodiments, the solvent or solvent mixture is a single solvent and a binary mixture. In some embodiments, the solvent or solvent mixture is a single solvent of water or an organic solvent and a binary mixture of water and an organic solvent.

In the present disclosure, the term "pharmaceutical composition" refers to a formulation formed from the compound of the present disclosure and a medium generally accepted in the art for delivering a bioactive compound to a mammal, such as a human. Such mediums include all pharmaceutically acceptable vehicles, diluents, and the like.

In the present disclosure, the term "effective amount" or "therapeutically effective amount" refers to an amount of a compound or a combination of compounds that ameliorates, alleviates, or eliminates a particular disease or condition and symptoms of a particular disease or condition, or averts or delays the onset of a particular disease or condition or symptoms of a particular disease or condition. The amount of the compound of the present disclosure that constitutes an "effective amount" or a "therapeutically effective amount" will vary depending on the compound, a disease state and severity thereof, and age and body weight of a mammal to be treated, but those skilled in the art can routinely determine the amount of the compound of the present disclosure based on their own knowledge and the contents of the present disclosure.

As used in the present disclosure, the term "treating" or "treatment" covers the treatment of the disease or condition of interest in a mammal, such as a human, having the disease or disorder of interest, and includes:
(i) preventing the disease or condition from occurring in a mammal, in particular, when such mammal is predisposed to the condition but has not yet been diagnosed as having it;
(ii) inhibiting the disease or condition, *i.e.,* arresting its development; or
(iii) relieving the disease or condition, *i.e.,* causing regression of the disease or condition.

As used herein, the terms "disease" and "condition" may be used interchangeably or may be different in that the particular malady or condition may not have a known causative agent (so that etiology has not yet been worked out) and it is therefore not yet recognized as a disease but only as an undesirable condition or syndrome, wherein a more or less specific set of symptoms have been identified by clinicians.

In the present disclosure, the term "physiologically acceptable" refers to a vehicle or diluent that does not eliminate the bioactivity and properties of the compound.

The compounds of the present disclosure or their pharmaceutically acceptable salt may contain one or more asymmetric centers and may thus give rise to enantiomers, diastereoisomers, and other stereoisomeric forms that may be defined, in terms of absolute stereochemistry, as (*R*)- or (*S*)-, or (D)- or (L)- for amino acids. The present disclosure is meant to include all such possible isomers, as well as their racemic and optically pure forms. Optically active (+) and (-), (*R*)- and (*S*)-, or (D)- and (L)- isomers may be prepared using chiral synthons or chiral reagents, or resolved using conventional techniques, such as HPLC using a chiral column. When the compounds described herein contain olefinic double bonds or other centers of geometric asymmetry, and unless specified otherwise, it is intended that the compounds include both E and Z geometric isomers. Likewise, all tautomeric forms are also intended to be included.

In the present disclosure, the term "stereoisomer" refers to a compound made up of the same atoms bonded by the same bonds but having different three-dimensional structures, which are not interchangeable. The present disclosure contemplates various stereoisomers and mixtures thereof.

### Compounds of General Formulas

The present disclosure relates to compounds represented by general formulas (A), (I), and (II), or stereoisomers, cis-trans isomers, or pharmaceutically acceptable salts thereof: 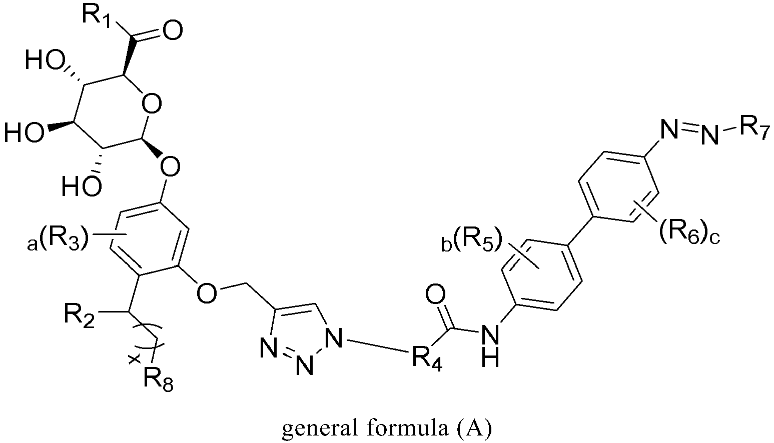 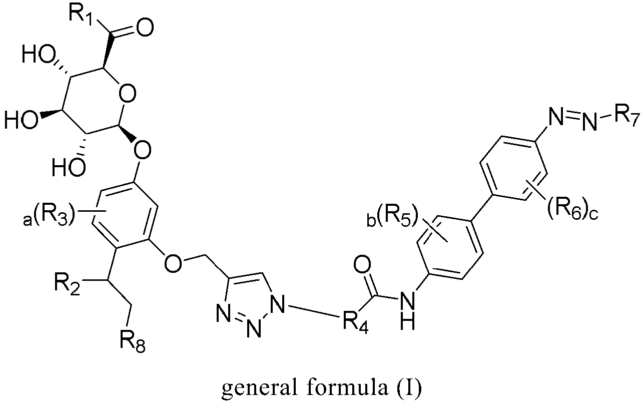 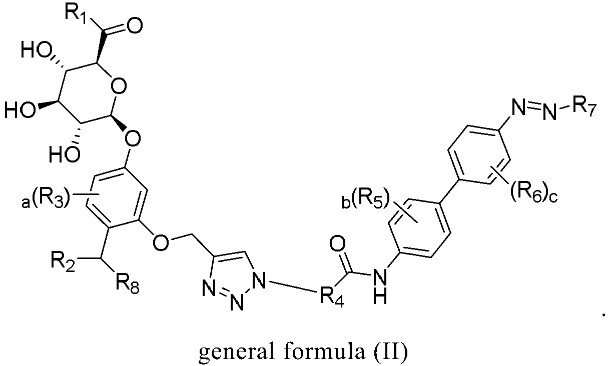 wherein
R₁ is selected from a hydroxy group, a C₁-C₁₀ hydrocarbyloxy group, an -O-alkali metal ion, an -O⁻ammonium ion, and -O⁻;
R₂ is selected from hydrogen and a C₁-C₁₀ hydrocarbyl group;
R₃ is, at each occurrence, independently selected from hydrogen, a halogen, a C₁-C₁₀ hydrocarbyl group, a C₁-C₁₀ halohydrocarbyl group, a C₁-C₁₀ hydrocarbyloxy group, a C₁-C₁₀ halohydrocarbyloxy group, a C₆-C₁₈ aryl group, a nitro group, and - NR₂₁R₂₂;
R₄ is selected from a C₁-C₁₀ hydrocarbylene group, a C₁-C₁₀ hydrocarbylenoxy group, a C₆-C₁₂ arylene group, a C₁-C₁₀ hydrocarbylene-C₆-C₁₂ arylene group, a C₁-C₁₀ hydrocarbylene-C₆-C₁₂ arylene-C₁-C₁₀ hydrocarbylene group, a (C₁-C₁₀ hydrocarbylenoxy)ₐ-C₁-C₁₀ hydrocarbylene group, a C₁-C₁₀ hydrocarbylenoxy-C₆-C₁₂ arylene group, -(CH₂)ₘ-CO-NR₂₃-(CHR₂₄)ⱼ-(CO-NR₂₅-(CHR₂₆)ₖ)ₚ-(CH₂)ₙ-, and - ((CH₂)ₘ-NR₂₃-(CHR₂₄)ⱼ)_{q}-(CH₂)ₙ-;
R₅ and R₆ are, at each occurrence, each independently selected from hydrogen, a halogen, a C₁-C₁₀ hydrocarbyl group, a C₁-C₁₀ halohydrocarbyl group, a C₁-C₁₀ hydrocarbyloxy group, a C₁-C₁₀ halohydrocarbyloxy group, and a C₆-C₁₈ aryl group;
R₇ is selected from: 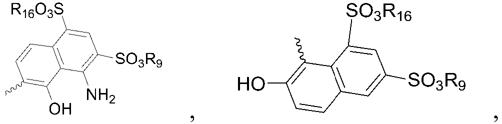 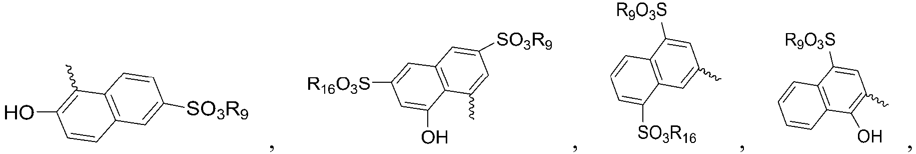 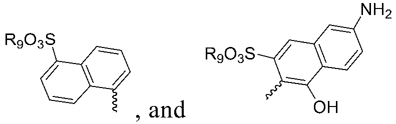
R₈ is selected from R₁₀, R₁₁, R₁₂, R₁₇, -OCO-R₁₀, -OCO-R₁₂, and -OCO-R₁₇: 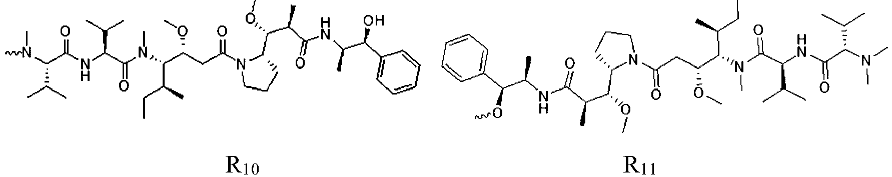 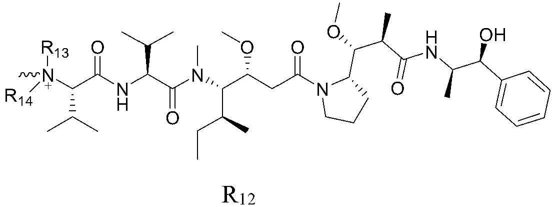 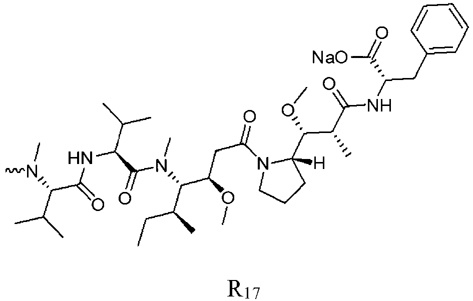
R₁₃ and R₁₄ are each independently selected from hydrogen, a C₁-C₈ hydrocarbyl group, a C₆-C₁₂ aryl group, and a C₁-C₈ hydrocarbylene-C₆-C₁₂ aryl group, and R₁₃ and R₁₄ optionally form, together with a nitrogen atom to which they are attached, a 3-17-membered nitrogen-containing heterocycle group;
R₉ and R₁₆ are each independently selected from hydrogen, an alkali metal ion, and an ammonium ion;
R₂₁ and R₂₂ are each independently selected from hydrogen and a C₁-C₆ hydrocarbyl group;
R₂₃ and R₂₅ are each independently selected from hydrogen, a C₁-C₆ hydrocarbyl group, and a C₁-C₆ hydrocarbyloxy group;
R₂₄ and R₂₆ are each independently selected from hydrogen, a C₁-C₆ hydrocarbyl group, a C₁-C₆ hydrocarbyloxy group, a C₆-C₁₂ aryl group, and a C₇-C₁₃ arylhydrocarbyl group; and
x is 0 or 1; a is an integer from 0 to 3; b and c are each independently an integer from 0 to 4; d is an integer from 0 to 4; j, k, m, n, and p are each independently an integer from 0 to 5, and q is an integer from 1 to 5;
wherein 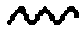 represents a linkage site.

In some embodiments, R₁ may be selected from a hydroxy group, a C₁-C₁₀ alkoxy group, -O⁻Li⁺, -O⁻Na⁺, -O⁻K⁺, -O⁻Cs⁺, -O⁻NH₄⁺, and -O⁻. For example, C₁-C₁₀ alkoxy may be, e.g., a methoxy group, an ethoxy group, a propoxy group, or a butoxy group. Where appropriate, R₁ may also be a C₂-C₁₀ hydrocarbyloxy group containing an unsaturated bond.

In some embodiments, R₂ may selected from hydrogen and a C₁-C₁₀ alkyl group. For example, C₁-C₁₀ alkyl group may be, e.g., a methyl group, an ethyl group, a n-propyl group, an isopropyl group, a n-butyl group, an isobutyl group, or a tert-butyl group.

In some embodiments, R₃ may be, at each occurrence, identical or different, and may be independently selected from hydrogen, F, Cl, Br, I, a C₁-C₁₀ alkyl group, a C₁-C₁₀ haloalkyl group, a C₁-C₁₀ alkoxy group, a C₁-C₁₀ haloalkoxy group, a C₆-C₁₈ aryl group, a nitro group, and -NR₂₁R₂₂. For example, C₁-C₁₀ alkyl group may be, e.g., a methyl group, an ethyl group, a n-propyl group, an isopropyl group, a n-butyl group, an isobutyl group, or a tert-butyl group. Correspondingly, C₁-C₁₀ haloalkyl group may be, e.g., a methyl group, an ethyl group, a n-propyl group, an isopropyl group, a n-butyl group, an isobutyl group, or a tert-butyl group substituted with one or more of fluorine, chlorine, bromine, iodine, or a combination thereof. C₁-C₁₀ alkoxy group may be, e.g., a methoxy group, an ethoxy group, a propoxy group, or a butoxy group, while C₁-C₁₀ haloalkoxy group may be, e.g., a methoxy group, an ethoxy group, a propoxy group, or a butoxy group substituted with one or more of fluorine, chlorine, bromine, iodine, or a combination thereof. C₆-C₁₈ aryl group may be, e.g., a phenyl group, a naphthyl group, or a biphenyl group. In -NR₂₁R₂₂, R₂₁ and R₂₂ are each independently selected from hydrogen and a C₁-C₆ alkyl group, such as hydrogen, a methyl group, an ethyl group, a n-propyl group, an isopropyl group, a n-butyl group, an isobutyl group, or a tert-butyl group.

In some embodiments, R₄ may be selected from a C₁-C₁₀ alkylene group, a C₁-C₁₀ alkylenoxy group, a C₆-C₁₂ arylene group, a C₁-C₁₀ alkylene-C₆-C₁₂ arylene group, a C₁-C₁₀ alkylene-C₆-C₁₂ arylene-C₁-C₁₀ alkylene group, a C₁-C₁₀ alkylenoxy-C₆-C₁₂ arylene group, -(CH₂)ₘ-CO-NR₂₃-(CHR₂₄)ⱼ-(CO-NR₂₅-(CHR₂₆)ₖ)ₚ-(CH₂)ₙ-, and - ((CH₂)ₘ-NR₂₃-(CHR₂₄)ⱼ)_{q}-(CH₂)ₙ-. For example, C₁-C₁₀ alkylene group may be, e.g., a methylene group, an ethylene group, a propylene group, a butylene group, or a tert-butylene group. C₁-C₁₀ alkylenoxy group may be, e.g., a methyleneoxy group, an ethyleneoxy group, a propyleneoxy group, or a butyleneoxy group. C₆-C₁₂ arylene group may be, e.g., a phenylene group, a naphthylene group, or a biphenylene group. The alkylene group, alkylenoxy group, and arylene group in C₁-C₁₀ alkylene-C₆-C₁₂ arylene group, C₁-C₁₀ alkylene-C₆-C₁₂ arylene-C₁-C₁₀ alkylene group, and C₁-C₁₀ alkylenoxy-C₆-C₁₂ arylene may be the above C₁-C₁₀ alkylene group, C₁-C₁₀ alkylenoxy group, and C₆-C₁₂ arylene group. In -(CH₂)ₘ-CO-NR₂₃-(CHR₂₄)ⱼ-(CO-NR₂₅-(CHR₂₆)ₖ)ₚ-(CH₂)ₙ- and -((CH₂)ₘ-NR₂₃-(CHR₂₄)ⱼ)_{q}-(CH₂)ₙ-, R₂₃ and R₂₅ are each independently selected from hydrogen, a C₁-C₆ alkyl group, and a C₁-C₆ alkoxy group, and R₂₄ and R₂₆ are each independently selected from hydrogen, a C₁-C₆ alkyl group, a C₁-C₆ alkoxy group, a C₆-C₁₂ aryl group, and a C₇-C₁₃ arylalkyl group, for example, C₁-C₆ alkyl group may be, e.g., a methyl group, an ethyl group, a n-propyl group, an isopropyl group, a n-butyl group, a isobutyl group, or a tert-butyl group, C₁-C₆ alkoxy group may be, e.g., a methoxy group, an ethoxy group, a propoxy group, or a butoxy group, C₆-C₁₂ aryl group may be a phenyl group, a naphthyl group, and a biphenyl group, and C₇-C₁₃ arylalkyl group may be, e.g., a benzyl group or a phenethyl group.

In some embodiments, R₅ and R₆ may be, at each occurrence, identical or different, and may each be independently selected from hydrogen, F, Cl, Br, I, a C₁-C₁₀ alkyl group, a C₁-C₁₀ haloalkyl group, a C₁-C₁₀ alkoxy group, a C₁-C₁₀ haloalkoxy group, and a C₆-C₁₈ aryl group. C₁-C₁₀ alkyl group may be, e.g., a methyl group, an ethyl group, a n-propyl group, an isopropyl group, a n-butyl group, an isobutyl group, or a tert-butyl group. Correspondingly, C₁-C₁₀ haloalkyl group may be, e.g., a methyl group, an ethyl group, a n-propyl group, an isopropyl group, a n-butyl group, an isobutyl group, or a tert-butyl group substituted with one or more of fluorine, chlorine, bromine, iodine, or a combination thereof. C₁-C₁₀ alkoxy group may be, e.g., a methoxy group, an ethoxy group, a propoxy group, or a butoxy group, while C₁-C₁₀ haloalkoxy group may be, e.g., a methoxy group, an ethoxy group, a propoxy group, or a butoxy group substituted with one or more of fluorine, chlorine, bromine, iodine, or a combination thereof. C₆-C₁₈ aryl group may be, e.g., a phenyl group, a naphthyl group, or a biphenyl group.

In some embodiments, R₇ may be selected from: 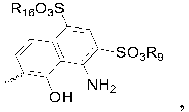 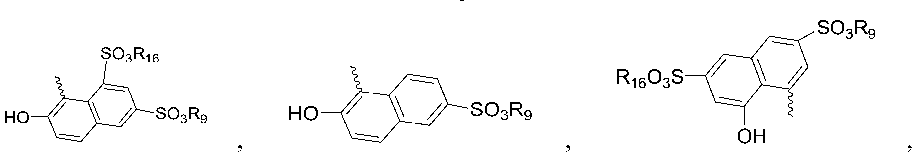 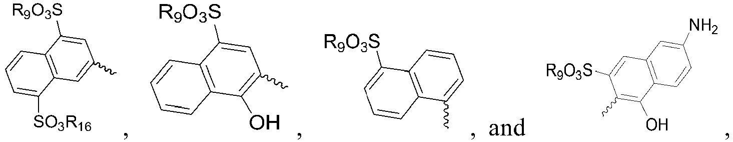 wherein R₉ and R₁₆ may each be independently selected from hydrogen, Li⁺, Na⁺, K⁺, Cs⁺, and NH₄⁺.

In some embodiments, R₈ may be selected from R₁₀, R₁₁, R₁₂, R₁₇, -OCO-R₁₀, -OCO-R₁₂, and -OCO-R₁₇: 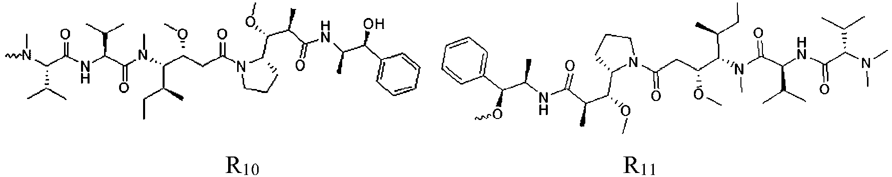 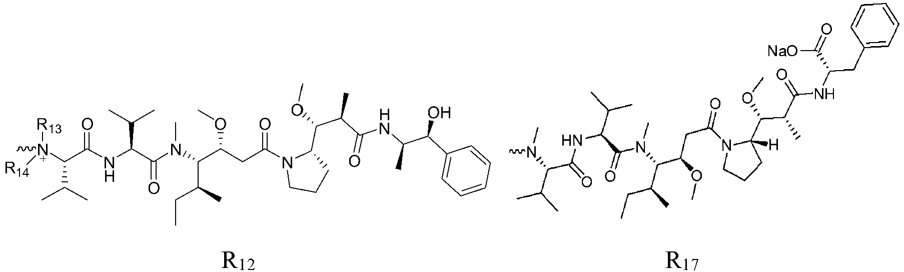 wherein R₁₃ and R₁₄ are each independently selected from hydrogen, a C₁-C₈ hydrocarbyl group, a C₆-C₁₂ aryl group, and a C₁-C₈ hydrocarbylene-C₆-C₁₂ aryl group, for example, hydrogen, a C₁-C₈ alkyl group, a C₆-C₁₂ aryl group, and a C₁-C₈ alkylene-C₆-C₁₂ aryl. More specifically, the R₁₃ and the R₁₄ are each independently selected from hydrogen, a methyl group, an ethyl group, an isopropyl group, a butyl group, a phenyl group, a benzyl group, and a phenethyl group.

In addition, R₁₃ and R₁₄ may also form, together with the nitrogen atom to which they are attached, a 3-17-membered nitrogen-containing heterocycle group; for example, a 3-15-membered, 3-12-membered, 3-10-membered, 3-6-membered, or 3-5-membered nitrogen-containing heterocycle. If a ring is formed, then R₁₂ is: 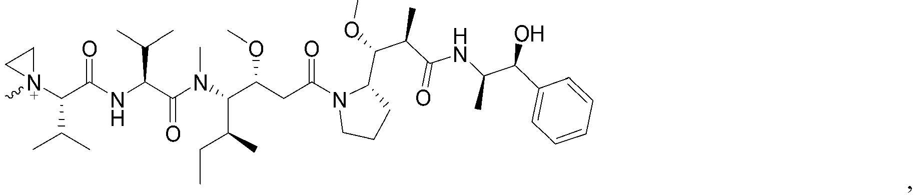 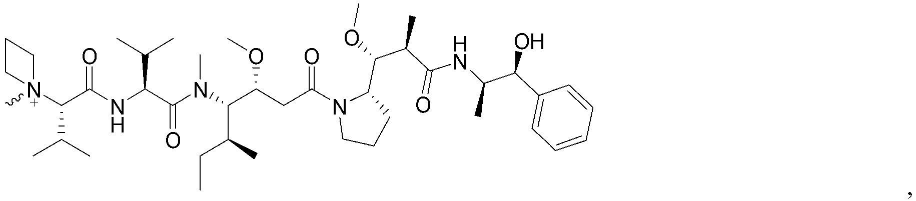 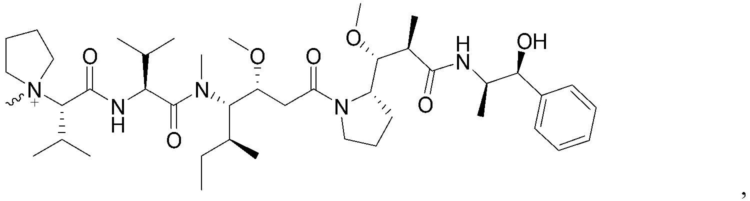 or

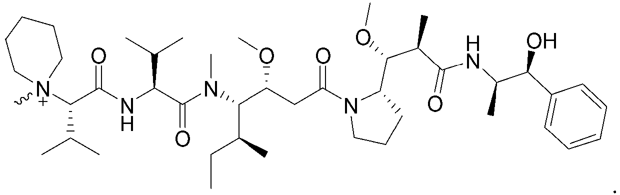

**Table 1**

| | | |
|---|---|---|
| 1 | 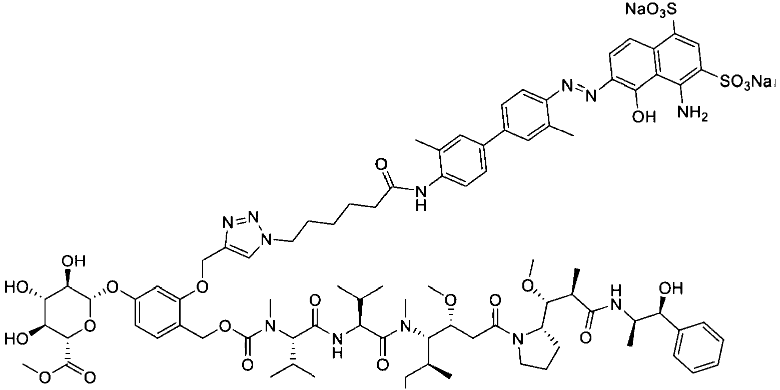 | sodium 4-amino-6-((E)-(4'-(6-(4-((2-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-(((2S,3R,4S,5S,6S)-3,4,5-trihydroxy-6-(methoxycarbonyl)tetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate |
| 2 | 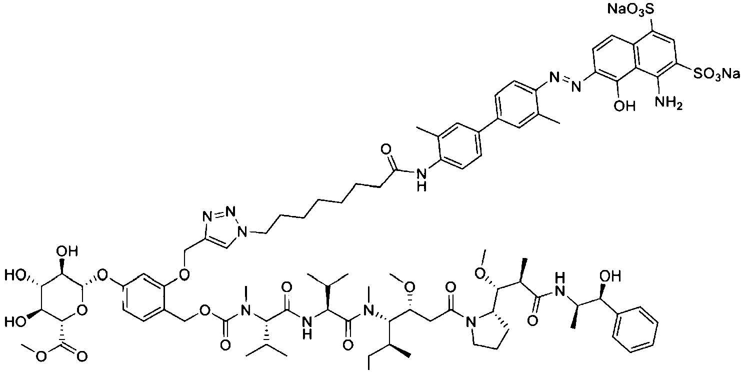 | sodium 4-amino-6-((E)-(4'-(8-(4-((2-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-(((2S,3R,4S,5S,6S)-3,4,5-trihydroxy-6-(methoxycarbonyl)tetrahydro-2H-pyran-2-yl)oxy)phenoxy)methyl)-1H-1,2,3-triazol-1-yl)octanamido)-3,3'-dimethyl-[1,1'-biphenyl]-4-yl)diazenyl)-5-hydroxynaphthalene-1,3-disulfonate |
| 3 | 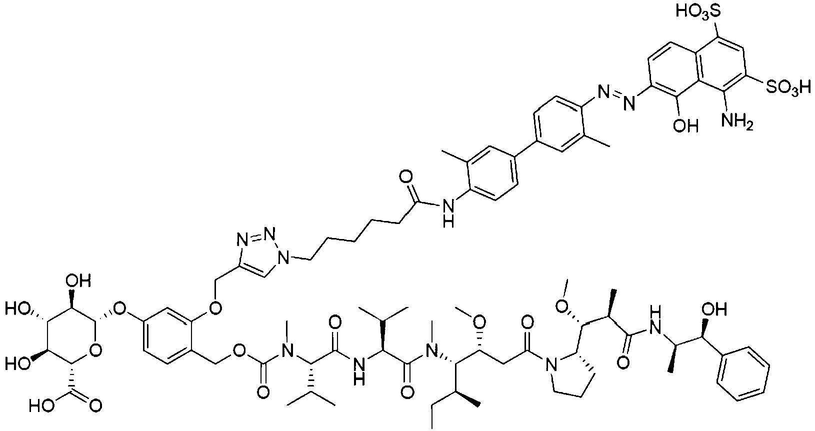 | (2S,3S,4S,5R,6S)-6-(3-((1-(6-((4'-((E)-(8-amino-1-hydroxy-5,7-disulfonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylic acid |
| 4 | 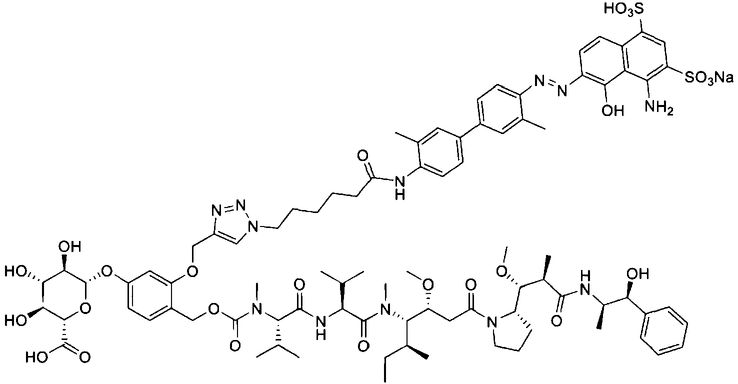 | sodium 1-amino-7-((E)-(4'-(6-(4-((2-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-(((2S,3R,4S,5S,6S)-6-carboxy-3,4,5-trihydroxytetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-8-hydroxy-4-sulfonaphthalene-2-sulfonate |
| 5 | 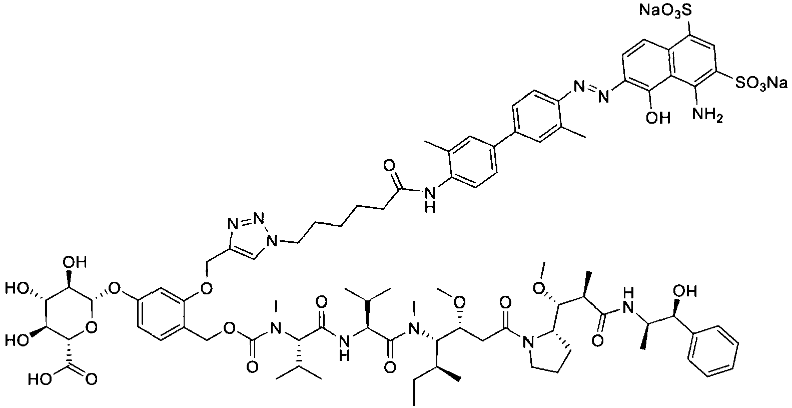 | sodium 4-amino-6-((E)-(4'-(6-(4-((2-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-(((2S,3R,4S,5S,6S)-6-carboxy-3,4,5-trihydroxytetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate |
| 6 | 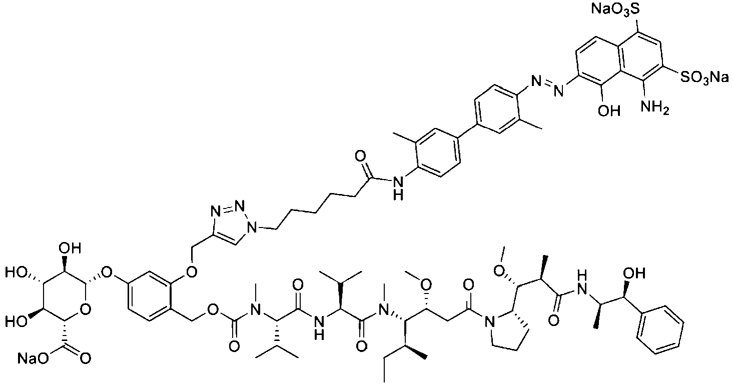 | sodium (2S,3S,4S,5R,6S)-6-(3-((1-(6-((4'-((E)-(8-amino-1-hydroxy-5,7-disulfonatonaphthalen-2-yl) diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 7 | 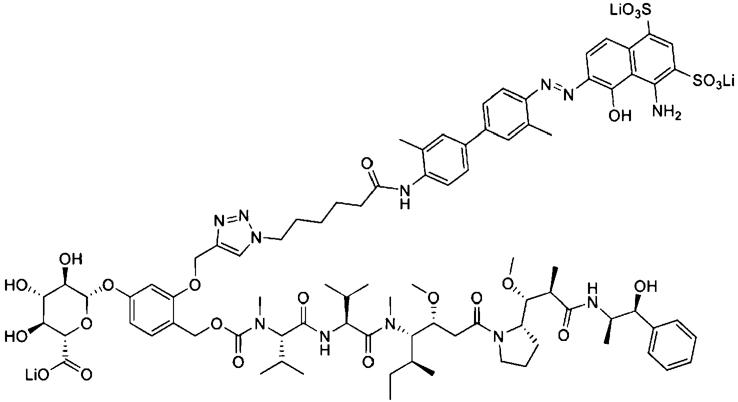 | lithium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 8 | 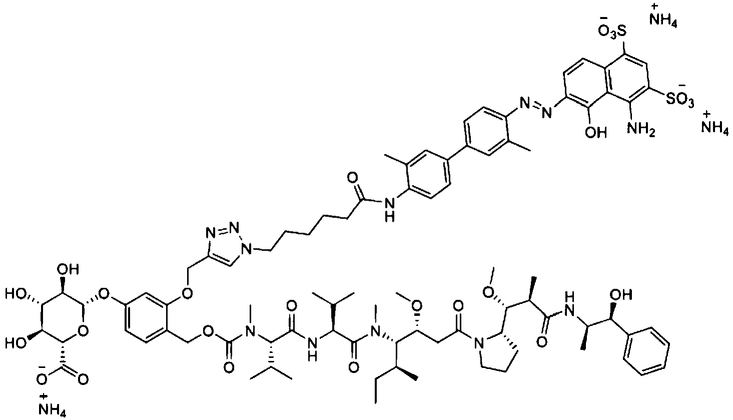 | ammonium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 9 | 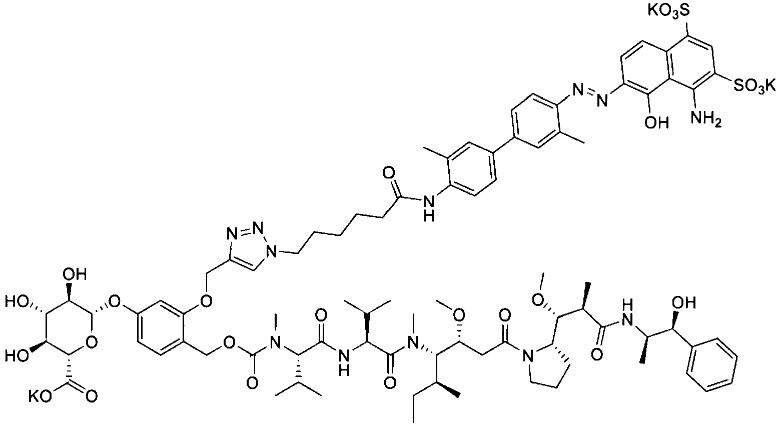 | potassium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 10 | 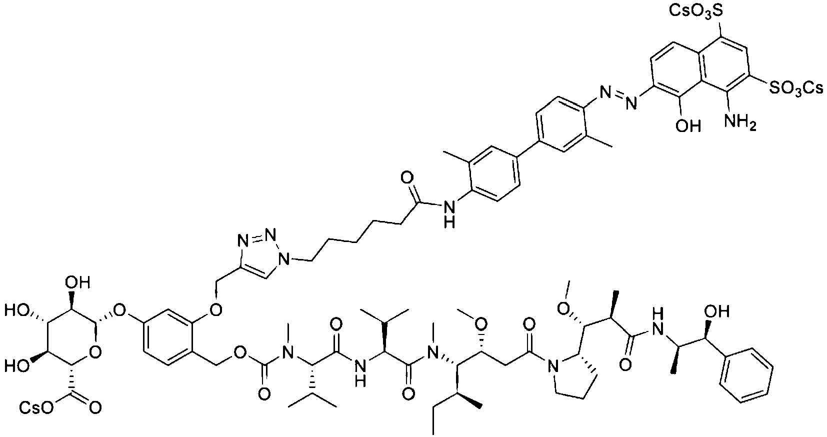 | cesium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,118,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 11 | 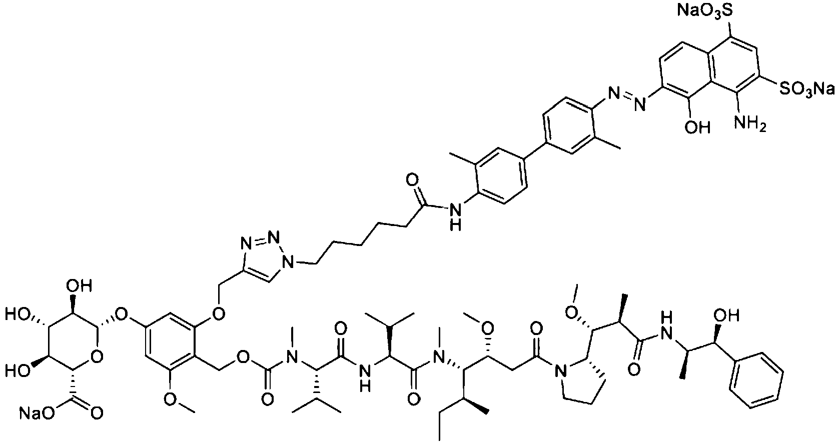 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-methoxyphenoxy)-3,4,5-trihydroxytetrahydro-2H-pyran-2-carboxylate |
| 12 | 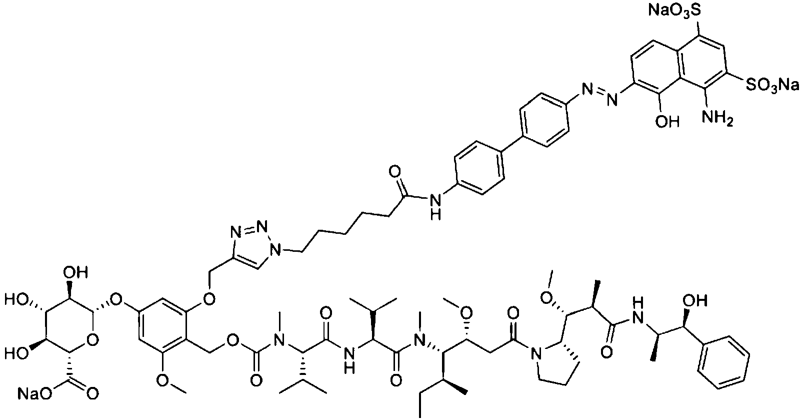 | sodium (2S,3S,4S,5R,6S)-6-(3-((1-(6-((4'-((E)-(8-amino-1-hydroxy-5,7-disulfonatonaphthalen-2-yl)diazenyl)-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-methoxyphenoxy)-3,4,5-trihydroxytetrahydro-2H-pyran-2-carboxylate |
| 13 | 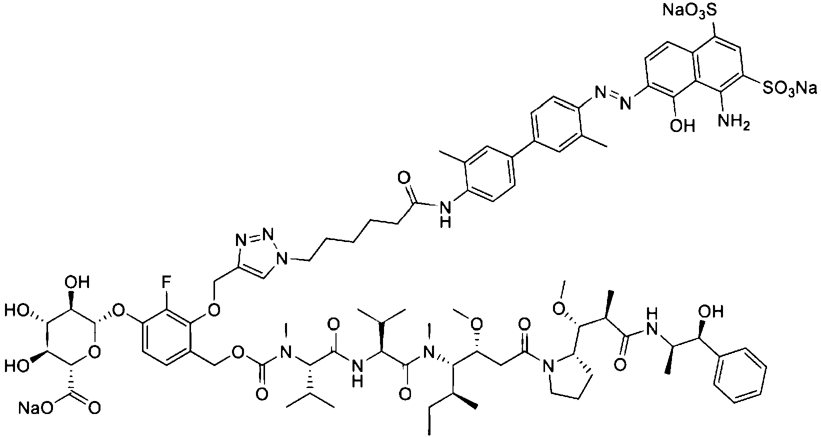 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-fluorophenoxy)-3,4,5-trihydroxytetrahydro-2H-pyran-2-carboxylate |
| 14 | 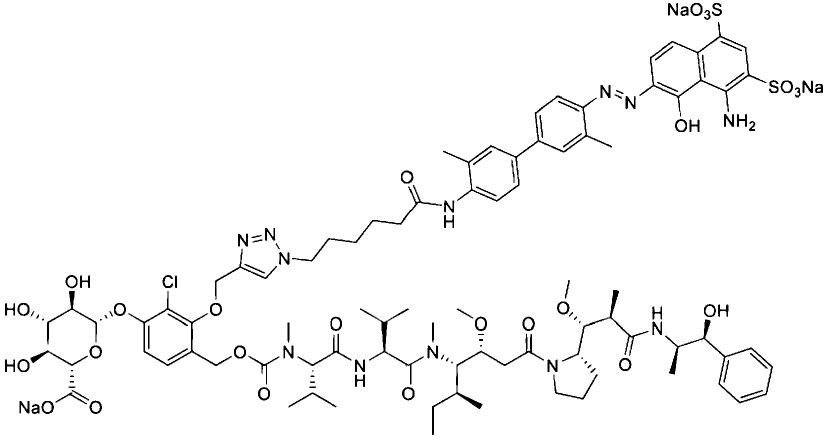 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-chlorophenoxy)-3,4,5-trihydroxytetrahydro-2H-pyran-2-carboxylate |
| 15 | 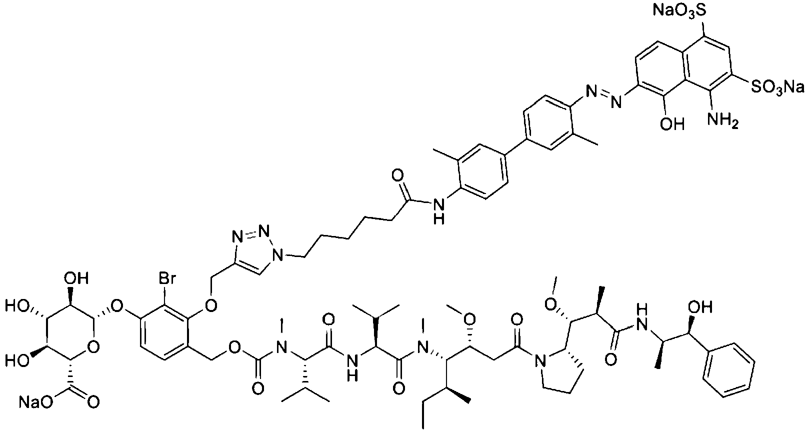 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-2-bromo-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 16 | 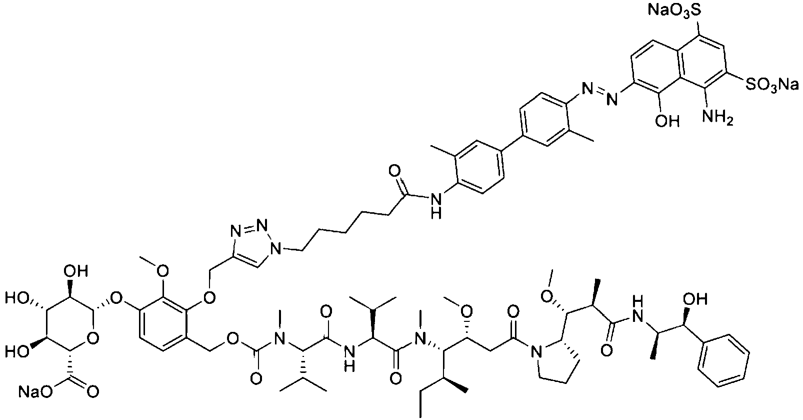 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-methoxyphenoxy)-3,4,5-trihydroxytetrahydro-2H-pyran-2-carboxylate |
| 17 | 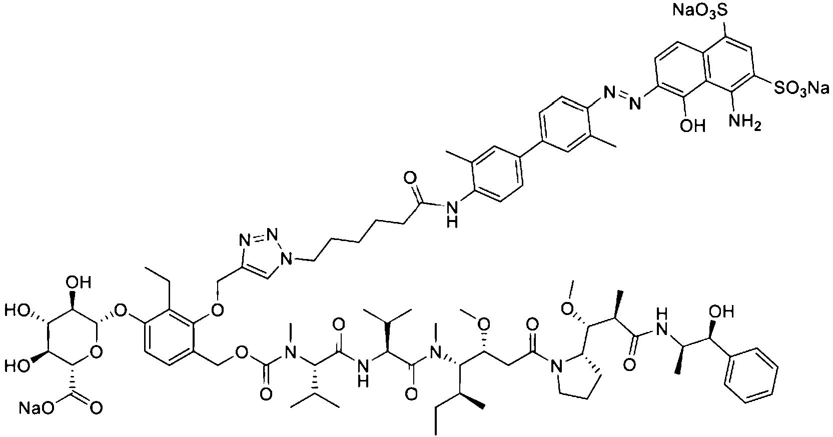 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-ethylphenoxy)-3,4,5-trihydroxytetrahydro-2H-pyran-2-carboxylate |
| 18 | 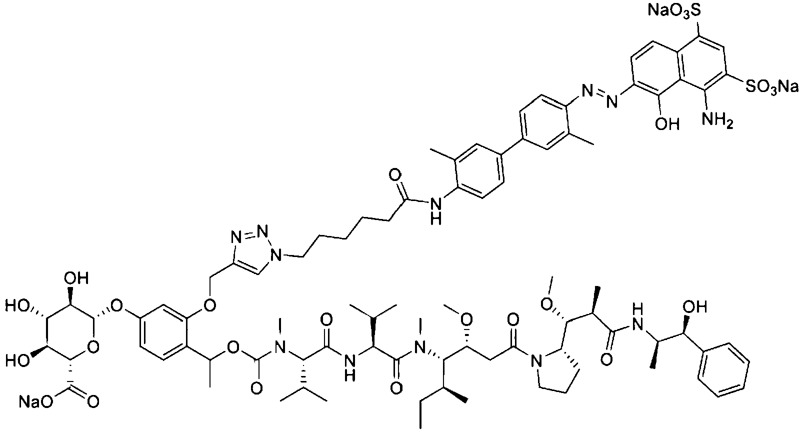 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3R,4S,7S,10S)-4-((S)-sec-butyl)-3-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-7,10-diisopropyl-5,11-dimethyl-6,9,12-trioxo-2,13-dioxa-5,8,11-triazapentadecan-14-yl)phenoxy)-3,4,5-trihydroxytetrahydro-2H-pyran-2-carboxylate |
| 19 | 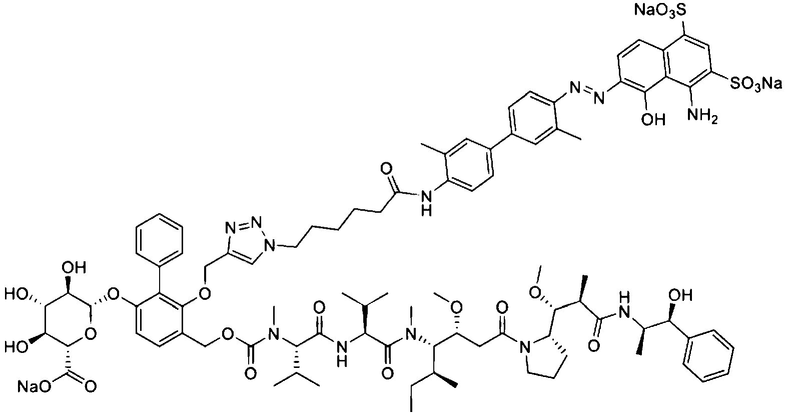 | sodium (2S,3S,4S,5R,6S)-6-((6-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-5-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-[1,1'-biphenyl]-2-yl)oxy)-3,4,5-trihydroxytetrahydro-2H-pyran-2-carboxylate |
| 20 | 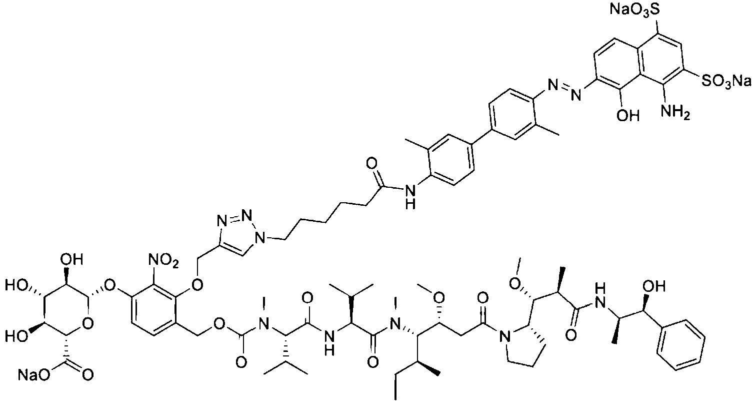 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-nitrophenoxy)-3,4,5-trihydroxytetrahydro-2H-pyran-2-carboxylate |
| 21 | 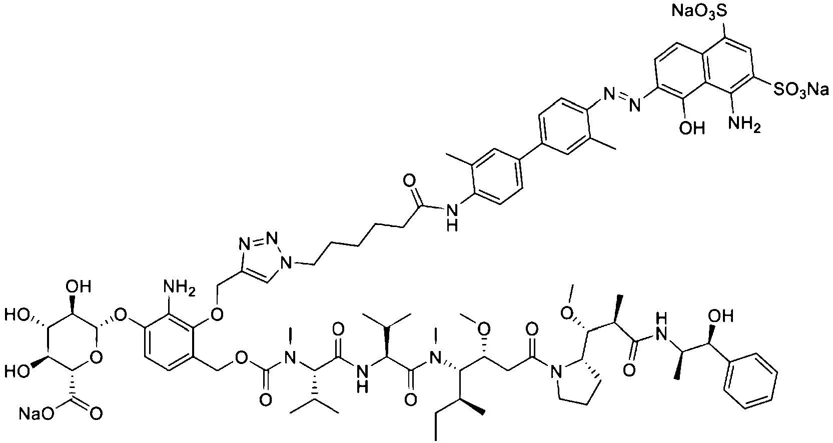 | sodium (2S,3S,4S,5R,6S)-6-(2-amino-3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 22 | 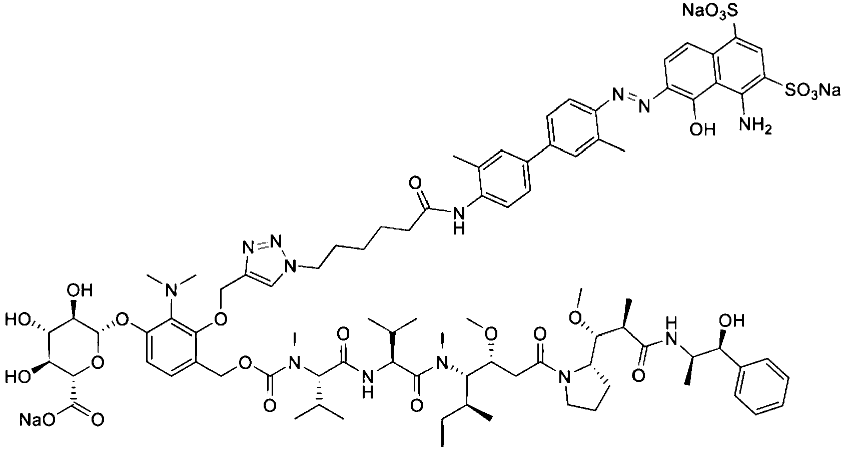 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-(dimethylamino)phenoxy)-3,4,5-trihydroxytetrahydro-2H-pyran-2-carboxylate |
| 23 | 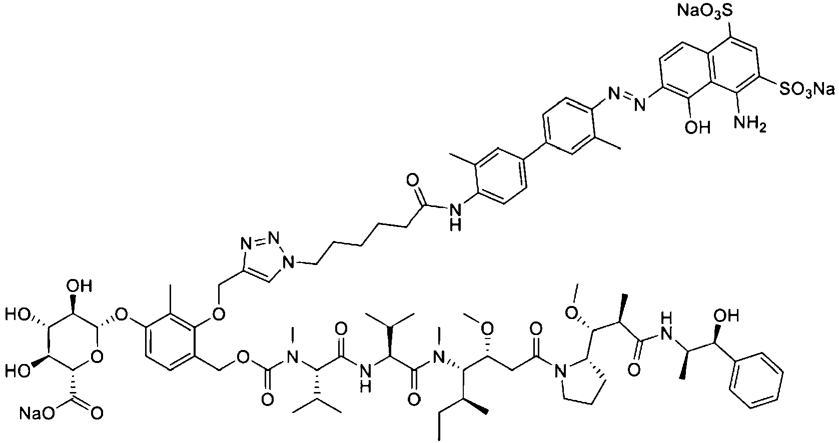 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-methylphenoxy)-3,4,5-trihydroxytetrahydro-2H-pyran-2-carboxylate |
| 24 | 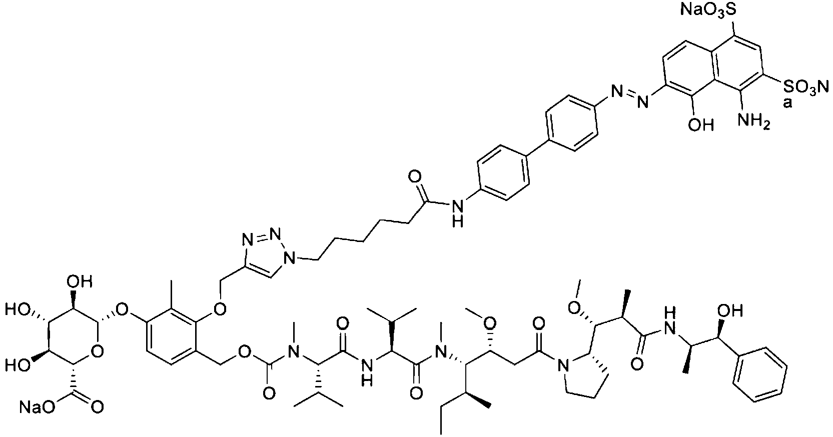 | sodium (2S,3S,4S,5R,6S)-6-(3-((1-(6-((4'-((E)-(8-amino-1-hydroxy-5,7-disulfonatonaphthalen-2-yl)diazenyl)-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-methylphenoxy)-3,4,5-trihydroxytetrahydro-2H-pyran-2-carboxylate |
| 25 | 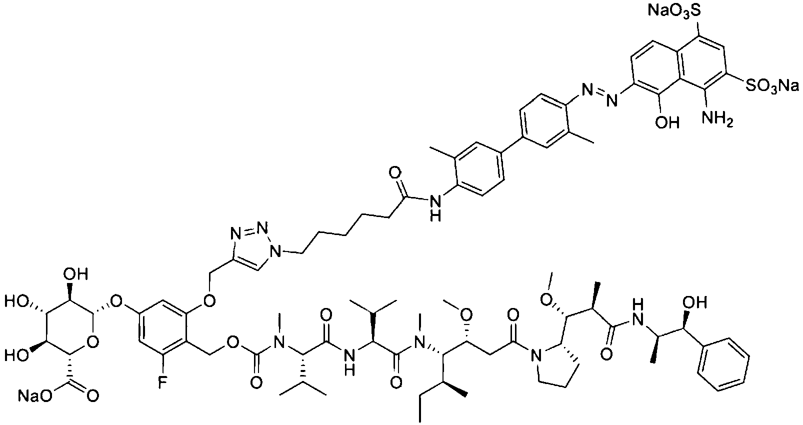 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-fluorophenoxy)-3,4,5-trihydroxytetrahydro-2H-pyran-2-carboxylate |
| 26 | 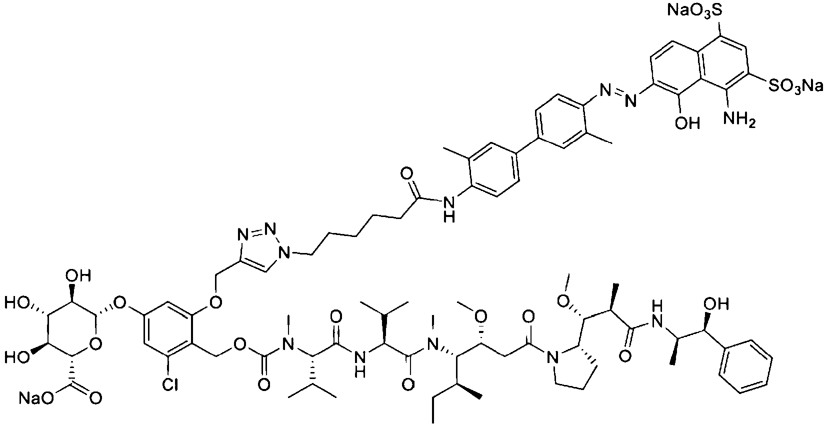 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-chlorophenoxy)-3,4,5-trihydroxytetrahydro-2H-pyran-2-carboxylate |
| 27 | 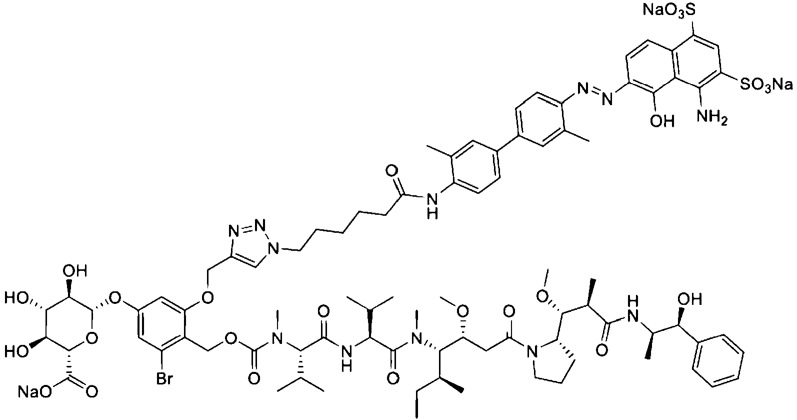 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-5-bromo-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 28 | 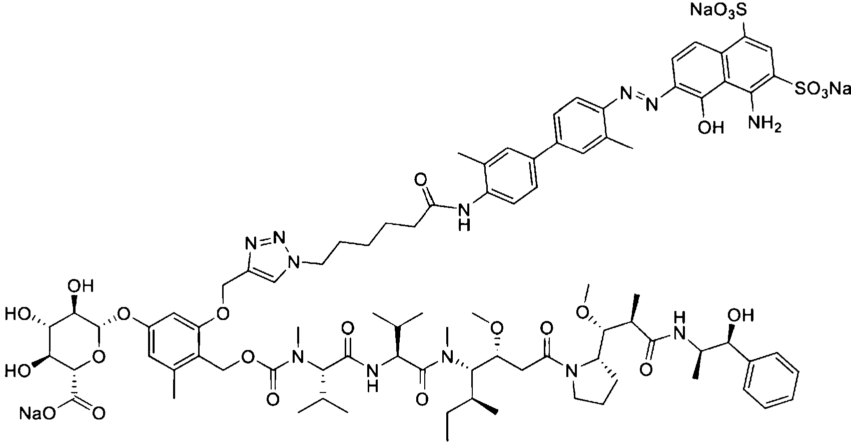 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-methylphenoxy)-3,4,5-trihydroxytetrahydro-2H-pyran-2-carboxylate |
| 29 | 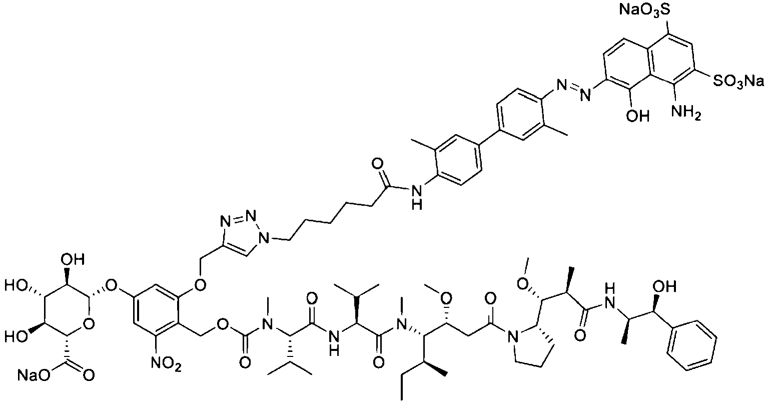 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-nitrophenoxy)-3,4,5-trihydroxytetrahydro-2H-pyran-2-carboxylate |
| 30 | 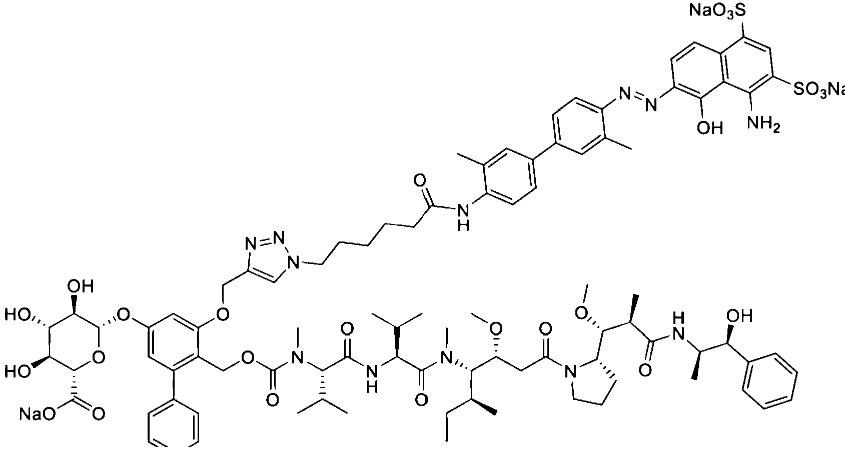 | sodium (2S,3S,4S,5R,6S)-6-((5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-6-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-[1,1'-biphenyl]-3-yl)oxy)-3,4,5-trihydroxytetrahydro-2H-pyran-2-carboxylate |
| 31 | 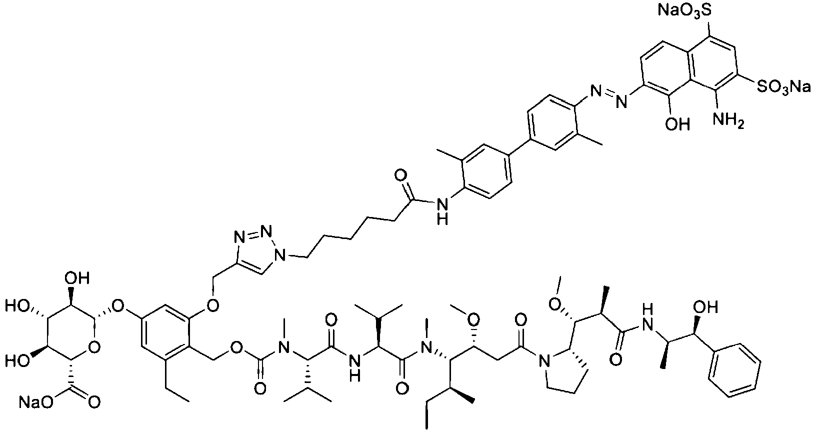 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-ethylphenoxy)-3,4,5-trihydroxytetrahydro-2H-pyran-2-carboxylate |
| 32 | 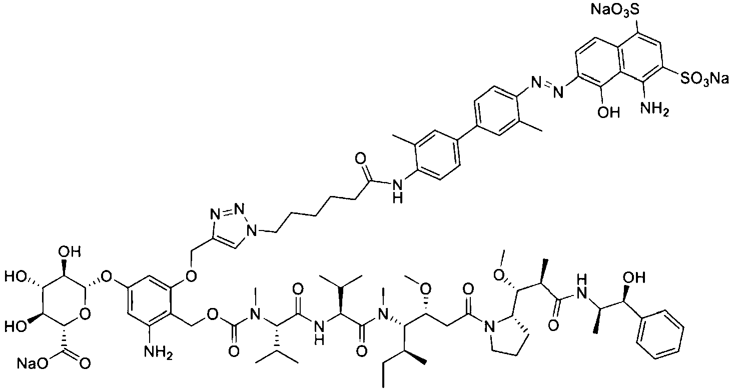 | sodium (2S,3S,4S,5R,6S)-6-(3-amino-5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 33 | 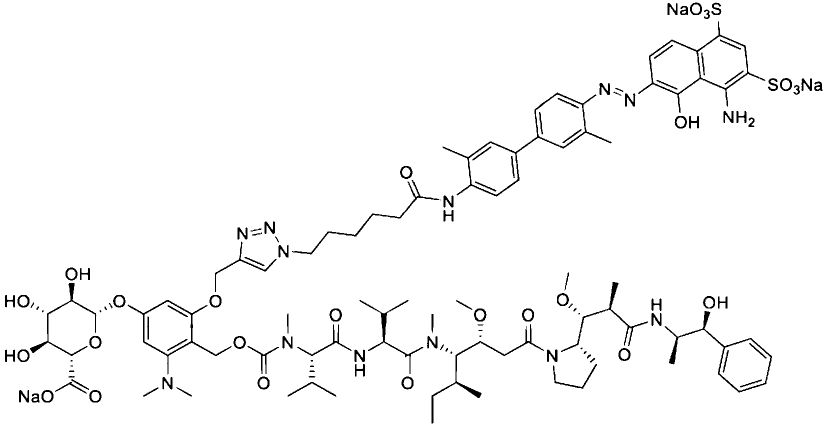 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-(dimethylamino)phenoxy)-3,4,5-trihydroxytetrahydro-2H-pyran-2-carboxylate |
| 34 | 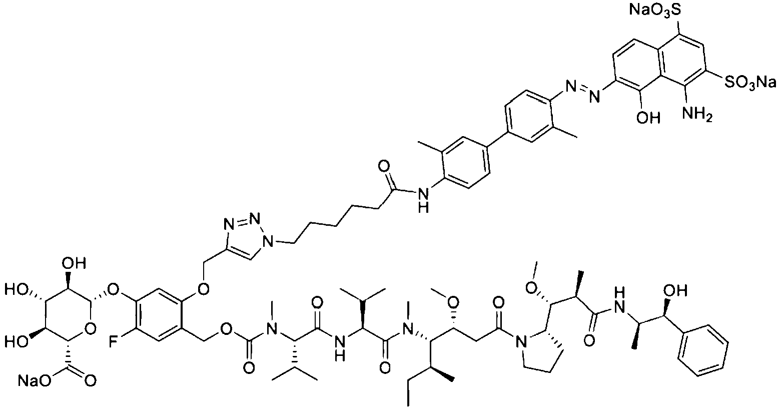 | sodium (2S,3S,4S,5R,6S)-6-(5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-fluorophenoxy)-3,4,5-trihydroxytetrahydro-2H-pyran-2-carboxylate |
| 35 | 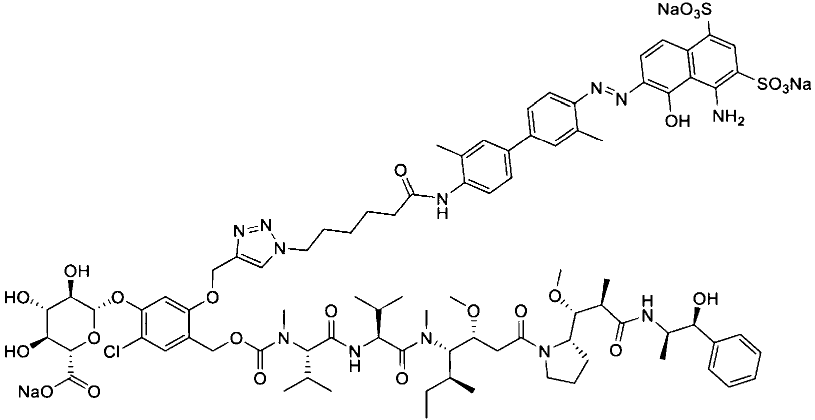 | sodium (2S,3S,4S,5R,6S)-6-(5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-chlorophenoxy)-3,4,5-trihydroxytetrahydro-2H-pyran-2-carboxylate |
| 36 | 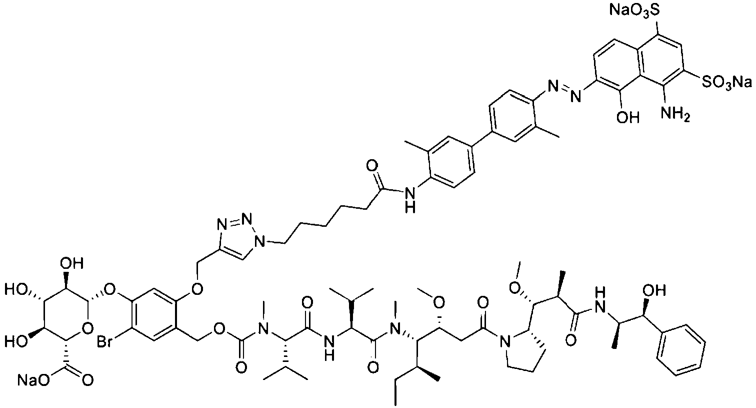 | sodium (2S,3S,4S,5R,6S)-6-(5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-2-bromo-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 37 | 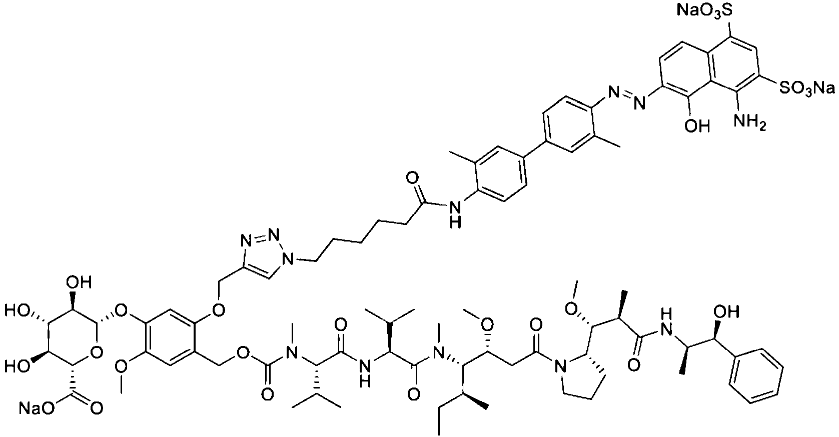 | sodium (2S,3S,4S,5R,6S)-6-(5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-methoxyphenoxy)-3,4,5-trihydroxytetrahydro-2H-pyran-2-carboxylate |
| 38 | 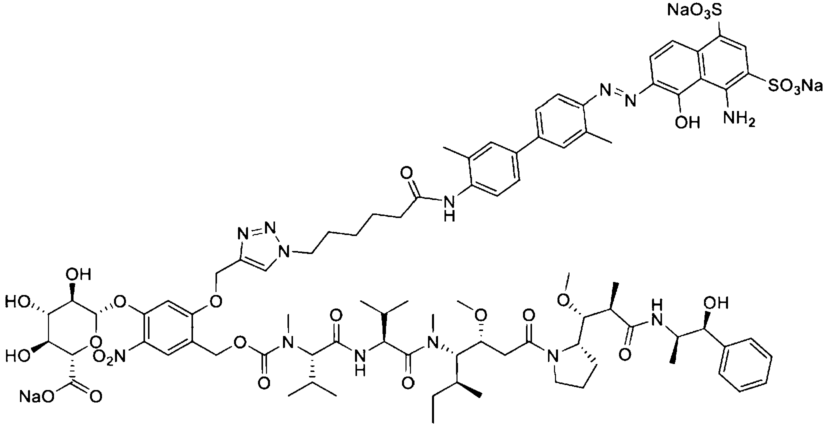 | sodium (2S,3S,4S,5R,6S)-6-(5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-nitrophenoxy)-3,4,5-trihydroxytetrahydro-2H-pyran-2-carboxylate |
| 39 | 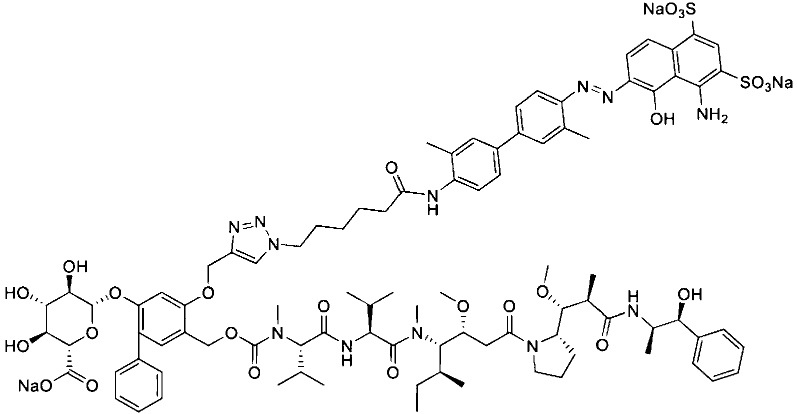 | sodium (2S,3S,4S,5R,6S)-6-((4-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-5-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-[1,1'-biphenyl]-2-yl)oxy)-3,4,5-trihydroxytetrahydro-2H-pyran-2-carboxylate |
| 40 | 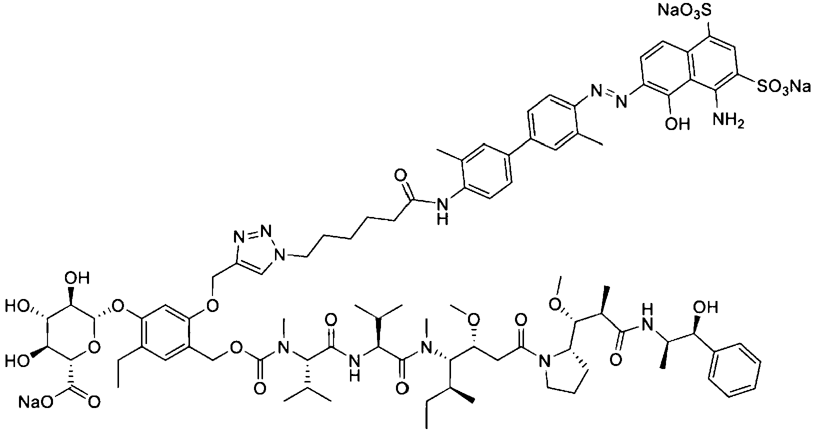 | sodium (2S,3S,4S,5R,6S)-6-(5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-ethylphenoxy)-3,4,5-trihydroxytetrahydro-2H-pyran-2-carboxylate |
| 41 | 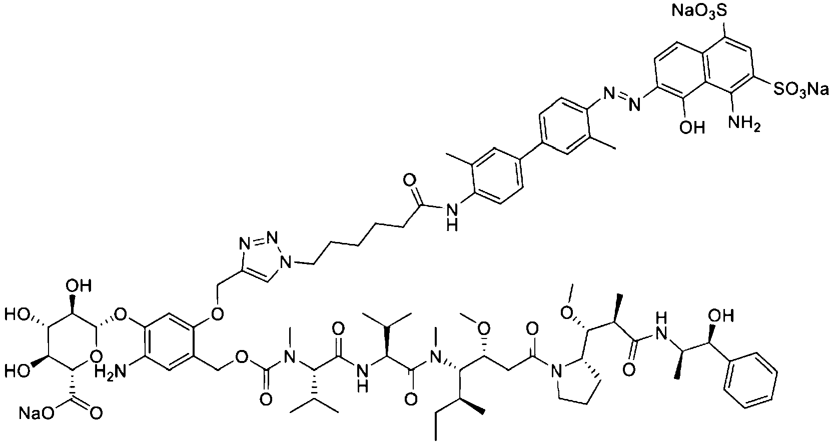 | sodium (2S,3S,4S,5R,6S)-6-(2-amino-5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 42 | 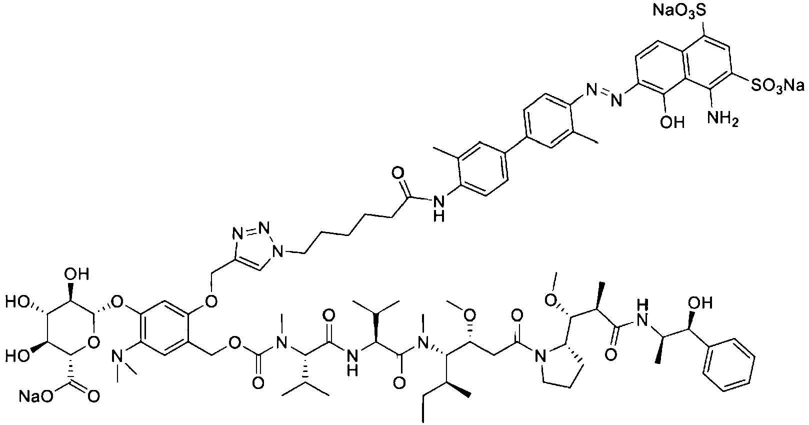 | sodium (2S,3S,4S,5R,6S)-6-(5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-(dimethylamino)phenoxy)-3,4,5-trihydroxytetrahydro-2H-pyran-2-carboxylate |
| 43 | 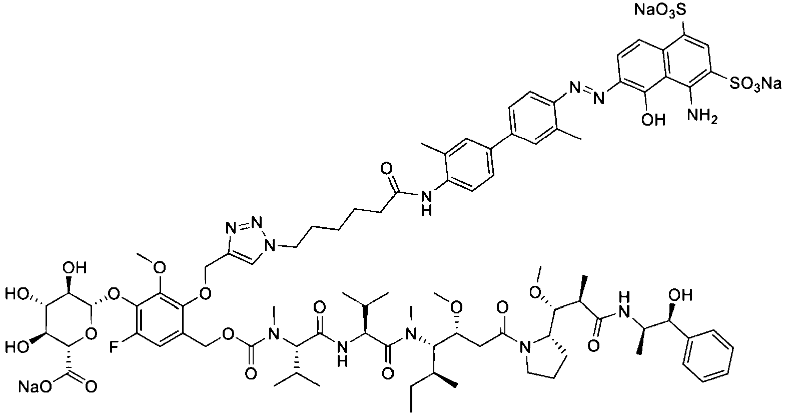 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-6-fluoro-2-methoxyphenoxy)-3,4,5-trihydroxytetrahydro-2H-pyran-2-carboxylate |
| 44 | 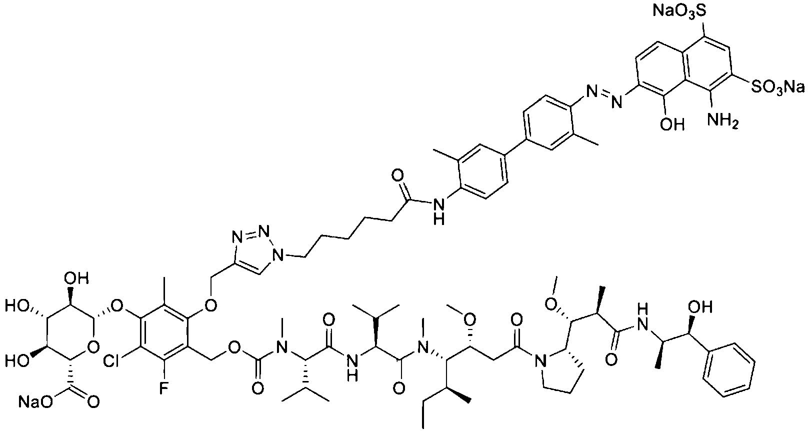 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-6-chloro-5-fluoro-2-methylphenoxy)-3,4,5-trihydroxytetrahydro-2H-pyran-2-carboxylate |
| 45 | 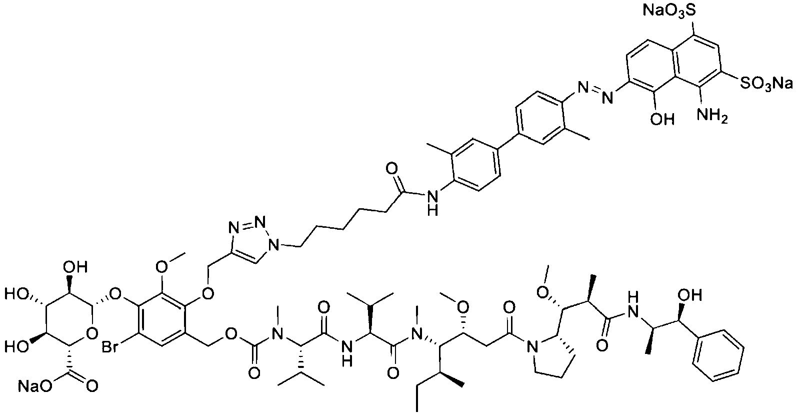 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-6-bromo-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-methoxyphenoxy)-3,4,5-trihydroxytetrahydro-2H-pyran-2-carboxylate |
| 46 | 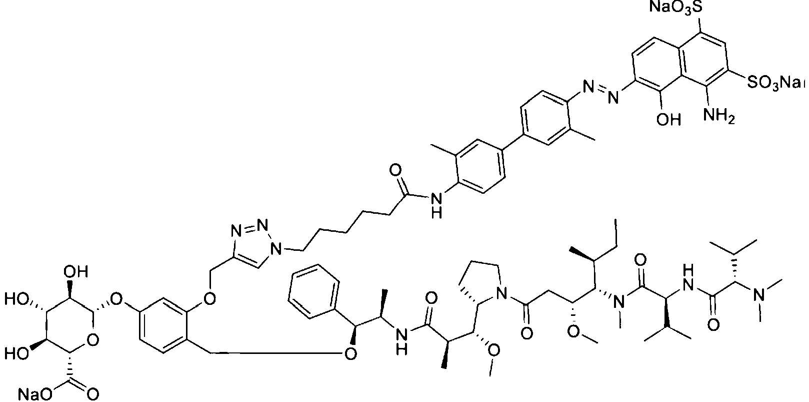 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-(((1S,2R)-2-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-1-phenylpropoxy)methyl)phenoxy)-3,4,5-trihydroxytetrahydro-2H-pyran-2-carboxylate |
| 47 | 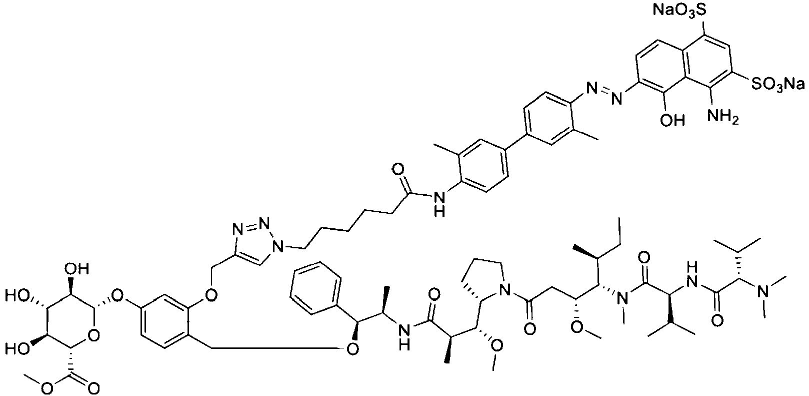 | sodium 4-amino-6-((E)-(4'-(6-(4-((2-(((1S,2R)-2-((2R,3R)-3-((S)-1-((3R,4S,5S)-4-((S)-2-( (S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-1-phenylpropoxy)methyl)-5-(((2S,3R,4S,5S,6S)-3,4,5-trihydroxy-6-(methoxycarbonyl)tetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate |
| 48 | 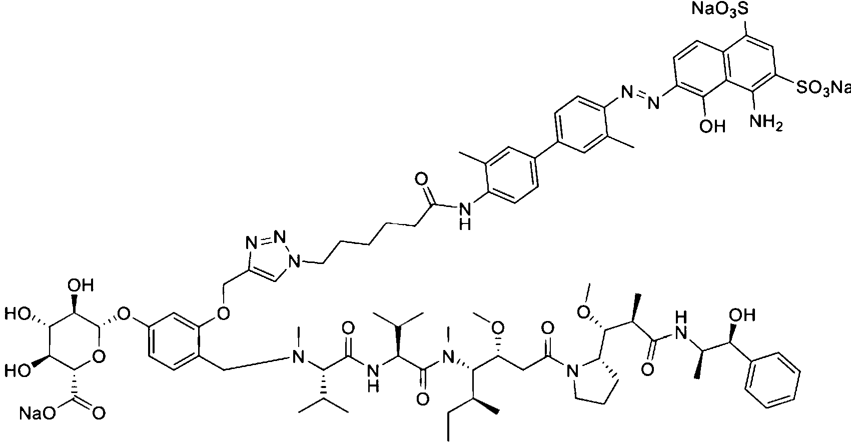 II | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecyl)phenoxy)-3,4,5-trihydroxytetrahydro-2H-pyran-2-carboxylate |
| 49 | 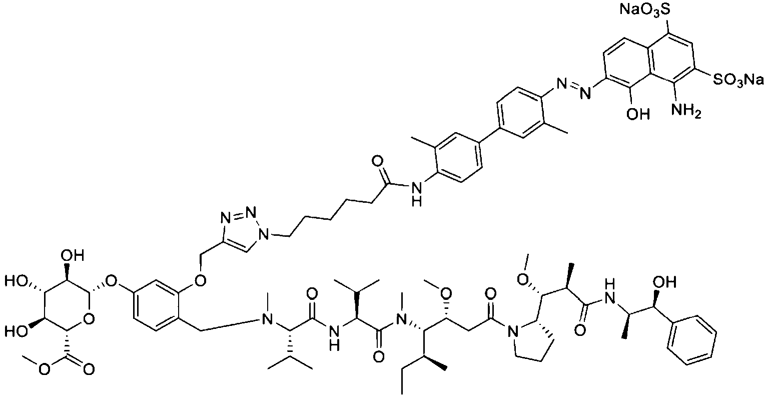 | sodium 4-amino-6-((E)-(4'-(6-(4-((2-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecyl)-5-(((2S,3R,4S,5 S,6S)-3,4,5-trihydroxy-6-(methoxycarbonyl)tetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate |
| 50 | 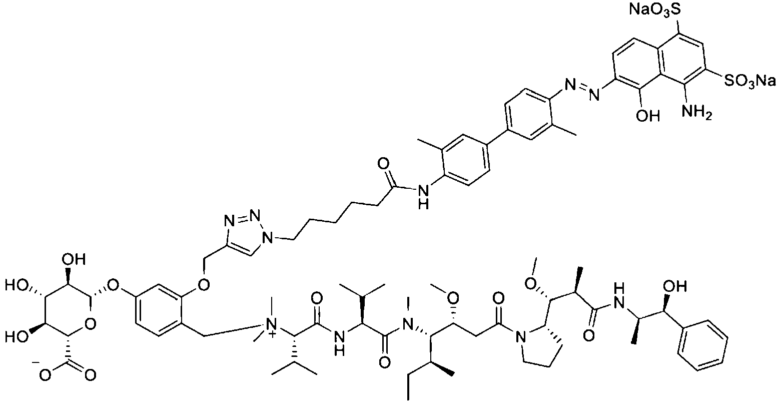 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate |
| 51 | 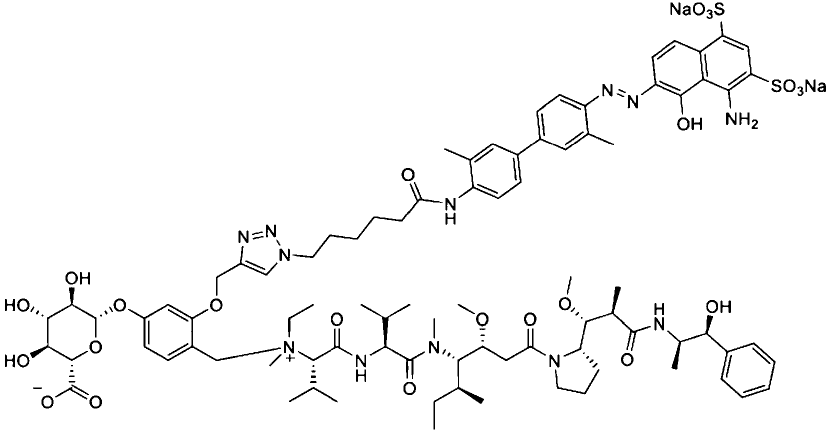 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-2-ethyl-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate |
| 52 | 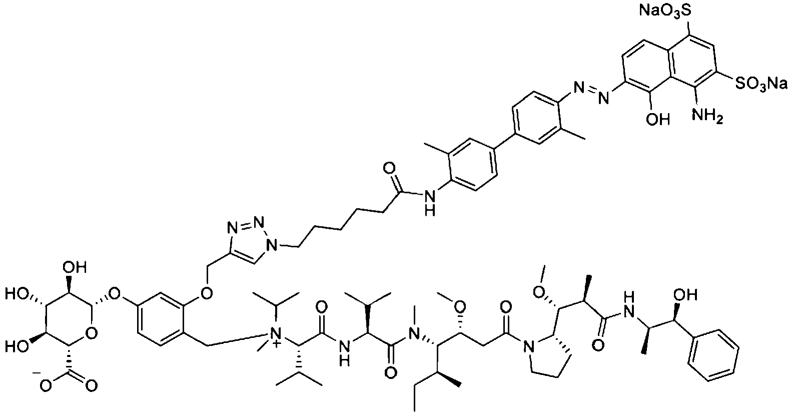 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-2,3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate |
| 53 | 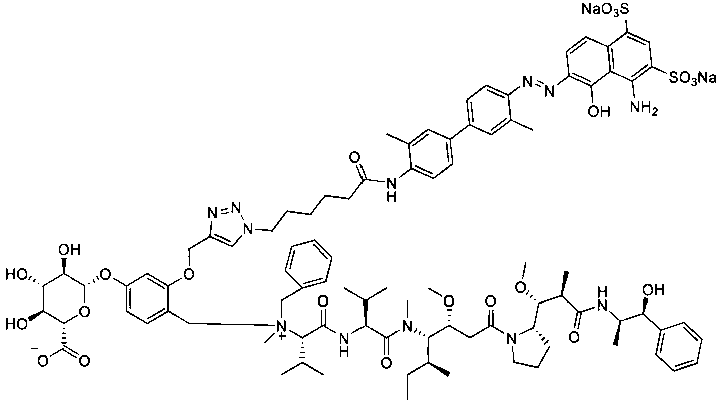 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-2-benzyl-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate |
| 54 | 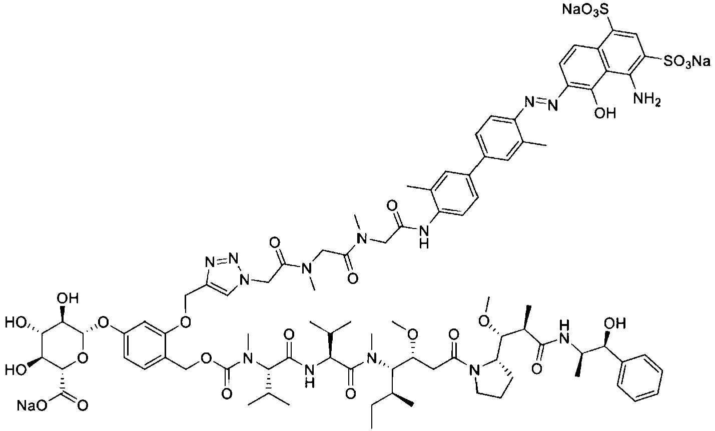 | sodium (2S,3S,4S,5R,6S)-6-(3-((1-(2-((2-((2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethyl)(methyl)amino)-2-oxoethyl)(methyl)amino)-2-oxoethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 55 | 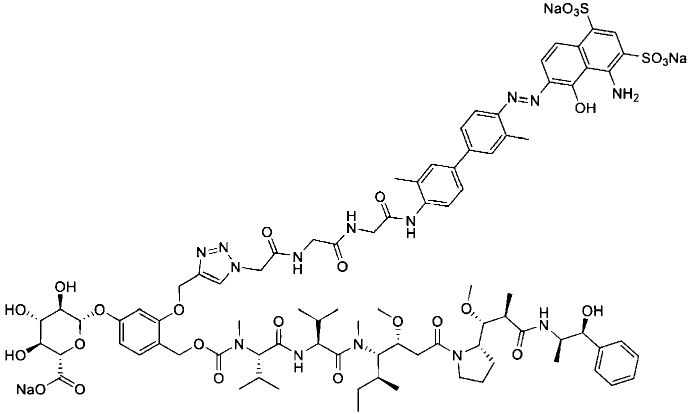 | sodium (2S,3S,4S,5R,6S)-6-(3-((1-(2-((2-((2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethyl)amino)-2-oxoethyl)amino)-2-oxoethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 56 | 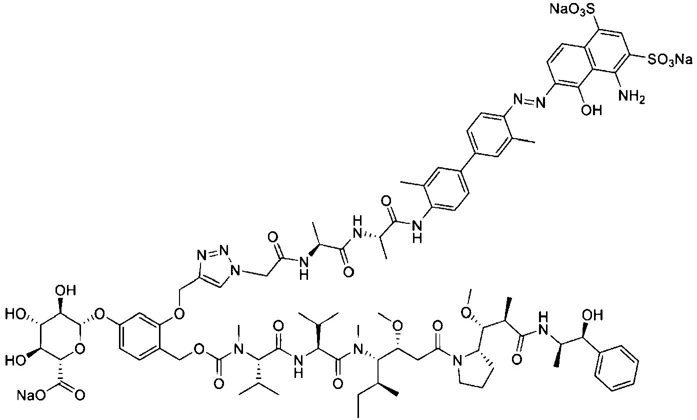 | sodium (2S,3S,4S,5R,6S)-6-(3-((1-(2-(((S)-1-(((S)-1-((4'-((E)-(8-amino-1-hydroxy-5,7-disulfonatonaphthalen-2-yl)diazenyl)-3,3'-dimethyl-[1,1'-biphenyl]-4-yl)amino)-1-oxopropan-2-yl)amino)-1-oxopropan-2-yl)amino)-2-oxoethyl)-1 H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 57 | 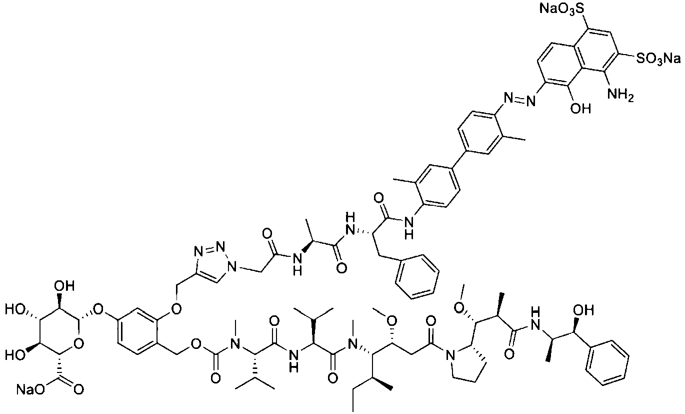 | sodium (2S,3S,4S,5R,6S)-6-(3-((1-(2-(((S)-1-(((S)-1-((4'-((E)-(8-amino-1-hydroxy-5,7-disulfonatonaphthalen-2-yl)diazenyl)-3,3'-dimethyl-[1,1'-biphenyl]-4-yl)amino)-1-oxo-3-phenylpropan-2-yl)amino)-1-oxopropan-2-yl)amino)-2-oxoethyl)-1 H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 58 | 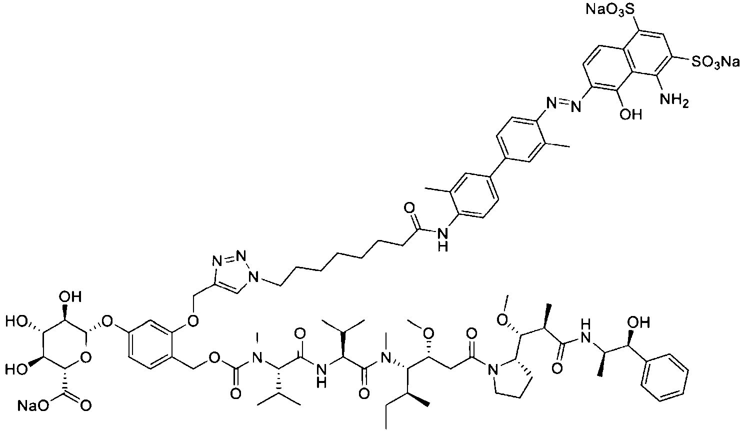 | sodium (2S,3S,4S,5R,6S)-6-(3-((1-(8-((4'-((E)-(8-amino-1-hydroxy-5,7-disulfonatonaphthalen-2-yl)diazenyl)-3,3'-dimethyl-[1,1'-biphenyl]-4-yl)amino)-8-oxooctyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 59 | 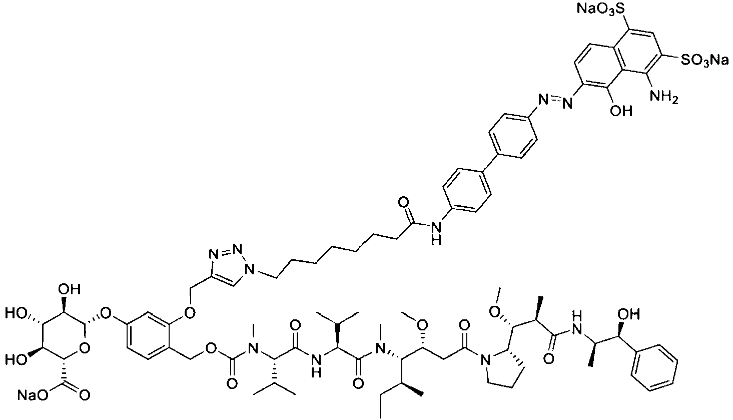 | sodium (2S,3S,4S,5R,6S)-6-(3-((1-(8-((4'-((E)-(8-amino-1-hydroxy-5,7-disulfonatonaphthalen-2-yl)diazenyl)-[1,1'-biphenyl]-4-yl)amino)-8-oxooctyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 60 | 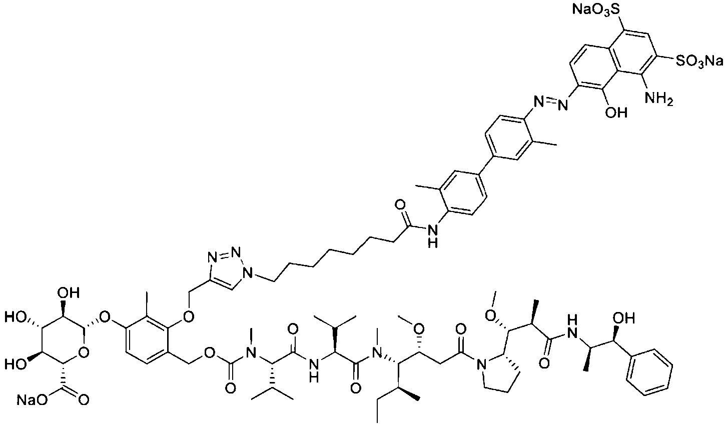 | sodium (2S,3S,4S,5R,6S)-6-(3-((1-(8-((4'-((E)-(8-amino-1-hydroxy-5,7-disulfonatonaphthalen-2-yl)diazenyl)-3,3'-dimethyl-[1,1'-biphenyl]-4-yl)amino)-8-oxooctyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-methylphenoxy)-3,4,5-trihydroxytetrahydro-2H-pyran-2-carboxylate |
| 61 | 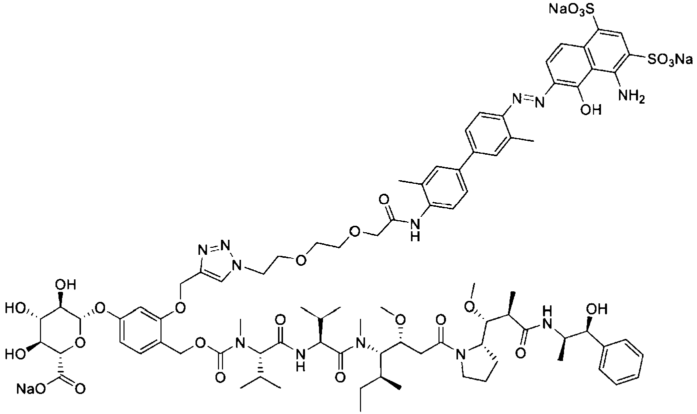 | sodium (2S,3S,4S,5R,6S)-6-(3-((1-(2-(2-(2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethoxy)ethoxy)ethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 62 | 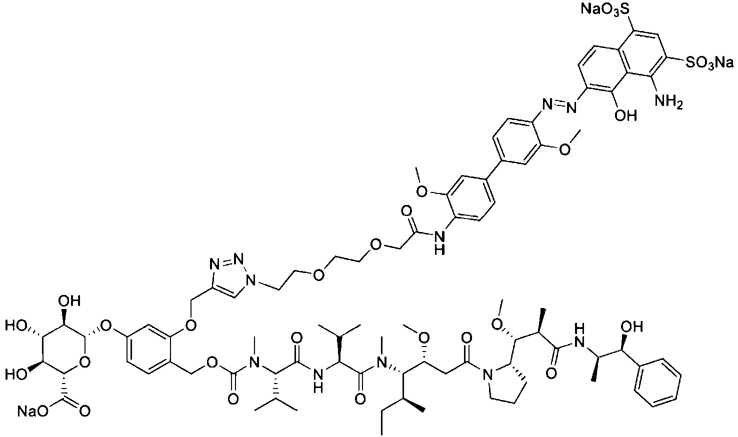 | sodium (2S,3S,4S,5R,6S)-6-(3-((1-(2-(2-(2-((4'-((E)-(8-amino-1-hydroxy-5,7-disulfonatonaphthalen-2-yl)diazenyl)-3,3'-dimethoxy-[1,1'-biphenyl]-4-yl)amino)-2-oxoethoxy)ethoxy)ethyl)-1 H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 63 | 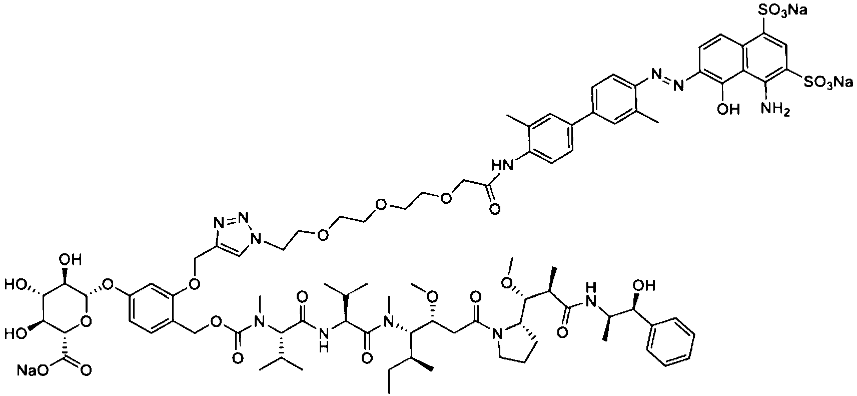 | sodium (2S,3S,4S,5R,6S)-6-(3-((1-(2-(2-(2-(2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethoxy)ethoxy)ethoxy)ethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 64 | 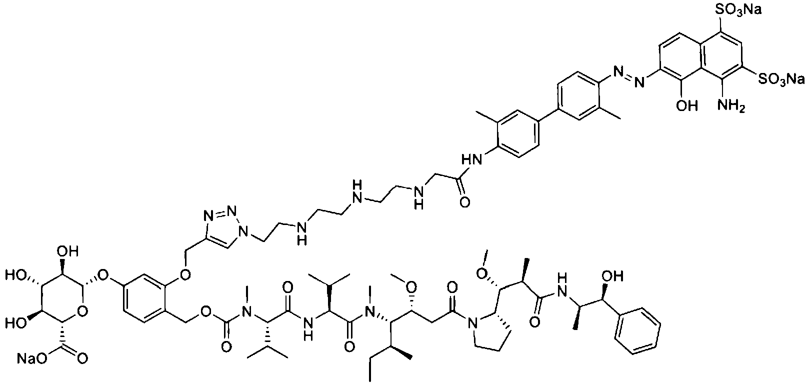 | sodium (2S,3S,4S,5R,6S)-6-(3-((1-(2-((2-((2-((2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethyl)amino)ethyl)amino)ethyl)amino)ethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 65 | 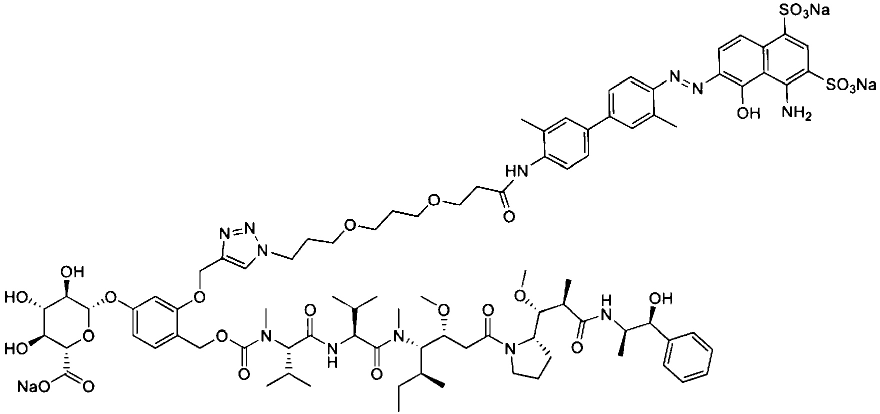 | sodium (2S,3S,4S,5R,6S)-6-(3-((1-(3-(3-(3-(( 4'-((E)-(8-amino-1-hydroxy-5,7-disulfonatonaphthalen-2-yl)diazenyl)-3,3'-dimethyl-[1,1'-biphenyl]-4-yl)amino)-3-oxopropoxy)propoxy)propyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 66 | 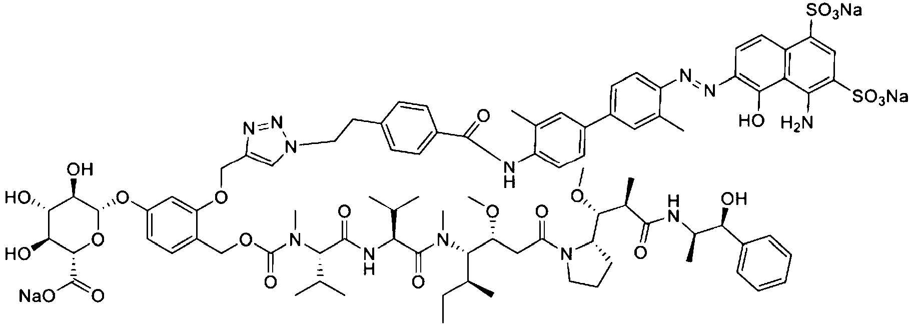 | sodium (2S,3S,4S,5R,65)-6-(3-((1-(4-((4'-((E)-(8-amino-1-hydroxy-5,7-disulfonatonaphthalen-2-yl)diazenyl)-3,3'-dimethyl-[1,1'-biphenyl]-4-yl)carbamoyl)phenethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 67 | 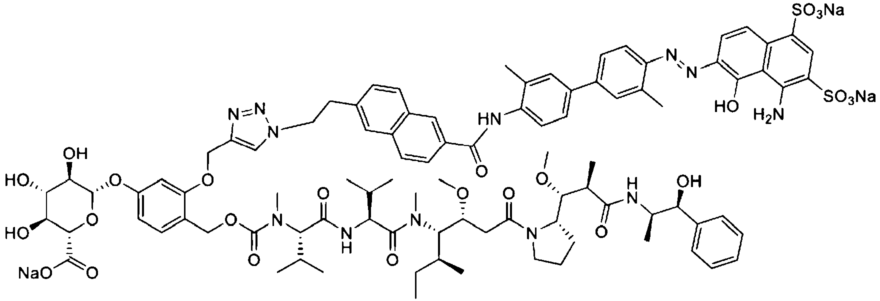 | sodium (2S,3S,4S,5R,6S)-6-(3-((1-(2-(6-((4'-((E)-(8-amino-1-hydroxy-5,7-disulfonatonaphthalen-2-yl)diazenyl)-3,3'-dimethyl-[1,1'-biphenyl]-4-yl)carbamoyl)naphthalen-2-yl)ethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 68 | 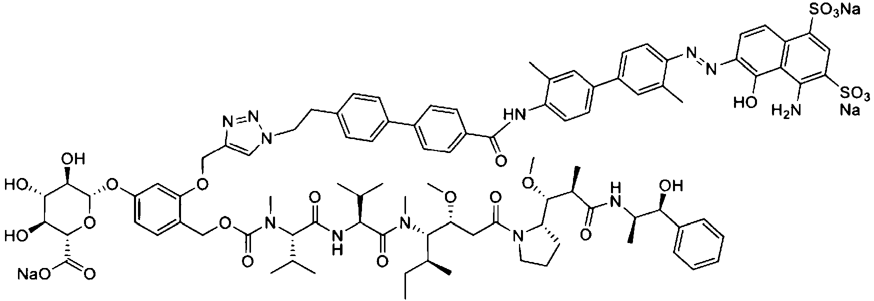 | sodium (2S,3S,4S,5R,6S)-6-(3-((1-(2-(4'-((4'-((E)-(8-amino-1-hydroxy-5,7-disulfonatonaphthalen-2-yl)diazenyl)-3,3'-dimethyl-[1,1'-biphenyl]-4-yl)carbamoyl)-[1,1'-biphenyl]-4-yl)ethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 69 | 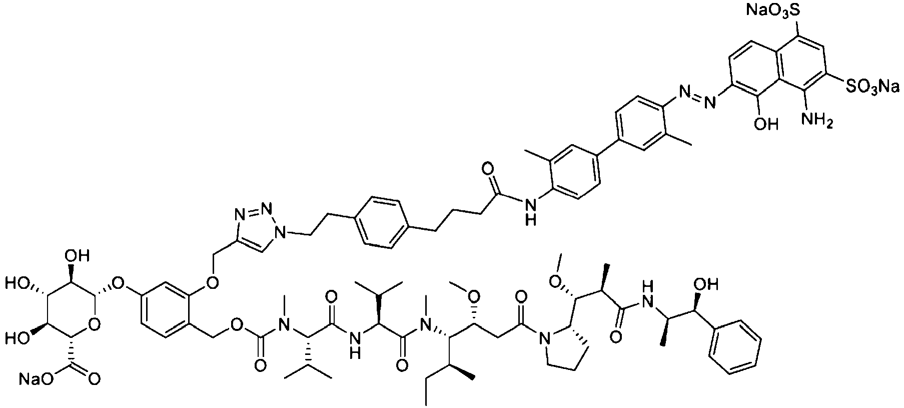 | sodium (2S,3S,4S,5R,6S)-6-(3-((1-(4-(4-((4'-((E)-(8-amino-1-hydroxy-5,7-disulfonatonaphthalen-2-yl)diazenyl)-3,3'-dimethyl-[1,1'-biphenyl]-4-yl)amino)-4-oxobutyl)phenethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 70 | 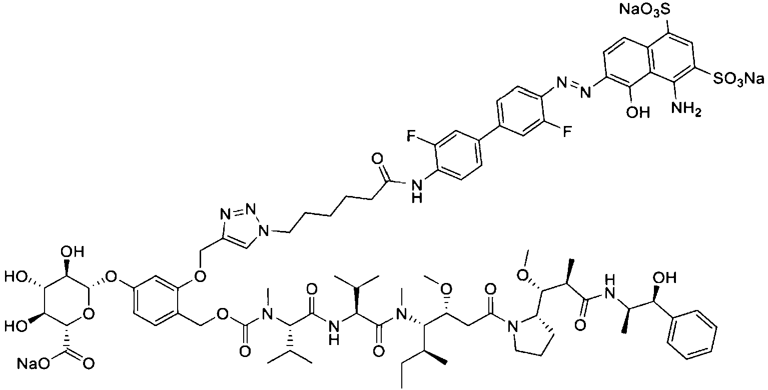 | sodium (2S,3S,4S,5R,65)-6-(3-((1-(6-((4'-((E)-(8-amino-1-hydroxy-5,7-disulfonatonaphthalen-2-yl)diazenyl)-3,3'-difluoro-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 71 | 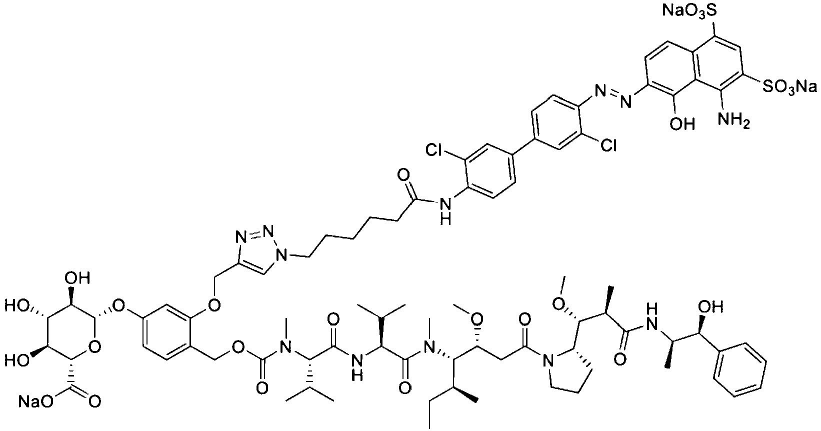 | sodium (2S,3S,4S,5R,65)-6-(3-((1-(6-((4'-((E)-(8-amino-1-hydroxy-5,7-disulfonatonaphthalen-2-yl)diazenyl)-3,3'-dichloro-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 72 | 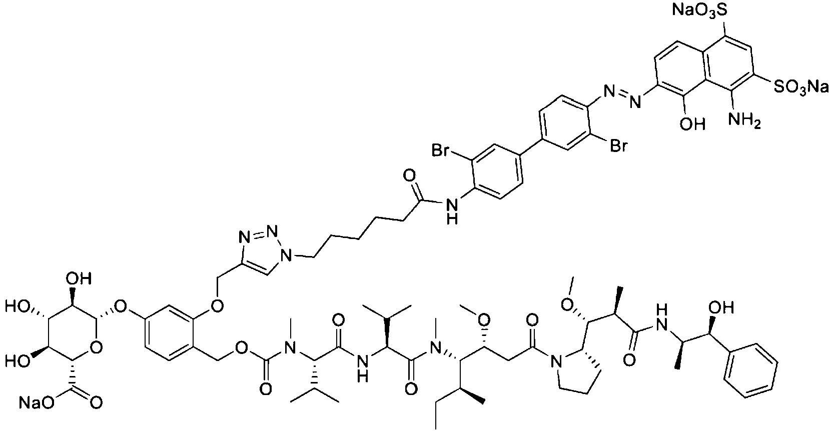 | sodium (2S,3S,4S,5R,65)-6-(3-((1-(6-((4'-((E)-(8-amino-1-hydroxy-5,7-disulfonatonaphthalen-2-yl)diazenyl)-3,3'-dibromo-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 73 | 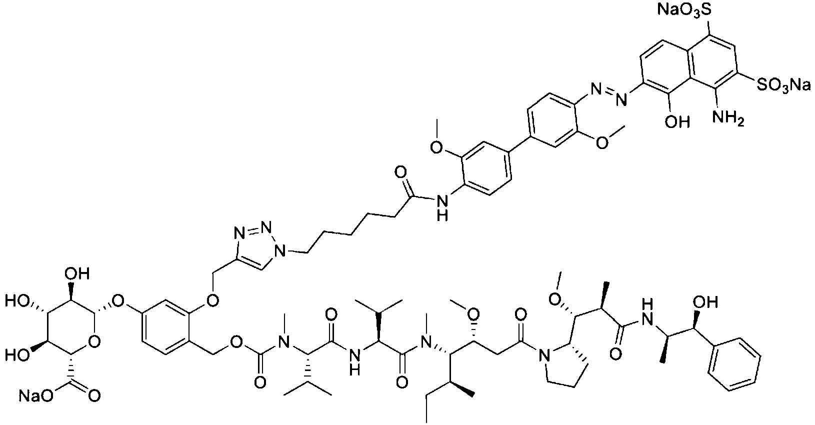 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethoxy-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 74 | 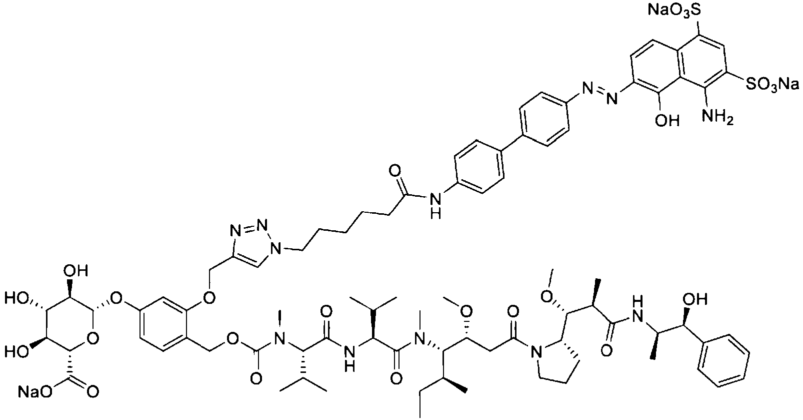 | sodium (2S,3S,4S,5R,6S)-6-(3-((1-(6-((4'-((E)-(8-amino-1-hydroxy-5,7-disulfonatonaphthalen-2-yl)diazenyl)-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 75 | 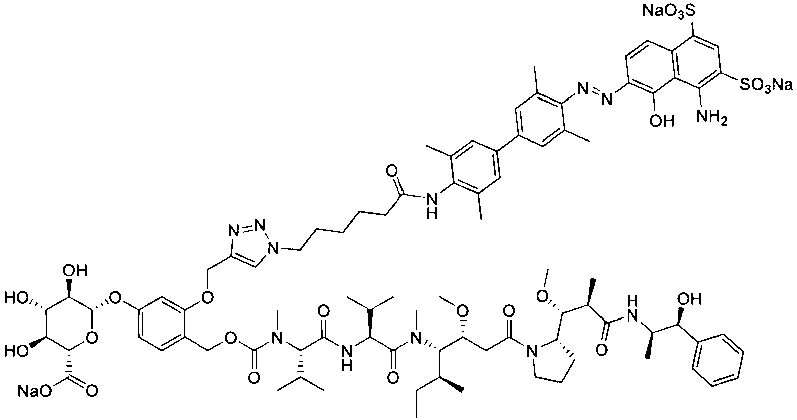 | sodium (2S,3S,4S,5R,6S)-6-(3-((1-(6-((4'-((E)-(8-amino-1-hydroxy-5,7-disulfonatonaphthalen-2-yl)diazenyl)-3,3',5,5'-tetramethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 76 | 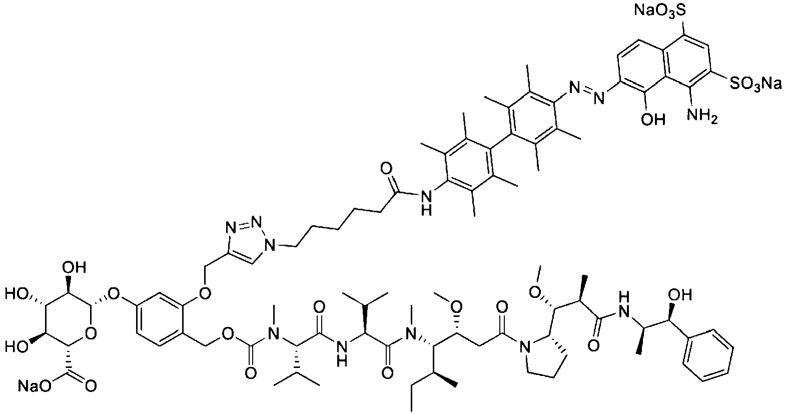 | sodium (2S,3S,4S,5R,6S)-6-(3-((1-(6-((4'-((E)-(8-amino-1-hydroxy-5,7-disulfonatonaphthalen-2-yl)diazenyl)-2,2',3,3',5,5',6,6'-octamethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 77 | 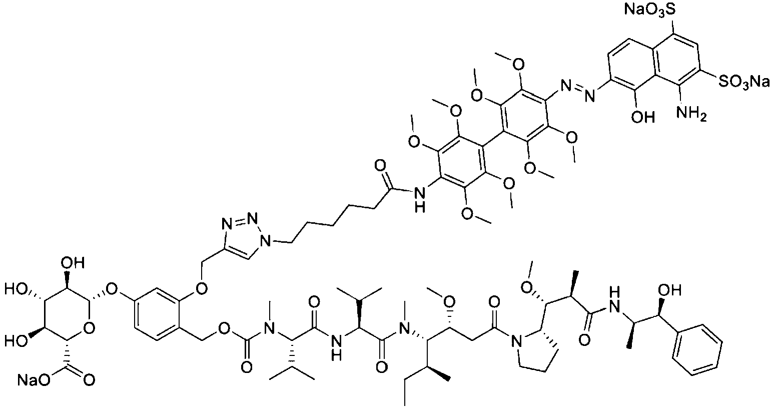 | sodium (2S,3S,4S,5R,6S)-6-(3-((1-(6-((4'-((E)-(8-amino-1-hydroxy-5,7-disulfonatonaphthalen-2-yl)diazenyl)-2,2',3,3',5,5',6,6'-octamethoxy-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 78 | 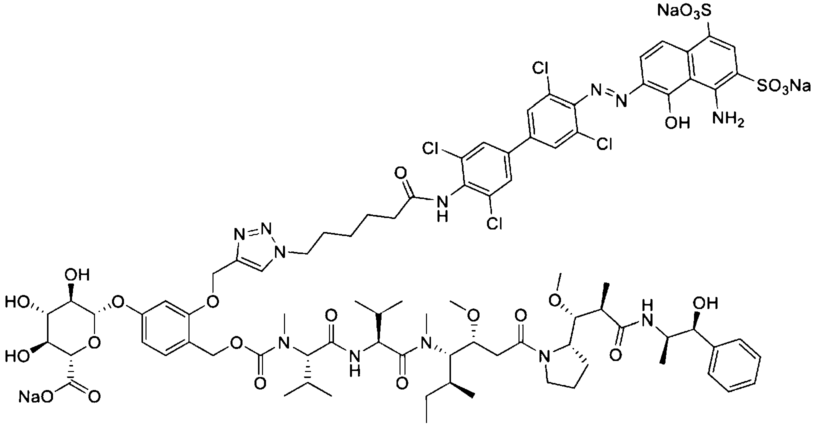 | sodium (2S,3S,4S,5R,6S)-6-(3-((1-(6-((4'-((E)-(8-amino-1-hydroxy-5,7-disulfonatonaphthalen-2-yl)diazenyl)-3,3',5,5'-tetrachloro-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 79 | 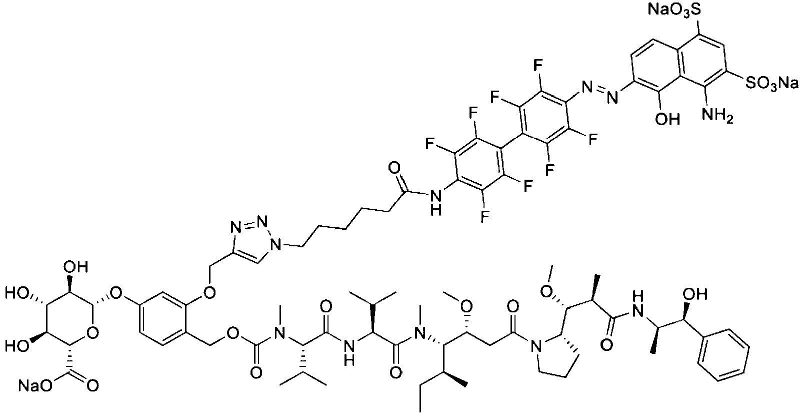 | sodium (2S,3S,4S,5R,65)-6-(3-((1-(6-((4'-((E)-(8-amino-1-hydroxy-5,7-disulfonatonaphthalen-2-yl)diazenyl)-2,2',3,3',5,5',6,6'-octafluoro-[ 1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 80 | 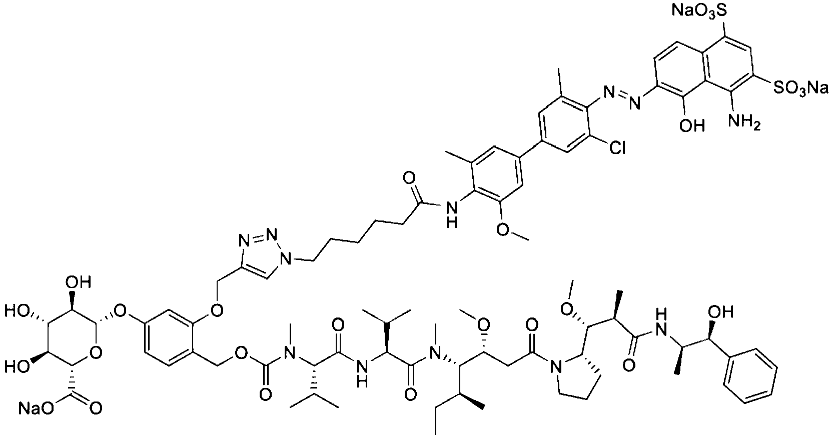 | sodium (2S,3S,4S,5R,6S)-6-(3-((1-(6-((4'-((E)-(8-amino-1-hydroxy-5,7-disulfonatonaphthalen-2-yl)diazenyl)-3'-chloro-3-methoxy-5,5'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 81 | 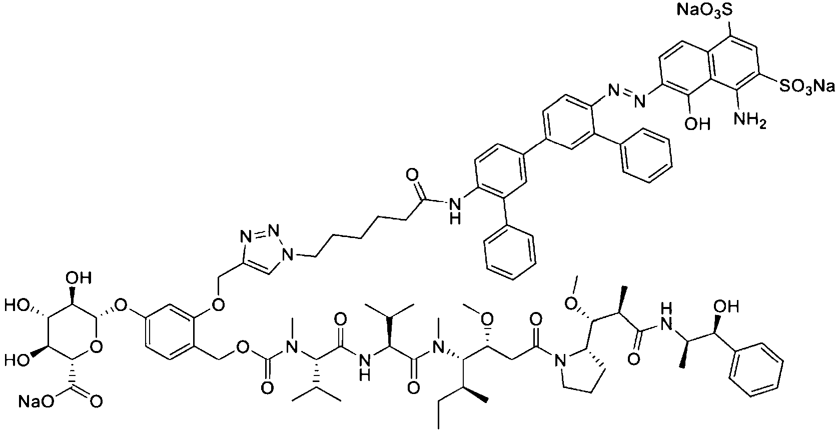 | sodium (2S,3S,4S,5R,6S)-6-(3-((1-(6-((6'-((E)-(8-amino-1-hydroxy-5,7-disulfonatonaphthalen-2-yl)diazenyl)-[1,1':3',1":3",1"'-quaterphenyl]-4"-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 82 | 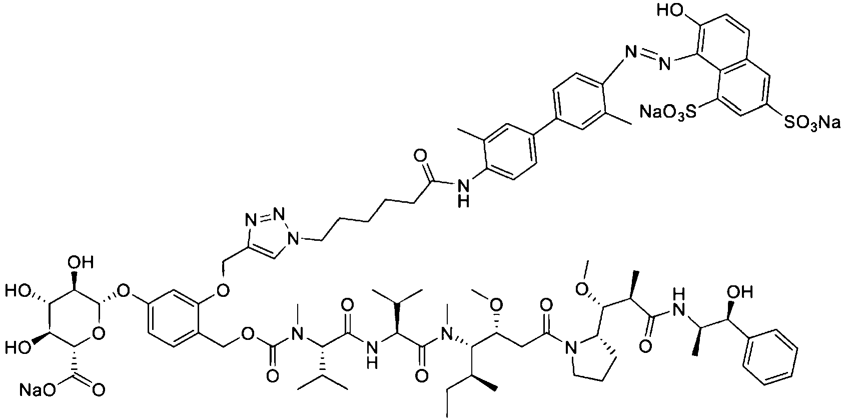 | sodium (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-((1-(6-((4'-((E)-(2-hydroxy-6,8-disulfonatonaphthalen-1-yl)diazenyl)-3,3'-dimethyl-[1,1'-biphenyl]-4-yl)amino)-6-oxohexyl)-1H-1,2,3-triazol-4-yl)methoxy)phenoxy)-3,4,5-trihydroxytetrahydro-2H-pyran-2-carboxylate |
| 83 | 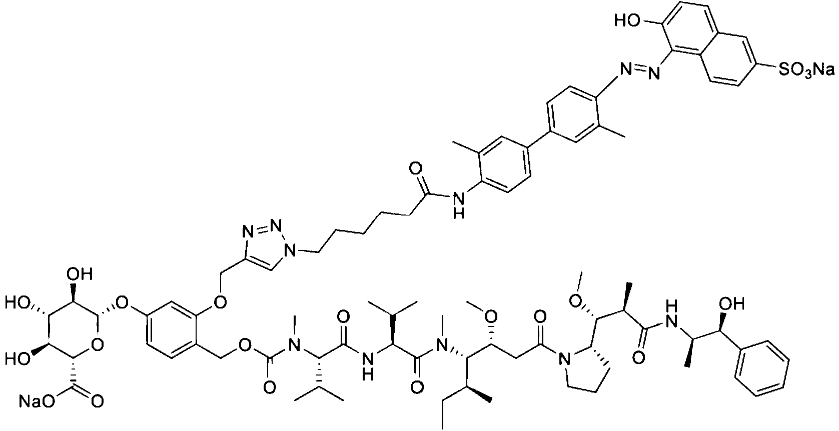 | sodium (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-((1-(6-((4'-((E)-(2-hydroxy-6-sulfonatonaphthalen-1-yl)diazenyl)-3,3'-dimethyl-[1,1'-biphenyl]-4-yl)amino)-6-oxohexyl)-1H-1,2,3-triazol-4-yl)methoxy)phenoxy)-3,4,5-trihydroxytetrahydro-2H-pyran-2-carboxylate |
| 84 | 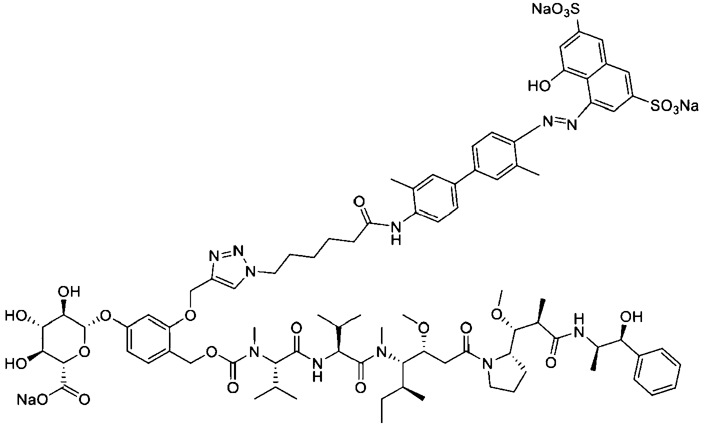 | sodium (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-((1-(6-((4'-((E)-(8-hydroxy-3,6-disulfonatonaphthalen-1-yl)diazenyl)-3,3'-dimethyl-[1,1'-biphenyl]-4-yl)amino)-6-oxohexyl)-1H-1,2,3-triazol-4-yl)methoxy)phenoxy)-3,4,5-trihydroxytetrahydro-2H-pyran-2-carboxylate |
| 85 | 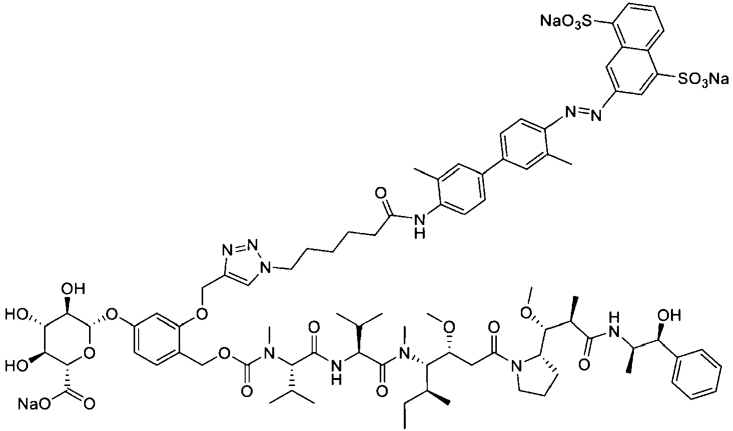 | sodium (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-((1-(6-((4'-((E)-(4,8-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)phenoxy)-3,4,5-trihydroxytetrahydro-2H-pyran-2-carboxylate |
| 86 | 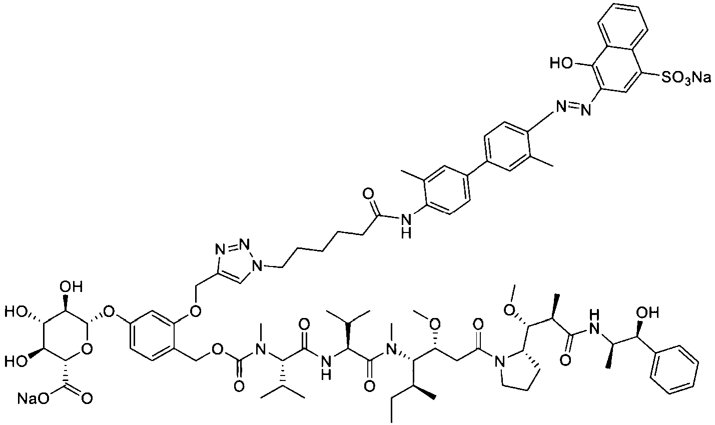 | sodium (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-((1-(6-((4'-((E)-(1-hydroxy-4-sulfonatonaphthalen-2-yl)diazenyl)-3,3'-dimethyl-[1,1'-biphenyl]-4-yl)amino)-6-oxohexyl)-1H-1,2,3-triazol-4-yl)methoxy)phenoxy)-3,4,5-trihydroxytetrahydro-2H-pyran-2-carboxylate |
| 87 | 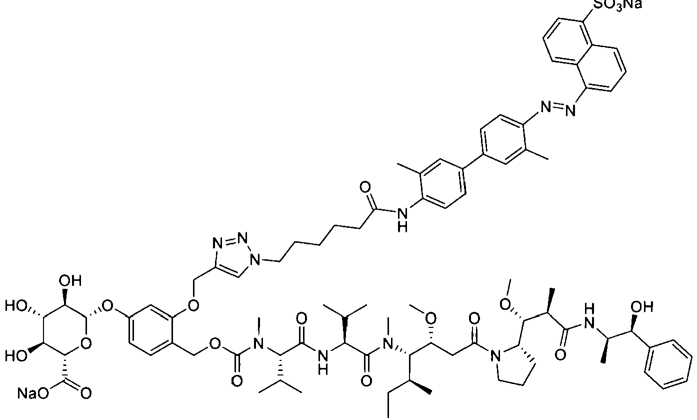 | sodium (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-((1-(6-((3,3'-dimethyl-4'-((E)-(5-sulfonatonaphthalen-1-yl)diazenyl)-[1,1'-biphenyl]-4-yl)amino)-6-oxohexyl)-1H-1,2,3-triazol-4-yl)methoxy)phenoxy)-3,4,5-trihydroxytetrahydro-2H-pyran-2-carboxylate |
| 88 | 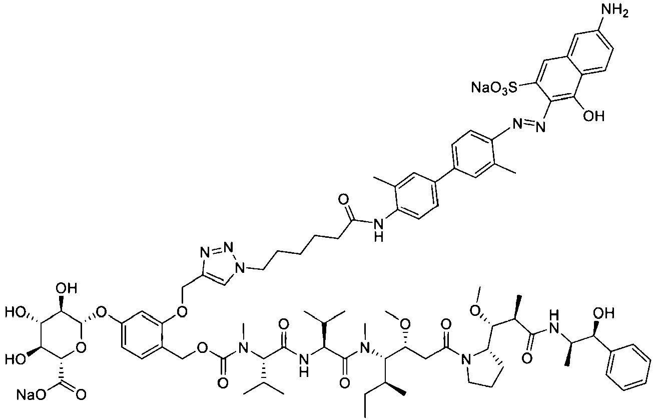 | sodium (2S,3S,4S,5R,65)-6-(3-((1-(6-((4'-((E)-(6-amino-1-hydroxy-3-sulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 89 | 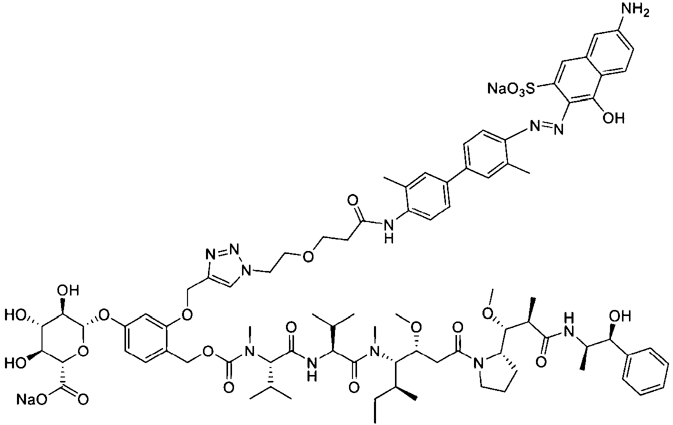 | sodium (2S,3S,4S,5R,6S)-6-(3-((1-(2-(3-((4'-((E)-(6-amino-1-hydroxy-3-sulfonatonaphthalen-2-yl)diazenyl)-3,3'-dimethyl-[1,1'-biphenyl]-4-yl)amino)-3-oxopropoxy)ethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 90 | 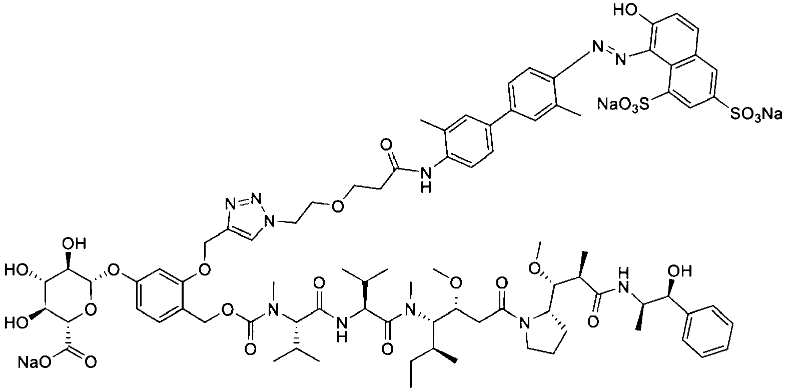 | sodium (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-((1-(2-(3-((4'-((E)-(2-hydroxy-6,8-disulfonatonaphthalen-1-yl)diazenyl)-3,3'-dimethyl-[1,1'-biphenyl]-4-yl)amino)-3-oxopropoxy)ethyl)-1H-1,2,3-triazol-4-yl)methoxy)phenoxy)-3,4,5-trihydroxytetrahydro-2H-pyran-2-carboxylate |
| 91 | 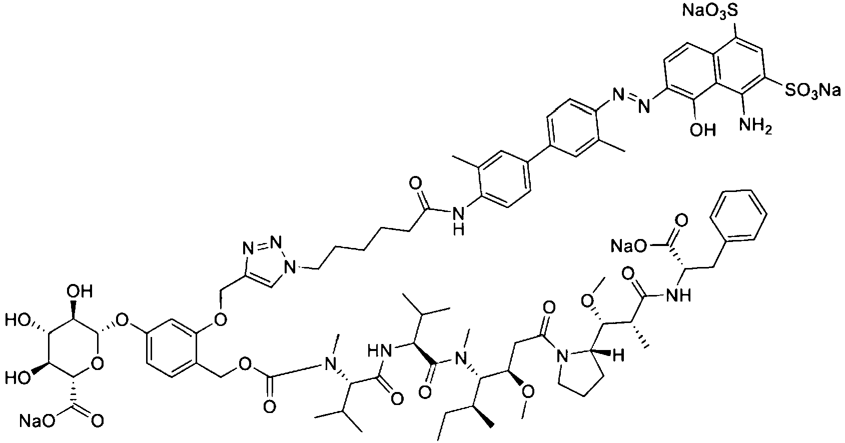 | sodium (2S,3S,4S,5R,65)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((S)-1-carboxylato-2-phenylethyl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 92 | 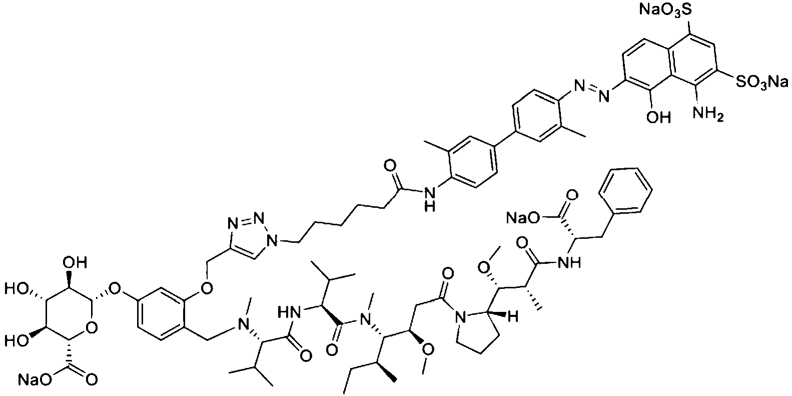 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((S)-1-carboxylato-2-phenylethyl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecyl)phenoxy)-3,4,5-trihydroxytetrahydro-2H-pyran-2-carboxylate |
| 93 | 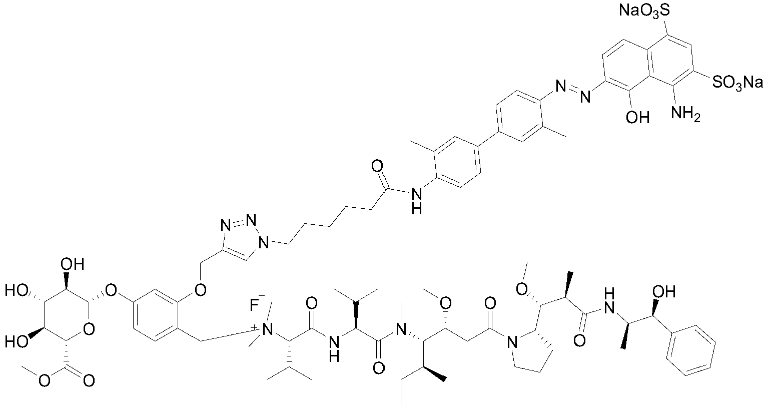 | sodium 4-amino-6-((E)-(4'-(6-(4-((2-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)-5-(((2S,3R,4S,5S,6S)-3,4,5-trihydroxy-6-(methoxycarbonyl)tetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate fluoride |
| 94 | 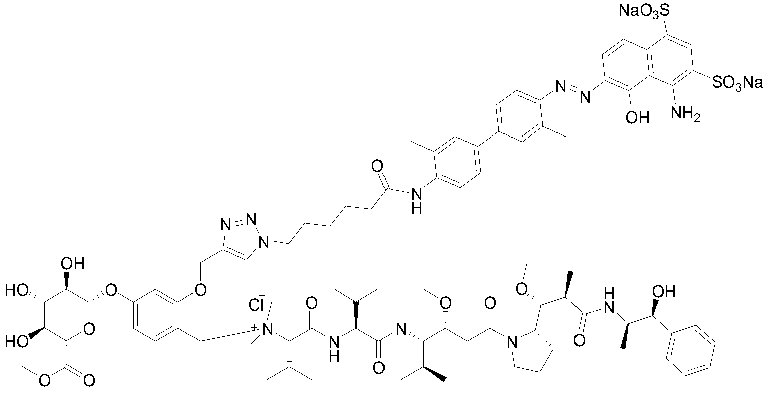 | sodium 4-amino-6-((E)-(4'-(6-(4-((2-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)-5-(((2S,3R,4S,5S,6S)-3,4,5-trihydroxy-6-(methoxycarbonyl)tetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate chloride |
| 95 | 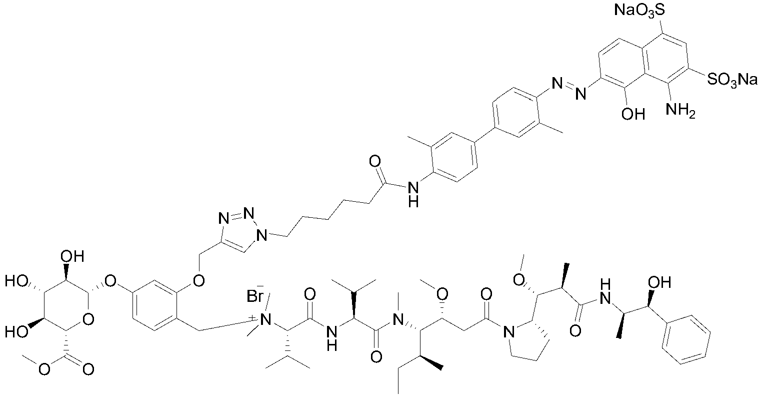 | sodium 4-amino-6-((E)-(4'-(6-(4-((2-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)-5-(((2S,3R,4S,5S,6S)-3,4,5-trihydroxy-6-(methoxycarbonyl)tetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate bromide |
| 96 | 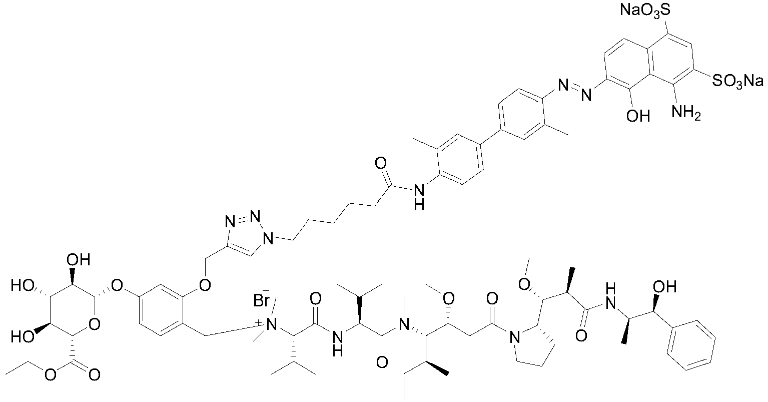 | sodium 4-amino-6-((E)-(4'-(6-(4-((2-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)-5-(((2S,3R,4S,5S,6S)-6-(ethoxycarbonyl)-3,4,5-trihydroxytetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate bromide |
| 97 | 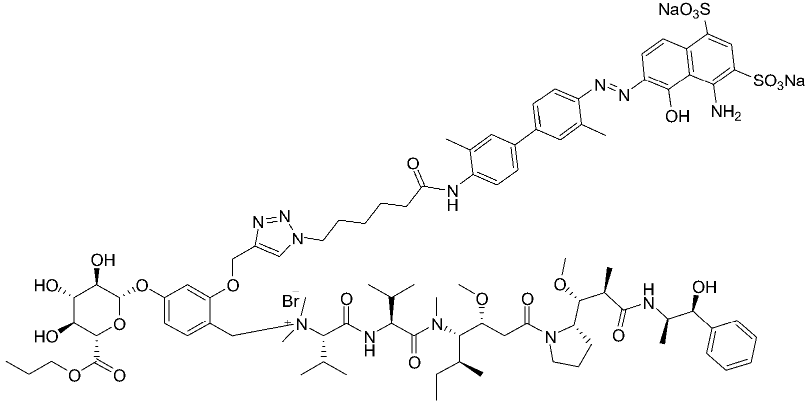 | sodium 4-amino-6-((E)-(4'-(6-(4-((2-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)-5-(((2S,3R,4S,5S,6S)-3,4,5-trihydroxy-6-(propoxycarbonyl)tetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate bromide |
| 98 | 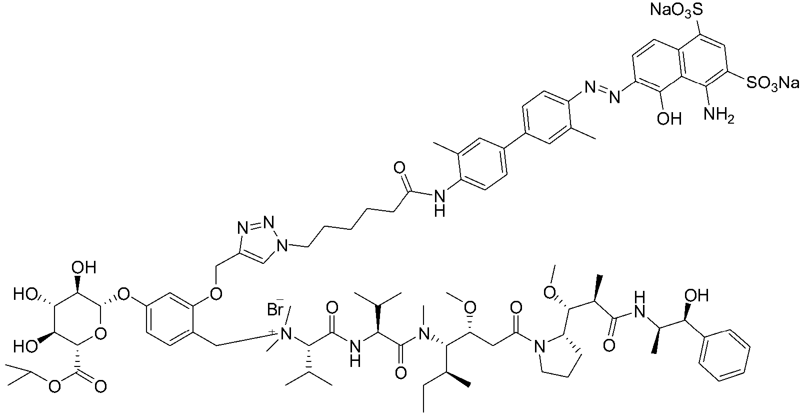 | sodium 4-amino-6-((E)-(4'-(6-(4-((2-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)-5-(((2S,3R,4S,5S,6S)-3,4,5-trihydroxy-6-(isopropoxycarbonyl)tetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate bromide |
| 99 | 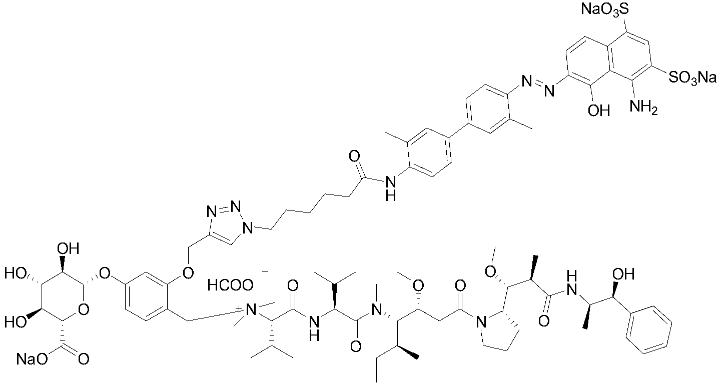 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate formate |
| 100 | 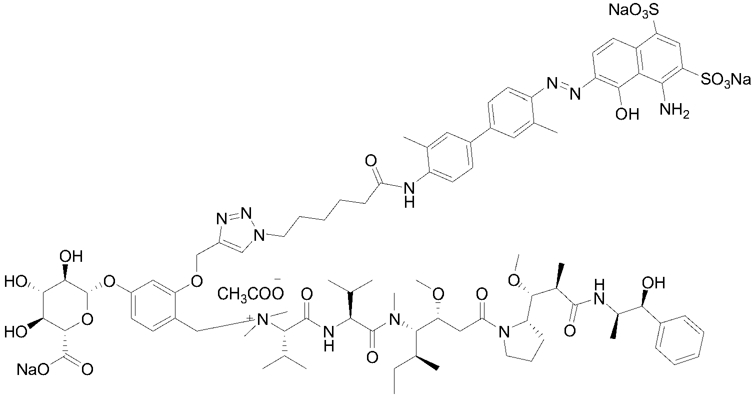 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate acetate |
| 101 | 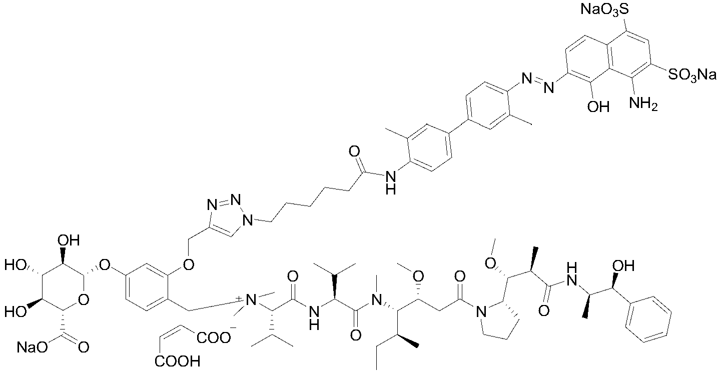 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate (Z)-3-carboxyacrylate |
| 102 | 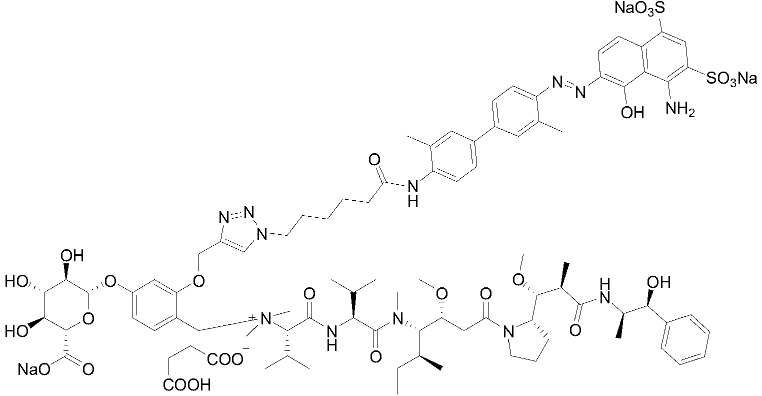 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate 3-carboxypropanoate |
| 103 | 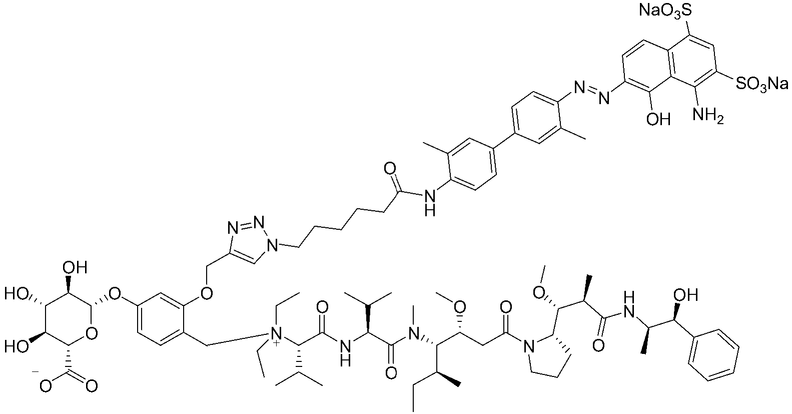 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-2,2-diethyl-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-8-methyl-4,7-dioxo-11-oxa-2,5,8-triazadodecan-2-ium-1-yl)phenoxy)-3,4,5-trihydroxytetrahydro-2H-pyran-2-carboxylate |
| 104 | 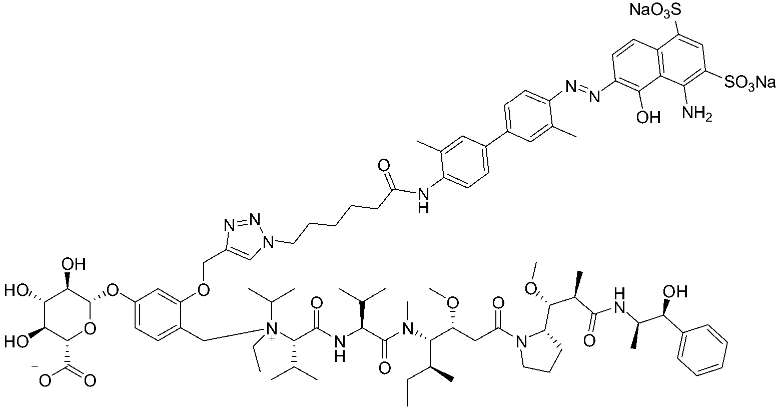 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-2-ethyl-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-2,3,6-triisopropyl-8-methyl-4,7-dioxo-11-oxa-2,5,8-triazadodecan-2-ium-1-yl)phenoxy)-3,4,5-trihydroxytetrahydro-2H-pyran-2-carboxylate |
| 105 | 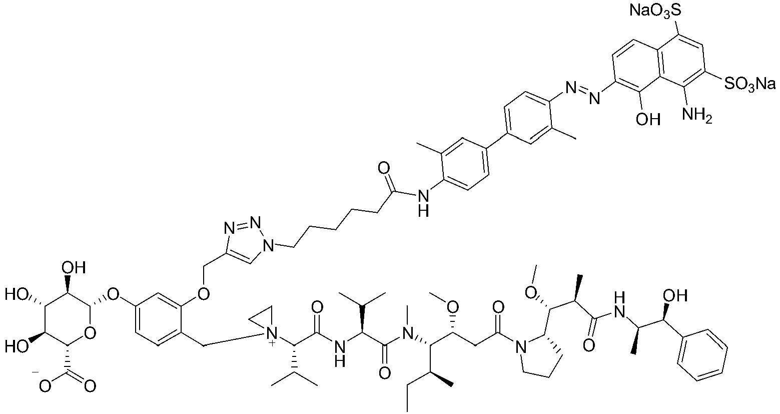 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((1-((S)-1-(((S)-1-(((3R,4S,5 S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)aziridin-1-ium-1-yl)methyl)phenoxy)-3,4,5-trihydroxytetrahydro-2H-pyran-2-carboxylate |
| 106 | 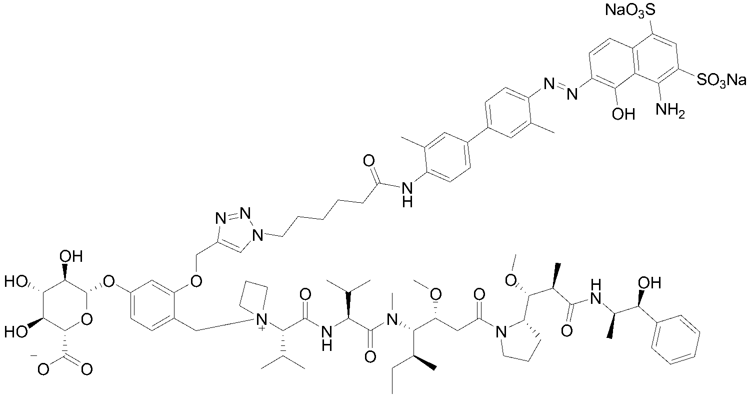 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((1-((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)azetidin-1-ium-1-yl)methyl)phenoxy)-3,4,5-trihydroxytetrahydro-2H-pyran-2-carboxylate |
| 107 | 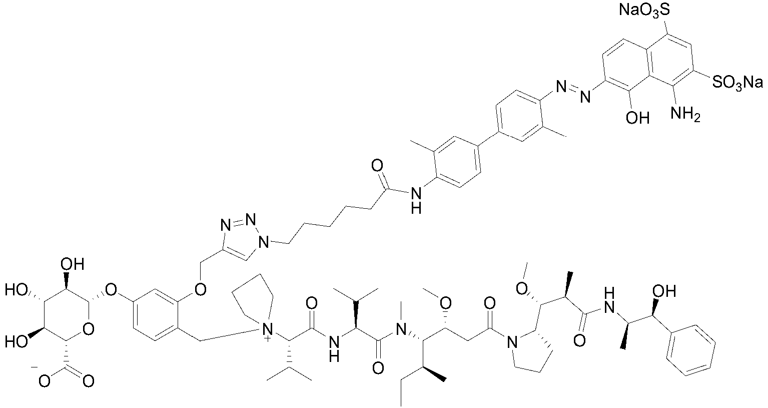 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((1-((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)pyrrolidin-1-ium-1-yl)methyl)phenoxy)-3,4,5-trihydroxytetrahydro-2H-pyran-2-carboxylate |
| 108 | 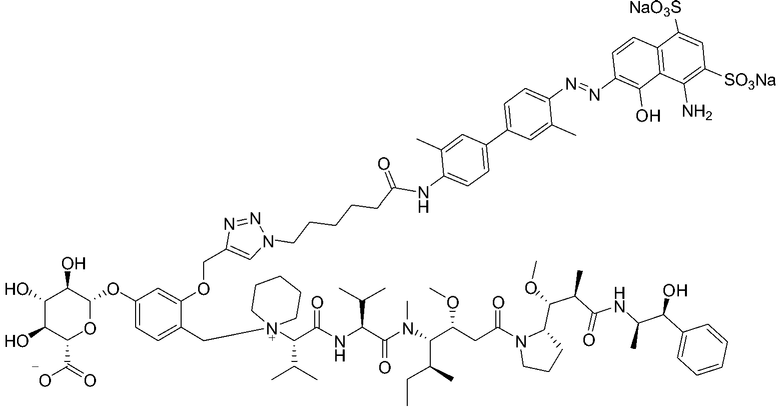 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((1-((S)-1-(((S)-1-(((3R,4S,5 S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)piperidin-1-ium-1-yl)methyl)phenoxy)-3,4,5-trihydroxytetrahydro-2H-pyran-2-carboxylate |
| 109 | 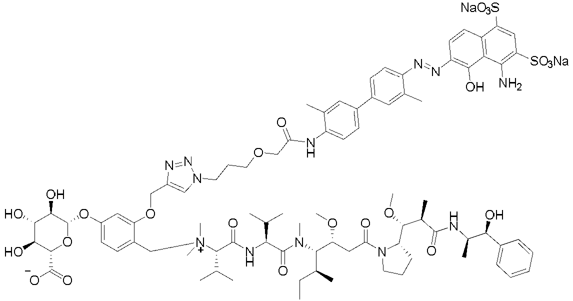 | sodium (2S,3S,4S,5R,6S)-6-(3-((1-(3-(2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethoxy)propyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate |
| 110 | 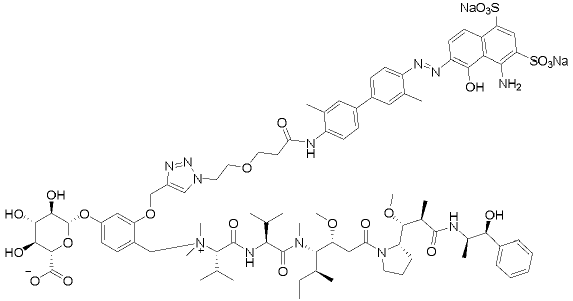 | sodium (2S,3S,4S,5R,6S)-6-(3-((1-(2-(3-((4'-((E)-(8-amino-1-hydroxy-5,7-disulfonatonaphthalen-2-yl)diazenyl)-3,3'-dimethyl-[1,1'-biphenyl]-4-yl)amino)-3-oxopropoxy)ethyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate |
| 111 | 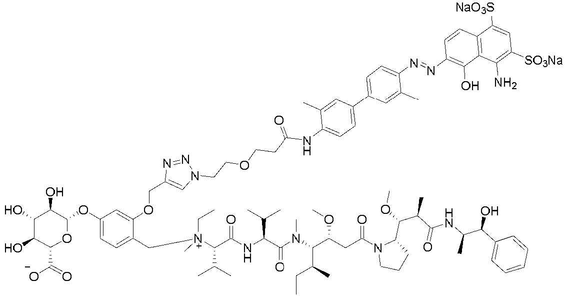 | sodium (2S,3S,4S,5R,6S)-6-(3-((1-(2-(3-((4'-((E)-(8-amino-1-hydroxy-5,7-disulfonatonaphthalen-2-yl)diazenyl)-3,3'-dimethyl-[1,1'-biphenyl]-4-yl)amino)-3-oxopropoxy)ethyl)-1H-1,2,3-triazol-4-yl)methoxy)-4-((3S,6S,9S,10R)-9-((S)-sec-butyl)-2-ethyl-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate |
| 112 | 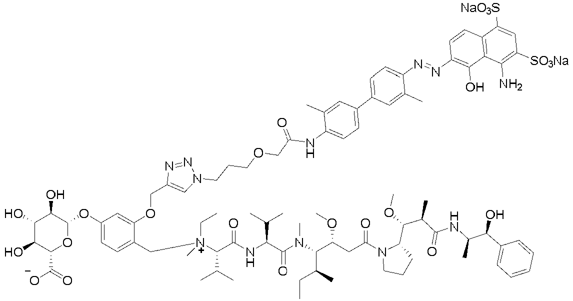 | sodium (2S,3S,4S,5R,6S)-6-(3-((1-(3-(2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethoxy)propyl)-1H-1,2,3-triazol-4-yl)methoxy)-4-((3S,6S,9S,10R)-9-((S)-sec-butyl)-2-ethyl-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate |
| 113 | 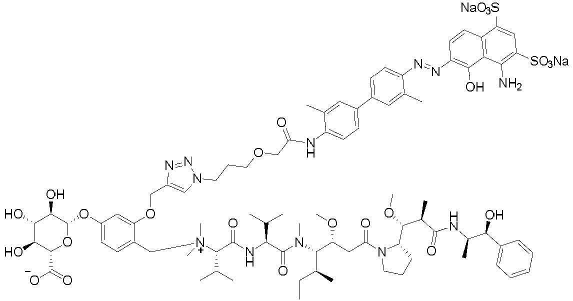 | sodium (2S,3S,4S,5R,6S)-6-(3-((1-(3-(2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethoxy)propyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate |
| 114 | 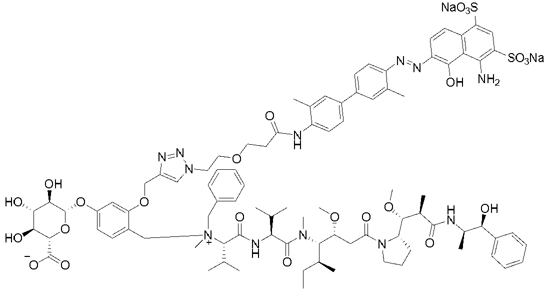 | sodium (2S,3S,4S,5R,6S)-6-(3-((1-(2-(3-((4'-((E)-(8-amino-1-hydroxy-5,7-disulfonatonaphthalen-2-yl)diazenyl)-3,3'-dimethyl-[1,1'-biphenyl]-4-yl)amino)-3-oxopropoxy)ethyl)-1H-1,2,3-triazol-4-yl)methoxy)-4-((3S,6S,9S,10R)-2-benzyl-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate |
| 115 | 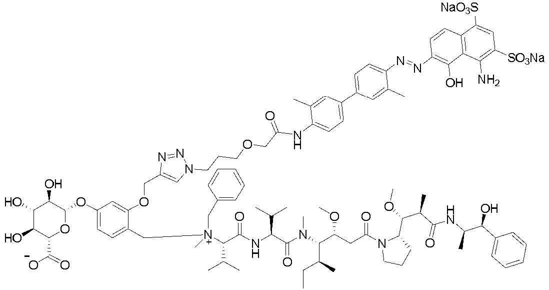 | sodium (2S,3S,4S,5R,6S)-6-(3-((1-(3-(2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethoxy)propyl)-1H-1,2,3-triazol-4-yl)methoxy)-4-((3S,6S,9S,10R)-2-benzyl-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate |

### Pharmaceutical Composition

In some embodiments, the pharmaceutical composition comprises the compound of the disclosure, or the stereoisomer, the cis-trans isomer, or the pharmaceutically acceptable salt thereof, and a pharmaceutically acceptable excipient.

In some embodiments, the administration route of the compound of the disclosure, or the stereoisomer, the cis-trans isomer, or the pharmaceutically acceptable salt thereof, when administered to a mammal for the treatment of a tumor or a cancer, may be the injection route.

The compound of the present disclosure may be prepared in any suitable form, such as a formulation for injection. Representative examples of the formulation for injection include, but are not limited to, an injection, a sterile powder for injection, and a concentrated solution for injection. The injection includes an injection of a solution type, an emulsion type (such as a fat emulsion injection) or a suspension type (such as some liposome injections or cortisone acetate injections), and may be used for, e.g., intramuscular injection, intravenous injection, or intravenous drip. A large-volume (unless otherwise specified, generally greater than or equal to 100 mL) injection for intravenous injection is also referred to as an intravenous infusion. Different from a large infusion, an ampoule-packed injection is commonly known as a small-volume injection. The sterile powder for injection includes a lyophilized powder for injection and a sterile bulk powder. The concentrated solution for injection is a sterile concentrated solution that is diluted right before use. Examples of the pharmaceutically acceptable vehicle that can be used in the pharmaceutical composition of the disclosure include, but are not limited to, various forms of vehicles that have been approved by the U.S. Food and Drug Administration for use in humans or animals and have no adverse effects on the ingredients of the pharmaceutical composition, such as any adjuvant, vehicle, carrier substance, solvent, osmotic pressure regulator, pH regulator, solubilizer, cosolvent, antioxidant, bacteriostat, emulsifier, suspending aid, filler, complexing agent, and chelating agent. An acceptable vehicle for therapeutic use is well known in the pharmaceutical field, and described, for example, in Remington's Pharmaceutical Sciences, 18th Ed., Mack Publishing Co., Easton, PA (1990), the entire contents of which are incorporated herein by reference.

The pharmaceutical composition of the disclosure may be administered using any method that achieves its intended purpose. The pharmaceutical composition of the disclosure may be administered by injection, such as intradermal injection, subcutaneous injection, intramuscular injection, intravenous drip, intraarterial injection, intracardiac injection, intraarticular injection, or subcutaneous infusion. The administered dosage depends on the recipient's age, health status, and body weight, and, if concurrent therapy is present, on the type of concurrent therapy, frequency of therapy, and nature of the desired therapeutic effect.

An appropriate dosage form includes, but is not limited to, an injection, a sterile powder for injection, and a concentrated solution for injection. The injection includes an injection of a solution type, an emulsion type (such as a fat emulsion injection) or a suspension type (such as some liposome injections or cortisone acetate injections), and may be used for, e.g., intramuscular injection, intravenous injection, or intravenous drip. The injection for intravenous injection includes an intravenous infusion and a small-volume injection. The sterile powder for injection includes a lyophilized powder for injection and a sterile bulk powder. The concentrated solution for injection is a sterile concentrated solution that is diluted right before use and can be prepared according to a method known in the art.

Desired appropriate vehicle, solvent, osmotic pressure regulator, pH regulator, solubilizer, cosolvent, antioxidant, bacteriostat, emulsifier, suspending aid, filler, complexing agent, and chelating agent may be provided in the pharmaceutical composition. For example, water for injection, a vegetable oil such as soybean oil for injection, ethanol, propylene glycol or polyethylene glycol may be added as a solvent; sodium chloride or glucose may be added as an osmotic pressure regulator; an inorganic acid or base, or an organic acid or base, such as hydrochloric acid, sodium hydroxide, sodium citrate, or citric acid, may be added as a pH adjuster; polyoxyethylene castor oil, polysorbate, or cyclodextrin such as hydroxypropyl-β-cyclodextrin may be added as a solubilizer; an organic acid or a sodium salt thereof, such as sodium benzoate, may be added; an amide or an amine, such as meglumine, may be used as a cosolvent; ascorbic acid, sodium sulfite, sodium bisulfite, or sodium metabisulfite may be added as an antioxidant; phenol, cresol, chlorobutanol, or thimerosal may be added as a bacteriostat; lecithin or poloxamer may be added as an emulsifier; polyvinylpyrrolidone or methylcellulose may be added as a suspending aid; mannitol or glucose may be added as a filler; and ethylenediaminetetraacetate may be added as a complexing agent and a chelating agent.

An appropriate administration route may include, for example, parenteral delivery including intradermal injection, subcutaneous injection, intramuscular injection, intravenous drip, intraarterial injection, intracardiac injection, intraarticular injection, subcutaneous infusion, intramedullary injection, intrathecal injection, direct intraventricular injection, intraperitoneal injection, intranasal injection, or intraocular injection. The compound may also be administered at a predetermined rate by extended and/or timed, pulsed administration in a sustained or controlled release dosage form including, e.g., a depot injection, an osmotic pump, a pill, and a transdermal patch (including an electromigration patch).

The pharmaceutical composition of the disclosure may be produced following a known method, for example, by conventional operations such as mixing, preparation, filtration, encapsulation, and sterilization.

Therefore, the pharmaceutical composition of the present disclosure may be prepared by a conventional method using one or more physiologically acceptable vehicles containing an auxiliary agent that facilitates processing an active compound into a pharmaceutically usable formulation. An appropriate formulation depends on a selected administration route. Any well-known technology, vehicle, etc. may be used as appropriate and as understood in the art.

An injection may be prepared into a conventional form such as a solution or a suspension, a solid dosage form suitable for preparation into a solution or a suspension right before injection, or an emulsion. An appropriate excipient is, for example, water, saline, glucose, mannitol, lactose, lecithin, albumin, sodium glutamate, or cysteine hydrochloride. In addition, if necessary, the pharmaceutical composition for injection may contain a small amount of non-toxic auxiliaries, such as a wetting agent or a pH buffer. A physiologically suitable buffer includes, but is not limited to, a Hank's solution, a Ringer's solution, or a normal saline buffer. If necessary, an absorption-enhancing reagent (e.g., a liposome) may be used.

In some embodiments, the pharmaceutical composition of the disclosure may contain 0.1% to 95% of the compound of the disclosure, a stereoisomer thereof, or a pharmaceutically acceptable salt thereof.

In some embodiments, the pharmaceutical composition of the disclosure may contain 1% to 70% of the compound of the disclosure, a stereoisomer thereof, or a pharmaceutically acceptable salt thereof.

In any case, the composition or formulation to be administered will contain a certain amount of the compound of the disclosure, the stereoisomer thereof, or the pharmaceutically acceptable salt thereof.

In any case, the administration amount of the compound of the disclosure, the stereoisomer thereof, or the pharmaceutically acceptable salt thereof is effective in treating a disease/condition of a to-be-treated subject.

### Administration Method

At least one compound of the disclosure, a stereoisomer thereof, or a pharmaceutically acceptable salt thereof, or a pharmaceutical composition comprising at least one compound of the disclosure, the stereoisomer thereof, or the pharmaceutically acceptable salt thereof, may be administered to a patient using any suitable method for systemically and/or locally delivering the compound of the disclosure, the stereoisomer thereof, or the pharmaceutically acceptable salt thereof. Non-limited examples of the administration method include (a) administration via an oral route, including administration in the form of a capsule, a tablet, a granule, a spray, a syrup, or such other forms; (b) administration via a non-oral route, such as rectal, vaginal, intraurethral, intraocular, intranasal, or intraaural administration, including administration in the form of an aqueous suspension or an oily preparation, or in the form of a drop, a spray, a suppository, an unguent, or an ointment; (c) administration via subcutaneous injection, intraperitoneal injection, intravenous injection, intramuscular injection, intradermal injection, intraorbital injection, intravesicular injection, intraspinal injection, or intrasternal injection, including delivery using an infusion pump; (d) local administration, such as direct injection in the renal region or the cardiac region, for example, by depot implant; and (e) topical administration. As recognized by those skilled in the art, an appropriate administration mode refers to contact of the compound of the present disclosure with a living tissue.

The most appropriate route depends on the nature and severity of the condition to be treated. Those skilled in the art are also familiar with determining an administration method (oral, intravenous, inhalation, subcutaneous, rectal, etc.), a dosage form, an appropriate pharmaceutical excipient, and other items associated with the delivery of the compound, the stereoisomer thereof, or the pharmaceutically acceptable salt thereof to a subject in need thereof.

The pharmaceutical composition suitable for administration includes a composition containing an effective amount of an active ingredient to achieve its intended effect. The desired dosage of the therapeutically effective amount of the pharmaceutical composition of the present disclosure depends on the administration route, the type of a treated animal (including a human), and physical features of an animal of interest. The dosage may be adjusted to achieve the desired effect, but this will depend on factors such as body weight, diet, concurrent therapy, and other factors recognized by those skilled in the medical field. More specifically, the therapeutically effective amount refers to an amount of a compound that effectively prevents, alleviates, or ameliorates disease symptoms, or prolongs the lifespan of a treated individual. The therapeutically effective amount can be well determined based on practical abilities of those skilled in the art, particularly in accordance with the detailed disclosure provided herein.

As apparent to those skilled in the art, the dosage and specific administration mode for in vivo administration will vary depending on the age, body weight, and species of a treated mammal, the used compound, and the specific use of the used compound. Those skilled in the art can determine the effective dosage level, that is, the dosage level necessary to determine the desired effect, using a conventional pharmacological method. Generally, human clinical application of a product is initiated at a lower dosage level, and the dosage level is gradually increased until the desired therapeutic effect is achieved. Alternatively, an effective dosage and an administration route for the composition to be identified can be established through an acceptable in vitro study using an established pharmacological method.

In a non-human animal study, the application of a potential product is initiated at a higher dosage level, and the dosage is gradually decreased until the desired effect is no longer achieved or undesirable side effects disappear. Depending on the desired effect and therapeutic indication, the dosage may be in a wide range. Generally, the dosage may be from about 10 µg/kg to 1,000 mg/kg body weight, and in some embodiments, from about 100 µg/kg to 300 mg/kg body weight. Alternatively, as understood by those skilled in the art, the dosage may be based on a patient's body surface area and calculated accordingly.

Each physician can select exact formulation, administration route, and dosage of the pharmaceutical composition of the present disclosure based on patient's conditions. Generally, the dosage of the composition administered to a patient may range from about 0.05 mg/kg to 3,000 mg/kg of the patient's body weight. The dosage may be given once, twice or more in a period of one or several days, depending on the patient's needs. Where a human dosage for the compound is determined for at least some conditions, the present disclosure will employ the identical dosage, or dosage ranging from about 0.1% to 500% of the determined human dosage, and in some embodiments, dosage ranging from 25% to 250% of the determined human dosage. In the absence of a determined human dosage, for example, for a new pharmaceutical compound, the appropriate human dosage can be inferred from values such as a median effective dose or a median infective dose, or other suitable values derived from in vitro or in vivo studies, as quantified from toxicity studies and efficacy studies in animals.

It should be pointed out that the attending physician will know how and when to terminate, interrupt, or adjust administration due to toxicity and organ dysfunction. Otherwise, if the clinical response is inadequate (excluding toxicity), the attending physician will also know to adjust therapy to a higher level. The magnitude of a dosage administered in the treatment of a condition of interest will vary with the severity of a condition to be treated and an administration route. The severity of the condition may be assessed, for example, partially using a standard prognostic assessment method. In addition, the dosage and a possible dosage frequency will also vary depending on the age, body weight, and response of an individual patient. A solution equivalent to the above discussed solution may be used in veterinary medicine.

An exact dosage can be determined based on drug-by-drug analysis, but in most cases, some generalizations can be made about medicaments. The daily dosage regimen for an adult patient is, for example, an oral dosage of 0.1 mg to 2,000 mg of each active ingredient, and in some embodiments, 1 mg to 2,000 mg of each active ingredient, e.g., 5 mg to 1,500 mg of each active ingredient. In other embodiments, the intravenous, subcutaneous, or intramuscular dosage of each active ingredient is 0.01 mg to 1,000 mg, and in some embodiments, 0.1 mg to 1,000 mg, such as 1 mg to 800 mg. Where an acceptable salt is administered, the dosage may be calculated based on free base. In some embodiments, the composition is administered from 1 to 4 times daily. Alternatively, the composition of the present disclosure may be administered by continuous intravenous infusion, and in some embodiments, at a daily dose of up to 2,000 mg of each active ingredient. As understood by those skilled in the art, in some cases, in order to effectively and rapidly treat a rapidly progressing disease or infection, it may be necessary to administer the compound of the present disclosure in an amount that exceeds or far exceeds the above dosage ranges. In some embodiments, the compound is administered over a continuous period of treatment, for example, for one week or several weeks, or for several months or years.

The dosage and dosing interval may be individually adjusted to provide plasma levels of the active ingredient sufficient to maintain the modification effect or minimum effective concentration (MEC). The MEC of each compound is different, but the MEC can be assessed based on in vitro data. A dosage required to achieve the MEC depends on individual features and an administration route. However, the plasma concentration can be determined by HPLC (high performance liquid chromatography) assays or bioassays.

The dosing interval may also be determined based on MEC values. The composition should be administered using a therapeutic regimen that maintains plasma levels above the MEC for 10-90% of the time, for 30-90% of the time in some embodiments, and for 50-90% of the time in some embodiments.

In the case of local administration or selective absorption, the effective local concentration of a drug is independent of its plasma concentration.

Of course, the amount of the administered composition depends on an individual to be treated such as body weight of the individual, severity, mode of administration, and judgment of the prescribing physician.

The efficacy and toxicity of the compound of the present disclosure can be assessed using a known method. For example, the toxicology of a particular compound or a subset of compounds that share some chemical moieties can be established by in vitro toxicity assay of cell lines, such as mammalian cell lines and, in some embodiments, human cell lines. The results of such studies are generally predictive of toxicity in an animal such as a mammal, or more specifically, predictive of toxicity in a human. Alternatively, the toxicity of a particular compound may be determined in an animal model such as mouse, rat, rabbit, or monkey using a known method. The efficacy of a particular compound can be determined using several recognized methods, such as in vitro assays, animal models, or human clinical trials. A recognized in vitro model is available for almost every category of conditions, including but not limited to cancers, cardiovascular diseases, and various immune disorders. Similarly, the efficacy of chemical drugs for treating these conditions can be determined using acceptable animal models. When a model is to be selected to measure efficacy, the skilled person could choose appropriate model, dosage, administration route and therapeutic regimen under the guidance of the prior art in this field. Of course, human clinical trials can be further used to determine the efficacy of the compound in a human.

The composition may be, if necessary, placed in a package or a dispensing device, the package or dispensing device may comprise one or more unit dosage forms containing the active ingredient. The package may include, for example, a metal or plastic foil, such as blister package. The package or dispensing device may be provided with a manual for administration. The package or dispensing device may be further provided with precautions associated with the container, which are prescribed by a government agency regulating the manufacture, use, or sale of the drug, and indicate that the form of the drug has been approved by the agency for human or veterinary administration. Such precautions, for example, may be a label for a prescription drug or a product manual approved by the State Food and Drug Administration or the U.S. Food and Drug Administration. The composition comprising the compound of the disclosure, the stereoisomer thereof, or the pharmaceutically acceptable salt thereof may also be prepared in an appropriate container or placed in a compatible pharmaceutical vehicle, and labeled for the treatment of a specified condition.

The present disclosure will be illustrated in detail below with reference to the examples to facilitate better understanding of various aspects of the present disclosure and advantages thereof. However, it should be understood that the examples below are non-limited, and merely used to illustrate some embodiments of the present disclosure.

### EXAMPLES

### Compound abbreviations:

aq: aqueous solution
mL: milliliter
g: gram
V: volume/weight
eq: equivalence ratio
L: liter
M: mol/liter
mg: milligram
µl: microliter
HOBt: 1-hydroxybenzotriazole
DIEA: diisopropylethylamine
DCM: dichloromethane
PE: petroleum ether
EA: ethyl acetate
DMSO: dimethyl sulfoxide
ACN: acetonitrile
MeOH: methanol
DMF: N,N-dimethylformamide
PPh₃: triphenylphosphine
THF: tetrahydrofuran
VCNa: sodium ascorbate
NaH: sodium hydride
CuSO₄: anhydrous copper sulfate
HCl: hydrochloric acid
NaOH: sodium hydroxide
CBr₄: carbon tetrabromide
AcOH: glacial acetic acid
N₂: nitrogen
NaCl: sodium chloride
Na₂CO₃: sodium carbonate
Na₂SO₄: sodium sulfate
CHCl₃: chloroform
NaBH₄: sodium borohydride
IPA: isopropanol
MTBE: methyl tert-butyl ether
HATU: 2-(7-azabenzotriazole)-N,N,N',N'-tetramethyluronium hexafluorophosphate
NH₄Cl: ammonium chloride
EtOH: ethanol
Py: pyridine
DBU: 1,8-diazabicycloundec-7-ene
Zn: zinc powder
DMAP: 4-dimethylaminopyridine
H₃PO₄: phosphoric acid
TEA: triethylamine
Ac₂O: acetic anhydride
H₂SO₄: concentrated sulfuric acid
TBSCl: tert-butyldimethylchlorosilane
KMnO₄: potassium permanganate
HF: hydrogen fluoride
SOCl₂: thionyl chloride
EDCI: 1-(3-dimethylaminopropyl)-3-ethylcarbodiimide hydrochloride
HPLC: high performance liquid chromatography
MS: mass spectrometry
1H NMR: Proton nuclear magnetic resonance spectroscopy
DMSO-d6: a form of dimethyl sulfoxide (DMSO), (CH₃)₂S=O, wherein a hydrogen (H) thereof is replaced with an isotope deuterium (D).

### Example 1

### 1-1: N-(4'-amino-3,3'-dimethyl-[1,1'-biphenyl]-4-yl)-6-bromohexanamide

A solution of 6-bromohexanoic acid (2 g, in dichloromethane (40 mL) was added dropwise to a reaction system of 3,3'-dimethylbenzidine (2.177 g), EDCI (2.359 g), HOBt (1.663 g), DIEA (1.590 g), and dichloromethane (60 mL) at 0-5°C. After the dropwise addition was completed, the mixture was naturally warmed to room temperature, and stirred for reaction overnight. A saturated aqueous solution of sodium bicarbonate (20 mL) was added to the system, and stirred for liquid separation. The aqueous phase was extracted with dichloromethane three times. The organic phases were collected, combined, dried, and concentrated under reduced pressure, to give a crude product, which was purified by column chromatography to give 2.047g of the product. HPLC purity: 93.09%.

MS (m/e): 389.20 [M+H]⁺. ¹HNMR (DMSO-d₆, 400MHz) δ: 9.214 (s, 1H), 7.373-7.295 (m, 3H), 7.245 (s, 1H), 7.218-7.193 (dd, 1H), 6.673-6.652 (d, 1H), 4.962 (s, 2H), 3.581-3.548 (t, 2H), 2.365-2.328 (t, 2H), 2.227 (s, 3H), 2.118 (s, 3H), 1.893-1.823 (m, 2H), 1.662-1.606 (m, 2H), 1.500-1.443 (m, 2H).

### 1-2: N-(4'-amino-3,3'-dimethyl-[1,1'-biphenyl]-4-yl)-6-azidohexanamide

N-(4'-amino-3,3'-dimethyl-[1,1'-biphenyl]-4-yl)-6-bromohexanamide (7.3 g) and dimethyl sulfoxide (51 mL) were stirred at room temperature until full dissolution, and sodium azide (1.83 g) was added. The mixture was stirred at room temperature overnight, and extracted with dichloromethane (200 mL) and water (200 mL). After liquid separation, the aqueous phase was extracted with dichloromethane (200 mL) twice. The organic phases were collected, combined, and washed with a saturated aqueous solution of sodium chloride (100 mL) four times. The organic phase was dried and concentrated under reduced pressure to give a crude product, which was purified by column chromatography to give 2.808 g of the product. HPLC purity: 93.32%.

MS (m/e): 352.24 [M+H]⁺. ¹H NMR (DMSO-d₆, 400 MHz) δ: 9.202 (s, 1 H), 7.367-7.292 (m, 3 H), 7.238-7.190 (m, 2 H), 6.666-6.646 (d, 1 H), 4.937 (s, 2 H), 3.362-3.327 (m, 2H), 2.358-2.322 (t, 2 H), 2.220 (s, 3 H), 2.112 (s, 3 H), 1.653-1.566 (m, 4 H), 1.427-1.369 (m, 2 H).

### 1-3: sodium (E)-4-amino-6-((4'-(6-azidohexanamido)-3,3'-dimethyl-[1.1'-biphenyl]-4-yl)diazenyl)-5-hydroxynaphthalene-1,3-disulfonate

Step 1: N-(4'-amino-3,3'-dimethyl-[1,1'-biphenyl]-4-yl)-6-azidohexanamide (1 g, 1.0 eq) was added to acetonitrile (16 mL), while stirring under argon protection until full dissolution. The system was cooled to 0-5°C in an ice-salt bath, and a hydrochloric acid solution (0.712 mL of 12M hydrochloric acid in 24 mL of deionized water) was added dropwise. After the dropwise addition was completed, the system was stirred for 5 min, and maintained at 0-5°C. A sodium nitrite solution (392 mg of sodium nitrite in 12 mL of deionized water) was added dropwise. After the dropwise addition was completed, the system was maintained at 0-5°C, and stirred for 30 min.

Step 2: sodium 4-amino-5-hydroxy-3-sulfonaphthalene-1-sulfonate (970 mg) was added to deionized water (19.4 mL). The system was stirred under argon protection, cooled in an ice-salt bath to 0-5°C, and maintained at 0-5°C. A sodium bicarbonate solution (956 mg of sodium bicarbonate in 20 mL of deionized water) was added dropwise. After the dropwise addition was completed, the system was maintained at 0-5°C, and stirred for 5 min.

Step 3: The system was maintained at 0-5°C, and the reaction mixture from step 1 was slowly added to the reaction system from step 2. The system was maintained at 0-5°C, and stirred for reaction for 3 h. An aqueous solution of sodium chloride (20%, 76 mL) was added to the system, and stirred at room temperature overnight. The reaction mixture was centrifuged, and the filter cake was collected, to give a purple solid, which was purified by preparative medium pressure liquid chromatography to give 1.64 g of the product. HPLC purity: 98.95%.

MS (m/e): 682.31 [M-2Na+3H]⁺. ¹HNMR (DMSO-d₆, 600MHz) δ: 15.954 (s, 1H), 9.643 (s, 1H), 9.277 (s, 1H), 8.362 (s, 1H), 8.042-8.025 (d, 1H), 7.902-7.887 (d, 1H), 7.652-7.650 (m, 2H), 7.572 (s, 1H), 7.500 (s, 2H), 7.003-6.987 (d, 1H), 3.366-3.344 (t, 2H), 2.517 (s, 3H), 2.385-2.361 (t, 2H), 2.277 (s, 3H), 1.661-1.581 (m, 4H), 1.422-1.397 (m, 2H).

### 1-4: (2S,3S,4S,5R,6S)-2-(methoxycarbonyl)-6-(4-((((4-nitrophenoxy)carbonyl)oxy)methyl)-3-(prop-2-yn-1-yloxy)phenoxy)tetrahydro-2H-pyran-3,4,5-triacetate

(2S,3R,4S,5S,6S)-2-(4-(hydroxymethyl)-3-(prop-2-yn-1-yloxy)phenoxy)-6-(methoxycarbonyl)tetrahydro-2H-pyran-3,4,5-triacetate (4.0 g), dichloromethane (240 mL), p-nitrophenyl chloroformate (3.26 g), and triethylamine (2.46 g) were stirred under nitrogen protection at room temperature overnight. The reaction liquid was concentrated under reduced pressure and purified by column chromatography to give 2.94 g of the product with an HPLC purity of 97.05%. MS (m/e): 682.36 [M+Na]⁺.

### 1-5: (2S,3R,4S,5S,6S)-2-(4-((5S,8S,11S,12R)-11-((S)-sec-butyl)-12-(2-((S)-2-((1R,2R)-3-(((1S,2R)-1-hydroxy-1-phenylprop-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-(prop-2-yn-1-yloxy)phenoxy)-6-(methoxy carbonyl)tetrahydro-2H-pyran-3,4,5-triacetate

(2S,3S,4S,5R,6S)-2-(methoxycarbonyl)-6-(4-((((4-nitrophenoxy)carbonyl) oxy)methyl)-3-(prop-2-yn-1-yloxy)phenoxy)tetrahydro-2H-pyran-3,4,5-triacetate (138 mg), (S)-N-((3R,4S,5S)-1-((S)-2-((1R,2R)-3-(((1S,2R)-1-hydroxy-1-phenylpropan -2-yl)amino)-1-methoxy-2-methyl-3-oxopropyl)pyrrolidin-1-yl)-3-methoxy-5-methyl-1-oxoheptan-4-yl)-N,3-dimethyl-2-((S)-3-methyl-2-(methylamino)butanamido) butanamide (100 mg), DMF (5 mL), HOBt (19 mg), DIEA (22 mg), and pyridine (1.25 mL) were stirred at room temperature overnight. The reaction liquid was concentrated under reduced pressure, and purified by preparative thin layer chromatography to give 124 mg of the product with an HPLC purity of 98.26%. MS (m/e): 1238.78[M+H]⁺.

### 1-6: (2S,3S,4S,5R,6S)-6-(4-((5S,8S,11S,12R)-11-((S)-sec-butyl)-12-(2-((S)-2-((1R,2R)-3-((15)2R):1-hydroxy-1-phenylpropan-2-yl)amino)-1-methoxy-2-methyl-3-oxopropyl)pyrrolidin-1-yl)-2-oxoethyl)5,8-diisopropyl-4, 10-dimethyl-3,6,9-trioxo- 2,13-dioxa-4,7,10-triazatetrad(prop-2-yn-11ýloxy)phenoxy)-3,4,5-trihydroxytetrahydro-2H-pyran-2-carboxylic acid

An aqueous solution of sodium hydroxide (6.5 mg in 0.65 mL of deionized water) was added dropwise to a mixture of (2S,3R,4S,5S,6S)-2-(4-((5S,8S, 11S,12R)-11-((S)-sec-butyl)-12-()2-((S)-2-((1R,2R)-3-((1522R)-1-hydroxy-1-phenylpropan-2-yl)amino)-1-methoxy-2-methyl-3-oxopropyl)pyrolidin-1-yl)-2-oxoethyl)-5,8-diisopropyl-4,10-dimethyl-3,6,9-trioxo-2,13-dioxa-4,7,10-triazatetradecyl)2-yn-1-yloxy)phenoxy)6-(methoxycarbonyl)tetrahydro-2H-pyran-3,4,5-triyl triacetate (50 mg) and tetrahydrofuran (0.65 mL) at a temperature of 0-5°C. After the dropwise addition was completed, the system was naturally warmed to room temperature, stirred for reaction for about 1.5 h, and cooled to 0°C. 1 M hydrochloric acid was added dropwise (system at a pH of about 4) to quench the reaction. The reaction liquid was concentrated under reduced pressure, and purified by preparative medium pressure liquid chromatography to give 40 mg of the product. HPLC purity: 98.60%. MS (m/e): 1098.66 [M+H]⁺.

### 1-7: sodium (2S,3S,4S,5R,6S)-6-(3-((1-(6-((4'-((E)-(8-amino-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate

(2S,3S,4S,SR,6S)-6-(4-((5S,8S,11S,12R)-11-((S)-sec-butyl)-122-(2-((S)-2((1R,2R)-3-((1S,2R)-1-hydroxy-1-phenylpropan-2-yl)amino)-1-methoxy-2methyl-3-oxopropyl)pyrrolidin-1-yl)-2-oxoethyl)-5,8-diisopropyl-4,10-dimethyl3,6,9-trioxo-2, 13-dioxa-4,7,10-triazatetradecyl)-3-(prop-2-yn-1-yloxy)phenoxy)3.4.5-trihydroxytetrahydro-2H-pyran-2-carboxylic acid (36 mg) and disodium (E)-4-amino-6-((4'-(6-azidohexanamido)-3,3'-dimethyl-[1,1'-biphenyl]-4-yl)diazenyl)-5-hydroxynaphthalene-1,3-disulfonate (29 mg) were added to dimethyl sulfoxide (2 mL) in dark place at room temperature, and dissolved while stirring. An aqueous solution of copper sulfate (4.2 mg, in 0.3 mL of deionized water) and an aqueous solution of sodium ascorbate (10.4 mg, in 0.3 mL of deionized water) were added sequentially, deionized water (1.6 mL) was added. The reaction liquid was stirred under argon protection at room temperature for reaction for 1 h. The crude reaction mixture was purified by preparative medium pressure liquid chromatography to give 25 mg of the product. HPLC purity: 93.85%. MS (m/e): 1779.67 [M-3Na+4H]⁺.

### Example 2

### 2-1: (2S,3S,4S,5R,6S)-2-(methoxycarbonyl)-6-(3-(prop-2-yn-1-yloxy)-4-((2,2,2-trichloro-1-iminoethoxy)methyl)phenoxy)tetrahydro-2H-pyran-3,4,5-trifyl triacetate

(2S,3R,4S,5S,6S)-2-(4-(hydroxymethyl)-3-(prop-2-yn-1-yloxy)phenoxy)-6-(methoxycarbonyl)tetrahydro-2H-pyran-3,4,5-triyl triacetate (1 g) was added to dichloromethane (10 mL) until full dissolution. Trichloroacetonitrile (365 mg) and DBU (30.8 mg) were added sequentially while stirring. The mixture was stirred at room temperature for reaction for 4 h, to give the product. The reaction liquid was directly used in the next reaction step, with a yield of 100%.

### 2-2: (25,3R,4S,5S,6S)-2-(4-(15,2R)-2-((2R,3R)-3-((S)-1-((3R,4S,5S)-4-(S)-4-(S)-S)-2(dimethylamino)-3-methylbutanamido)-N,3-dimethylbutanamido)-3-methoxy-5-methylheptanoyl)pyrrolidin-2-yl)-3-methoxy-2-methylpropanamido)-1-phenylpropoxy)methyl)-3-(prop-2-yn-1-yloxy)phenoxy)-6-(methoxycarbonyl)tetdro2H-pyran-3.4.5-triyl triacetate

(2S,3S,4S,5R,6S)-2-(methoxycarbonyl)-6-(3-(prop-2-yn-1-yloxy)-4-((2,2222-trichloro-1-iminoethoxy)methyl)phenoxy)tetrahydro-2H-pyran-3.4.5-triyl triacetate (52 mg) and (S)-2-((S)-2-(dimethylamino)-3-methylbutanamido)-N-((3R,4S,5S)-1-((S)-2-((1R,2R)-3-((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethylbutanamide (30 mg) were dissolved in anhydrous dichloromethane (2 mL). D-camphorsulfonic acid (1.1 mg) was added while stirring at room temperature. The reaction mixture was stirred under argon protection at room temperature for 4 h, and concentrated under reduced pressure to give a crude product, which was purified by preparative thin layer chromatography to give 30 mg of the product, with an HPLC purity of 78.44%. MS (m/e): 1208.85 [M+H]⁺.

### 2-3: (2S,3S,4S,5R,6S)-6-(4-(((1S,2R)-2-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropionamido)-1-phenylpropoxy)methyl)-3-(prop-2-yn-1-yloxy)phenoxy)-3,4,5-trihydroxytetrahydro-2H-pyran-2-carboxylic acid

(2S,3R,4S,5S,6S)-2-(4-115,2R)-2-((2R,3R)-3-((S)-1-((3R,4S,5S)4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-1-phenylpropoxy)methyl)-3-(prop-2-yn-1-yloxy)phenoxy)-6-(methoxycarbonyl)tetrahydro-2H-pyran-3,4,55-triyl triacetate (30 mg) was added to methanol (2 mL) while stirring. At 0-5°C, an aqueous solution of sodium hydroxide (10 mg, in 2 mL of deionized water) was added dropwise. After the addition was completed, the system was naturally warmed to room temperature, stirred for reaction for about 2 h, and cooled to 0°C. 1 M hydrochloric acid was added dropwise (system at a pH of about 4) to quench the reaction. The reaction liquid was concentrated under reduced pressure, and purified by preparative medium pressure liquid chromatography to give 27 mg of the product. HPLC purity: 72.35%. MS (m/e): 1068.92 [M+H]⁺.

### 2-4: sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-(((1S,2R)-2-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-1-phenylpropoxy)methyl)phenoxy)-3,4,5-trihydroxytetrahydro-2H-pyran-2-carboxylate

14 mg of the product was synthesized from (2S,3S,4S,5R,6S)-6-(4-(((1S,2R)-2-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropionamido)-1-phenylpropoxy)methyl)-3-(prop-2-yn-1-yloxy)phenoxy)-3,4,5-trihydroxytetrahydro-2H-pyran-2-carboxylic acid and disodium (E)-4-amino-6-((4'-(6-azidohexanamido)-3,3'-dimethyl-[1,1'-biphenyl]-4-yl)diazenyl)-5-hydroxynaphthalene-1,3-disulfonate using the synthesis method of example 1-7. HPLC purity: 91.58%. MS (m/e): 1749.80 [M-3Na+4H]⁺.

### Example 3

### 3-1: (2S,3R,4S,5S,6S)-2-(4-(bromomethyl)-3-(prop-2-yn-1-yloxy)phenoxy)-6-(methoxycarbonyl)tetrahydro-2H-pyran-3,4,5-triyl triacetate

(2S,3R,4S,5S,6S)-2-(4-(hydroxymethyl)-3-(prop-2-yn-1-yloxy)phenoxy)-6-(methoxycarbonyl)tetrahydro-2H-pyran-3,4,5-triyl triacetate (1.0 g), carbon tetrabromide (1.01 g), and dichloromethane (8 m) were stirred. Triphenylphosphine (796 mg) was added batchwise at 0-5°C. After the addition was completed, the system was naturally warmed to room temperature, stirred for reaction for 2 h, concentrated under reduced pressure, and purified by column chromatography to give 743 mg of the product. HPLC purity: 98.43%.

MS (m/e): 579.15 [M+Na]⁺. ¹HNMR (CDCl₃, 400MHz) δ: 7.277-7.263 (d, 1H), 6.752-6.721 (d, 1H), 6.640-6.586 (dd, 1H), 5.354-5.337 (m, 2H), 5.292-5.263 (m, 1H), 5.159-5.146 (d, 1H), 4.758-4.754 (d, 2H), 4.542-4.506 (d, 2H), 4.189-4.173 (m, 1H), 3.730 (s, 3H), 2.541-2.533 (t, 1H), 2.064 (s, 3H), 2.051 (s, 3H), 2.043 (s, 3H).

### 3-2: (2S,3R,4S,5S,6S)-2-(4-((3S,6S,9S, 10R)-9-((S)-sec-butyl)-10-(2-((S)-2-((1R.2R)-3-(((1S.2R)-1-hydroxy-1-phenylpropan-2-yl)amino)-1-methoxy-2-methyl-3oxopropyl)pyrrolidin-1-yl)-2-oxoethyl)-3,6-diisopropyl-2,8-dimethyi-4,7-dioxo-11-oxa-2,5,8-triazadodecyl)-3-(prop-2-yn-1-yloxy)phenoxy)-6-(methoxycarbonyl)tetrahydro-2Hpyran-3.4.5-triyl triacetate

(2S,3R,4S,5S,6S)-2-(4-(bromomethyl)-3-(prop-2-yn-1-yloxy)phenoxy)-6-(methoxycarbonyl)tetrahydro-2H-pyran-3.4.5-triyl triacetate (116 mg) and (S)-N-((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethyl-2-((S)-3-methyl-2-(methylamino)butanamido)butanamide (50 mg) were dissolved in dichloromethane (5 mL). DIEA (27 mg) was added. The reaction liquid was stirred at room temperature overnight, concentrated under reduced pressure, and purified by preparative thin layer chromatography to give 62 mg of the product with an HPLC purity of 99.36%. MS (m/e): 1194.74 [M+H]⁺.

### 3-3: (2S,3S,4S,5R,6S)-6-(4-((3S,6S,9S,10R)-9-((S)-sec-butyl)-10-(2-((S)-2-((1R,2R)-3-(((1S,2R)-1-hydroxy-1-phenylprop-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecyl)-3-(prop-2-yn-1-yloxy)phenoxy)-3,4,5-trihydroxytetrahydro-2H-pyran-2-carboxylic acid

55 mg of the product was prepared by hydrolyzing (2S,3R,4S,5S,6S)-2-(4-((3S,6S,9S, 10R)-9-((S)-sec-butyl)-10-(2-((S)-22-((1R,2R)-3-(15,2R)1-hydroxy-1-phenylpropan-2-yl)amino)-1-methoxy-2-methyl-3-oxopropbyl)pyrrolidin-1-yl)-2-oxoethyl)-3,6-diisopropyl-2,8-dimethyl-4,7-dioxo-11-oxa-2,5,8-triazadodecyp-2-yn-1-yloxy)phenoxy)-6-(methoxycarbonyl)tetrahydro-2H-pyran-3,4,55-triyl triacetate using the synthesis method of example 1-6. HPLC purity: 79.39%. MS (m/e): 1054.79 [M+H]⁺.

### 3-4: sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecyl)phenoxy)-3,4,5-trihydroxytetrahydro-2H-pyran-2-carboxylate

20 mg of the product was prepared from (2S,3S,4S,5R,6S)-6-(4-((3S,6S,9S,10R)-9-((S)-sec-butyl)-10-(2-((S)-2-((1R,2R)-3-(((1S,2R)-1-hydroxy-1-phenylprop-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecyl)-3-(prop-2-yn-1-yloxy)phenoxy)-3,4,5-trihydroxytetrahydro-2H-pyran-2-carboxylic acid and sodium (E)-4-amino-6-((4'-(6-azidohexanamido)-3,3'-dimethyl-[1,1'-biphenyl]-4-yl)diazenyl)-5-hydroxynaphthalene-1,3-disulfonate using the synthesis method of example 1-7. HPLC purity: 96.89%. MS (m/e): 1735.77 [M-3Na+4H]⁺.

### Example 4

### 4-1: (S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-N,N,3-trimethyl-1-oxo-N-(2-(prop-2-yn-1-yloxy)-4-(((2S,3R,4S,5S,6S)-3,4,5-triacetoxy-6-(methoxycarbonyl)tetrahydro-2H-pyran-2-yl)oxy)benzyl)butan-2-aminium bromide

(2S.3R.4S.5S.6S)-2-(4-(bromomethyl)-3-(prop-2-yn-1-yloxy)phenoxy)-6-(methoxycarbonyl)tetrahydro-2H-pyran-3.4.5-triyl triacetate (114 mg), (S)-2-((S)-2-(dimethylamino)-3-methylbutanamido)-N-((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethylbutanamide (100 mg), and 2-butanone (10 mL) were stirred at 80°C for reaction for 12 h. The reaction liquid was concentrated under reduced pressure, and purified by preparative medium pressure liquid chromatography to give 70 mg of the product. HPLC purity: 97.09%. MS (m/e): 1208.75 [M-Br]⁺.

### 4-2: (2S,3S,4S,5R,65)-6-(4-((3S,6S,9S,10R)-9-((S)-sec-butyl)-10-(2-((S)-2-((1R,2R)-3-(((1S,2R)-1-hydroxy-1-phenylprop-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ammonio-1-yl)-3-(prop-2-yn-1-yloxy)phenoxy)-3,4,5-trihydroxytetrahydro-2H-pyran-2-carboxylate

36 mg of the product was prepared by hydrolyzing (S)-1-((IS)-1-((I)R,4S,5S)-1-((S)-2-((1R,2R)-3-((1S,2R)-1-hydroxy-1-phenylpropan-2-(1)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-N,N,3-trimethyl-1-oxo-N-(2-(prop-2-yn-1-yloxy)-4-((2S,3R,4S,5S,6S)-3,4,5triacetoxy-6-(methoxycarbonyl)tetrahydro-2H-pyran-2-yl)oxy)benzyl)butan-2-aminium bromide using the synthesis method of example 1-6. HPLC purity: 98.38%. MS (m/e): 1068.78 [M+H]⁺.

### 4-3: sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate

54 mg of the product was prepared from (2S,3S,4S,5R,6S)-6-(4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ammonio-1-yl)-3-(prop-2-yn-1-yloxy)phenoxy)-3,4,5-trihydroxytetrahydro-2H-pyran-2-carboxylate and sodium (E)-4-amino-6-((4'-(6-azidohexanamido)-3,3'-dimethyl-[1,1'-biphenyl]-4-yl)diazenyl)-5-hydroxynaphthalene-1,3-disulfonate using the synthesis method of example 1-7. HPLC purity: 99.16%. MS (m/e): 1749.86 [M-2Na+3H]⁺.

### Example 5

### 5-1: methyl ((2R,3R)-3-((S)-1-((3R,4S,SS)-4-((S)-N,3-dimethyl-2-((S)-3-methyl-2-(methylamino)butanamido)butanamido)-3-methoxy-5-methylheptanoyl)pyrrolidin-2-yl)-3-methoxy-2-methylpropionyl)-L-phenylalaninate

Thionyl chloride (49 mg) was added dropwise to ((2R,3R)-3-((S)-1-((3R,4S,5S)-4-((S)-N,3-dimethyl-2-((S)-3-methyl-2-(methylamino)butanamido)butanamido)-3-methoxy-5-methylheptanoyl)pyrrolidin-2-yl)-3-methoxy-2-methylpropionamido)-L-phenylalanine (100 mg) and methanol (5 mL) at 0-5°C. After the dropwise addition was completed, the system was naturally warmed to room temperature, and stirred for reaction overnight. The reaction liquid was concentrated under reduced pressure, dissolved in ethyl acetate, washed with a saturated aqueous solution of sodium bicarbonate and a saturated aqueous solution of sodium chloride sequentially, and dried over anhydrous sodium sulfate. The organic phase was concentrated under reduced pressure to give 101 mg of the product. HPLC purity: 95.40%. MS (m/e): 746.63 [M+H]⁺.

### 5-2: (2S,3R,4S,5S,6S)-2-(4-((5S,8S,11S,12R)-11-((S)-sec-butyl)-5,8-diisopropy)-methoxy-3-((S)-1-methoxy-1-oxo-3-phenylpropan-2-yl)amino)-2-methyl-3-oxoprl)pyrrolidin-1yl)-2-oxoethyl)-4,10-dimethyl-3,6,9-trioxo-2,13-dioxa-4,7,10-triazatetradecyl)-3(prop-1-yn-1-yloxy)phenoxy)-6-(methoxycarbonyl)tetrahydro-2H-pyran-3,4,5-triyl triacetate

40 mg of the product was prepared from methyl ((2R,3R)-3-((S)-1-((3R,4S,5S)-4-((S)-N,3-dimethyl-2-((S)-3-methyl-2-(methylamino)butanamido)butanamido)-3-methoxy-5-methylheptanoyl)pyrrolidin-2-yl)-3-methoxy-2-methylpropionyl)-L-phenylalaninate and (2S,3S,4S,5R,6S)-2-(methoxycarbonyl)-6-(4-((((4-nitrophenoxy)carbonyl)oxy)methyl)-3-(prop-2-yn-1-yloxy)phenoxy)tetrahydro-2H-pyran-3,4,5-triyl triacetate using the synthesis method of example 1-5, HPLC purity: 81.73%. MS (m/e): 1266.81 [M+H]⁺.

### 5-3: (2S,3S,4S,5R,6S)-6-(4-((5S,8S,11S,12R)-11-((S)-sec-butyl)-12-(2-((S)-2-((1R,2R)-3-(((S)-1-carboxy-2-phenethyl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-(prop-2-yn-1-yloxy)phenoxy)-3,4,5-trihydroxytetrahydro-2H-pyran-2-carboxylic acid

7 mg of the product was prepared from (2S,3R,4S,5S,6S)-2-(4-((5S,8S,11S,12R)-11-((S)-sec-butyl)-5,8-diisopropyl-12-(2-((S)-2-((1R,2R)-1-methoxy-3-(((S)-1-methoxy-1-oxo-3-phenylprop-2-yl)amino)-2-methyl-3-oxopropyl)pyrrolidin-1-yl)-2-oxoethyl)-4,10-dimethyl-3,6,9-trioxo-2,13-dioxa-4,7,10-triazatetradecyl)-3-(prop-2-yn-1-yloxy)phenoxy)-6-(methoxycarbonyl)tetrahydro-2H-pyran-3,4,5-triyl triacetate using the synthesis method of example 1-6. HPLC purity: 97.20%. MS (m/e): 1112.82 [M+H]⁺.

### 5-4: sodium (2S,3S,4S,5R,6S)-6-(3-((1-(6-((4'-((E)-(8-amino-1-hydroxy-5,7-disulfonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-1 1-((S)-sec-butyl)-12-(2-((S)-2-((1R,2R)-3-(((S)-1-carboxylate-2-phenethyl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate

1 mg of the product was prepared from (2S,3S,4S,5R,6S)-6-(4-((5S,8S,11S,12R)-11-((S)-sec-butyl)-12-(2-((S)-2-((1R,2R)-3-(((S)-1-carboxy-2-phenethyl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-(prop-2-yn-1-yloxy)phenoxy)-3,4,5-trihydroxytetrahydro-2H-pyran-2-carboxylic acid and sodium (E)-4-amino-6-((4'-(6-azidohexanamido)-3,3'-dimethyl-[1,1'-biphenyl]-4-yl)diazenyl)-5-hydroxynaphthalene-1,3-disulfonate using the synthesis method of example 1-7. HPLC purity: 89.97%. MS (m/e): 1793.88 [M-4Na+5H]⁺.

### Example 6

### 6-1: (2S,3R,4S,5S,6S)-2-(4-((3S,6S,9S,10R)-9-((S)-sec-butyl)-3,6-diisopropyl-10-(2-((S)-2-((1R,2R)-1-methoxy-3-(((S)-1-methoxy-1-oxo-3-phenylprop-2-yl)amino)-2-methyl-3-oxopropyl)pyrrolidin-1-yl)-2-oxoethyl)-2,8-dimethyl-4,7-dioxo-11-oxa-2,5,8-triazadodecyl)-3-(prop-2-yn-1-yloxy)phenoxy)-6-(methoxycarbonyl)tetrahydro-2H-pyran-3,4,5-triyl triacetate

(2S,3R,4S,5S,6S)-2-(4-(bromomethyl)-3-(prop-2-ynyloxy)phenoxy)-6-(methoxycarbonyl)tetrahydro-2H-pyran-3,4,5-triyl triacetate and the compound methyl ((2R,3R)-3-((S)-1-((3R,4S,5S)-4-((S)-N,3-dimethyl-2-((S)-3-methyl-2-(methylamino)butanamido)butanamido)-3-methoxy-5-methylheptanoyl)pyrrolidin-2-yl)-3-methoxy-2-methylpropionyl)-L-phenylalaninate (50 mg) was dissolved in DMF (1 mL). DIEA (26 mg) was added. The reaction liquid was stirred at room temperature overnight, concentrated under reduced pressure, and purified by preparative medium pressure liquid chromatography to give 40 mg of the product with an HPLC purity of 94.66%. MS (m/e): 1222.80 [M+H]⁺.

### 6-2: (2S,3S,4S,5R,6S)-6-(4-((3S,6S,9S,10R)-9-((S)-sec-butyl)-10-(2-((S)-2-((1R,2R)-3-(((S)-1-carboxy-2-phenethyl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecyl)-3-(prop-2-yn-1-yloxy)phenoxy)-3,4,5-trihydroxytetrahydro-2H-pyran-2-carboxylic acid

23 mg of the product was prepared by hydrolyzing (2S,3R,4S,5S,6S)-2-(4-((3S,6S,9S,10R)-9-((S)-sec-butyl)-3,6-diisopropyl-10-(2-((S)-2-((1R,2R)-1-methoxy-3-(((S)-1-methoxy-1-oxo-3-phenylprop-2-yl)amino)-2-methyl-3-oxopropyl)pyrrolidin-1-yl)-2-oxoethyl)-2,8-dimethyl-4,7-dioxo-11-oxa-2,5,8-triazadodecyl)-3-(prop-2-yn-1-yloxy)phenoxy)-6-(methoxycarbonyl)tetrahydro-2H-pyran-3,4,5-triyl triacetate using the synthesis method of example 1-6. HPLC purity: 91.34%. MS (m/e): 1068.72 [M+H]⁺.

### 6-3: sodium (2S,3S,4S,5R,6S)-6-(3-((1-(6-((4'-((E)-(8-amino-1-hydroxy-5,7-disulfonaphthalen-2-yl)diazenyl)-3,3'-dimethyl-[1,1'-biphenyl]-4-yl)amino)-6-oxohexyl)-1H-1,2,3-triazol-4-yl)methoxy)4-((3S,6S,9S,10R)-9-((S)-sec-butyl)-10-(2-((S)-2-((1R,2R)-3-(((S)-1-carboxylate-2-phenylethyl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecyl)phenoxy)-3,4,5-trihydroxytetrahydro-2H-pyran-2-carboxylate

4 mg of the product was prepared from (2S,3S,4S,5R,6S)-6-(4-((3S,6S,9S,10R)-9-((S)-sec-butyl)-10-(2-((S)-2-((1R,2R)-3-(((S)-1-carboxy-2-phenylethyl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecyl)-3-(prop-2-yn-1-yloxy)phenoxy)-3,4,5-trihydroxytetrahydro-2H-pyran-2-carboxylic acid and the compound sodium (E)-4-amino-6-((4'-(6-azidohexanamido)-3,3'-dimethyl-[1,1'-biphenyl]-4-yl)diazenyl)-5-hydroxynaphthalene-1,3-disulfonate using the synthesis method of example 1-7. HPLC purity: 89.39%. MS (m/e): 1749.91 [M-4Na+5H]⁺.

### Example 7

### 7-1: (S)-2-((S)-2-(benzyl(methyl)amino)-3-methylbutanamido)-N-((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethylbutanamide

(S)-N-((3R,4S,5S)-1-((S)-2-((1R,2R)-3-((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethyl-2-((S)-3-methyl-2-(methylamino)butanamido)butanamide (200 mg) and sodium triacetoxyborohydride (590 mg) were dissolved in a solution containing N,N-dimethylformamide: acetic acid=10:1 (2.2 mL) while stirring at room temperature, until the solution became clear, and then benzaldehyde (355 mg) was added. The system was kept for reaction for 16 h, and concentrated with an oil pump to give a crude product, which was purified by column chromatography to give 211 mg of the product, MS (m/e): 808.64 [M+H]⁺.

### 7-2: (2S)-N-benzyl-1-(((S)-1-(((3R,4S,5S)-1-((S)-2-((1R,2R)-3-(((1S,2R)-1-hydroxy-1-phenylprop-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutyl-2-yl)amino)-N,3-dimethyl-1-oxo-N-(2-(prop-2-yn-1-yloxy)-4-(((2S,3R,4S,5S,6S)-3,4,5-triacetyloxy-6-(methoxycarbonyl)tetrahydro-2H-pyran-2-yl)oxy)benzyl)butan-2-aminium bromide

(S)-2-((S)-2-(benzyl(methyl)amino)-3-methylbutanamido)-N-((3R,4S,5S)-1-((S)-2-((1R,2R)-3-(((1S,2R)-1-hydroxy-1-phenylprop-2-yl)amino)-1-methoxy-2-methyl-3-oxopropyl)pyrrolidin-1-yl)-3-methoxy-5-methyl-1-oxoheptan-4-yl)-N,3-dimethylbutanamide (656 mg), (2S,3R,4S,5S,6S)-2-(4-(bromomethyl)-3-(prop-2-ynyloxy)phenoxy)-6-(methoxycarbonyl)tetrahydro-2H-pyran-3,4,5-triacetate (1.36 g), and potassium carbonate (56 mg) were dissolved in acetonitrile (6.5 mL). The system was stirred and refluxed in an oil bath at 80°C for 20 h. After the reaction was completed, the system was concentrated to dryness, and the crude product was purified by column chromatography to give 171 mg of the product. MS (m/e): 1285.06 [M-Br]⁺.

### 7-3: (2S,3S,4S,5R,6S)-6-(4-((3S,6S,9S,10R)-2-benzyl-9-((S)-sec-butyl)-10-(2-((S)-2-((1R,2R)-3-(((1S,2R)-1-hydroxy-1-phenylprop-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ammonio-1-yl)-3-(prop-2-yn-1-yloxy)phenoxy)-3,4,5-trihydroxytetrahydro-2H-pyran-2-carboxylate

70 mg of the product was prepared by hydrolyzing (2S)-N-benzyl-1-(((S)-1-(((3R,4S,5S)-1-((S)-2-((1R,2R)-3-(((1S,2R)-1-hydroxy-1-phenylprop-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutyl-2-yl)amino)-N,3-dimethyl-1-oxo-N-(2-(prop-2-yn-1-yloxy)-4-(((2S,3R,4S,5S,6S)-3,4,5-triacetyloxy-6-(methoxycarbonyl)tetrahydro-2H-pyran-2-yl)oxy)benzyl)butan-2-aminium bromide using the synthesis method of example 1-6. MS (m/e): 1144.71 [M+H]⁺.

### 7-4: sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-2-benzyl-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate

83 mg of the product was prepared from (2S,3S,4S,5R,6S)-6-(4-((3S,6S,9S,10R)-2-benzyl-9-((S)-sec-butyl)-10-(2-((S)-2-((1R,2R)-3-(((1S,2R)-1-hydroxy-1-phenylprop-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ammonio-1-yl)-3-(prop-2-yn-1-yloxy)phenoxy)-3,4,5-trihydroxytetrahydro-2H-pyran-2-carboxylate and sodium (E)-4-amino-6-((4'-(6-azidohexanamido)-3,3'-dimethyl-[1,1'-biphenyl]-4-yl)diazenyl)-5-hydroxynaphthalene-1,3-disulfonate using the synthesis method of example 1-7. MS (m/e): 1825.76 [M-2Na+3H]⁺.

### Example 8

### 8-1: (S)-2-((S)-2-(ethyl(methyl)amino)-3-methylbutanamido)-N-((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethylbutanamide

247 mg of the product was prepared from (S)-N-((3R,4S,SS)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethyl-2-((S)-3-methyl-2-(methylamino)butanamido)butanamide and acetaldehyde using the synthesis method of example 7-1. MS (m/e): 746.68 [M+H]⁺.

### 8-2: (2S)-N-ethyl-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-N,3-dimethyl-1-oxo-N-(2-(prop-2-yn-1-yloxy)-4-(((2S,3R,4S,5S,6S)-3,4,5-triacetyloxy-6-(methoxycarbonyl)tetrahydro-2H-pyran-2-yl)oxy)benzyl)butan-2-aminium bromide

(2S,3R,4S,5S,6S)-2-(4-(bromomethyl)-3-(prop-2-ynyloxy)phenoxy)-6-(methoxycarbonyl)tetrahydro-2H-pyran-3,4,5-triacetate (201 mg), (S)-2-((S)-2-(ethyl(methyl)amino)-3-methylbutanamido)-N-((3R,4S,5S)-1-((S)-2-((1R,2R)-3-(((1S,2R)-1-hydroxy-1-phenylprop-2-yl)amino)-1-methoxy-2-methyl-3-oxopropyl)pyrrolidin-1-yl)-3-methoxy-5-methyl-1-oxoheptan-4-yl)-N,3-dimethylbutanamide (207 mg), acetone (4 mL), and N,N-diisopropylethylamine (9 mg) were stirred at 55°C for reaction for 17 h. The reaction liquid was concentrated to dryness. The crude product was purified by column chromatography to give 87 mg of the product. MS (m/e): 1223.05 [M-Br]⁺.

### 8-3: (2S,3S,4S,5R,6S)-6-(4-((3S,6S,9S,10R)-9-((S)-sec-butyl)-2-ethyl-10-(2-((S)-2-((1R,2R)-3-(((1S,2R)-1-hydroxy-1-phenylprop-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)-3-(prop-2-yn-1-yloxy)phenoxy)-3,4,5-trihydroxytetrahydro-2H-pyran-2-carboxylate

54 mg of the product was prepared from (2S)-N-ethyl-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-N,3-dimethyl-1-oxo-N-(2-(prop-2-yn-1-yloxy)-4-(((2S,3R,4S,5S,6S)-3,4,5-triacetyloxy-6-(methoxycarbonyl)tetrahydro-2H-pyran-2-yl)oxy)benzyl)butan-2-aminium bromide using the synthesis method of example 1-6. MS (m/e): 1082.74 [M+H]⁺.

### 8-4: sodium (2S,3S,4S,5R,6S)-6-(3-((1-(2-(3-((4'-((E)-(8-amino-1-hydroxy-5,7-disulfonatonaphthalen-2-yl)diazenyl)-3,3'-dimethyl-[1,1'-biphenyl]-4-yl)amino)-3-oxopropoxy)ethyl)-1H-1,2,3-triazol-4-yl)methoxy)-4-((3S,6S,9S,10R)-9-((S)-sec-butyl)-2-ethyl-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate

70 mg of the product was prepared from (2S,3S,4S,5R,6S)-6-(4-((3S,6S,9S,10R)-9-((S)-sec-butyl)-2-ethyl-10-(2-((S)-2-((1R,2R)-3-(((1S,2R)-1-hydroxy-1-phenylprop-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)-3-(prop-2-yn-1-yloxy)phenoxy)-3,4,5-trihydroxytetrahydro-2H-pyran-2-carboxylate and sodium (E)-4-amino-6-((4'-(3-(2-azidoethoxy)propionamido)-3,3'-dimethyl-[1,1'-biphenyl]-4-yl)diazenyl)-5-hydroxynaphthalene-1,3-disulfonate using the synthesis method of example 1-7. MS (m/e): 1765.98 [M-2Na+3H]⁺.

### Example 9

### 9-1: tert-butyl (4'-amino-3,3'-dimethyl-[1,1'-biphenyl]-4-yl)carbamate

3,3'-dimethylbenzidine (10 g) was dissolved in 1,4-dioxane (170 mL) while stirring. Then, ditert-butyl dicarbonate (11 mL) was diluted with 1,4-dioxane (30 mL), and the diluted solution was slowly added dropwise to the reaction liquid. The mixture was stirred under nitrogen protection at room temperature for reaction for 20 h. The crude product was purified by column chromatography to give 7.4 g of the product.

### 9-2: tert-butyl (4'-(3-(2-bromoethoxy)propionamido)-3,3'-dimethyl-[1,1'-biphenyl]-4-yl)carbamate

tert-butyl (4'-amino-3,3'-dimethyl-[1,1'-biphenyl]-4-yl)carbamate (1.2 g), 3-(2-bromoethoxy)propionic acid (0.84 g), tetrahydrofuran (20 mL), 2-(7-azabenzotriazole)-N,N,N',N'-tetramethyluronium hexafluorophosphate (1.7 g), and diisopropylethylamine (0.46 g) were stirred at room temperature for reaction for 18 h. After the reaction was completed, the reaction mixture was extracted with dichloromethane. The organic phase was dried, filtered, concentrated to dryness, and purified by column chromatography to give 1.6 g of the product. MS (m/e): 491.21 [M+H]⁺.

### 9-3: N-(4'-amino-3,3'-dimethyl-[1,1'-biphenyl]-4-yl)-3-(2-bromoethoxy)propionamide

tert-butyl (4'-(3-(2-bromoethoxy)propionamide)-3,3'-dimethyl-[1,1'-biphenyl]-4-yl)carbamate (1.6 g) was dissolved in dichloromethane (19.2 mL), and then trifluoroacetic acid (1.92 mL) was added. The mixture was stirred at room temperature for 17 h. After the reaction was completed, the mixture was concentrated to dryness. The crude product was purified by column chromatography to give 1.2 g of the product. MS (m/e): 391.27 [M+H]⁺.

### 9-4: N-(4'-amino-3,3'-dimethyl-[1,1'-biphenyl]-4-yl)-3-(2-azidoethoxy)propionamide

770 mg of the product was prepared from N-(4'-amino-3,3'-dimethyl-[1,1'-biphenyl]-4-yl)-3-(2-bromoethoxy)propionamide using the synthesis method of example 1-2. MS (m/e): 354.32 [M+H]⁺.

### 9-5: sodium (E)-4-amino-6-((4'-(3-(2-azidoethoxy)propionamide)-3,3'-dimethyl-[1,1'-biphenyl]-4-yl)diazenyl)-5-hydroxynaphthalene-1,3-disulfonate

379 mg of the product was prepared from N-(4'-amino-3,3'-dimethyl-[1,1'-biphenyl]-4-yl)-3-(2-azidoethoxy)propionamide and monosodium 4-amino-5-hydroxy-1,3-naphthalenedisulfonate using the synthesis method of example 1-3. MS (m/e): 684.63 [M-2Na+3H]⁺.

### 9-6: sodium (2S,3S,4S,5R,6S)-6-(3-((1-(2-(3-((4'-((E)-(8-amino-1-hydroxy-5,7-disulfonatonaphthalen-2-yl)diazenyl)-3,3'-dimethyl-[1,1'-biphenyl]-4-yl)amino)-3-oxopropoxy)ethyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate

109 mg of the product was prepared from (2S,3S,4S,5R,6S)-6-(4-((3S,6S,9S,10R)-9-((S)-sec-butyl)-10-(2-((S)-2-((1R,2R)-3-(((1S,2R)-1-hydroxy-1-phenylprop-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)-3-(prop-2-yn-1-yloxy)phenoxy)-3,4,5-trihydroxytetrahydro-2H-pyran-2-carboxylate and disodium (E)-4-amino-6-((4'-(3-(2-azidoethoxy)propionamido)-3,3'-dimethyl-[1,1'-biphenyl]-4-yl)diazenyl)-5-hydroxynaphthalene-1,3-disulfonate using the synthesis method of example 1-7. MS (m/e): 1751.81 [M-2Na+3H]⁺.

### Example 10

### 10-1: 2-(3-chloropropoxy)acetic acid

ethyl 2-(3-chloropropoxy)acetate (2.5 g) was dissolved in tetrahydrofuran (25 mL) while stirring, and the system was cooled to 0°C in an ice-salt bath. Sodium hydroxide (665 mg) was dissolved in water (25 mL), and then slowly added dropwise to the reaction liquid. The mixture was stirred for reaction for 1.5 h. After the reaction was completed, the reaction liquid was concentrated, adjusted with 1 mol/L HCl to a pH=1, extracted with ethyl acetate 3 times, and subjected to liquid separation. The organic phase was dried over anhydrous magnesium sulfate, filtered, and concentrated, to give 1.7 g of the product.

### 10-2: tert-butyl (4'-(2-(3-chloropropoxy)acetamido)-3,3'-dimethyl-[1,1'-biphenyl]-4-yl)carbamate

2.87 g of the product was prepared from tert-butyl (4'-amino-3,3'-dimethyl-[1,1'-biphenyl]-4-yl)carbamate and 2-(3-chloropropoxy)acetic acid using the synthesis method of example 9-2.

### 10-3: N-(4'-amino-3,3'-dimethyl-[1,1'-biphenyl]-4-yl)-2-(3-chloropropoxy)acetamide

1.315 g of the product was prepared from tert-butyl (4'-(2-(3-chloropropoxy)acetamido)-3,3'-dimethyl-[1,1'-biphenyl]-4-yl)carbamate using the synthesis method of example 9-3. MS (m/e): 347.32 [M+H]+.

### 10-4: N-(4'-amino-3,3'-dimethyl-[1,1'-biphenyl]-4-yl)-2-(3-azidopropoxy)acetamide

N-(4'-amino-3,3'-dimethyl-[1,1'-biphenyl]-4-yl)-2-(3-chloropropoxy)acetamide (1.315 g) was fully dissolved in dimethyl sulfoxide (10 mL) while stirring at room temperature, sodium azide (287 mg) and sodium iodide (441 mg) were added, and the mixture was stirred in an oil bath at 80°C for 15 h. After the reaction was completed, the mixture was extracted with dichloromethane (200 mL) and water (200 mL). After liquid separation, the aqueous phase was extracted with dichloromethane (200 mL) twice. The organic phases were collected, combined, and washed with a saturated aqueous solution of sodium chloride four times. The organic phase was dried and concentrated under reduced pressure to give a crude product, which was purified by column chromatography to give 914 mg of the product. MS (m/e): 354.32 [M+H]⁺.

### 10-5: sodium (E)-4-amino-6-((4'-(2-(3-azidopropoxy)acetamido)-3,3'-dimethyl-[1,1'-biphenyl]-4-yl)diazenyl)-5-hydroxynaphthalene-1,3-disulfonate

345 mg of the product was prepared from N-(4'-amino-3,3'-dimethyl-[1,1'-biphenyl]-4-yl)-2-(3-azidopropoxy)acetamide and monosodium 4-amino-5-hydroxy-1,3-naphthalenedisulfonate using the synthesis method of example 1-3. MS (m/e): 684.34 [M-2Na+3H]+.

### 10-6: sodium (2S,3S,4S,5R,6S)-6-(3-((1-(3-(2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethoxy)propyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate

128 mg of the product was prepared from (2S,3S,4S,5R,6S)-6-(4-((3S,6S,9S,10R)-9-((S)-sec-butyl)-10-(2-((S)-2-((1R,2R)-3-(((1S,2R)-1-hydroxy-1-phenylprop-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)-3-(prop-2-yn-1-yloxy)phenoxy)-3,4,5-trihydroxytetrahydro-2H-pyran-2-carboxylate and disodium (E)-4-amino-6-((4'-(2-(3-azidopropoxy)acetamido)-3,3'-dimethyl-[1,1'-biphenyl]-4-yl)diazenyl)-5-hydroxynaphthalene-1,3-disulfonate using the synthesis method of example 1-7. MS (m/e): 1752.15 [M-2Na+3H]⁺.

### Example 11

106 mg of sodium (2S,3S,4S,5R,6S)-6-(3-((1-(3-(2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethoxy)propyl)-1H-1,2,3-triazol-4-yl)methoxy)-4-((3S,6S,9S,10R)-9-((S)-sec-butyl)-2-ethyl-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate was prepared from (2S,3S,4S,5R,6S)-6-(4-((3S,6S,9S,10R)-9-((S)-sec-butyl)-2-ethyl-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)-3-(prop-2-yn-1-yloxy)phenoxy)-3,4,5-trihydroxytetrahydro-2H-pyran-2-carboxylate and disodium (E)-4-amino-6-((4'-(2-(3-azidopropoxy)acetamido)-3,3'-dimethyl-[1,1'-biphenyl]-4-yl)diazenyl)-5-hydroxynaphthalene-1,3-disulfonate using the synthesis method of example 1-7. MS (m/e): 1766.04 [M-2Na+3H]⁺.

### Example 12

51 mg of sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-2-ethyl-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate was prepared from (2S,3S,4S,5R,6S)-6-(4-((3S,6S,9S,10R)-9-((S)-sec-butyl)-2-ethyl-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)-3-(prop-2-yn-1-yloxy)phenoxy)-3,4,5-trihydroxytetrahydro-2H-pyran-2-carboxylate and disodium (E)-4-amino-6-((4'-(6-azidohexanamido)-3,3'-dimethyl-[1,1'-biphenyl]-4-yl)diazenyl)-5-hydroxynaphthalene-1,3-disulfonate using the synthesis method of example 1-7. MS (m/e): 1763.98 [M-2Na+3H]⁺.

### Example 13

40 mg of sodium (2S,3S,4S,5R,6S)-6-(3-((1-(2-(3-((4'-((E)-(8-amino-1-hydroxy-5,7-disulfonatonaphthalen-2-yl)diazenyl)-3,3'-dimethyl-[1,1'-biphenyl]-4-yl)amino)-3-oxopropoxy)ethyl)-1H-1,2,3-triazol-4-yl)methoxy)-4-((3S,6S,9S,10R)-2-benzyl-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate was prepared from (2S,3S,4S,5R,6S)-6-(4-((3S,6S,9S,10R)-2-benzyl-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)-3-(prop-2-yn-1-yloxy)phenoxy)-3,4,5-trihydroxytetrahydro-2H-pyran-2-carboxylate and disodium (E)-4-amino-6-((4'-(3-(2-azidoethoxy)propionamido)-3,3'-dimethyl-[1,1'-biphenyl]-4-yl)diazenyl)-5-hydroxynaphthalene-1,3-disulfonate using the synthesis method of example 1-7. MS (m/e): 1827.99 [M-2Na+3H]⁺.

### Example 14

51 mg of sodium (2S,3S,4S,5R,6S)-6-(3-((1-(3-(2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethoxy)propyl)-1H-1,2,3-triazol-4-yl)methoxy)-4-((3S,6S,9S,10R)-2-benzyl-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate was prepared from (2S,3S,4S,5R,6S)-6-(4-((3S,6S,9S,10R)-2-benzyl-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)-3-(prop-2-yn-1-yloxy)phenoxy)-3,4,5-trihydroxytetrahydro-2H-pyran-2-carboxylate and disodium (E)-4-amino-6-((4'-(2-(3-azidopropoxy)acetamido)-3,3'-dimethyl-[1,1'-biphenyl]-4-yl)diazenyl)-5-hydroxynaphthalene-1,3-disulfonate using the synthesis method of example 1-7. MS (m/e): 1827.95 [M-2Na+3H]⁺.

### Biological Example 1: Study on in vitro inhibitory effect against human tumor cells

### 1. Experimental materials

### 1.1 Test compounds

The compounds of examples 1 to 14 of the present disclosure are named example compounds 1 to 14 for use in experiments, and hereinafter referred to as the test compounds.

### 1.2 Experimental cells

Human colorectal cancer cell lines HCT116 and SW620, human liver cancer cell line HepG2, human gastric cancer cell line NCI-N87, human triple-negative breast cancer cell line MDA-MB-468, human pharyngeal squamous cell carcinoma cell line Fadu, human ovarian cancer cell line SK-OV-3, human pancreatic cancer cell line BxPC-3, human prostate cancer cell line 22RV1, human non-small cell lung carcinomas cell lines NCI-H1975 and HCC827, and human malignant melanoma cell line A375 were all purchased from the Cell Bank of the Chinese Academy of Sciences.

### 1.3 Main reagents and devices

RPMI-1640 medium: Gibco, with original samples being dry powder, prepared into a liquid medium based on the manual, and supplemented with 2 g/L NaHCO₃ and 5.958 g/L HEPES as required for cell culture.

EMEM (Minimum Essential Medium with Earle's salts): Gibco, with original samples being dry powder, prepared into a liquid medium based on the manual, and supplemented with 2.2 g/L NaHCO₃ and 5.958 g/L HEPES as required for cell culture.

L-15 medium: Gibco, with original samples being dry powder, prepared into a liquid medium based on the manual, and supplemented with 2 g/L NaHCO₃ and 5.958 g/L HEPES as required for cell culture.

McCoy'5A medium: Sigma, with original samples being dry powder, prepared into a liquid medium based on the manual, and supplemented with 1.5 g/L NaHCO₃ and 2.383 g/L HEPES as required for cell culture.

DMEM (Minimum Essential Medium with Earle's salts): Gibco, with original samples being dry powder, prepared into a liquid medium based on the manual, and supplemented with 2.2 g/L NaHCO₃ and 5.958 g/L HEPES as required for cell culture.

FBS (fetal bovine serum): Biological Industries (BI).

βGU (β-glucuronidase): Sigma-Aldrich, prepared into 2 mg/mL stock solution with PBS.

MTT (tetrazolium salt): Sigma-Aldrich, prepared into 5 mg/mL stock solution with PBS.

SRB (Sulforhodamine): Sigma-Aldrich, prepared into 0.4% (w/v) working solution with 1% acetic acid.

Vertical pressure steam sterilizer: Shanghai Shenan Medical Apparatus Factory, model LDZX-50KBS.

CO₂ cell incubator: Thermo, model 311, model 371.

Super-clean bench: Donglian Har Instrument Manufacture Co., Ltd, model DL-CJ-2N, model DL-CJ-1ND.

Microplate reader: Bio-Rad, model 680.

96-well plate washer: Thermo, model N21535.

### 2. Experimental method

A cell culture medium was prepared according to the formulation shown in Table 2 below. Cells were cultured with 5% CO₂ at 37°C and saturated humidity until they reached a logarithmic growth phase (usually adherent cells were nearly fully confluent). After removing adherent cells by trypsin/EDTA digestion, the cells were collected for subsequent experiments.

The cells were inoculated into a 96-well cell culture plate (hereinafter referred to as the 96-well plate) based on the cell count shown in Table 2, cultured overnight to achieve cell adherence, and then, after different concentrations of the test compound were added, further cultured for a total of 3 days. Finally, the total cell protein in the assay wells was assayed by SRB, or the cell viability was assessed by MTT:

2.1 Assaying total cell protein by SRB: the modification is based on the method in the literature (V. Vichai and K. Kirtikara. Sulforhodamine B colorimetric assay for cytotoxicity screening. Nature Protocols. 2006, 1: 1112-1116). Specific method: the culture medium was pipetted out from the wells, the cells were fixed with 10% trichloroacetic acid for more than 1 h, and trichloroacetic acid was removed; the cells were washed with H₂O, and stained with 0.4% SRB for 15-30 min; after excess SRB was removed, the cells were washed with 1% acetic acid; 100 µl of 10 mM tris(trihydroxymethylaminomethane) was added to dissolve the protein-bound SRB, followed by assay at a wavelength of 570 nm.

2.2 Assaying cell viability by MTT: the modification is based on the method in the literature (J. Carmichael, W. DeGraff, A. Gazdar et al., Evaluation of a tetrazolium-based semiautomated colorimetric assay: assessment of chemosensitivity testing. Cancer Research. 1987, 47: 936-942). Specific method:
For well-adherent cells: the medium was pipetted out, 100 µL of a basal medium containing 0.5 mg/mL MTT (medium free of fetal bovine serum (FBS) and other additives) was added to each well, and culture was continued for additional 3 h. The MTT-containing basal medium was pipetted out, and 100 µL of DMSO was added to each well to dissolve formazan, followed by assay at a wavelength of 490 nm.

For poorly-adherent cells: MTT solution was directly added to a concentration of 0.5 mg/mL, culture was continued for additional 3 h, and an equal volume of triple solvent was added to the system to dissolve formazan, followed by assay at a wavelength of 570 nm.

2.3: 1-2 mg of β-glucuronidase was weighed, and dissolved in PBS to prepare 2 mg/mL stock solution. Prior to use, the stock solution was diluted with a complete medium to prepare 12.5 µg/mL enzyme working solution. For assay wells requiring the enzyme, 80 µL of the enzyme working solution was added, with the β-glucuronidase at a concentration of 1 µg/well; and for assay wells not requiring the enzyme, 80 µL of the complete medium was added.

1-3 mg of the test compound was weighed, and dissolved in DMSO to prepare 20 mM stock solution. The stock solution was diluted in a 3-fold gradient with DMSO, then diluted 100-fold with RPMI1640 basal medium to obtain 10× working solutions, with a total of 8 concentrations. 20 µL of the 10× working solutions were add to the assay wells, with the final volume of each well of 200 µL, and with the DMSO at a concentration of 0.1%.

2.4 Calculation of inhibitory activity of compound: the experiment includes a compound test group, a compound-free positive solvent control group (PC), and a negative solvent control group (NC) without cells and the compound. $Inhibition rate = \left[1-\left(test value-NC mean\right)/\left(PC mean-NC mean\right)\right] \times 100 %$

The mean and SD of the inhibition rate were obtained from replicate assay wells. A curve was plotted with the compound concentrations distributed in a logarithmic form as the abscissa and with the mean inhibition rates as the ordinate, and was fitted with a 4-parameter logistic equation. The compound concentration corresponding to 50% inhibition rate on the curve is the IC₅₀ value.

**Table 2: Culture medium formulation and cell inoculation concentration for human tumor cell lines**

| Cell line name | Culture medium formulation | Cell inoculation concentration (count/well) |
|---|---|---|
| HCT116 | 90% McCoy'5A, 10% FBS | 5×10³ |
| SW620 | 90% L-15, 10% FBS | 5×10³ |
| HepG2 | EMEM+10% FBS | 8×10⁴ |
| NCI-N87 | 90% RPMI-1640, 10% FBS, 1 mM sodium pyruvate, 2.5g/L glucose | 1.5×10⁴ |
| MDA-MB-468 | 90% L-15, 10% FBS | 8×10³ |
| Fadu | 90% EMEM, 10% FBS, 1 mM sodium pyruvate | 7×10³ |
| SK-OV-3 | 90% McCoy'5A, 10% FBS | 6×10³ |
| BxPC-3 | 90% RPMI-1640, 10% FBS, 1 mM sodium pyruvate, 2.5g/L glucose | 8×10³ |
| NCI-H1975 | 90% RPMI-1640, 10% FBS, 1 mM sodium pyruvate, 2.5g/L glucose | 5×10⁴ |
| HCC827 | 90% RPMI-1640, 10% FBS, 1 mM sodium pyruvate, 2.5g/L glucose | 8×10⁴ |
| A375 | DMEM+10% FBS | 8×10⁴ |
| 22RV1 | 90% RPMI-1640, 10% FBS, 1 mM sodium pyruvate, 2.5g/L glucose | 7×10³ |

### 3. Experimental results

The IC₅₀ values of the test compounds against human tumor cell lines are as shown in Tables 3-7.

**Table 3: Inhibitory activity of test compounds against human tumor cells (IC₅₀: nM)**

| Cell lines | HCT116 (human colorectal cancer) | | SW620 (human colorectal cancer) | | Fadu (human pharyngeal squamous cell carcinoma) | |
|---|---|---|---|---|---|---|
| Test method | MTT | | SRB | | MTT | |
| | βGU-free | With addition of βGU | βGU-free | With addition of βGU | βGU-free | With addition of βGU |
| Example compound 1 | 88 | 6.6 | 45 | 5.9 | 34 | 7.7 |
| Example compound 2 | 143 | 6.1 | 81 | 6.7 | 38 | 2.7 |
| Example compound 3 | | | | | 159 | 7.8 |
| Example compound 4 | 139 | 6.6 | 67 | 3.4 | 21 | 1.7 |
| Example compound 10 | 87 | 3.4 | | | 14 | 0.64 |
| Example compound 9 | 69 | 2.9 | | | 25 | 0.35 |
| Example compound 12 | 84 | 1.6 | | | 36 | 0.21 |
| Example compound 8 | 51 | 2.1 | | | 19 | 0.79 |
| Example compound 11 | 55 | 1.1 | | | 21 | 0.61 |

**Table 4: Inhibitory activity of test compounds against human tumor cells (IC₅₀: nM)**

| Cell lines | HepG2 (human liver cancer cell) | | NCI-N87 (human gastric cancer cell) | | MDA-MB-468 (human triple-negative breast cancer) | |
|---|---|---|---|---|---|---|
| Test method | SRB | | MTT | | SRB | |
| | βGU-free | With addition of βGU | βGU-free | With addition of βGU | βGU-free | With addition of βGU |
| Example compound 1 | 47 | 3.8 | 45 | 7.3 | 6.2 | 1.1 |
| Example compound 2 | 120 | 4.3 | 81 | 5.6 | 24 | 2.7 |
| Example compound 3 | | | | | 50 | 0.77 |
| Example compound 4 | 72 | 4.7 | 58 | 2.3 | 8.8 | 0.96 |
| Example compound 10 | 93 | 4.1 | | | | |
| Example compound 9 | 88 | 3.8 | | | | |
| Example compound 12 | 106 | 3 | | | | |
| Example compound 8 | 86 | 4 | | | | |
| Example compound 11 | 66 | 3 | | | | |

**Table 5: Inhibitory activity of test compounds against human tumor cells (IC₅₀: nM)**

| Cells | NCI-H1975 (human non-small cell lung cancer) | | HCC827 (human non-small cell lung cancer) | | SK-OV-3 (human ovarian cancer) | |
|---|---|---|---|---|---|---|
| Test method | MTT | | SRB | | MTT | |
| | βGU-free | With addition of βGU | βGU-free | With addition of βGU | βGU-free | With addition of βGU |
| Example compound 1 | 38 | 4.2 | 51 | 2.9 | 201 | 7.2 |
| Example compound 2 | 114 | 4.3 | 110 | 3.8 | 211 | 6.1 |
| Example compound 4 | 86 | 5.5 | 54 | 3.4 | 215 | 5.1 |
| Example compound 10 | 50 | 1.9 | | | 74 | 2.7 |
| Example compound 9 | 69 | 4.7 | | | 65 | 2.4 |
| Example | 74 | 1.2 | | | 37 | 1.1 |
| compound 12 | | | | | | |
| Example compound 8 | 72 | 3.4 | | | 61 | 2.6 |
| Example compound 11 | 64 | 1.7 | | | 77 | 1.7 |

**Table 6: Inhibitory activity of test compounds against human tumor cells (IC₅₀: nM)**

| Cells | BxPC-3 (human pancreatic cancer in situ)SRB | | 22RV1 (human prostate cancer) | |
|---|---|---|---|---|
| Test method | MTT | | SRB | |
| | βGU-free | With addition of βGU | βGU-free | With addition of βGU |
| Example compound 1 | 29 | 4.7 | 99 | 7.2 |
| Example compound 2 | 43 | 3.9 | 176 | 7.5 |
| Example compound 3 | 106 | 9.7 | | |
| Example compound 4 | 44 | 1.9 | 115 | 7.0 |
| Example compound 10 | 29 | 0.71 | 94 | 4.4 |
| Example compound 9 | 28 | 0.86 | 86 | 3.8 |
| Example compound 12 | 28 | 0.53 | 77 | 2.5 |
| Example compound 8 | 21 | 0.54 | 63 | 3 |
| Example compound 11 | 15 | 0.4 | 63 | 2.2 |

**Table 7: Inhibitory activity of test compounds against human tumor cells (IC₅₀: nM)**

| Cells | A375 (human malignant melanoma) | |
|---|---|---|
| Test method | SRB | |
| | βGU-free | With addition of βGU |
| Example compound 1 | 107 | 6.4 |
| Example compound 2 | 212 | 6.7 |
| Example compound 4 | 66 | 5.0 |
| Example compound 10 | 33 | 1.4 |
| Example compound 9 | 27 | 2.7 |
| Example compound 12 | *50* | 1 |
| Example compound 8 | 43 | 2.4 |
| Example compound 11 | 45 | 1.6 |

### Biological Example 2: In vivo study on xenograft model of human gastric cancer NCI-N87

### 1. Experimental materials

Example compound 1, purity 98.08%, kept in a light-proof sealed container at 2-8°C
Experimental animals: SPF (specific pathogen-free) male Balb/c-nu nude mice, with body weight of 18-20 g, raised in an SPF-grade barrier system, with the barrier environment controlled at a temperature of 20-26°C, relative humidity of 40-70%, and a day/night cycle of 12/12 h.

Test tumor strain: human gastric cancer NCI-N87 cell strain, sourced from the Shanghai Cell Bank.

### 2. Experimental method and dosage regimen

First, the xenograft model of human gastric cancer NCI-N87 was established subcutaneously in nude mice by inoculation with a cell-matrigel mixture. When the xenograft tumor grew to a volume of 100-300 mm³ and maintained stable growth, the experimental animals were randomly grouped using remainder method based on the tumor volume and administered with drugs. The tumor volume was measured 2 to 3 times every week. After the dosing period was completed, the experimental animals were killed, and the subcutaneous tumors were excised and weighed. The anti-tumor activity of the drug in this model was evaluated based on relevant data of the tumor volumes and the tumor weights.

**Table 8: List of experimental animal groups and dosage regimens**

| Group | | Dosage (mg/kg) | Solvent | Dosing volume (mL/kg) | Dosing concentration (mg/mL) | Dosing period | Administration route | Number of animals |
|---|---|---|---|---|---|---|---|---|
| Model control group | G1 | - | - | - | - | - | - | |
| Example compound 1 | G2 | 15 | 0.9% sodium chloride injection | 10 | 1.5 | qw×2 | i.v. | 8 |

| | | | | | | | | |
|---|---|---|---|---|---|---|---|---|
| Note: "-" means blank or no valid data, the dosage or dosing period may be adjusted based on the animal tolerance and tumor growth during administration. | | | | | | | | |

The example compound 1 was fully dissolved in sterile 0.9% sodium chloride injection to prepare a drug solution at a concentration of 1.5 mg/mL, which was protected from light and prepared immediately before use.

### 3. Evaluation indicators

Calculation formula of the tumor volume: volume=0.5× long diameter × short diameter². $Relative tumor volume \left(RTV\right) : RTV = {V}_{t /} {V}_{0}$

V₀ is the tumor volume measured at the time of grouping for dosing (i.e., d0), and Vₜ is the tumor volume measured each time. $Relative tumor proliferation rate \left(T/C\right) : T / C = {T}_{RTV} / {C}_{RTV} \times 100 %$

T is the mean RTV of the test compound, and C is the mean RTV of the model control group. 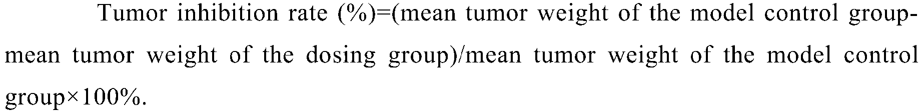 Tumor inhibition rate (%)=(mean tumor weight of the model control group-mean tumor weight of the dosing group)/mean tumor weight of the model control group×100%. $Body weight change percentage = {W}_{n} / {W}_{0} \times 100 %$

Wₙ: mean body weight of each group of experimental animals on day n, and W₀: mean body weight of each group of experimental animals on day 0.

### Statistical method:

Relevant data of the tumor volumes and the animal body weights were analyzed statistically using the general linear model test in SPSS 22.0 software. Sphericity test was first performed using repeated measures ANOVA. If P>0.05, it was considered that no correlation was present among repeated measurement data, and one-way Anova was used for intergroup statistical analysis. If P≤0.05, it was considered that correlation was present among repeated measurement data, and multivariate analysis of variance was used for intergroup statistical analysis.

Tumor weight data was tested for homogeneity of variance using Levene's Test in SPSS 22.0 software. If the variances were homogeneous (P>0.05), one-way Anova was used for intergroup statistical analysis. If the variances were heterogeneous (P≤0.05), the Kruskal-Wallis nonparametric test was performed. If the Kruskal-Wallis nonparametric test result was significant (P≤0.05), Mann-Whitney U test of the nonparametric test was used for intergroup pairwise comparison.

### 4. Experimental process and results

The purchased experimental animals were subjected to balanced feeding for 3 days. A xenograft model was established in nude mice by inoculation with a cell-matrigel mixture: conventionally cultured NCI-N87 cells in the logarithmic growth phase were sampled, digested, collected, and adjusted with PBS to a cell density of 6×10⁷ cells/mL. In an ice water bath, the cell suspension was slowly added to an equal volume of matrigel, the system was fully mixed, and then the xenograft model was established by subcutaneous inoculation in the right armpit of male nude mice, with 0.15 mL/mouse and a cell inoculum size of approximately 4.5 million cells/mouse.

The tumor growth of the model animals was regularly observed. When the mean tumor volume of the model nude mice reached about 100-300 mm³, the experimental animals were randomly grouped using the remainder method based on the tumor volume, and administered with drugs. The day on which the animals were grouped was day 0. After grouping, drugs were administered to each group according to Table 8. The dosage or dosing period can be appropriately adjusted based on the tumor growth and the animal state. After administration, the tumor volumes of the experimental animals were measured 2-3 times every week until the experiment was completed. After the dosing period was completed, the experimental animals were killed, and the subcutaneous tumors were excised and weighed. The anti-tumor activity of each compound in this model was evaluated based on relevant data of the tumor volumes and tumor weights. The experimental results are as shown below.

**Table 9: Effect on growth of xenograft model of human gastric cancer NCI-N87**

| Group | Host (nude mouse) response | | | | | Tumor response | | | |
|---|---|---|---|---|---|---|---|---|---|
| | Number of animals | Body weight (g, x̅±S.D.) | | Final body weight change (%) | Maximum body weight change (%) | Tumor weight (g, x̅±S.D.) | Tumor inhibition rate (%) | Relative volume RTV (x̅±S.D.) | T/C (%) |
| | Start/end | Start | End | | | | | | |
| G1 | 8/8 | 23.2±2.3 | 25.9±4.3 | 10.8 | 10.8, d24 | 1.511±0.920 | - | 7.13±1.38 | 100.0 |
| G2 | 8/8 | 23.4±1.1 | 25.1±2.4 | 7.2 | 7.2, d24 | 0.118±0.036*** | 92.2 | 1.55±0.19*** | 21.7 |

| | | | | | | | | | |
|---|---|---|---|---|---|---|---|---|---|
| Note: "-" means no valid data or blank; and ***p<0.001 means comparison to the model control group. | | | | | | | | | |

### Biological Example 3: In vivo study on xenograft model of human gastric cancer NCI-N87

The test was performed based on the dosage regimen in Table 10, following the method in Biological Example 2.

**Table 10: List of dosage regimens**

| Group | | Dosage (mg/kg) | Solvent | Dosing volume (mL/kg) | Dosing concentration (mg/mL) | Administration route | Dosing period | Number of animals |
|---|---|---|---|---|---|---|---|---|
| Model control group | G1 | - | - | - | - | - | - | 7 |
| Example compound 2 | G2 | 15 | 0.9% sodium chloride injection | 10 | 1.5 | i.v. | qw×2 | |

| | | | | | | | | |
|---|---|---|---|---|---|---|---|---|
| Note: "-" means blank. | | | | | | | | |

The experimental results are as shown below.

**Table 11: Effect on growth of xenograft model of human gastric cancer NCI-N87**

| Group | Host (nude mouse) response | | | | | Tumor response | | | |
|---|---|---|---|---|---|---|---|---|---|
| | Number of animals | Body weight (g, x̅±S.D.) | | Final body weight change (%) | Maximum body weight change (%) | Tumor weight (g, x̅±S.D.) | Tumor inhibition rate (%) | Relative volume RTV (x̅±S.D.) | T/C (%) |
| | Start/end | Start | End | | | | | | |
| G1 | 7/7 | 25.5±1.9 | 29.0±2.1 | 13.9 | 13.9, d26 | 1.355±0.807 | - | 8.07±3.36 | 100 |
| G2 | 7/7 | 24.2±1.5 | 26.9±1.3 | 11.4 | 11.4, d26 | 0.048±0.009** | 96.4 | 0.48±0.07* | 5.9 |

| | | | | | | | | | |
|---|---|---|---|---|---|---|---|---|---|
| Note: "-" means no valid data or blank; and *p<0.05 and **p<0.01 mean comparison to the model control group. | | | | | | | | | |

### Biological Example 4: In vivo study on xenograft model of human gastric cancer NCI-N87

The test was performed based on the dosage regimen in Table 12, following the method in Biological Example 2.

**Table 12: List of experimental animal groups and dosage regimens**

| Group | | Dosage (mg/kg) | Solvent | Dosing volume (mL/kg) | Dosing concentration (mg/mL) | Administration route | Dosing period | Number of animals |
|---|---|---|---|---|---|---|---|---|
| Model control group | G1 | - | - | - | - | - | - | 6 |
| Example compound (group 4) | G2 | 0.4 | 0.9% sodium chloride injection | 10 | 0.04 | i.v. | qw×2 | |

| | | | | | | | | |
|---|---|---|---|---|---|---|---|---|
| Note: "-" means no valid data or blank. | | | | | | | | |

The experimental results are as shown below.

**Table 13: Effect on growth of xenograft model of human gastric cancer NCI-N87**

| Group | Host (nude mouse) response | | | | | Tumor response | | | |
|---|---|---|---|---|---|---|---|---|---|
| | Number of animals | Body weight (g, x̅±S.D.) | | Final body weight change (%) | Maximum body weight change (%) | Tumor weight (g, x̅±S.D.) | Tumor inhibition rate (%) | Relative volume RTV (x̅±S.D.) | T/C (%) |
| | Start/end | Start | End | | | | | | |
| G1 | 6/6 | 22.5±0.9 | 27.3±0.8 | 21.5 | 22.7, d18 | 1.369±0.572 | - | 4.96±1.30 | 100 |
| G2 | 6/6 | 23.7±2.3 | 27.9±3.2 | 17.4 | 22.6, d18 | 0.064±0.019** | 95.3 | 0.44±0.08** | 9.0 |

| | | | | | | | | | |
|---|---|---|---|---|---|---|---|---|---|
| Note: "-" means no valid data or blank; and *p<0.05 and **p<0.01 mean comparison to the model control group. | | | | | | | | | |

### Biological Example 5: In vivo study on xenograft model of human ovarian cancer SK-OV-3

The test was performed based on the dosage regimen in Table 14, following the method in Biological Example 2.

**Table 14: List of experimental animal groups and dosage regimens**

| Group | | Dosage (mg/kg) | Solvent | Dosing volume (mL/kg) | Dosing concentration (mg/mL) | Administration route | Dosing period | Number of animals |
|---|---|---|---|---|---|---|---|---|
| Model control group | G1 | - | - | - | - | - | - | |
| Example compound 10 | G2 | 0.8 | 0.9% sodium chloride injection | 10 | 0.08 | i.v. | qw×3 | |
| Example compound 9 | G3 | | | | | | | 6 |
| Example compound 12 | G4 | | | | | | | |
| Example compound 8 | G5 | | | | | | | |
| Example compound 11 | G6 | | | | | | | |

| | | | | | | | | |
|---|---|---|---|---|---|---|---|---|
| Note: "-" means no valid data or blank. | | | | | | | | |

The experimental results are as shown below.

**Table 15 Effect on growth of xenograft model of human ovarian cancer SK-OV-3**

| Group | Host (nude mouse) response | | | | | Tumor response | | | |
|---|---|---|---|---|---|---|---|---|---|
| | Number of animals | Body weight (g, x̅±S.D.) | | Final body weight change (%) | Maximum body weight change (%) | Tumor weight (g, x̅±S.D.) | Tumor inhibition rate (%) | Relative volume RTV (x̅±S.D.) | T/C (%) |
| | Start/end | Start | End | | | | | | |
| G1 | 6/6 | 23.1±1.2 | 26.4±1.3 | 14.2 | 14.2, d21 | 1.708±0.235 | - | 11.57+3.21 | 100 |
| G2 | 6/6 | 23.7±1.1 | 24.7±0.9 | 4.3 | 4.4, d18 | 0.035±0.077** | 97.9 | 0.37±0.73** | 3.2 |
| G3 | 6/6 | 24.5±0.6 | 25.8±0.7 | 5.3 | 5.3, d21 | 0.002±0.001** | 99.9 | 0.05±0.03** | 0.4 |
| G4 | 6/6 | 24.1±1.2 | 25.1±1.4 | 4.4 | 4.4, d21 | 0.005±0.001** | 99.7 | 0.07±0.04** | 0.6 |
| G5 | 6/5 | 22.9±1.6 | 24.1±1.6 | 4.6 | 4.6, d21 | 0.005±0.003** | 99.7 | 0.08±0.02** | 0.7 |
| G6 | 6/6 | 24.4±0.9 | 25.7±0.8 | 5.7 | 5.7, d21 | 0.011±0.013** | 99.3 | 0.19±0.23** | 1.7 |

| | | | | | | | | | |
|---|---|---|---|---|---|---|---|---|---|
| Note: "-" means no valid data or blank; and **p<0.01 means comparison to the model control group. | | | | | | | | | |

One animal in group G5 died on day 18; during the experiment, injury was resulted from animal biting and fighting in this group on day 7, thereby decreasing body weights of some animals, but the body weights of other animals in the same group recovered well in subsequent experiments, suggesting that the death of the animal was independent of drug administration.

### Biological Example 6: In vivo study on xenograft model of human ovarian cancer SK-OV-3

The test was performed based on the dosage regimen in Table 16, following the method in Biological Example 2.

**Table 16: List of experimental animal groups and dosage regimens**

| Group | | Dosage (mg/kg) | Solvent | Dosing volume (mL/kg) | Dosing concentration (mg/mL) | Administration route | Dosing period | Number of animals |
|---|---|---|---|---|---|---|---|---|
| Model control group | G1 | - | - | - | - | - | - | 7 |
| Example compound 4 | G2 | 0.8 | 0.9% sodium chloride injection | 10 | 0.08 | i.v. | qw×3 | |

| | | | | | | | | |
|---|---|---|---|---|---|---|---|---|
| Note: "-" means no valid data or blank. | | | | | | | | |

The experimental results are as shown below.

**Table 17 Effect on growth of xenograft model of human ovarian cancer SK-OV-3**

| Group | Host (nude mouse) response | | | | | Tumor response | | | |
|---|---|---|---|---|---|---|---|---|---|
| | Number of animals | Body weight (g, x̅±S.D.) | | Final body weight change (%) | Maximum body weight change (%) | Tumor weight (g, x̅±S.D.) | Tumor inhibition rate (%) | Relative volume RTV (x̅±S.D.) | T/C (%) |
| | Start/end | Start | End | | | | | | |
| G1 | 7/7 | 23.3±1.2 | 26.2±2.0 | 12.4 | 12.4, d21 | 1.399±1.107 | - | 10.05±6.85 | 100 |
| G2 | 7/7 | 23.3±0.8 | 24.2±0.9 | 4.2 | 4.8, d17 | 0.055±0.077** | 96.0 | 0.79±0.79*** | 7.6 |

| | | | | | | | | | |
|---|---|---|---|---|---|---|---|---|---|
| Note: "-" means no valid data or blank; and **p<0.01 and ***p<0.001 mean comparison to the model control group. | | | | | | | | | |

### Biological Example 7: In vivo study on xenograft model of human non-small cell lung cancer NCI-H1975

The test was performed based on the dosage regimen in Table 18, following the method in Biological Example 2.

**Table 18: List of experimental animal groups and dosage regimens**

| Group | | Dosage (mg/kg) | Solvent | Dosing volume (mL/kg) | Dosing concentration (mg/mL) | Administration route | Dosing period | Number of animals |
|---|---|---|---|---|---|---|---|---|
| Model control group | G1 | - | - | - | - | - | - | |
| Example compound 10 | G2 | 0.8 | 0.9% sodium chloride injection | 10 | 0.08 | i.v. | qw×3 | |
| Example compound 9 | G3 | | | | | | | 6 |
| Example compound 12 | G4 | | | | | | | |
| Example compound 11 | G5 | | | | | | | |

| | | | | | | | | |
|---|---|---|---|---|---|---|---|---|
| Note: "-" means no valid data or blank. | | | | | | | | |

The experimental results are as shown below.

**Table 19 Effect on growth of xenograft model of human non-small cell lung cancer NCI-H1975**

| Group | Host (nude mouse) response | | | | | Tumor response | | | |
|---|---|---|---|---|---|---|---|---|---|
| | Number of animals | Body weight (g, x̅±S.D.) | | Final body weight change (%) | Maximum body weight change (%) | Tumor weight (g, x̅±S.D.) | Tumor inhibition rate (%) | Relative volume RTV (x̅±S.D.) | T/C (%) |
| | Start/end | Start | End | | | | | | |
| G1 | 6/6 | 23.9±0.6 | 27.6±1.1 | 15.1 | 15.1, d21 | 1.787±0.329 | - | 14.18±4.66 | 100 |
| G2 | 6/6 | 23.7±1.3 | 26.0±1.0 | 10.2 | 10.2, d21 | 0.041±0.022** | 97.7 | 0.66±0.23** | 4.6 |
| G3 | 6/6 | 26.1±2.8 | 28.3±2.9 | 8.8 | 8.8, d21 | 0.041±0.051** | 97.7 | 0.45±0.30** | 3.2 |
| G4 | 6/6 | 24.1±1.6 | 25.6±2.4 | 5.8 | 5.8, d21 | 0.039±0.039** | 97.8 | 0.46±0.35** | 3.2 |
| G5 | 6/6 | 25.3±1.6 | 27.0±2.2 | 6.7 | 6.7, d21 | 0.061±0.049** | 96.6 | 0.74±0.51** | 5.2 |

| | | | | | | | | | |
|---|---|---|---|---|---|---|---|---|---|
| Note: "-" means no valid data or blank; and **p<0.01 means comparison to the model control group. | | | | | | | | | |

### Biological Example 8: In vivo study on xenograft model of human non-small cell lung cancer NCI-H1975

The test was performed based on the dosage regimen in Table 20, following the method in Biological Example 2.

**Table 20: List of experimental animal groups and dosage regimens**

| Group | | Dosage (mg/kg) | Solvent | Dosing volume (mL/kg) | Dosing concentration (mg/mL) | Administration route | Dosing period | Number of animals |
|---|---|---|---|---|---|---|---|---|
| Model control group | G1 | - | - | - | - | - | - | 7 |
| Example compound 4 | G2 | 0.8 | 0.9% sodium chloride injection | 10 | 0.08 | i.v. | qw×3 | |

| | | | | | | | | |
|---|---|---|---|---|---|---|---|---|
| Note: "-" means no valid data or blank. | | | | | | | | |

The experimental results are as shown below.

**Table 21 Effect on growth of xenograft model of human non-small cell lung cancer NCI-H1975**

| Group | Host (nude mouse) response | | | | | Tumor response | | | |
|---|---|---|---|---|---|---|---|---|---|
| | Number of animals | Body weight (g, x̅±S.D.) | | Final body weight change (%) | Maximum body weight change (%) | Tumor weight (g, x̅±S.D.) | Tumor inhibition rate (%) | Relative volume RTV (x̅±S.D.) | T/C (%) |
| | Start/end | Start | End 24d | | | | | | |
| G1 | 7/7 | 24.8±1.4 | 28.0±1.5 | 13.0 | 13.0, d24 | 2.693±1.388 | - | 12.53±6.91 | 100 |
| G2 | 7/7 | 24.8±0.8 | 26.0±1.1 | 4.9 | 4.9, d24 | 0.072±0.046** | 97.3 | 0.33±0.17* | 2.7 |

| | | | | | | | | | |
|---|---|---|---|---|---|---|---|---|---|
| Note: "-" means no valid data or blank; and *p<0.05 and **p<0.01 mean comparison to the model control group. | | | | | | | | | |

### Biological Example 9: In vivo study on xenograft model of human non-small cell lung cancer NCI-H1975

The test was performed based on the dosage regimen in Table 22, following the method in Biological Example 2.

**Table 22: List of experimental animal groups and dosage regimens**

| Group | | Dosage (mg/kg) | Solvent | Dosing volume (mL/kg) | Dosing concentration (mg/mL) | Administration route | Dosing period | Number of animals |
|---|---|---|---|---|---|---|---|---|
| Model control group | G1 | - | - | - | - | - | - | 6 |
| Example compound 8 | G2 | 0.8 | 0.9% sodium chloride injection | 10 | 0.08 | i.v. | qw×3 | |

| | | | | | | | | |
|---|---|---|---|---|---|---|---|---|
| Note: "-" means no valid data or blank. | | | | | | | | |

The experimental results are as shown below.

**Table 23 Effect on growth of xenograft model of human non-small cell lung cancer NCI-H1975**

| Group | Host (nude mouse) response | | | | | Tumor response | | | |
|---|---|---|---|---|---|---|---|---|---|
| | Number of animals | Body weight (g, **x̅**±S.D.) | | Final body weigh | Maximu m body weight | Tumor weight (g, **x̅**±S.D.) | Tumor inhibition rate | Relative volume RTV | T/C (%) |
| | Start/end | Start | End 24d | t change (%) | change (%) | | (%) | (**x̅**±S.D.) | |
| G1 | 6/6 | 25.8±1.6 | 28.4±1.7 | 10.1 | 10.1, d26 | 1.274±0.395 | - | 7.47±0.90 | 100 |
| G2 | 6/6 | 25.3±1.1 | 26.3±1.5 | 4 | 4.3, d17 | 0.022±0.038* * | 98.3 | 0.19±0.27*** | 2.5 |

| | | | | | | | | | |
|---|---|---|---|---|---|---|---|---|---|
| Note: "-" means no valid data or blank; and *p<0.05, **p<0.01, and ***p<0.001 mean comparison to the model control group. | | | | | | | | | |

### Biological Example 10: In vivo study on xenograft model of human non-small cell lung cancer NCI-H1975

The test was performed based on the dosage regimen in Table 24, following the method in Biological Example 2.

**Table 24: List of experimental animal groups and dosage regimens**

| Group | | Dosage (mg/kg) | Solvent | Dosing volume (mL/kg) | Dosing concentration (mg/mL) | Administration route | Dosing period | Number of animals |
|---|---|---|---|---|---|---|---|---|
| Model control group | G1 | - | - | - | - | - | - | 6 |
| Example compound 10 | G2 | 0.4 | 0.9% sodium chloride injection | 10 | 0.04 | i.v. | qw×2 | |
| Example compound 9 | G3 | | | | | | | |
| Example compound 12 | G4 | | | | | | | |

| | | | | | | | | |
|---|---|---|---|---|---|---|---|---|
| Note: "-" means no valid data or blank. | | | | | | | | |

The experimental results are as shown below.

**Table 25 Effect on growth of xenograft model of human non-small cell lung cancer NCI-H1975**

| Group | Host (nude mouse) response | | | | | Tumor response | | | |
|---|---|---|---|---|---|---|---|---|---|
| | Number of animals | Body weight (g, x̅±S.D.) | | Final body weight change (%) | Maximum body weight change (%) | Tumor weight (g, x̅±S.D.) | Tumor inhibition rate (%) | Relative volume RTV (x̅±S.D.) | T/C (%) |
| | Start/end | Start | End 24d | | | | | | |
| G1 | 6/6 | 21.8±1.1 | 23.4±0.9 | 7.6 | 7.6, d28 | 1.095±0.085 | - | 4.93±0.41 | 100 |
| G2 | 6/6 | 21.9±1.3 | 22.9±0.8 | 4.6 | 4.6, d28 | 0.047±0.020** | 95.7 | 0.26±0.02*** | 5.2 |
| G3 | 6/6 | 22.5±1.4 | 23.5±0.6 | 4.8 | 4.8, d28 | 0.073±0.016** | 93.4 | 0.38±0.05*** | 7.7 |
| G4 | 6/6 | 21.8±0.6 | 23.1±0.7 | 5.7 | 5.7, d28 | 0.086±0.009** | 92.1 | 0.44±0.06*** | 8.8 |

| | | | | | | | | | |
|---|---|---|---|---|---|---|---|---|---|
| Note: "-" means no valid data or blank; and **p<0.01 and ***p<0.001 mean comparison to the model control group. | | | | | | | | | |

The above description only provides example embodiments of the present disclosure, and is not intended to limit the scope of the present disclosure. Any modification, equivalent replacement, improvement, and the like made within the spirit and principles of the present disclosure should be encompassed within the scope of protection of the present disclosure.

## Claims

1. A compound represented by general formula (A), or a stereoisomer, a cis-trans isomer, or a pharmaceutically acceptable salt thereof: 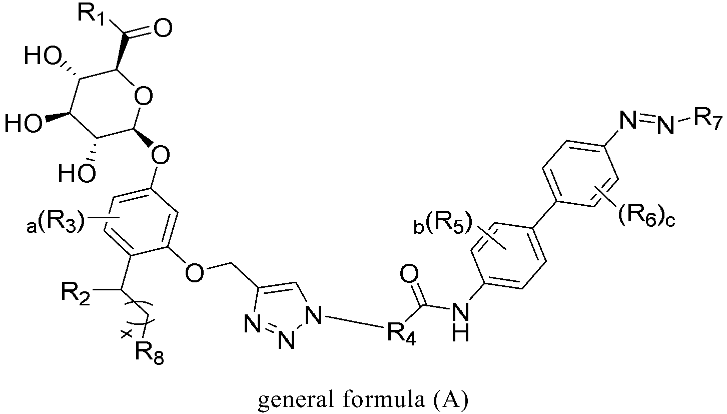 wherein
R₁ is selected from a hydroxy group, a C₁-C₁₀ hydrocarbyloxy group, an -O⁻ alkali metal ion, an -O⁻ammonium ion, and -O⁻;
R₂ is selected from hydrogen and a C₁-C₁₀ hydrocarbyl group;
R₃ is, at each occurrence, independently selected from hydrogen, a halogen, a C₁-C₁₀ hydrocarbyl group, a C₁-C₁₀ halohydrocarbyl group, a C₁-C₁₀ hydrocarbyloxy group, a C₁-C₁₀ halohydrocarbyloxy group, a C₆-C₁₈ aryl group, a nitro group, and - NR₂₁R₂₂;
R₄ is selected from a C₁-C₁₀ hydrocarbylene group, a C₁-C₁₀ hydrocarbylenoxy group, a C₆-C₁₂ arylene group, a C₁-C₁₀ hydrocarbylene-C₆-C₁₂ arylene group, a C₁-C₁₀ hydrocarbylene-C₆-C₁₂ arylene-C₁-C₁₀ hydrocarbylene group, a (C₁-C₁₀ hydrocarbylenoxy)_{d}-C₁-C₁₀ hydrocarbylene group, a C₁-C₁₀ hydrocarbylenoxy-C₆-C₁₂ arylene group, -(CH₂)ₘ-CO-NR₂₃-(CHR₂₄)-(CO-NR₂₅-(CHR₂₆)ₖ)ₚ-(CH₂)ₙ-, and - ((CH₂)ₘ-NR₂₃-(CHR₂₄)ⱼ)_{q}-(CH₂)ₙ-;
R₅ and R₆ are, at each occurrence, each independently selected from hydrogen, a halogen, a C₁-C₁₀ hydrocarbyl group, a C₁-C₁₀ halohydrocarbyl group, a C₁-C₁₀ hydrocarbyloxy group, a C₁-C₁₀ halohydrocarbyloxy group, and a C₆-C₁₈ aryl group;
R₇ is selected from: 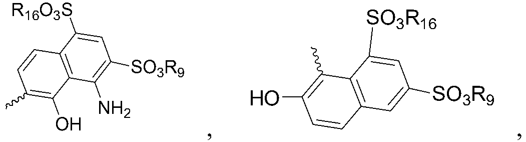 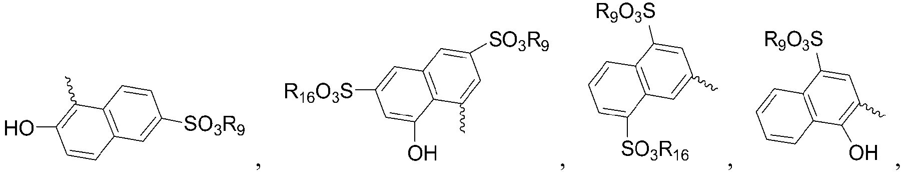 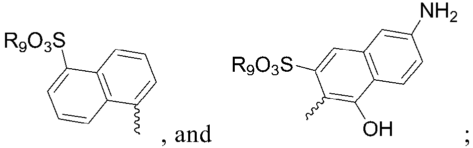
R₈ is selected from R₁₀, R₁₁, R₁₂, R₁₇, -OCO-R₁₀, -OCO-R₁₂, and -OCO-R₁₇: 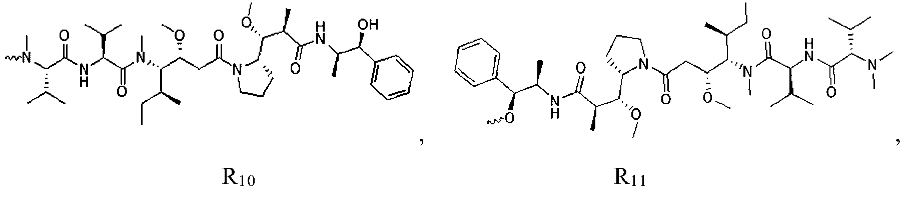 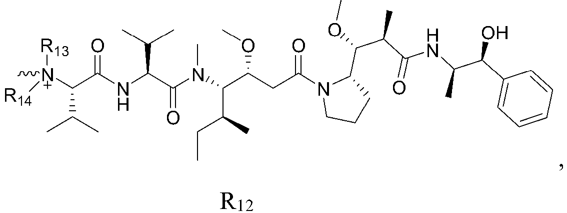 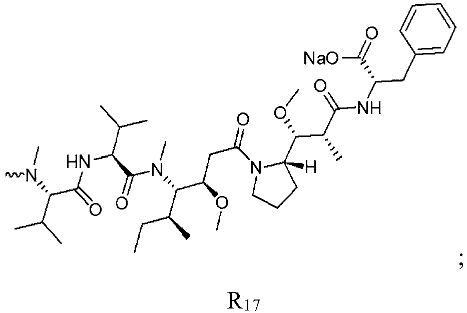
R₁₃ and R₁₄ are each independently selected from hydrogen, a C₁-C₈ hydrocarbyl group, a C₆-C₁₂ aryl group, and a C₁-C₈ hydrocarbylene-C₆-C₁₂ aryl group, R₁₃ and R₁₄ optionally form, together with a nitrogen atom to which they are attached, a 3-17-membered nitrogen-containing heterocycle group;
R₉ and R₁₆ are each independently selected from hydrogen, an alkali metal ion, and an ammonium ion;
R₂₁ and R₂₂ are each independently selected from hydrogen and a C₁-C₆ hydrocarbyl group;
R₂₃ and R₂₅ are each independently selected from hydrogen, a C₁-C₆ hydrocarbyl group, and a C₁-C₆ hydrocarbyloxy group;
R₂₄ and R₂₆ are each independently selected from hydrogen, a C₁-C₆ hydrocarbyl group, a C₁-C₆ hydrocarbyloxy group, a C₆-C₁₂ aryl group, and a C₇-C₁₃ arylhydrocarbyl group; and
x is 0 or 1; a is an integer from 0 to 3; b and c are each independently an integer from 0 to 4; d is an integer from 0 to 4; j, k, m, n, and p are each independently an integer from 0 to 5, and q is an integer from 1 to 5;
wherein 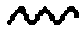 represents a linkage site.

2. The compound represented by general formula (A) according to claim 1, or the stereoisomer, the cis-trans isomer, or the pharmaceutically acceptable salt thereof, wherein general formula (A) is general formula (I): 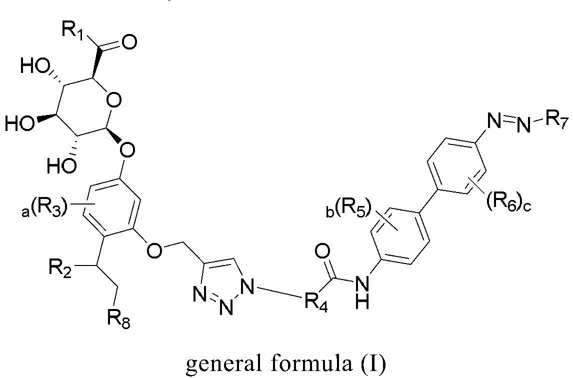 wherein
R₁ is selected from a hydroxy group, a C₁-C₁₀ hydrocarbyloxy group, an -O⁻ alkali metal ion, an -O⁻ammonium ion, and -O⁻;
R₂ is selected from hydrogen and a C₁-C₁₀ hydrocarbyl group;
R₃ is, at each occurrence, independently selected from hydrogen, a halogen, a C₁-C₁₀ hydrocarbyl group, a C₁-C₁₀ halohydrocarbyl group, a C₁-C₁₀ hydrocarbyloxy group, a C₁-C₁₀ halohydrocarbyloxy group, a C₆-C₁₈ aryl group, a nitro group, and - NR₂₁R₂₂;
R₄ is selected from a C₁-C₁₀ hydrocarbylene group, a C₁-C₁₀ hydrocarbylenoxy group, a C₆-C₁₂ arylene group, a C₁-C₁₀ hydrocarbylene-C₆-C₁₂ arylene group, a C₁-C₁₀ hydrocarbylene-C₆-C₁₂ arylene-C₁-C₁₀ hydrocarbylene group, a (C₁-C₁₀ hydrocarbylenoxy)_{d}-C₁-C₁₀ hydrocarbylene group, a C₁-C₁₀ hydrocarbylenoxy-C₆-C₁₂ arylene group, -(CH₂)ₘ-CO-NR₂₃-(CHR₂₄)ⱼ-(CO-NR₂₅-(CHR₂₆)ₖ)ₚ-(CH₂)ₙ-, and - ((CH₂)ₘ-NR₂₃-(CHR₂₄)ⱼ)_{q}-(CH₂)ₙ-;
R₅ and R₆ are, at each occurrence, each independently selected from hydrogen, a halogen, a C₁-C₁₀ hydrocarbyl group, a C₁-C₁₀ halohydrocarbyl group, a C₁-C₁₀ hydrocarbyloxy group, a C₁-C₁₀ halohydrocarbyloxy group, and a C₆-C₁₈ aryl group;
R₇ is selected from: 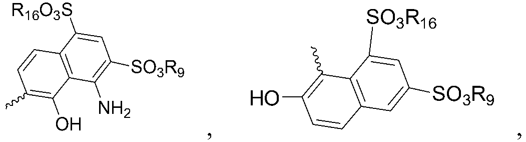 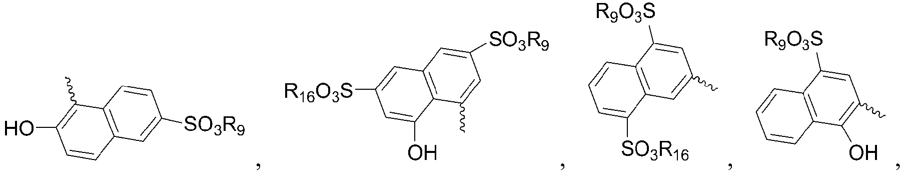 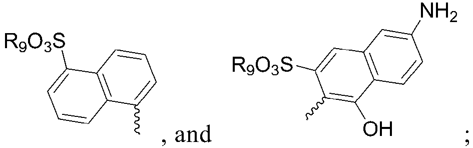
R₈ is selected from R₁₀, R₁₁, R₁₂, R₁₇, -OCO-R₁₀, -OCO-R₁₂, and -OCO-R₁₇: 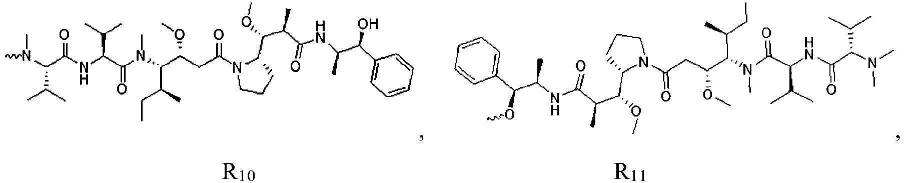 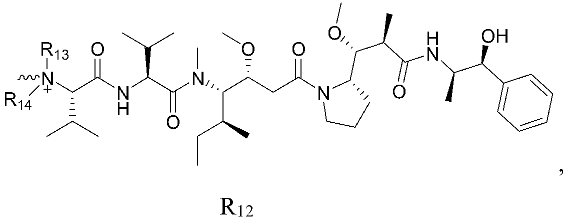 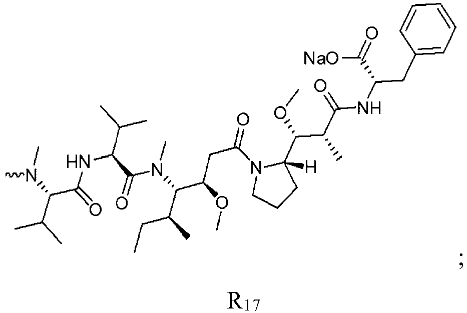
R₁₃ and R₁₄ are each independently selected from hydrogen, a C₁-C₈ hydrocarbyl group, a C₆-C₁₂ aryl group, and a C₁-C₈ hydrocarbylene-C₆-C₁₂ aryl group, and R₁₃ and R₁₄ optionally form, together with a nitrogen atom to which they are attached, a 3-17-membered nitrogen-containing heterocycle group;
R₉ and R₁₆ are each independently selected from hydrogen, an alkali metal ion, and an ammonium ion;
R₂₁ and R₂₂ are each independently selected from hydrogen and a C₁-C₆ hydrocarbyl group;
R₂₃ and R₂₅ are each independently selected from hydrogen, a C₁-C₆ hydrocarbyl group, and a C₁-C₆ hydrocarbyloxy group;
R₂₄ and R₂₆ are each independently selected from hydrogen, a C₁-C₆ hydrocarbyl group, a C₁-C₆ hydrocarbyloxy group, a C₆-C₁₂ aryl group, and a C₇-C₁₃ arylhydrocarbyl group; and
a is an integer from 0 to 3; b and c are each independently an integer from 0 to 4; d is an integer from 0 to 4; j, k, m, n and p are each independently an integer from 0 to 5, and q is an integer from 1 to 5;
wherein 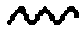 represents a linkage site.

3. The compound represented by general formula (A) according to claim 1 or 2, or the stereoisomer, the cis-trans isomer, or the pharmaceutically acceptable salt thereof, wherein
R₁ is selected from a hydroxy group, a C₁-C₁₀ alkoxy group, -O⁻Li⁺, -O⁻Na⁺, - O⁻K⁺, -O⁻Cs⁺, -O⁻NH₄⁺, and -O⁻;
R₂ is selected from hydrogen and a C₁-C₁₀ alkyl group;
R₃ is, at each occurrence, independently selected from hydrogen, F, Cl, Br, I, a C₁-C₁₀ alkyl group, a C₁-C₁₀ haloalkyl group, a C₁-C₁₀ alkoxy group, a C₁-C₁₀ haloalkoxy group, a C₆-C₁₈ aryl group, a nitro group, and -NR₂₁R₂₂;
R₄ is selected from a C₁-C₁₀ alkylene group, a C₁-C₁₀ alkylenoxy group, a C₆-C₁₂ arylene group, a C₁-C₁₀ alkylene-C₆-C₁₂ arylene group, a C₁-C₁₀ alkylene-C₆-C₁₂ arylene-C₁-C₁₀ alkylene group, a (C₁-C₁₀ hydrocarbylenoxy)_{d}-C₁-C₁₀ hydrocarbylene group, a C₁-C₁₀ alkylenoxy-C₆-C₁₂ arylene group, -(CH₂)ₘ-CO-NR₂₃-(CHR₂₄)ⱼ-(CO-NR₂₅-(CHR₂₆)ₖ)ₚ-(CH₂)ₙ-, and -((CH₂)ₘ-NR₂₃-(CHR₂₄)ⱼ)_{q}-(CH₂)ₙ-;
R₅ and R₆ are, at each occurrence, each independently selected from hydrogen, F, Cl, Br, I, a C₁-C₁₀ alkyl group, a C₁-C₁₀ haloalkyl group, a C₁-C₁₀ alkoxy group, a C₁-C₁₀ haloalkoxy group, and a C₆-C₁₈ aryl group;
R₇ is selected from: 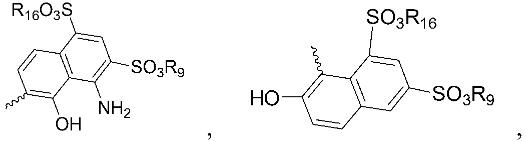 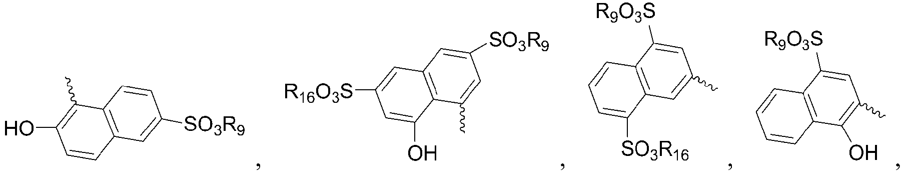 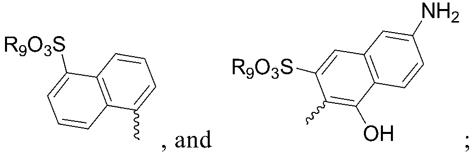
R₈ is selected from R₁₀, R₁₁, R₁₂, R₁₇, -OCO-R₁₀, -OCO-R₁₂, and -OCO-R₁₇: 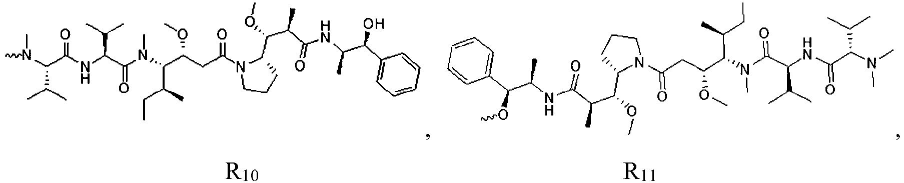 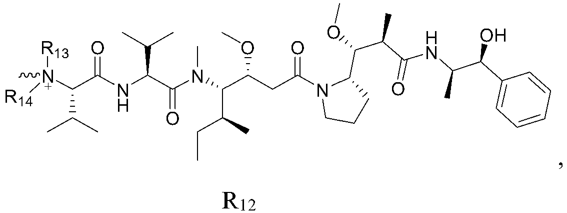 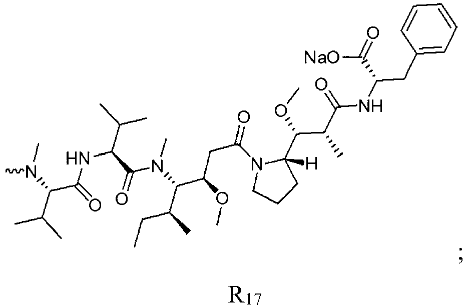
R₁₃ and R₁₄ are each independently selected from hydrogen, a C₁-C₈ alkyl group, a C₆-C₁₂ aryl group, and a C₁-C₈ alkylene-C₆-C₁₂ aryl group, and R₁₃ and R₁₄ optionally form, together with the nitrogen atom to which they are attached, a 3-6-membered nitrogen-containing heterocycle group;
R₉ and R₁₆ are each independently selected from hydrogen, Li⁺, Na⁺, K⁺, Cs⁺, and NH₄⁺;
R₂₁ and R₂₂ are each independently selected from hydrogen and a C₁-C₆ alkyl group;
R₂₃ and R₂₅ are each independently selected from hydrogen, a C₁-C₆ alkyl group, and a C₁-C₆ alkoxy group;
R₂₄ and R₂₆ are each independently selected from hydrogen, a C₁-C₆ alkyl group, a C₁-C₆ alkoxy group, a C₆-C₁₂ aryl group, and a C₇-C₁₃ arylalkyl group; and
a is an integer from 0 to 3; b and c are each independently an integer from 0 to 4; d is an integer from 0 to 4; j, k, m, n, and p are each independently an integer from 0 to 5, and q is an integer from 1 to 5;
wherein 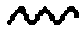 represents a linkage site.

4. The compound represented by general formula (A) according to any one of claims 1 to 3, or the stereoisomer, the cis-trans isomer, or the pharmaceutically acceptable salt thereof, wherein
R₁ is selected from -OH, -OCH₃, -OCH₂CH₃, -O⁻Li⁺, -O⁻Na⁺, -O⁻K⁺, -O⁻Cs⁺, - O⁻NH₄⁺, and -O⁻;
R₂ is selected from hydrogen, -CH₃, and -CH₂CH₃;
R₃ is, at each occurrence, independently selected from hydrogen, F, Cl, Br, - CH₃, -CH₂CH₃, -CH₂CH₂CH₃, -OCH₃, -OCH₂CH₃, a phenyl group, -NO₂, -NH₂, - NH(CH₃), and -N(CH₃)₂;
R₄ is selected from -CH₂CH₂CH₂CH₂CH₂-, -CH₂CH₂CH₂CH₂CH₂CH₂-, - CH₂CH₂CH₂CH₂CH₂CH₂CH₂-, -(CH₂CH₂O)₃CH₂-, -(CH₂CH₂CH₂O)₂CH₂CH₂-, - (CH₂CH₂NH)₃CH₂-, -CH₂CH₂C₆H₄-, -CH₂CH₂C₁₀H₆-, -CH₂CH₂C₆H₄C₆H₄-, - CH₂CH₂C₆H₄CH₂CH₂CH₂-, -CH₂CH₂OCH₂CH₂-, -CH₂CH₂OCH₂CH₂CH₂-, - CH₂CH₂CH₂OCH₂CH₂CH₂-, -(CON(CH₃)CH₂)₂-, -(CH₂CONH)₂CH₂-, - CH₂CONHCH(CH₃)CONHCH(CH₃)-, and -CH₂CONHCH(CH₃)CONHCH(CH₂C₆H₅)-;
R₅ and R₆ are, at each occurrence, each independently selected from hydrogen, F, Cl, Br, -CH₃, -CH₂CH₃, -OCH₃, -OCH₂CH₃, and a phenyl group;
R₇ is selected from: 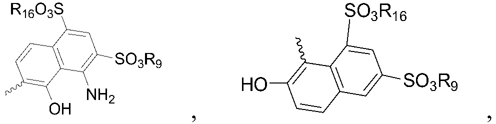 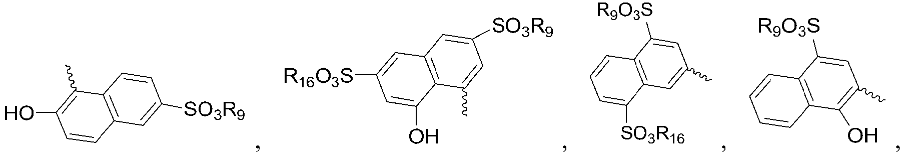 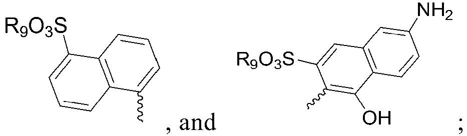
R₈ is selected from R₁₀, R₁₁, R₁₂, R₁₃, R₁₄, R₁₅, R₁₇, -OCO-R₁₀, -OCO-R₁₂, and -OCO-R₁₇: 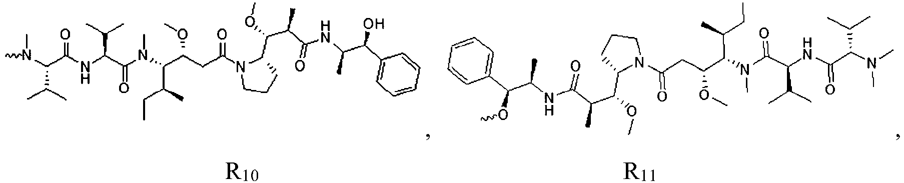 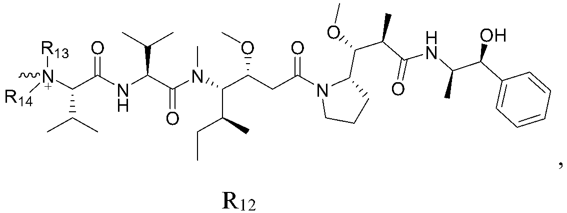 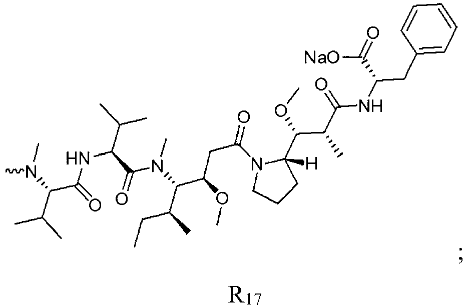
R₁₃ and R₁₄ are each independently selected from hydrogen, a methyl group, an ethyl group, an isopropyl group, a butyl group, a phenyl group, a benzyl group, and a phenethyl group;
when R₁₃ and R₁₄ together with the nitrogen atom to which they are attached form a ring, R₁₂ is: 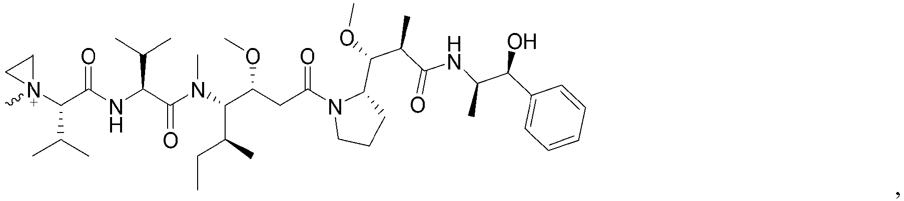 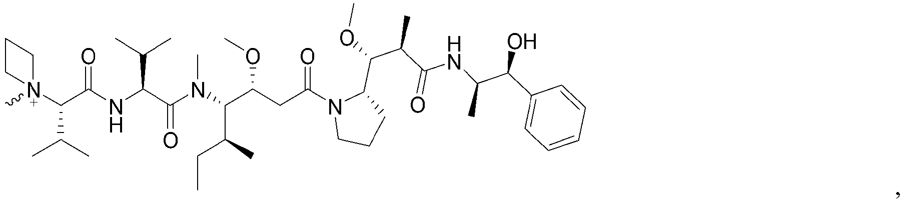 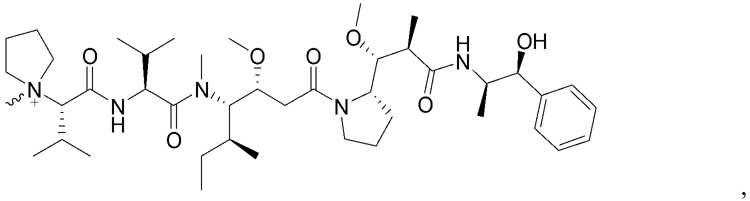 or 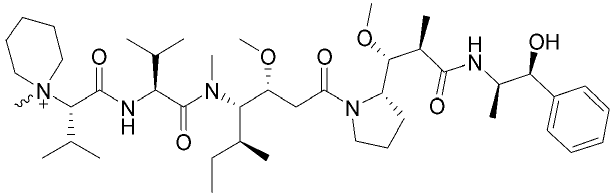
R₉ and R₁₆ are each independently selected from hydrogen, Li⁺, Na⁺, K⁺, Cs⁺, and NH₄⁺; and
a is an integer from 0 to 3; and b and c are each independently an integer from 0 to 4;
wherein 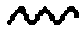 represents a linkage site.

5. A compound, or a stereoisomer, a cis-trans isomer, or a pharmaceutically acceptable salt thereof, wherein the compound is selected from:
| serial No. | structural formula | chemical name |
|---|---|---|
| 1 | 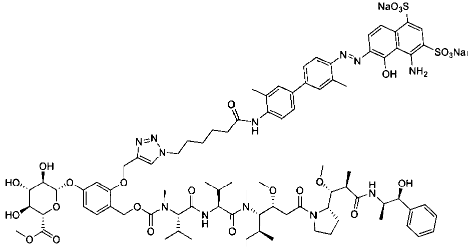 | sodium 4-amino-6-((E)-(4'-(6-(4-((2-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-(((2S,3R,4S,5S,6S)-3,4,5-trihydroxy-6-(methoxycarbonyl)tetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate |
| 2 | 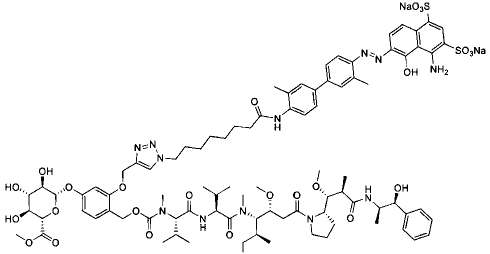 | sodium 4-amino-6-((E)-(4'-(8-(4-((2-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5- |
| | | (((2S,3R,4S,5S,6S)-3,4,5-trihydroxy-6-(methoxycarbonyl)tetrahydro-2H-pyran-2-yl)oxy)phenoxy)methyl)-1H-1,2,3-triazol-1-yl)octanamido)-3,3'-dimethyl-[1,1'-biphenyl]-4-yl)diazenyl)-5-hydroxynaphthalene-1,3-disulfonate |
| 3 | 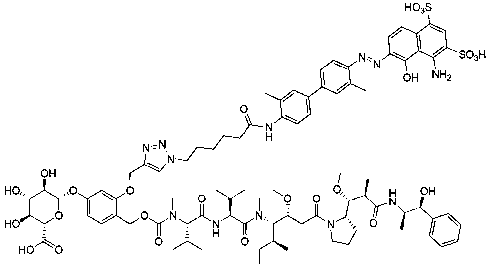 | (2S,3S,4S,5R,6S)-6-(3-((1-(6-((4'-((E)-(8-amino-1-hydroxy-5,7-disulfonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylic acid |
| 4 | 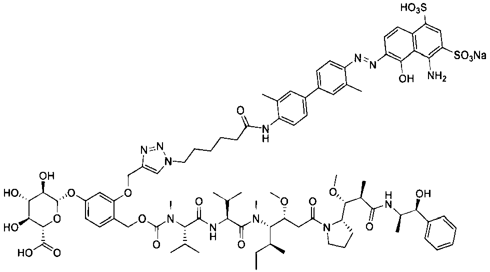 | sodium 1-amino-7-((E)-(4'-(6-(4-((2-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-(((2S,3R,4S,5S,6S)-6-carboxy- |
| | | 3,4,5-trihydroxytetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-8-hydroxy-4-sulfonaphthalene-2-sulfonate |
| 5 | 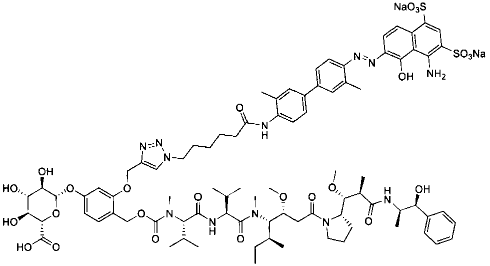 | sodium 4-amino-6-((E)-(4'-(6-(4-((2-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-(((2S,3R,4S,5S,6S)-6-carboxy-3,4,5-trihydroxytetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate |
| 6 | 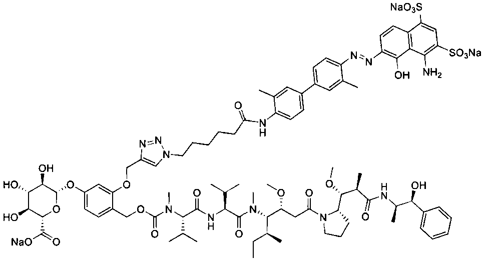 | sodium (2S,3S,4S,5R,6S)-6-(3-((1-(6-((4'-((E)-(8-amino-1-hydroxy-5,7-disulfonatonaphthalen-2-yl) diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3- |
| | | oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 7 | 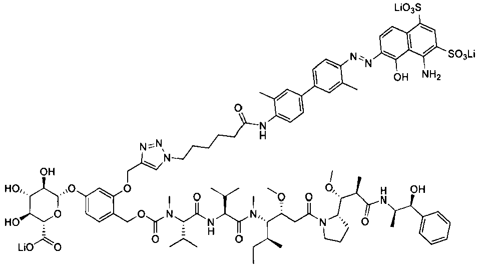 | lithium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 8 | 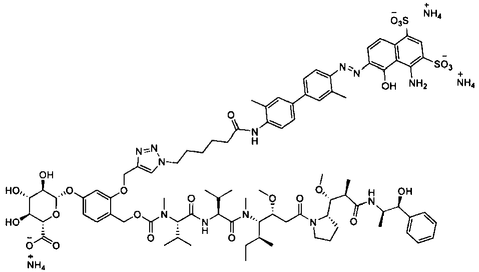 | ammonium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2- |
| | | oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 9 | 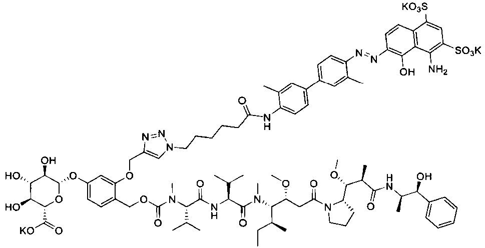 | potassium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 10 | 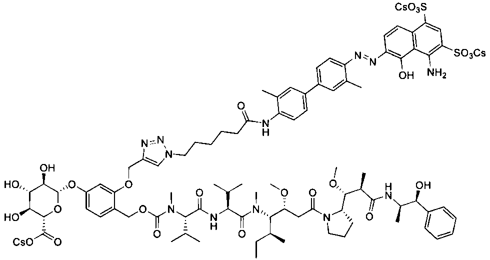 | cesium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10- |
| | | dimethyl-3,6,9-trioxo-2,13-dioxa-4,7,10-triazatetradecyl)phenoxy)-3,4,5-trihydroxytetrahydro-2H-pyran-2-carboxylate |
| 11 | 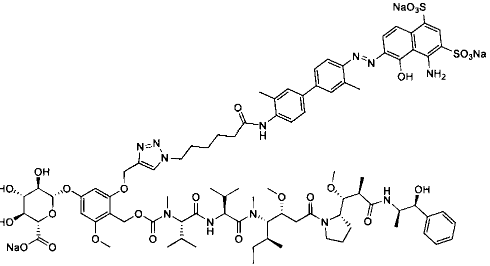 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-methoxyphenoxy)-3,4,5-trihydroxytetrahydro-2H-pyran-2-carboxylate |
| 12 | 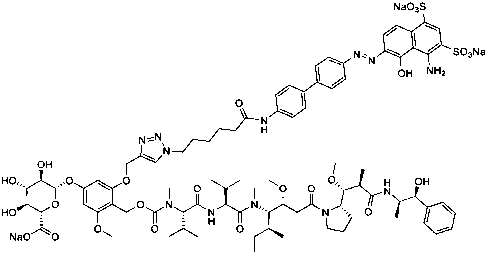 | sodium (2S,3S,4S,5R,6S)-6-(3-((1-(6-((4'-((E)-(8-amino-1-hydroxy-5,7-disulfonatonaphthalen-2-yl)diazenyl)-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10- |
| | | dimethyl-3,6,9-trioxo-2,13-dioxa-4,7,10-triazatetradecyl)-5-methoxyphenoxy)-3,4,5-trihydroxytetrahydro-2H-pyran-2-carboxylate |
| 13 | 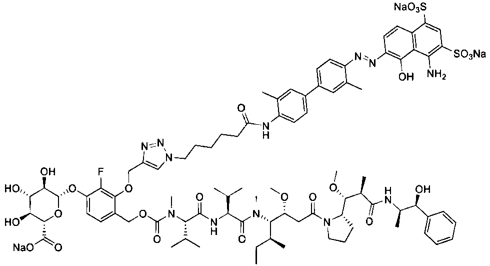 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-fluorophenoxy)-3,4,5-trihydroxytetrahydro-2H-pyran-2-carboxylate |
| 14 | 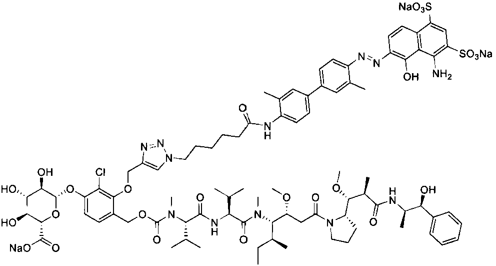 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2- |
| | | oxoethyl)-5,8-diisopropyl-4,10-dimethyl-3,6,9-trioxo-2,13-dioxa-4,7,10-triazatetradecyl)-2-chlorophenoxy)-3,4,5-trihydroxytetrahydro-2H-pyran-2-carboxylate |
| 15 | 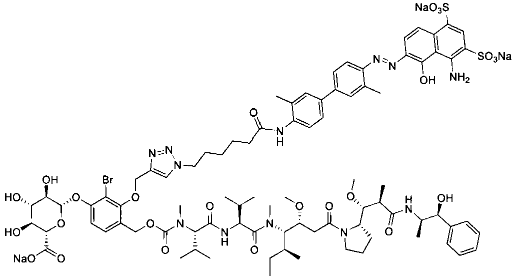 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-2-bromo-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 16 | 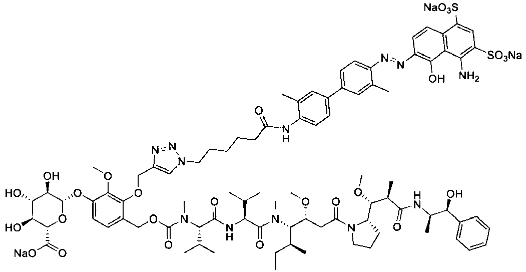 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3- |
| | | oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-methoxyphenoxy)-3,4,5-trihydroxytetrahydro-2H-pyran-2-carboxylate |
| 17 | 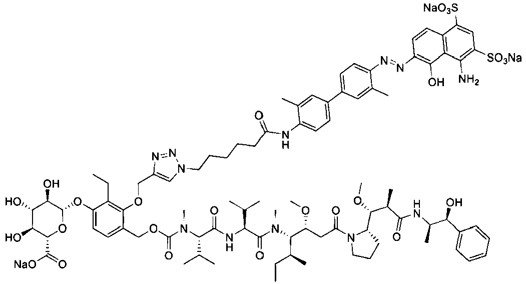 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-ethylphenoxy)-3,4,5-trihydroxytetrahydro-2H-pyran-2-carboxylate |
| 18 | 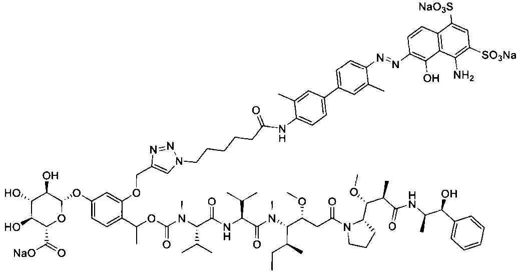 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3R,4S,7S,10S)-4-((S)-sec-butyl)-3-(2-((S)-2-((1R,2R)-3-(((1S,2R)-1-hydroxy-1-phenylpropan-2-yl)amino)-1- |
| | | methoxy-2-methyl-3-oxopropyl)pyrrolidin-1-yl)-2-oxoethyl)-7,10-diisopropyl-5,11-dimethyl-6,9,12-trioxo-2,13-dioxa-5,8,11-triazapentadecan-14-yl)phenoxy)-3,4,5-trihydroxytetrahydro-2H-pyran-2-carboxylate |
| 19 | 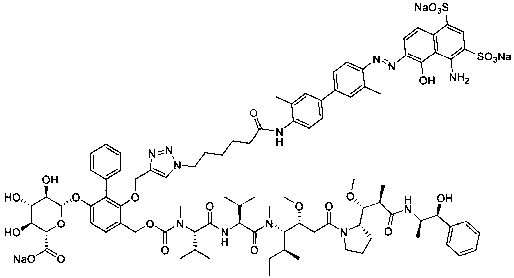 | sodium (2S,3S,4S,5R,6S)-6-((6-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-5-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-[1,1'-biphenyl]-2-yl)oxy)-3,4,5-trihydroxytetrahydro-2H-pyran-2-carboxylate |
| 20 | 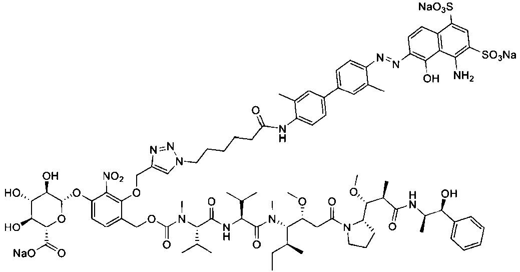 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy- |
| | | 1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-nitrophenoxy)-3,4,5-trihydroxytetrahydro-2H-pyran-2-carboxylate |
| 21 | 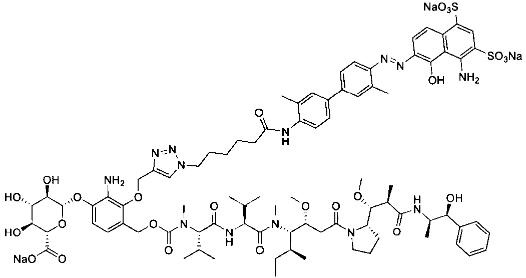 | sodium (2S,3S,4S,5R,6S)-6-(2-amino-3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 22 | 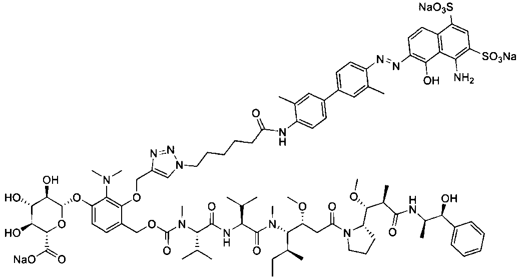 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy- |
| | | 1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-(dimethylamino)phenoxy)-3,4,5-trihydroxytetrahydro-2H-pyran-2-carboxylate |
| 23 | 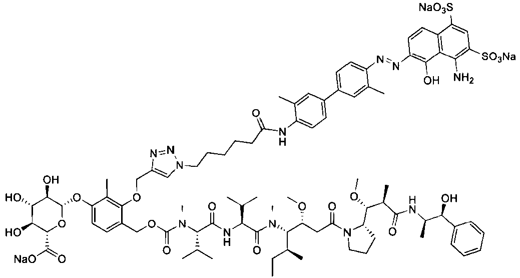 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-methylphenoxy)-3,4,5-trihydroxytetrahydro-2H-pyran-2-carboxylate |
| 24 | 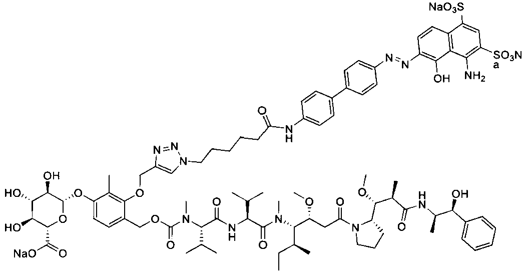 | sodium (2S,3S,4S,5R,6S)-6-(3-((1-(6-((4'-((E)-(8-amino-1-hydroxy-5,7-disulfonatonaphthalen-2-yl)diazenyl)-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3- |
| | | (((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-methylphenoxy)-3,4,5-trihydroxytetrahydro-2H-pyran-2-carboxylate |
| 25 | 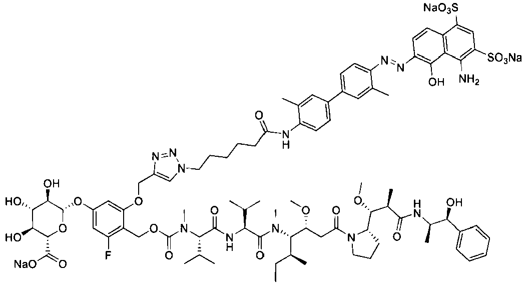 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-fluorophenoxy)-3,4,5-trihydroxytetrahydro-2H-pyran-2-carboxylate |
| 26 | 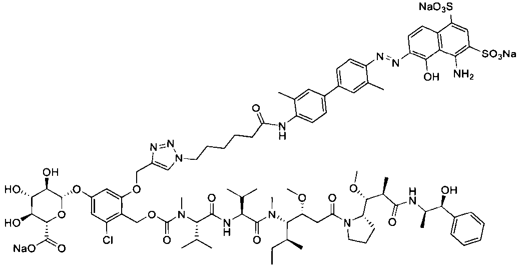 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)- |
| | | 11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-chlorophenoxy)-3,4,5-trihydroxytetrahydro-2H-pyran-2-carboxylate |
| 27 | 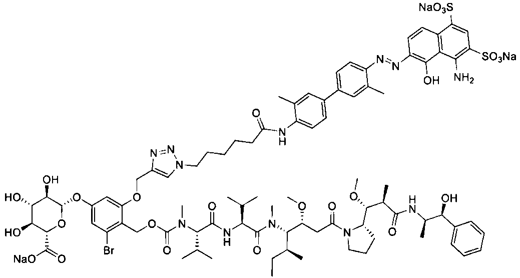 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-5-bromo-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 28 | 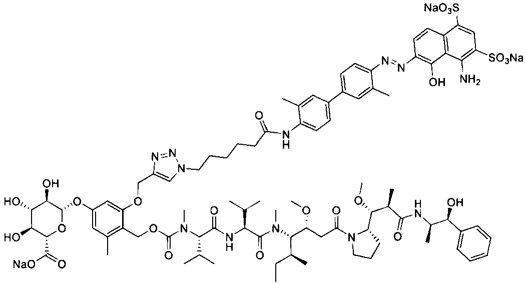 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-methylphenoxy)-3,4,5-trihydroxytetrahydro-2H-pyran-2-carboxylate |
| 29 | 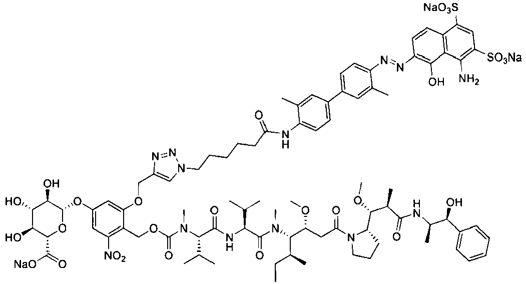 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-nitrophenoxy)-3,4,5-trihydroxytetrahydro-2H-pyran-2-carboxylate |
| 30 | 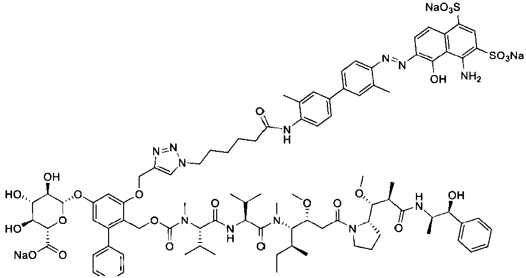 | sodium (2S,3S,4S,5R,6S)-6-((5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-6-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-[1,1'-biphenyl]-3-yl)oxy)-3,4,5-trihydroxytetrahydro-2H-pyran-2-carboxylate |
| 31 | 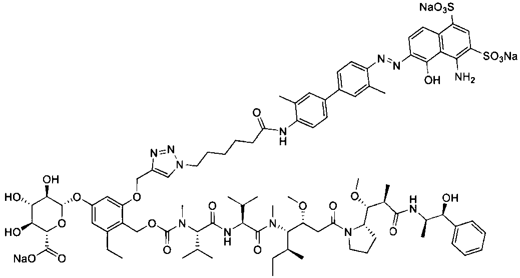 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-ethylphenoxy)-3,4,5-trihydroxytetrahydro-2H-pyran-2- |
| | | carboxylate |
| 32 | 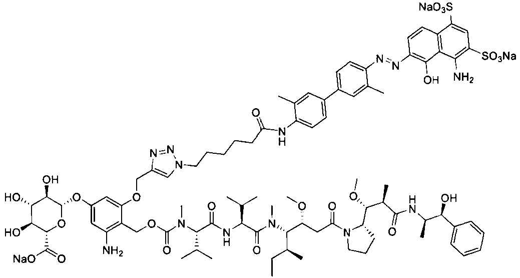 | sodium (2S,3S,4S,5R,6S)-6-(3-amino-5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 33 | 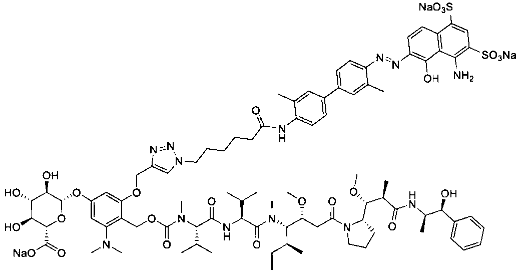 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-5-(dimethylamino)phenoxy)-3,4,5-trihydroxytetrahydro-2H-pyran-2- |
| | | carboxylate |
| 34 | 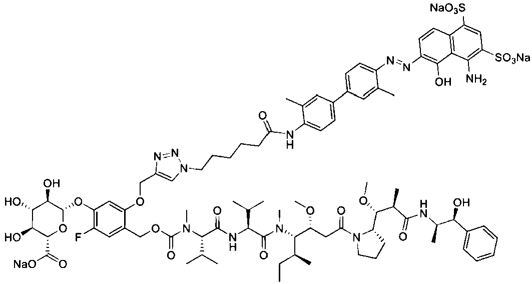 | sodium (2S,3S,4S,5R,6S)-6-(5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-fluorophenoxy)-3,4,5-trihydroxytetrahydro-2H-pyran-2-carboxylate |
| 35 | 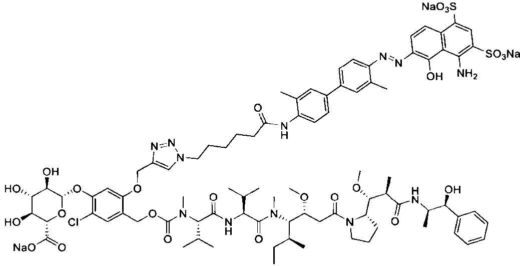 | sodium (2S,3S,4S,5R,6S)-6-(5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-chlorophenoxy)-3,4,5- |
| | | trihydroxytetrahydro-2H-pyran-2-carboxylate |
| 36 | 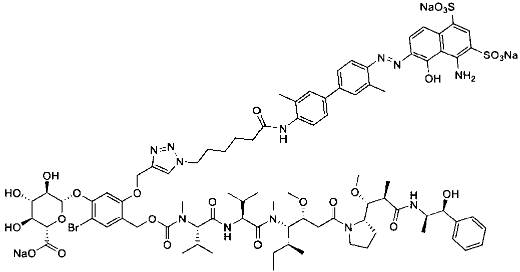 | sodium (2S,3S,4S,5R,6S)-6-(5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-2-bromo-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 37 | 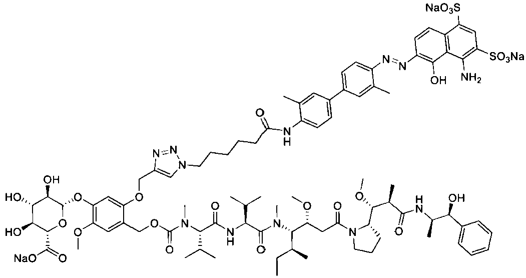 | sodium (2S,3S,4S,5R,6S)-6-(5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2- |
| | | methoxyphenoxy)-3,4,5-trihydroxytetrahydro-2H-pyran-2-carboxylate |
| 38 | 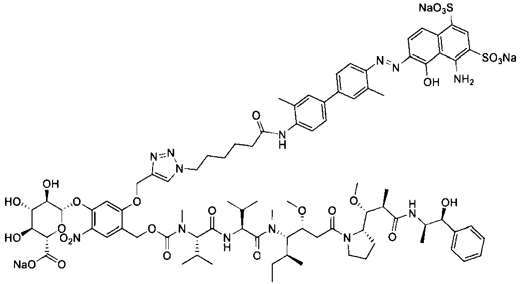 | sodium (2S,3S,4S,5R,6S)-6-(5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-nitrophenoxy)-3,4,5-trihydroxytetrahydro-2H-pyran-2-carboxylate |
| 39 | 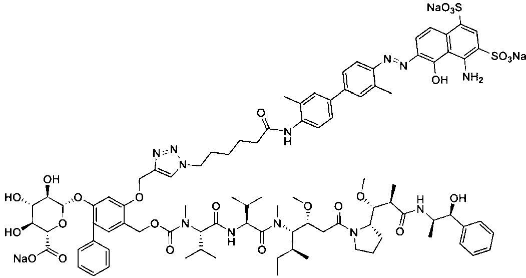 | sodium (2S,3S,4S,5R,6S)-6-((4-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-5-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa- |
| | | 4,7,10-triazatetradecyl)-[1,1'-biphenyl]-2-yl)oxy)-3,4,5-trihydroxytetrahydro-2H-pyran-2-carboxylate |
| 40 | 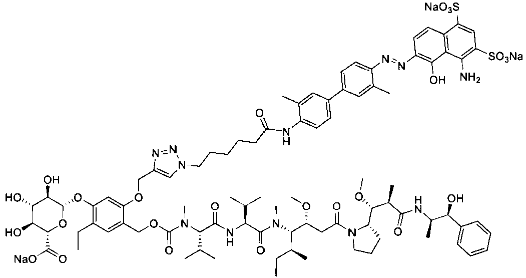 | sodium (2S,3S,4S,5R,6S)-6-(5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-ethylphenoxy)-3,4,5-trihydroxytetrahydro-2H-pyran-2-carboxylate |
| 41 | 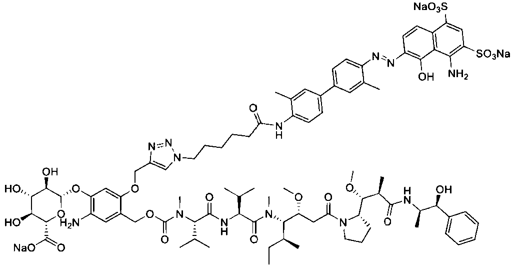 | sodium (2S,3S,4S,5R,6S)-6-(2-amino-5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10- |
| | | dimethyl-3,6,9-trioxo-2,13-dioxa-4,7,10-triazatetradecyl)phenoxy)-3,4,5-trihydroxytetrahydro-2H-pyran-2-carboxylate |
| 42 | 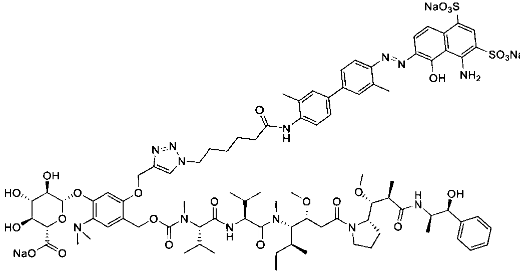 | sodium (2S,3S,4S,5R,6S)-6-(5-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-(dimethylamino)phenoxy)-3,4,5-trihydroxytetrahydro-2H-pyran-2-carboxylate |
| 43 | 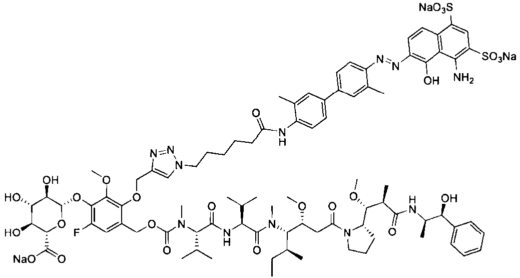 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10- |
| | | dimethyl-3,6,9-trioxo-2,13-dioxa-4,7,10-triazatetradecyl)-6-fluoro-2-methoxyphenoxy)-3,4,5-trihydroxytetrahydro-2H-pyran-2-carboxylate |
| 44 | 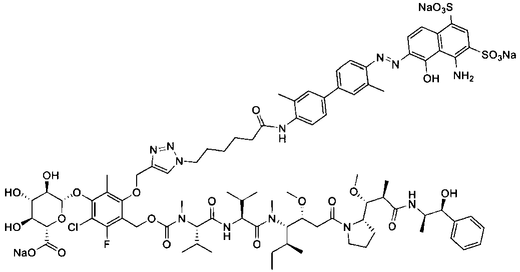 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-6-chloro-5-fluoro-2-methylphenoxy)-3,4,5-trihydroxytetrahydro-2H-pyran-2-carboxylate |
| 45 | 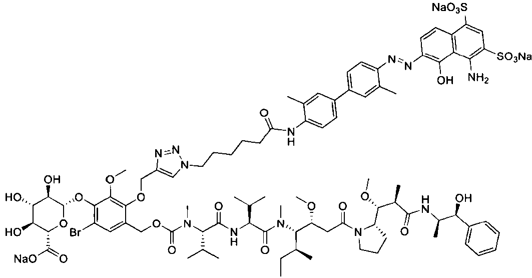 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-6-bromo-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3- |
| | | oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-methoxyphenoxy)-3,4,5-trihydroxytetrahydro-2H-pyran-2-carboxylate |
| 46 | 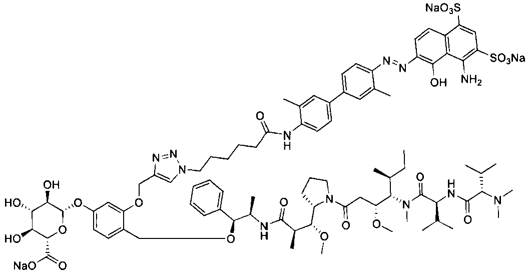 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-(((1S,2R)-2-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-1-phenylpropoxy)methyl)phenoxy)-3,4,5-trihydroxytetrahydro-2H-pyran-2-carboxylate |
| 47 | 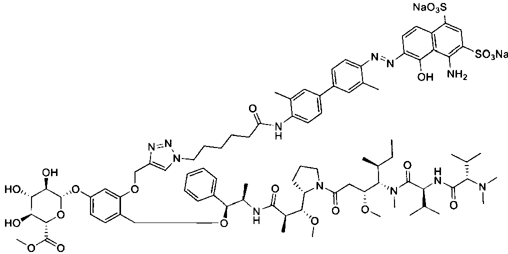 | sodium 4-amino-6-((E)-(4'-(6-(4-((2-(((1S,2R)-2-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-1-phenylpropoxy)methyl)-5-(((2S,3R,4S,5S,6S)-3,4,5-trihydroxy-6- |
| | | (methoxycarbonyl)tetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate |
| 48 | 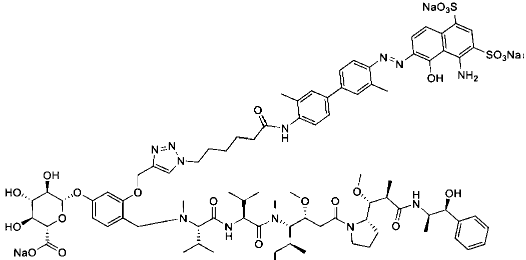 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecyl)phenoxy)-3,4,5-trihydroxytetrahydro-2H-pyran-2-carboxylate |
| 49 | 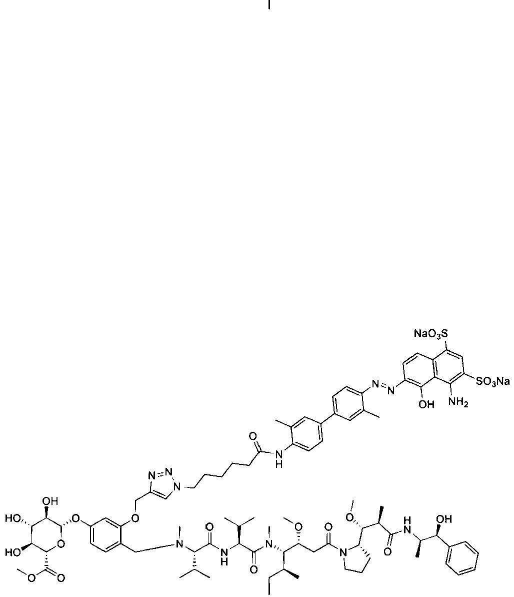 | sodium 4-amino-6-((E)-(4'-(6-(4-((2-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecyl)-5-(((2S,3R,4S,5S,6S)-3,4,5-trihydroxy-6- |
| | | (methoxycarbonyl)tetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate |
| 50 | 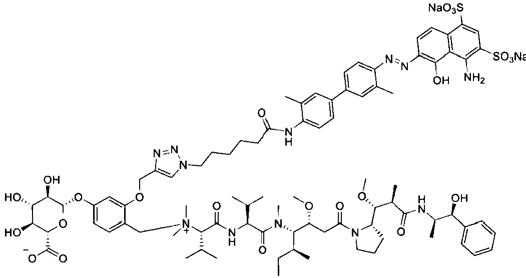 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate |
| 51 | 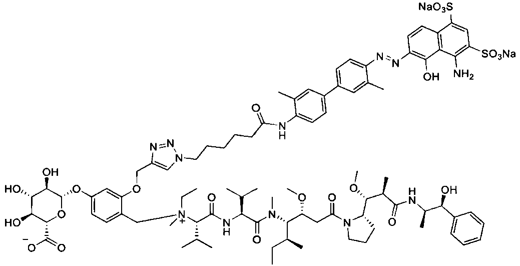 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-2-ethyl-10-(2-((S)-2-((1R,2R)-3-(((1S,2R)-1-hydroxy-1-phenylpropan-2- |
| | | yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate |
| 52 | 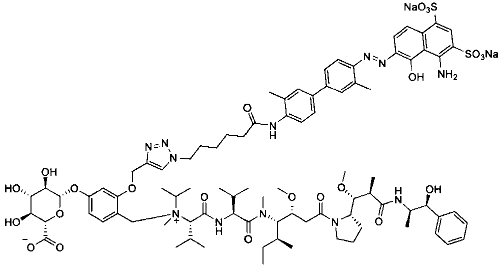 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-2,3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate |
| 53 | 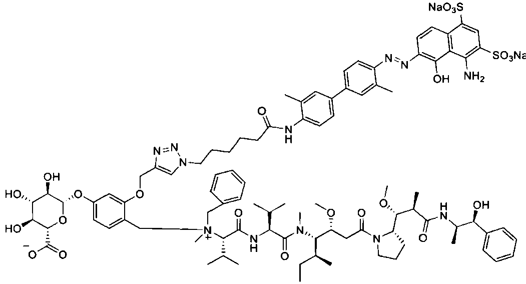 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-2-benzyl-9-((S)-sec-butyl)-10-(2-((S)-2-((1R,2R)-3-(((1S,2R)-1- |
| | | hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate |
| 54 | 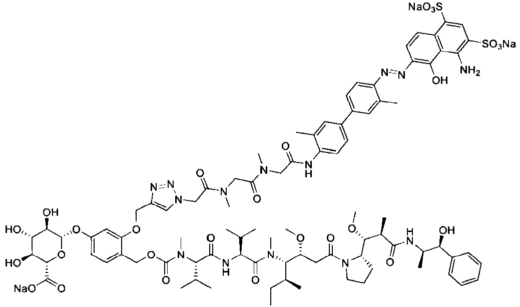 | sodium (2S,3S,4S,5R,6S)-6-(3-((1-(2-((2-((2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethyl)(methyl)amino)-2-oxoethyl)(methyl)amino)-2-oxoethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 55 | 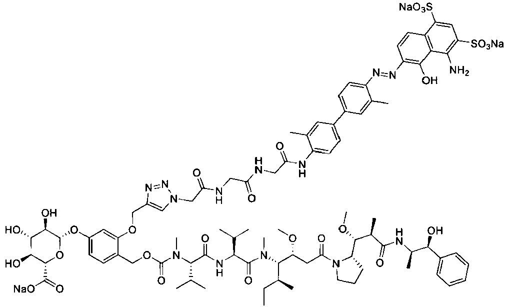 | sodium (2S,3S,4S,5R,6S)-6-(3-((1-(2-((2-((2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethyl)amino)-2-oxoethyl)amino)-2-oxoethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 56 | 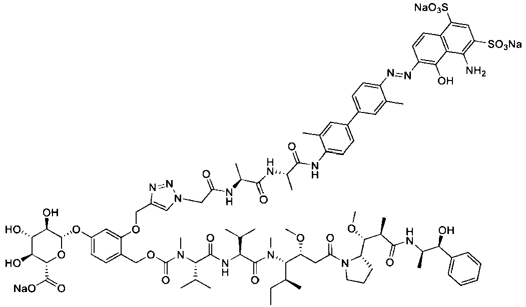 | sodium (2S,3S,4S,5R,6S)-6-(3-((1-(2-(((S)-1-(((S)-1-((4'-((E)-(8-amino-1-hydroxy-5,7-disulfonatonaphthalen-2-yl)diazenyl)-3,3'-dimethyl-[1,1'-biphenyl]-4-yl)amino)-1-oxopropan-2-yl)amino)-1-oxopropan-2-yl)amino)-2-oxoethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)- |
| | | 3,4,5-trihydroxytetrahydro-2H-pyran-2-carboxylate |
| 57 | 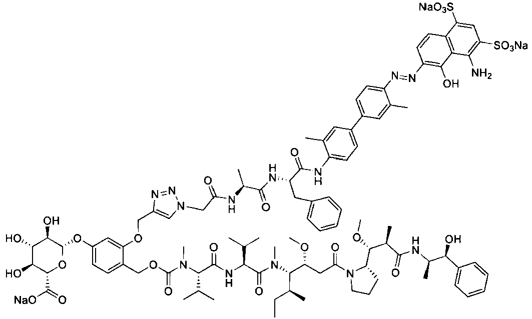 | sodium (2S,3S,4S,5R,6S)-6-(3-((1-(2-(((S)-1-(((S)-1-((4'-((E)-(8-amino-1-hydroxy-5,7-disulfonatonaphthalen-2-yl)diazenyl)-3,3'-dimethyl-[1,1'-biphenyl]-4-yl)amino)-1-oxo-3-phenylpropan-2-yl)amino)-1-oxopropan-2-yl)amino)-2-oxoethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 58 | 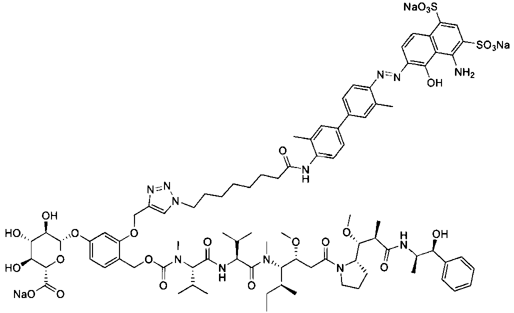 | sodium (2S,3S,4S,5R,6S)-6-(3-((1-(8-((4'-((E)-(8-amino-1-hydroxy-5,7-disulfonatonaphthalen-2-yl)diazenyl)-3,3'-dimethyl-[1,1'-biphenyl]-4-yl)amino)-8-oxooctyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa- |
| | | 4,7,10-triazatetradecyl)phenoxy)-3,4,5-trihydroxytetrahydro-2H-pyran-2-carboxylate |
| 59 | 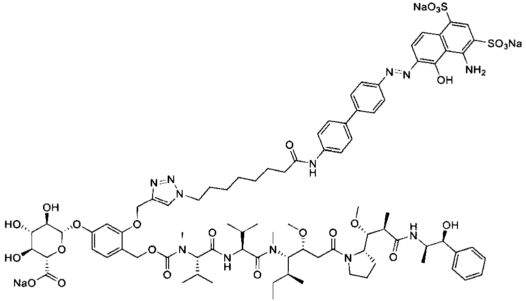 | sodium (2S,3S,4S,5R,6S)-6-(3-((1-(8-((4'-((E)-(8-amino-1-hydroxy-5,7-disulfonatonaphthalen-2-yl)diazenyl)-[1,1'-biphenyl]-4-yl)amino)-8-oxooctyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 60 | 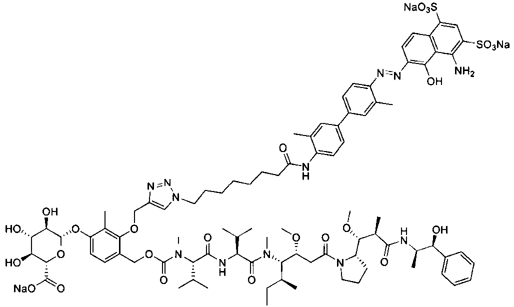 | sodium (2S,3S,4S,5R,6S)-6-(3-((1-(8-((4'-((E)-(8-amino-1-hydroxy-5,7-disulfonatonaphthalen-2-yl)diazenyl)-3,3'-dimethyl-[1,1'-biphenyl]-4-yl)amino)-8-oxooctyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2- |
| | | methylphenoxy)-3,4,5-trihydroxytetrahydro-2H-pyran-2-carboxylate |
| 61 | 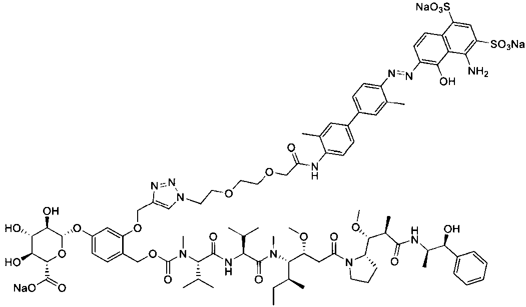 | sodium (2S,3S,4S,5R,6S)-6-(3-((1-(2-(2-(2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethoxy)ethoxy)ethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 62 | 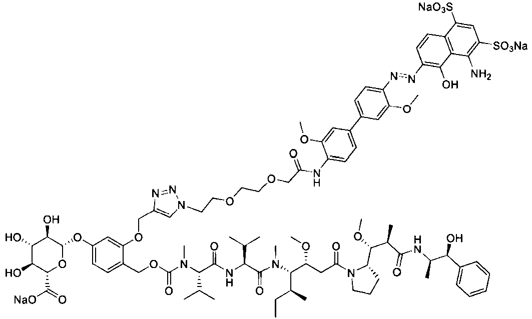 | sodium (2S,3S,4S,5R,6S)-6-(3-((1-(2-(2-(2-((4'-((E)-(8-amino-1-hydroxy-5,7-disulfonatonaphthalen-2-yl)diazenyl)-3,3'-dimethoxy-[1,1'-biphenyl]-4-yl)amino)-2-oxoethoxy)ethoxy)ethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10- |
| | | dimethyl-3,6,9-trioxo-2,13-dioxa-4,7,10-triazatetradecyl)phenoxy)-3,4,5-trihydroxytetrahydro-2H-pyran-2-carboxylate |
| 63 | 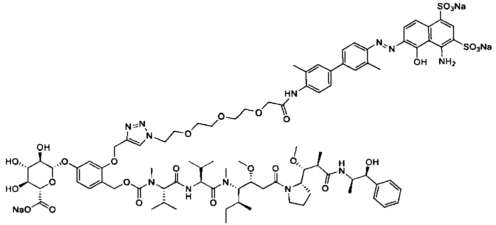 | sodium (2S,3S,4S,5R,6S)-6-(3-((1-(2-(2-(2-(2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethoxy)ethoxy)ethoxy)ethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 64 | 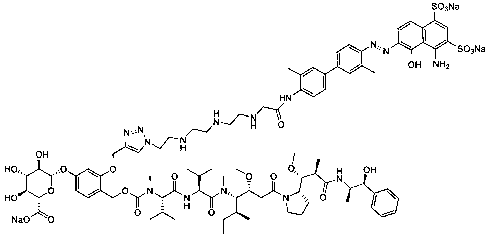 | sodium (2S,3S,4S,5R,6S)-6-(3-((1-(2-((2-((2-((2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethyl)amino)ethyl)amino)ethy 1)amino)ethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3- |
| | | oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 65 | 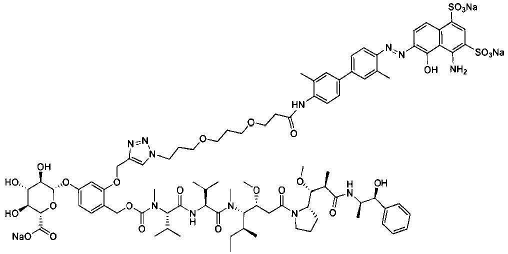 | sodium (2S,3S,4S,5R,6S)-6-(3-((1-(3-(3-(3-((4'-((E)-(8-amino-1-hydroxy-5,7-disulfonatonaphthalen-2-yl)diazenyl)-3,3'-dimethyl-[1,1'-biphenyl]-4-yl)amino)-3-oxopropoxy)propoxy)propyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 66 | 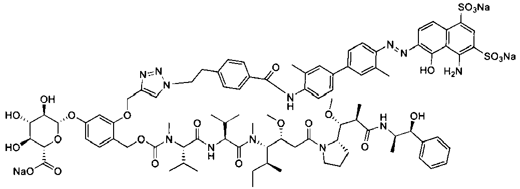 | sodium (2S,3S,4S,5R,6S)-6-(3-((1-(4-((4'-((E)-(8-amino-1-hydroxy-5,7-disulfonatonaphthalen-2-yl)diazenyl)-3,3'-dimethyl-[1,1'-biphenyl]-4-yl)carbamoyl)phenethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1- |
| | | methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 67 | 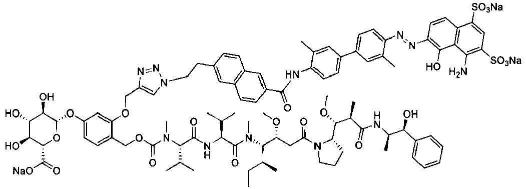 | sodium (2S,3S,4S,5R,6S)-6-(3-((1-(2-(6-((4'-((E)-(8-amino-1-hydroxy-5,7-disulfonatonaphthalen-2-yl)diazenyl)-3,3'-dimethyl-[1,1'-biphenyl]-4-yl)carbamoyl)naphthalen-2-yl)ethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 68 | 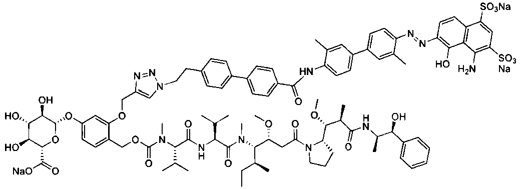 | sodium (2S,3S,4S,5R,6S)-6-(3-((1-(2-(4'-((4'-((E)-(8-amino-1-hydroxy-5,7-disulfonatonaphthalen-2-yl)diazenyl)-3,3'-dimethyl-[1,1'-biphenyl]-4-yl)carbamoyl)-[1,1'-biphenyl]-4-yl)ethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1- |
| | | phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 69 | 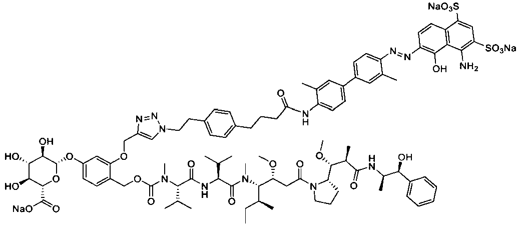 | sodium (2S,3S,4S,5R,6S)-6-(3-((1-(4-(4-((4'-((E)-(8-amino-1-hydroxy-5,7-disulfonatonaphthalen-2-yl)diazenyl)-3,3'-dimethyl-[1,1'-biphenyl]-4-yl)amino)-4-oxobutyl)phenethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 70 | 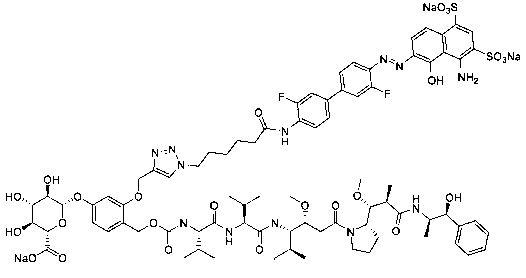 | sodium (2S,3S,4S,5R,6S)-6-(3-((1-(6-((4'-((E)-(8-amino-1-hydroxy-5,7-disulfonatonaphthalen-2-yl)diazenyl)-3,3'-difluoro-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy- |
| | | 1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 71 | 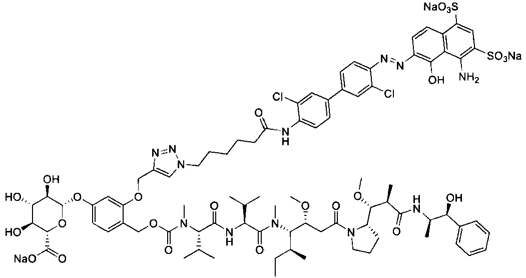 | sodium (2S,3S,4S,5R,6S)-6-(3-((1-(6-((4'-((E)-(8-amino-1-hydroxy-5,7-disulfonatonaphthalen-2-yl)diazenyl)-3,3'-dichloro-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 72 | 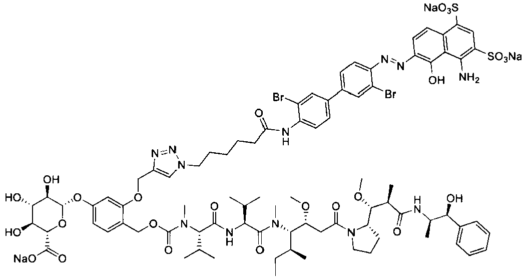 | sodium (2S,3S,4S,5R,6S)-6-(3-((1-(6-((4'-((E)-(8-amino-1-hydroxy-5,7-disulfonatonaphthalen-2-yl)diazenyl)-3,3'-dibromo-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1- |
| | | methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 73 | 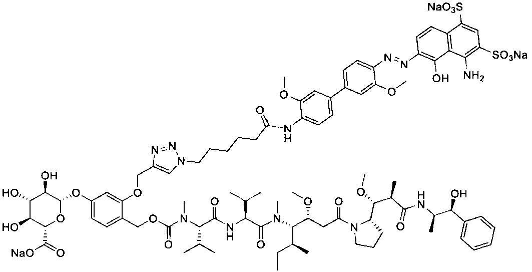 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethoxy-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 74 | 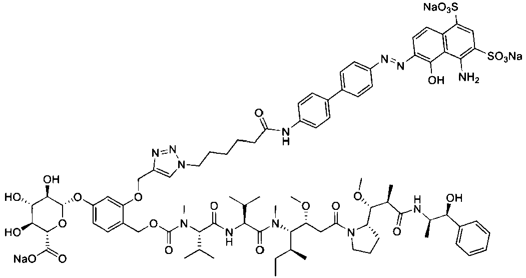 | sodium (2S,3S,4S,5R,6S)-6-(3-((1-(6-((4'-((E)-(8-amino-1-hydroxy-5,7-disulfonatonaphthalen-2-yl)diazenyl)-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3- |
| | | oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 75 | 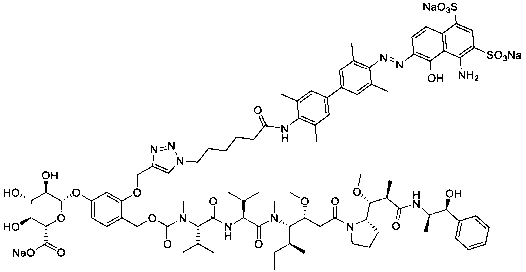 | sodium (2S,3S,4S,5R,6S)-6-(3-((1-(6-((4'-((E)-(8-amino-1-hydroxy-5,7-disulfonatonaphthalen-2-yl)diazenyl)-3,3',5,5'-tetramethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 76 | 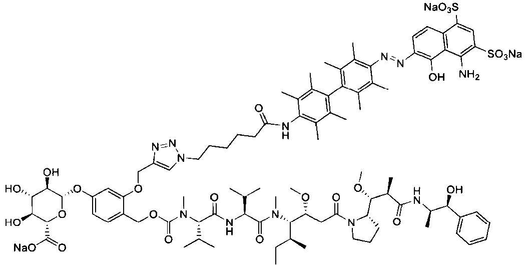 | sodium (2S,3S,4S,5R,6S)-6-(3-((1-(6-((4'-((E)-(8-amino-1-hydroxy-5,7-disulfonatonaphthalen-2-yl)diazenyl)-2,2',3,3',5,5',6,6'-octamethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3- |
| | | oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 77 | 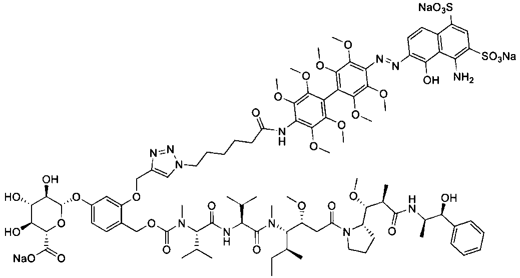 | sodium (2S,3S,4S,5R,6S)-6-(3-((1-(6-((4'-((E)-(8-amino-1-hydroxy-5,7-disulfonatonaphthalen-2-yl)diazenyl)-2,2',3,3',5,5',6,6'-octamethoxy-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 78 | 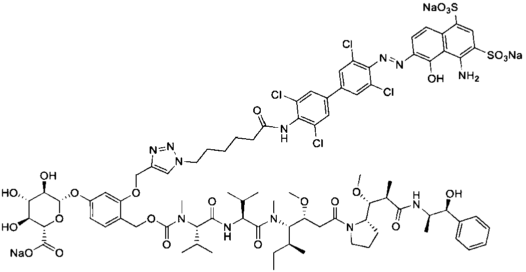 | sodium (2S,3S,4S,5R,6S)-6-(3-((1-(6-((4'-((E)-(8-amino-1-hydroxy-5,7-disulfonatonaphthalen-2-yl)diazenyl)-3,3',5,5'-tetrachloro-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3- |
| | | oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 79 | 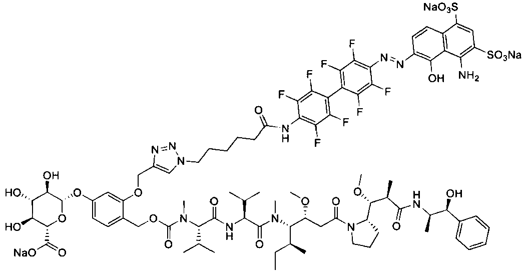 | sodium (2S,3S,4S,5R,6S)-6-(3-((1-(6-((4'-((E)-(8-amino-1-hydroxy-5,7-disulfonatonaphthalen-2-yl)diazenyl)-2,2',3,3',5,5',6,6'-octafluoro-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 80 | 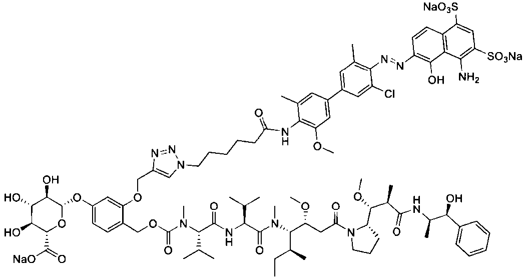 | sodium (2S,3S,4S,5R,6S)-6-(3-((1-(6-((4'-((E)-(8-amino-1-hydroxy-5,7-disulfonatonaphthalen-2-yl)diazenyl)-3'-chloro-3-methoxy-5,5'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1- |
| | | methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 81 | 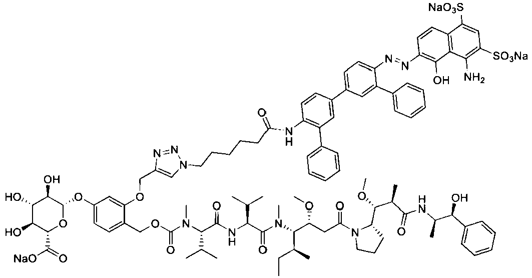 | sodium (2S,3S,4S,5R,6S)-6-(3-((1-(6-((6'-((E)-(8-amino-1-hydroxy-5,7-disulfonatonaphthalen-2-yl)diazenyl)-[1,1':3',1":3",1"'-quaterphenyl]-4"-yl)amino)-6-oxohexyl)-1H-1,2,3-triazol-4-yl)methoxy)-4-((55,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 82 | 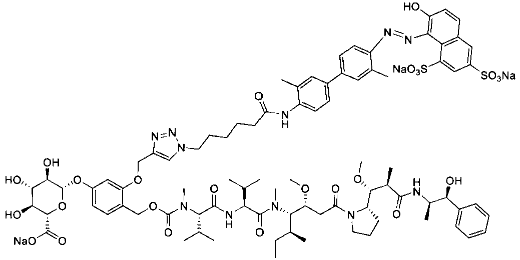 | sodium (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-((1-(6-((4'-((E)-(2-hydroxy-6,8-disulfonatonaphthalen-1- |
| | | yl)diazenyl)-3,3'-dimethyl-[1,1'-biphenyl]-4-yl)amino)-6-oxohexyl)-1H-1,2,3-triazol-4-yl)methoxy)phenoxy)-3,4,5-trihydroxytetrahydro-2H-pyran-2-carboxylate |
| 83 | 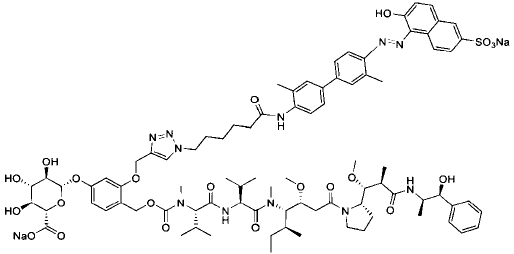 | sodium (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-((1-(6-((4'-((E)-(2-hydroxy-6-sulfonatonaphthalen-1-yl)diazenyl)-3,3'-dimethyl-[1,1'-biphenyl]-4-yl)amino)-6-oxohexyl)-1H-1,2,3-triazol-4-yl)methoxy)phenoxy)-3,4,5-trihydroxytetrahydro-2H-pyran-2-carboxylate |
| 84 | 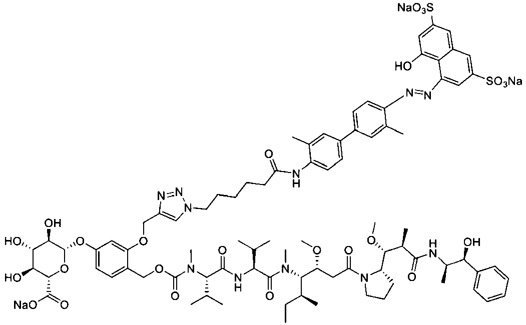 | sodium (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-((1-(6-((4'-((E)-(8-hydroxy-3,6-disulfonatonaphthalen-1-yl)diazenyl)-3,3'-dimethyl-[1,1'- |
| | | biphenyl]-4-yl)amino)-6-oxohexyl)-1H-1,2,3-triazol-4-yl)methoxy)phenoxy)-3,4,5-trihydroxytetrahydro-2H-pyran-2-carboxylate |
| 85 | 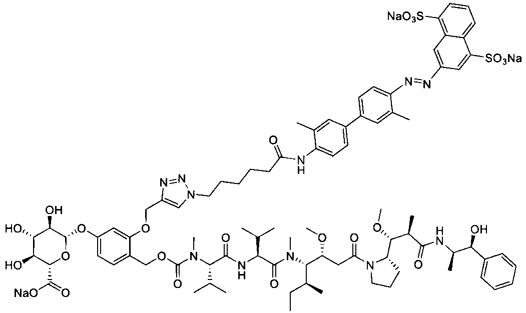 | sodium (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-((1-(6-((4'-((E)-(4,8-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)phenoxy)-3,4,5-trihydroxytetrahydro-2H-pyran-2-carboxylate |
| 86 | 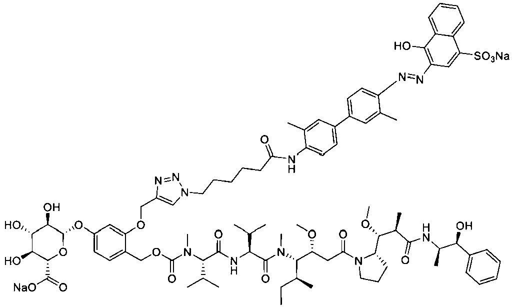 | sodium (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-((1-(6-((4'-((E)-(1-hydroxy-4-sulfonatonaphthalen-2-yl)diazenyl)-3,3'-dimethyl-[1,1'-biphenyl]-4-yl)amino)-6- |
| | | oxohexyl)-1H-1,2,3-triazol-4-yl)methoxy)phenoxy)-3,4,5-trihydroxytetrahydro-2H-pyran-2-carboxylate |
| 87 | 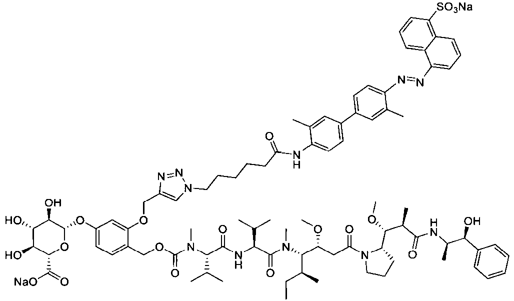 | sodium (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-((1-(6-((3,3'-dimethyl-4'-((E)-(5-sulfonatonaphthalen-1-yl)diazenyl)-[1,1'-biphenyl]-4-yl)amino)-6-oxohexyl)-1H-1,2,3-triazol-4-yl)methoxy)phenoxy)-3,4,5-trihydroxytetrahydro-2H-pyran-2-carboxylate |
| 88 | 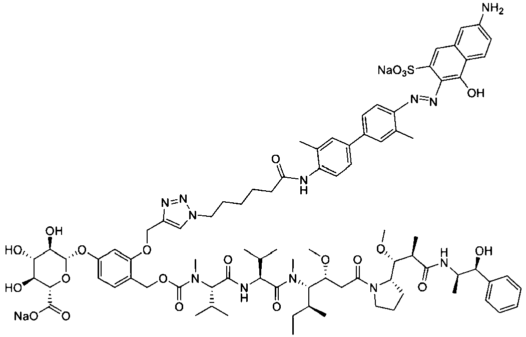 | sodium (2S,3S,4S,5R,6S)-6-(3-((1-(6-((4'-((E)-(6-amino-1-hydroxy-3-sulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H- |
| | | pyran-2-carboxylate |
| 89 | 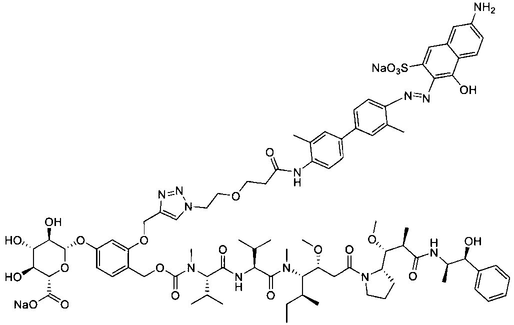 | sodium (2S,3S,4S,5R,6S)-6-(3-((1-(2-(3-((4'-((E)-(6-amino-1-hydroxy-3-sulfonatonaphthalen-2-yl)diazenyl)-3,3'-dimethyl-[1,1'-biphenyl]-4-yl)amino)-3-oxopropoxy)ethyl)-1H-1,2,3-triazol-4-yl)methoxy)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 90 | 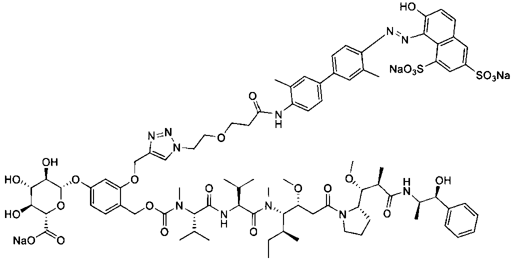 | sodium (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-3-((1-(2-(3-((4'-((E)-(2-hydroxy-6,8-disulfonatonaphthalen-1-yl)diazenyl)-3,3'-dimethyl-[1,1'-biphenyl]-4-yl)amino)-3-oxopropoxy)ethyl)-1H-1,2,3-triazol-4-yl)methoxy)phenoxy)-3,4,5-trihydroxytetrahydro-2H-pyran-2-carboxylate |
| 91 | 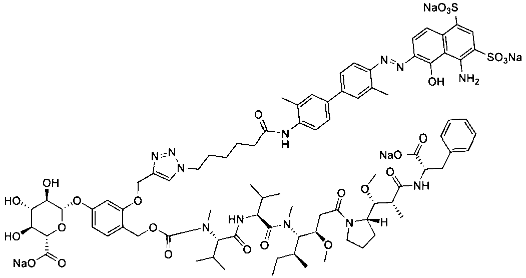 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((5S,8S,11S,12R)-11-((S)-sec-butyl)-12-(2-((S)-2-((1R,2R)-3-(((S)-1-carboxylato-2-phenylethyl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylate |
| 92 | 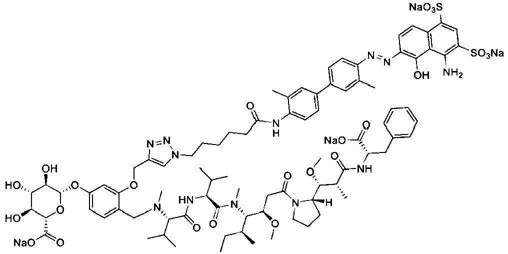 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((S)-1-carboxylato-2-phenylethyl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecyl)phenoxy)-3,4,5-trihydroxytetrahydro-2H-pyran-2-carboxylate |
| 93 | 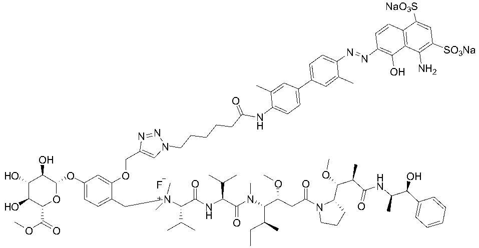 | sodium 4-amino-6-((E)-(4'-(6-(4-((2-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)-5-(((2S,3R,4S,5S,6S)-3,4,5-trihydroxy-6-(methoxycarbonyl)tetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate fluoride |
| 94 | 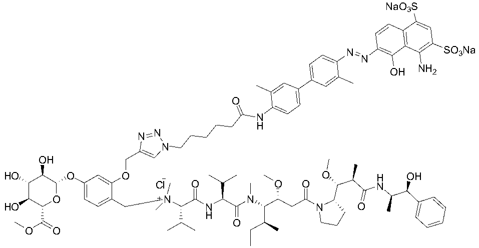 | sodium 4-amino-6-((E)-(4'-(6-(4-((2-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)-5-(((2S,3R,4S,5S,6S)-3,4,5-trihydroxy-6-(methoxycarbonyl)tetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate chloride |
| 95 | 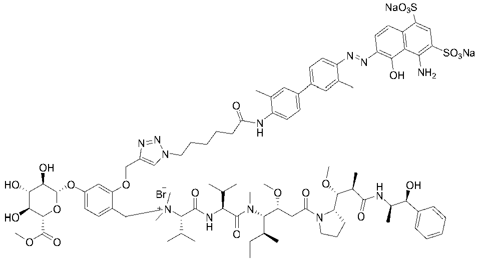 | sodium 4-amino-6-((E)-(4'-(6-(4-((2-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)-5-(((2S,3R,4S,5S,6S)-3,4,5-trihydroxy-6-(methoxycarbonyl)tetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate bromide |
| 96 | 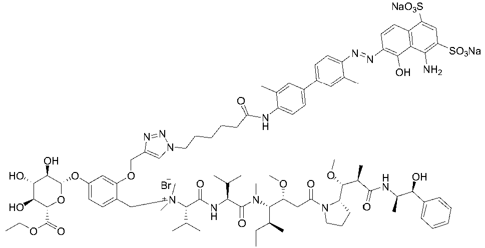 | sodium 4-amino-6-((E)-(4'-(6-(4-((2-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)-5-(((2S,3R,4S,5S,6S)-6-(ethoxycarbonyl)-3,4,5-trihydroxytetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate bromide |
| 97 | 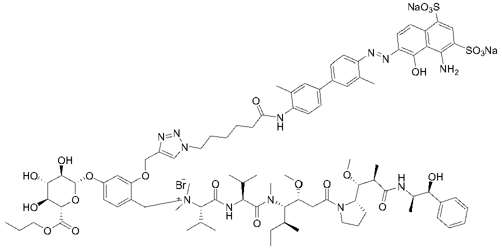 | sodium 4-amino-6-((E)-(4'-(6-(4-((2-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)-5-(((2S,3R,4S,5S,6S)-3,4,5-trihydroxy-6-(propoxycarbonyl)tetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate bromide |
| 98 | 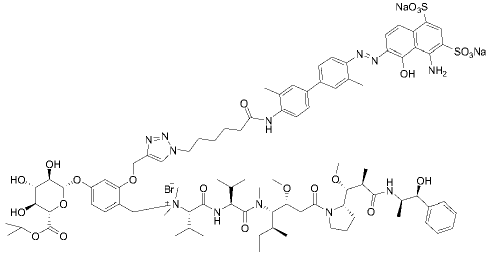 | sodium 4-amino-6-((E)-(4'-(6-(4-((2-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)-5-(((2S,3R,4S,5S,6S)-3,4,5-trihydroxy-6-(isopropoxycarbonyl)tetrahydro-2H-pyran-2-yl)oxy)phenoxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate bromide |
| 99 | 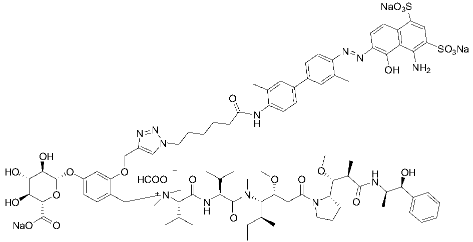 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate formate |
| 100 | 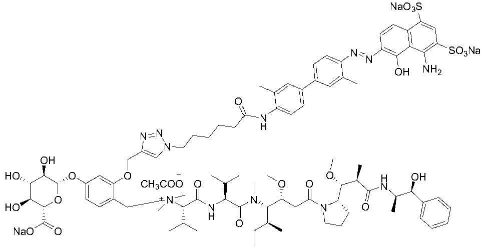 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate acetate |
| 101 | 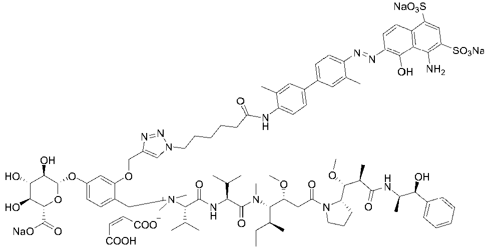 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate (Z)-3-carboxyacrylate |
| 102 | 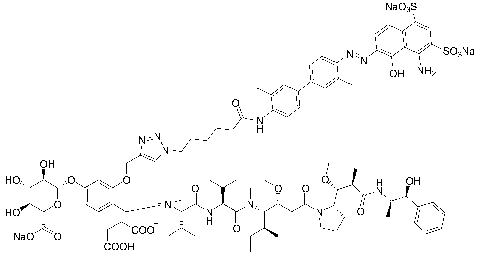 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate 3-carboxypropanoate |
| 103 | 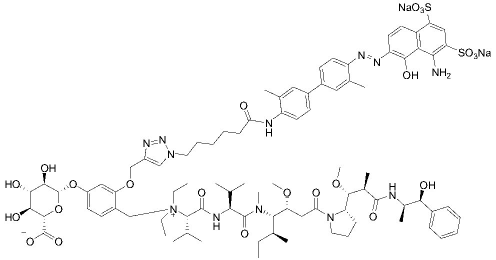 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-2,2-diethyl-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-8-methyl-4,7-dioxo-11-oxa-2,5,8-triazadodecan-2-ium-1-yl)phenoxy)-3,4,5-trihydroxytetrahydro-2H-pyran-2-carboxylate |
| 104 | 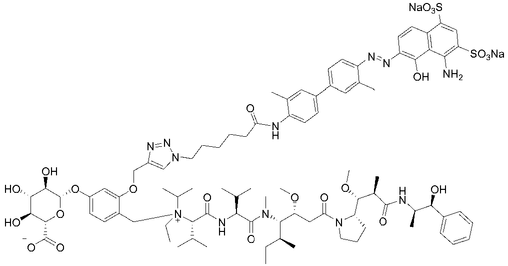 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((3S,6S,9S,10R)-9-((S)-sec-butyl)-2-ethyl-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-2,3,6-triisopropyl-8-methyl-4,7-dioxo-11-oxa-2,5,8-triazadodecan-2-ium-1-yl)phenoxy)-3,4,5-trihydroxytetrahydro-2H-pyran-2-carboxylate |
| 105 | 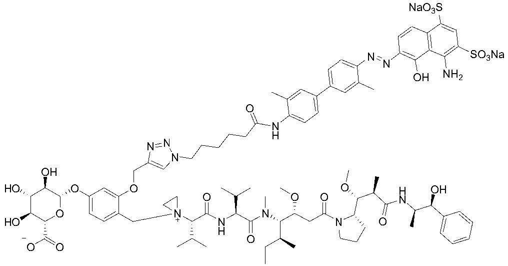 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((1-((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)aziridin-1-ium-1-yl)methyl)phenoxy)-3,4,5-trihydroxytetrahydro-2H-pyran-2-carboxylate |
| 106 | 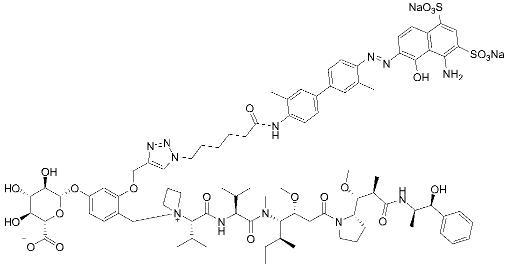 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((1-((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)azetidin-1-ium-1-yl)methyl)phenoxy)-3,4,5-trihydroxytetrahydro-2H-pyran-2-carboxylate |
| 107 | 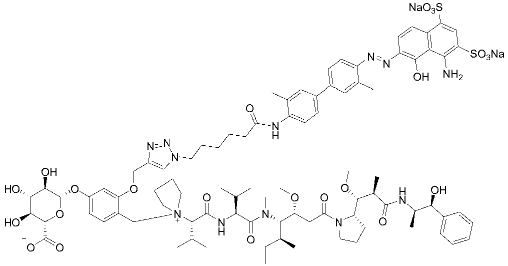 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((1-((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)pyrrolidin-1-ium-1-yl)methyl)phenoxy)-3,4,5-trihydroxytetrahydro-2H-pyran-2-carboxylate |
| 108 | 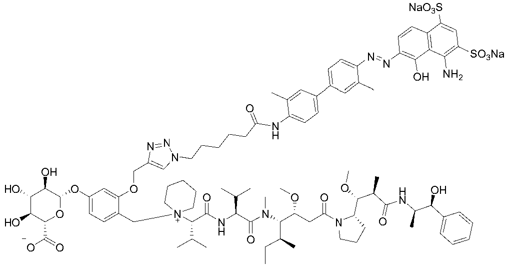 | sodium (2S,3S,4S,5R,6S)-6-(3-((1-(6-((4'-((E)-(8-amino-1-hydroxy-5,7-disulfonatonaphthalen-2-yl)diazenyl)-3,3'-dimethyl-[1,1'-biphenyl]-4-yl)amino)-6-oxohexyl)-1H-1,2,3-triazol-4-yl)methoxy)-4-((1-((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)piperidin-1-ium-1-yl)methyl)phenoxy)-3,4,5-trihydroxytetrahydro-2H-pyran-2-carboxylate. |

6. A compound, or a stereoisomer, a cis-trans isomer, or a pharmaceutically acceptable salt thereof, wherein the compound is selected from:
| serial No. | structural formula | chemical name |
|---|---|---|
| 1 | 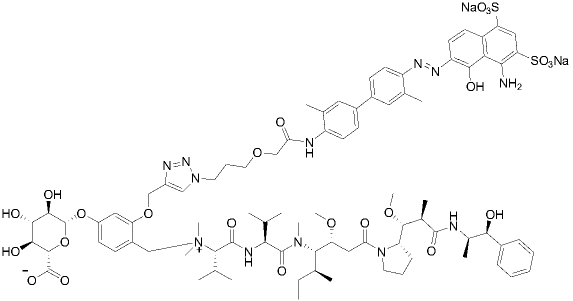 | sodium (2S,3S,4S,5R,6S)-6-(3-((1-(3-(2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethoxy)propyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate |
| 2 | 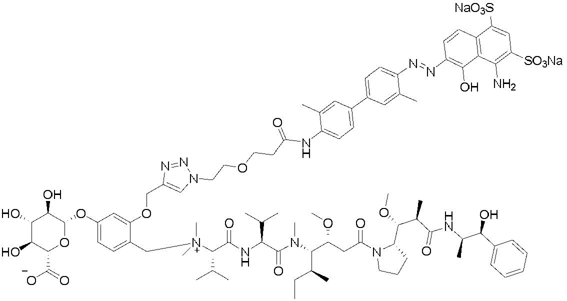 | sodium (2S,3S,4S,5R,6S)-6-(3-((1-(2-(3-((4'-((E)-(8-amino-1-hydroxy-5,7-disulfonatonaphthalen-2-yl)diazenyl)-3,3'-dimethyl-[1,1'-biphenyl]-4-yl)amino)-3-oxopropoxy)ethyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate |
| 3 | 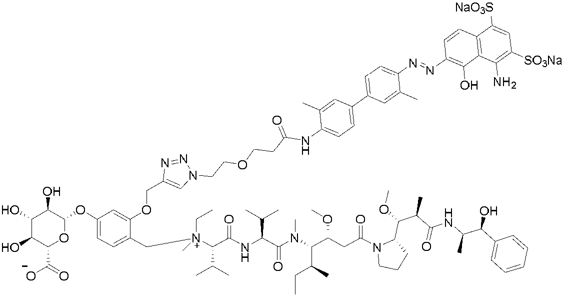 | sodium (2S,3S,4S,5R,6S)-6-(3-((1-(2-(3-((4'-((E)-(8-amino-1-hydroxy-5,7-disulfonatonaphthalen-2-yl)diazenyl)-3,3'-dimethyl-[1,1'-biphenyl]-4-yl)amino)-3-oxopropoxy)ethyl)-1H-1,2,3-triazol-4-yl)methoxy)-4-((3S,6S,9S,10R)-9-((S)-sec-butyl)-2-ethyl-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate |
| 4 | 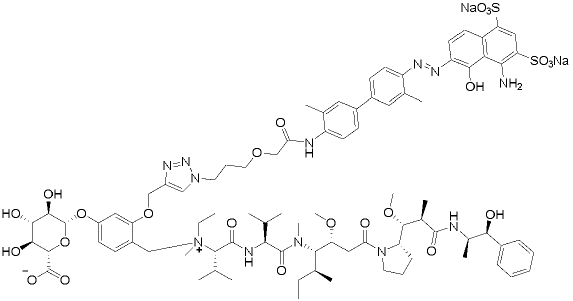 | sodium (2S,3S,4S,5R,6S)-6-(3-((1-(3-(2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethoxy)propyl)-1H-1,2,3-triazol-4-yl)methoxy)-4-((3S,6S,9S,10R)-9-((S)-sec-butyl)-2-ethyl-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate |
| 5 | 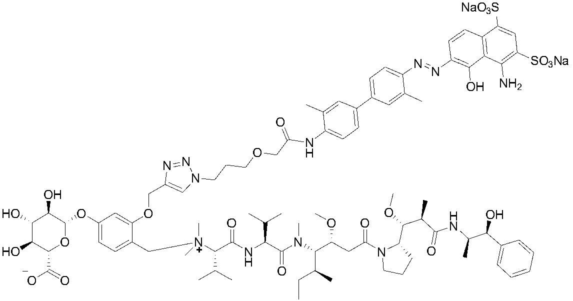 | sodium (2S,3S,4S,5R,6S)-6-(3-((1-(3-(2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethoxy)propyl)-1H-1,2,3-triazol-4-yl)methoxy)-4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,2,8-trimethyl-4,7-dioxo-11-oxa-2,5,8-triazadodecan-2-ium-1-yl)phenoxy)-3,4,5-trihydroxytetrahydro-2H-pyran-2-carboxylate |
| 6 | 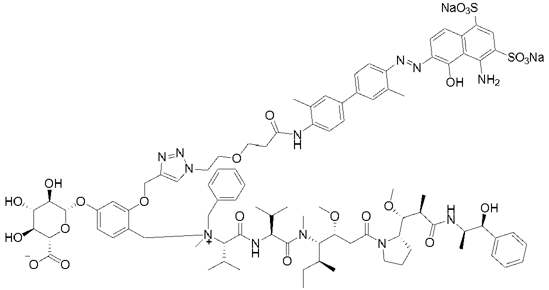 | sodium (2S,3S,4S,5R,6S)-6-(3-((1-(2-(3-((4'-((E)-(8-amino-1-hydroxy-5,7-disulfonatonaphthalen-2-yl)diazenyl)-3,3'-dimethyl-[1,1'-biphenyl]-4-yl)amino)-3-oxopropoxy)ethyl)-1H-1,2,3-triazol-4-yl)methoxy)-4-((3S,6S,9S,10R)-2-benzyl-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate |
| 7 | 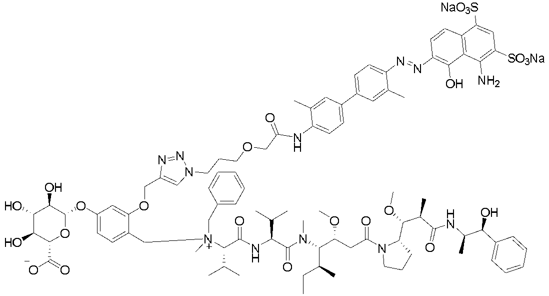 | sodium (2S,3S,4S,5R,6S)-6-(3-((1-(3-(2-((4'-((E)-(8-amino-1-hydroxy-5,7-disulfonatonaphthalen-2-yl)diazenyl)-3,3'-dimethyl-[1,1'-biphenyl]-4-yl)amino)-2-oxoethoxy)propyl)-1H-1,2,3-triazol-4-yl)methoxy)-4-((3S,6S,9S,10R)-2-benzyl-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecan-2-ium-1-yl)phenoxy)-3,4,5-trihydroxytetrahydro-2H-pyran-2-carboxylate. |

7. A pharmaceutical composition, comprising the compound according to any one of claims 1 to 6, or the stereoisomer, the cis-trans isomer, or the pharmaceutically acceptable salt thereof, and a pharmaceutically acceptable excipient.

8. The pharmaceutical composition according to claim 7, wherein the excipient is a sodium chloride solution vehicle.

9. The pharmaceutical composition according to claim 7, wherein the pharmaceutical composition is prepared into a formulation for injection.

10. The pharmaceutical composition according to claim 9, wherein the formulation for injection is an injection, a sterile powder for injection, and a concentrated solution for injection.

11. Use of the compound according to any one of claims 1 to 6, or the stereoisomer, the cis-trans isomer, or the pharmaceutically acceptable salt thereof, or the pharmaceutical composition according to any one of claims 7 to 10 for the manufacture of a medicament for treating a tumor or a cancer.

12. The use according to claim 11, wherein the tumor or the cancer is selected from colorectal cancer, liver cancer, cervical cancer, ovarian cancer, gastric cancer, esophagus cancer, breast cancer, pancreatic cancer, bladder cancer, liver cancer, intestinal cancer, head and neck cancer, uterine cancer, urothelial carcinoma, osteosarcoma, sarcoma, renal cancer, melanoma, prostate cancer, glioma, neuroglioma, and leukemia.
